# EUROPEAN PATENT APPLICATION

(11) **EP 4 534 541 A1**
(43) Date of publication of application: **09.04.2025**
(21) Application number: 23815319.1
(22) Date of filing: 02.06.2023
(51) Int. Cl.: C07F 9/54, A61K 31/67, A61K 31/665, A61K 31/675, A61K 31/66, A61P 35/00

(54) **PHOSPHORUS COMPOUND AND USE THEREOF**

(30) Priority: 02.06.2022 CN 202210619968; 19.01.2023 CN 202310075094
(71) Applicant: Shanghai Shijiang Biotechnology Co., Ltd, Shanghai 200333 (CN)
(72) Inventor: SHI, Yufeng, Nanjing, Jiangsu 210032 (CN); MA, Wenjiang, Nanjing, Jiangsu 210032 (CN); WANG, Yingcai, Nanjing, Jiangsu 210032 (CN)
(74) Representative: reuteler & cie SA
(86) International application number: PCT/CN2023/098077
(87) International publication number: WO 2023/232143

(57) **Abstract**

The present invention relates to a phosphorus compound and the use thereof. Specifically, provided in the present invention is a compound of formula (I), or an optical isomer, racemate, solvate, pharmaceutically acceptable salt or deuterated compound thereof. The compound of the present invention has significant and excellent precision treatment effects on tumors that have low expression, no expression, low activity or no activity of mitochondrial permeability transition pores. low expression, no expression, low activity or no activity of peptidyl prolyl isomerase F, low expression or no expression of NNMT gene, high expression of DNA methylase, high expression of UHRFI, high methylation level at a nucleotide site in NNMT gene, and/or high methylation level of DNA at CpG site in NNMT gene region.

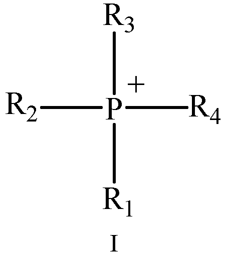

## Description

### FIELD OF THE INVENTION

The present invention relates to the field of medicine. Specifically, the present invention relates to phosphorus compound and use thereof.

### BACKGROUND TECHNOLOGY

Tumor is a common disease that seriously endangers human health, and the mortality rate of malignant tumors is rising. Due to the heterogeneity of tumor, simply using the same treatment method or medication based on source or pathological characteristic of tumors can easily lead to improper treatment, which can delay valuable treatment time and opportunities for patient. Therefore, it is very necessary to take precision treatment according to the different features of tumor. With the development of biological technology, tumors are typed at the molecular level such as gene and protein level, etc, and more and more changes in tumor-related gene and the expression and activity of protein have been discovered, changes in tumor-related gene and the expression and activity of protein play an important role in the development of malignant tumors, the discovery and application of biomarkers can provide precise guidance for the application of related drug and make precision treatment of tumor possible, thereby achieving targeted administration of drug, significantly improving treatment effect, reducing the dose of drug and reducing toxic side effects.

Therefore, there is an urgent need in the art to develop a drug that can achieve precision treatment on tumor.

### SUMMARY OF THE INVENTION

The present invention provide a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In the first aspect of the present invention, it provides a compound of formula I, or an optical isomer thereof, or a racemate thereof, or a solvate thereof, or a pharmaceutically acceptable salt thereof, or a deuterated compound thereof; 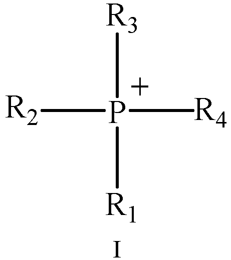 wherein,
R₁, R₂, R₃ and R₄ are each independently substituted or unsubstituted C3-C16 cycloalkyl, substituted or unsubstituted 3-16 membered heterocycloalkyl, substituted or unsubstituted C6-C16 aryl, substituted or unsubstituted 5-16 membered heteroaryl, or substituted or unsubstituted 5-16 membered heteroaryl-substituted or unsubstituted C1-C8 alkyl-.

In another preferred embodiment, each "substituted" means that one or more (preferably 1, 2, 3, 4, 5, 6, 7 or 8) hydrogen atoms on the group are each independently substituted by a substituent.

In another preferred embodiment, R₁, R₂, R₃ and R₄ are each independently substituted or unsubstituted C3-C14 cycloalkyl, substituted or unsubstituted 3-14 membered heterocycloalkyl, substituted or unsubstituted C6-C14 aryl, substituted or unsubstituted 5-14 membered heteroaryl, or substituted or unsubstituted 5-14 membered heteroaryl-substituted or unsubstituted C1-C8 alkyl-.

In another preferred embodiment, R₁, R₂, R₃ and R₄ are each independently substituted or unsubstituted C3-C12 cycloalkyl, substituted or unsubstituted 3-12 membered heterocycloalkyl, substituted or unsubstituted C6-C12 aryl, substituted or unsubstituted 5-12 membered heteroaryl, or substituted or unsubstituted 5-12 membered heteroaryl-substituted or unsubstituted C1-C6 alkyl-.

In another preferred embodiment, R₁, R₂, R₃ and R₄ are each independently substituted or unsubstituted C3-C10 cycloalkyl, substituted or unsubstituted 3-10 membered heterocycloalkyl, substituted or unsubstituted C6-C10 aryl, substituted or unsubstituted 5-10 membered heteroaryl, or substituted or unsubstituted 5-10 membered heteroaryl-substituted or unsubstituted C1-C4 alkyl-.

In another preferred embodiment, R₁, R₂, R₃ and R₄ are each independently substituted or unsubstituted 3 membered heterocycloalkyl, substituted or unsubstituted 4 membered heterocycloalkyl, substituted or unsubstituted 5 membered heterocycloalkyl, substituted or unsubstituted 6 membered heterocycloalkyl, substituted or unsubstituted 7 membered heterocycloalkyl, substituted or unsubstituted 8 membered heterocycloalkyl, substituted or unsubstituted 9 membered heterocycloalkyl, substituted or unsubstituted 10 membered heterocycloalkyl, substituted or unsubstituted 11 membered heterocycloalkyl, substituted or unsubstituted 12 membered heterocycloalkyl, substituted or unsubstituted 13 membered heterocycloalkyl, substituted or unsubstituted 14 membered heterocycloalkyl, substituted or unsubstituted 15 membered heterocycloalkyl,substituted or unsubstituted 16 membered heterocycloalkyl, substituted or unsubstituted C6 aryl, substituted or unsubstituted C7 aryl, substituted or unsubstituted C8 aryl, substituted or unsubstituted C9 aryl, substituted or unsubstituted C10 aryl, substituted or unsubstituted C11 aryl, substituted or unsubstituted C12 aryl, substituted or unsubstituted C13 aryl, substituted or unsubstituted C14 aryl, substituted or unsubstituted C15 aryl, substituted or unsubstituted C16 aryl, substituted or unsubstituted 5 membered heteroaryl, substituted or unsubstituted 6 membered heteroaryl, substituted or unsubstituted 7 membered heteroaryl, substituted or unsubstituted 8 membered heteroaryl, substituted or unsubstituted 9 membered heteroaryl, substituted or unsubstituted 10 membered heteroaryl, substituted or unsubstituted 11 membered heteroaryl, substituted or unsubstituted 12 membered heteroaryl, substituted or unsubstituted 13 membered heteroaryl, substituted or unsubstituted 14 membered heteroaryl, substituted or unsubstituted 15 membered heteroaryl, substituted or unsubstituted 16 membered heteroaryl, or substituted or unsubstituted 6-10 membered heteroaryl-substituted or unsubstituted C1-C2 alkyl-

In another preferred embodiment, R₁, R₂, R₃ and R₄ are each independently phenyl, methoxyl-phenyl-, indolyl, isoindolyl, methoxyl-indolyl-, methyl-indolyl-, hydroxyl-ethyl-indolyl-, sulfhydryl-ethyl-indolyl-, piperidinyl-ethyl-indolyl-, morpholinyl-ethyl-indolyl-, haloethyl-indolyl-, pyrrolopyridine group, methyl-pyrrolopyridine group-, benzimidazole group, methyl-benzimidazole group-, indazolyl, methyl-indazolyl-, pyridinyl, *3H*-indolyl, *7H*-pyrrolo[3,4-b]pyridine group, *3H-*pyrrolo[3,4-c]pyridine group, *1H*-pyrrolo[3,4-c] pyridine group, *3H*-pyrrolo[3,2-b]pyridine group, *3H*-pyrrolo[2,3-c]pyridine group, *3H*-indazolyl, *2H*-indazolyl, *1H*-pyrrolo[2,3-b]pyridine group, monomethyl-substituted *3H*-indolyl, monomethyl-substituted *7H*-pyrrolo[3,4-b]pyridine group, monomethyl-substituted *3H*-pyrrolo[3,4-c]pyridine group, monomethyl-substituted *1H*-pyrrolo[3,4-c]pyridine group, monomethyl-substituted *3H*-pyrrolo[3,2-b]pyridine group, monomethyl-substituted *3H*-pyrrolo[2,3-c]pyridine group, monomethyl-substituted *3H*-indazolyl, monomethyl-substituted *2H*-indazolyl, monomethyl-substituted *1H*-pyrrolo[2,3-b]pyridine group, benzofuran group, furyl, benzothiazole group, benzothiophene group, thiazolyl, haloindolyl-methyl-, dihydropyranyl.

In another preferred embodiment, the methoxyl-phenyl- is

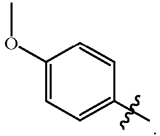

In another preferred embodiment, the indolyl is *1H*-indolyl or *3H* indolyl.

In another preferred embodiment, the methoxyl-indolyl- is

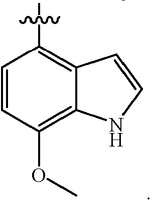

In another preferred embodiment, the pyrrolopyridine group is pyrrolo[2, 3-*c*]pyridine group, pyrrolo[2, 3-*b*]pyridine group, pyrrolo[3, 2-*b*]pyridine group, pyrrolo[3, 2-*c*]pyridine group, pyrrolo[3, 4-*b*]pyridine group, pyrrolo[3, 4-*c*]pyridine group, pyrrolo[3, 2-*b*]pyridine group or pyrrolo[2, 3-*c*]pyridine group.

In another preferred embodiment, the pyrrolopyridine group is *1H*-pyrrolo[2, 3-*c*]pyridine group, *1H*-pyrrolo[2, 3-*b*]pyridine group, *1H*-pyrrolo[3, 2-*b*]pyridine group, *1H*-pyrrolo[3, 2-c]pyridine group, *7H*-pyrrolo[3,4-*b*]pyridine group, *3H*-pyrrolo[3, 4-*c*]pyridine group, *1H-*pyrrolo[3, 4-*c*]pyridine group, *3H* -pyrrolo[3, 2-*b*]pyridine group, *3H*-pyrrolo[2, 3-*c*]pyridine group.

In another preferred embodiment, the benzimidazole group is benzo[*d*]imidazole group.

In another preferred embodiment, the benzimidazole group is *1H*-benzo[d]imidazole group.

In another preferred embodiment, the indazolyl is *1H*-indazolyl or *3H*-indazolyl.

In another preferred embodiment, the isoindazolyl is *1H*-isoindazolyl.

In another preferred embodiment, the benzothiazole group is benzo[*d*]thiazole group.

In another preferred embodiment, the benzothiophene group is benzo[*b*]thiophene group.

In another preferred embodiment, the haloindolyl-methyl- is 3-bromo-indolyl-methyl-.

In another preferred embodiment, the haloindolyl-methyl- is 3-bromo-*1H*-indolyl-methyl-.

In another preferred embodiment, the dihydropyranyl is 3, 6-dihydropyranyl.

In another preferred embodiment, the dihydropyranyl is 3, 6-dihydro-2*H*-pyranyl.

In another preferred embodiment, 1, 2, 3 or 4 of R₁, R₂, R₃ and R₄ are heteroaryl.

In another preferred embodiment, R₁, R₂, R₃ and R₄ are each independently 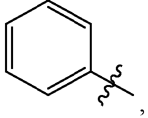 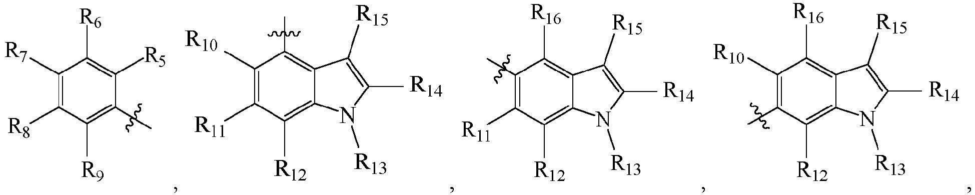 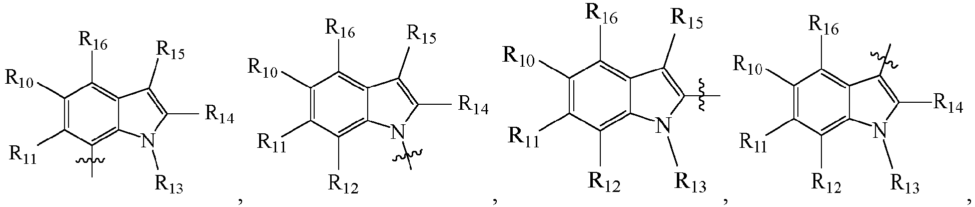 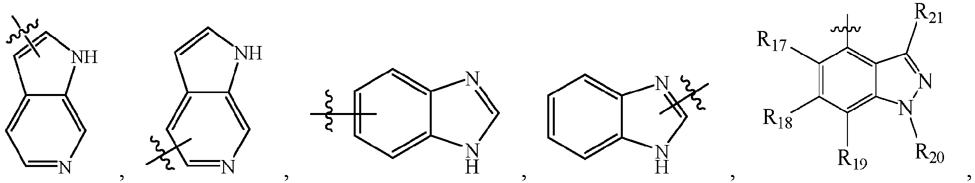 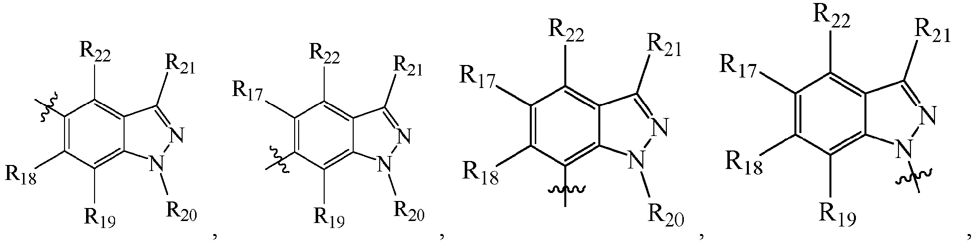 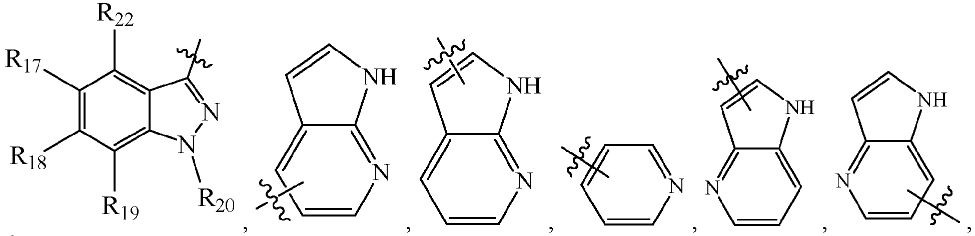 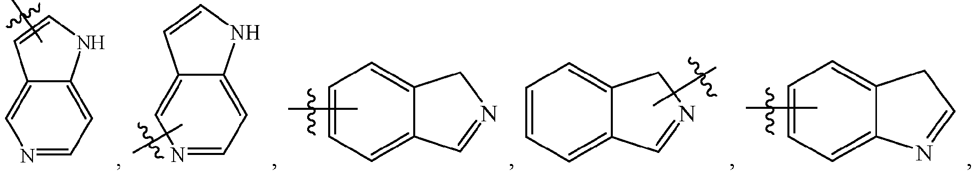 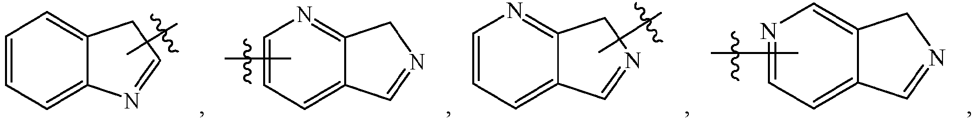 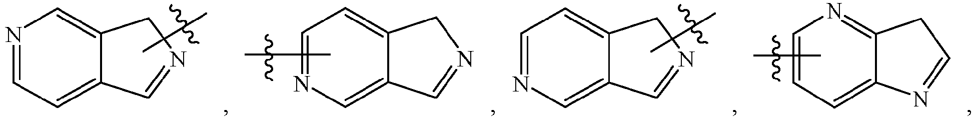 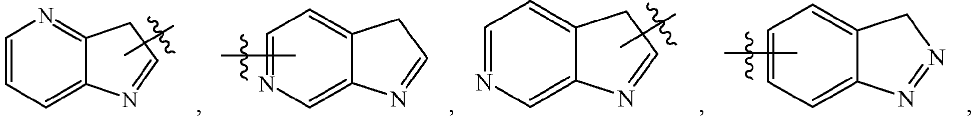 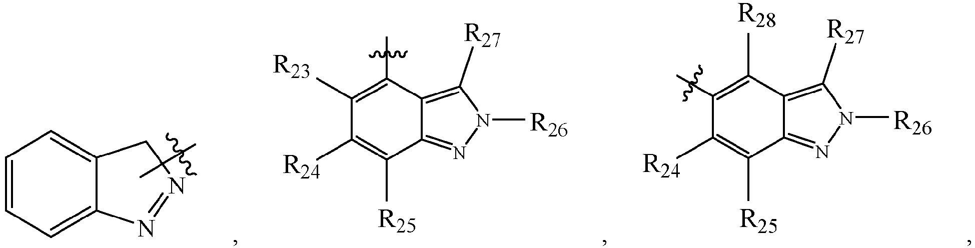 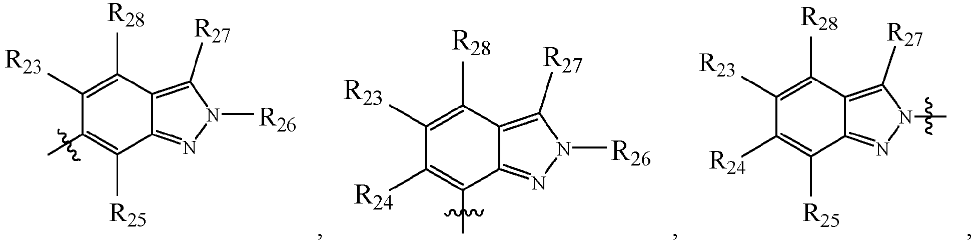 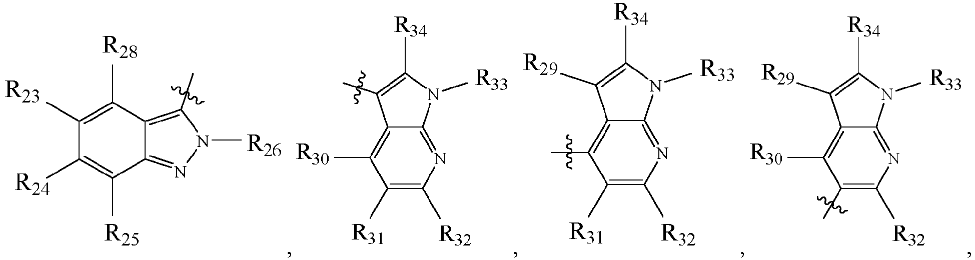 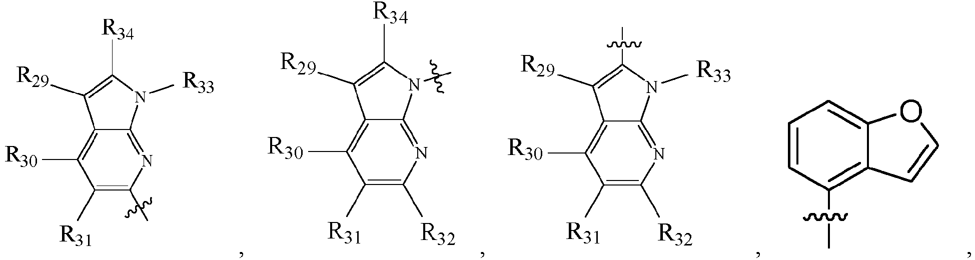 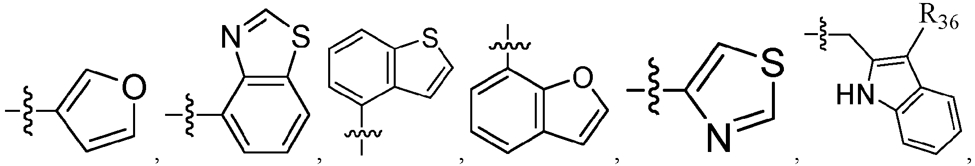 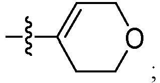
R₅, R₆, R₇, R₈ and R₉ are each independently hydrogen, C1-C10 alkyl, C1-C10 alkyl-O-, C1-C10 alkyl-S-;
R₁₀, R₁₁, R₁₂, R₁₃, R₁₄, R₁₅ and R₁₆ are each independently hydrogen, C1-C10 alkyl, C1-C10 alkyl-O-, C1-C10 alkyl-S-, 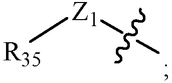
R₁₇, R₁₈, R₁₉, R₂₀, R₂₁ and R₂₂ are each independently hydrogen, C1-C10 alkyl, C1-C10 alkyl-O-, C1-C10 alkyl-S-;
R₂₃, R₂₄, R₂₅, R₂₆, R₂₇ and R₂₈ are each independently hydrogen, C1-C10 alkyl, C1-C10 alkyl-O-, C1-C10 alkyl-S-;
R₂₉, R₃₀, R₃₁, R₃₂, R₃₃ and R₃₄ are each independently hydrogen, C1-C10 alkyl, C1-C10 alkyl-O-, C1-C10 alkyl-S-;
R₃₅ is hydrogen, hydroxyl, sulfhydryl, 3-12 membered heterocycloalkyl or halogen;
R₃₆ is hydrogen or halogen;
Z₁ is C1-C8 alkylidene.

In another preferred embodiment, R₁, R₂, R₃ and R₄ are each independently

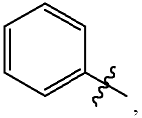

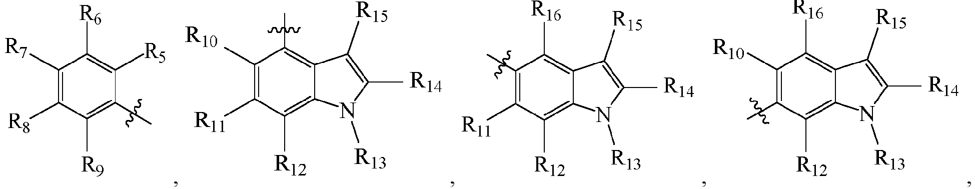

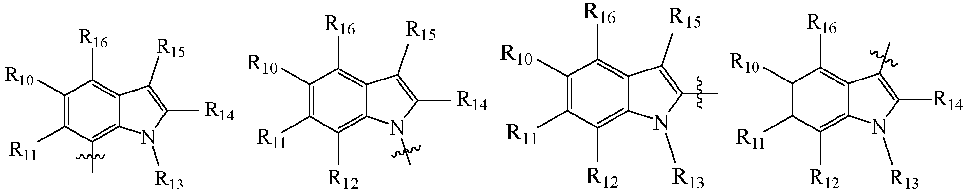

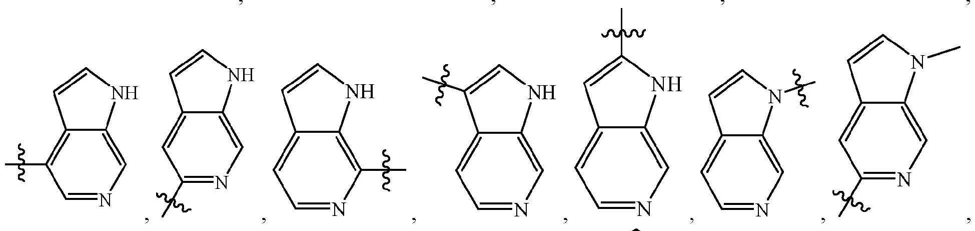

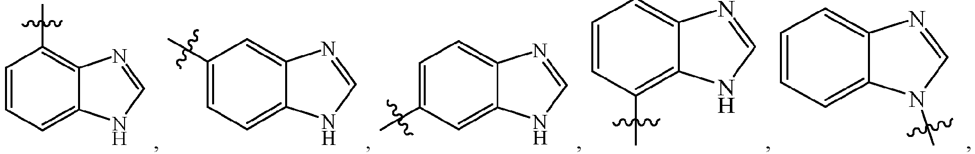

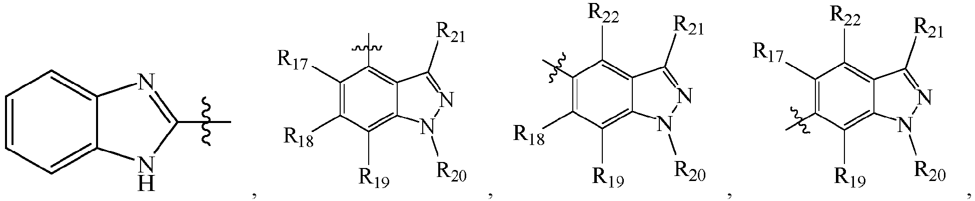

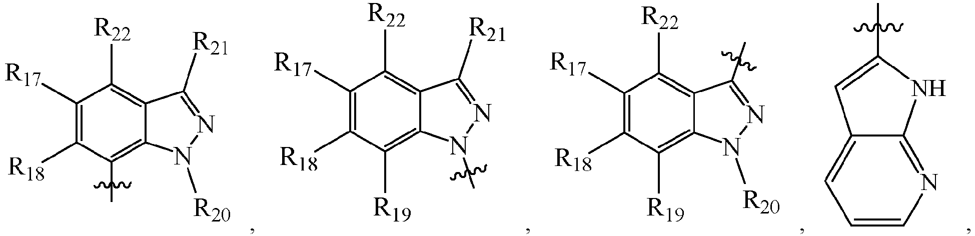

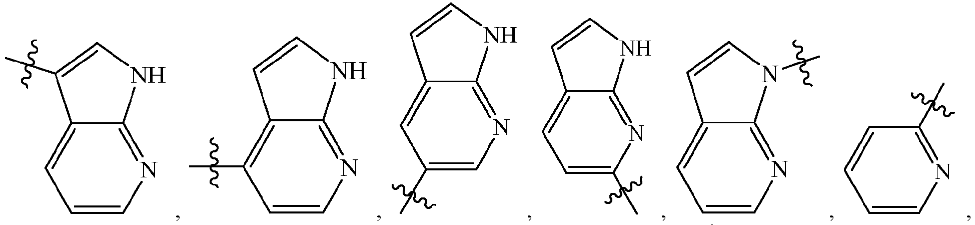

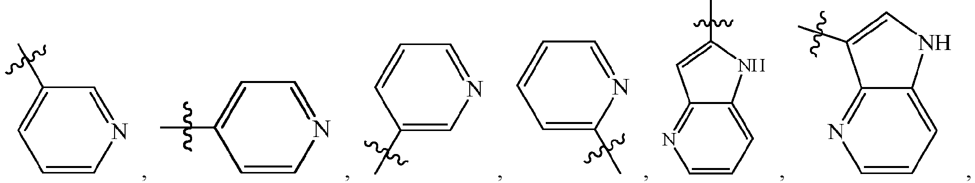

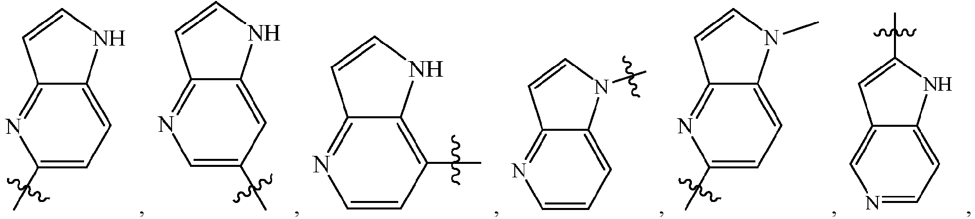

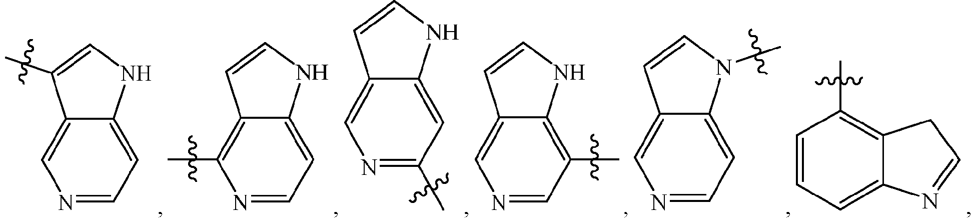

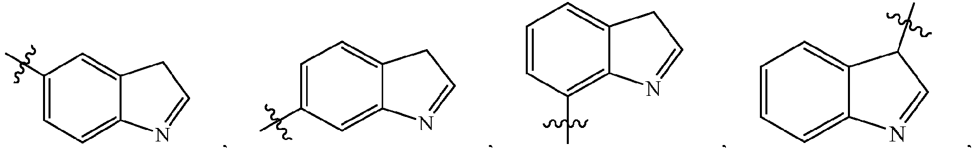

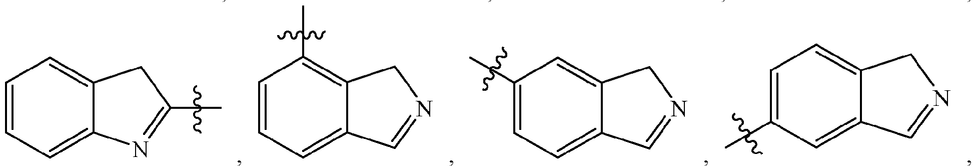

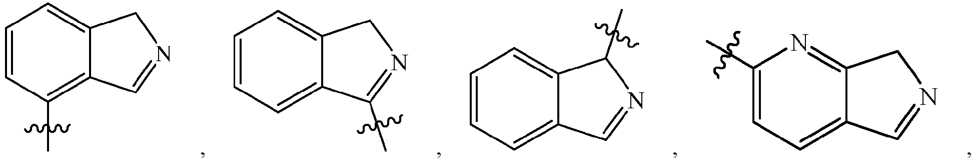

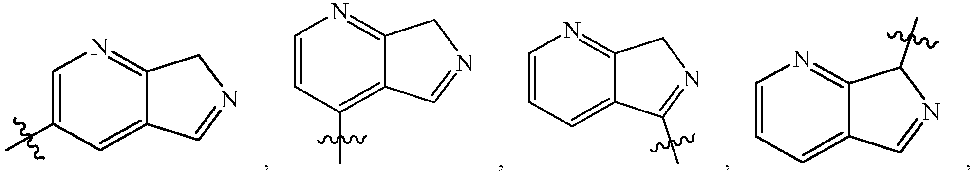

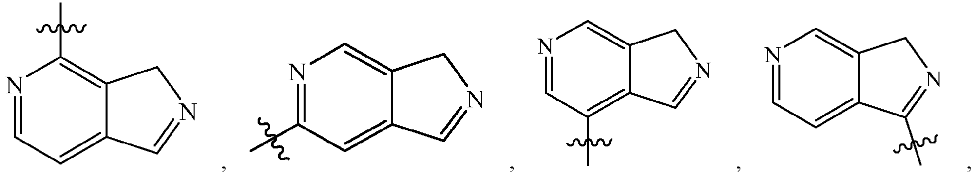

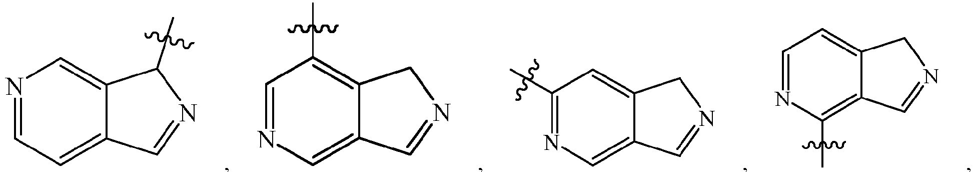

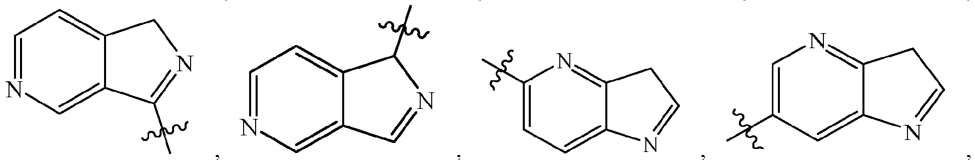

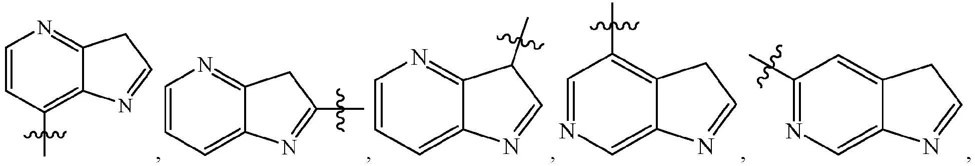

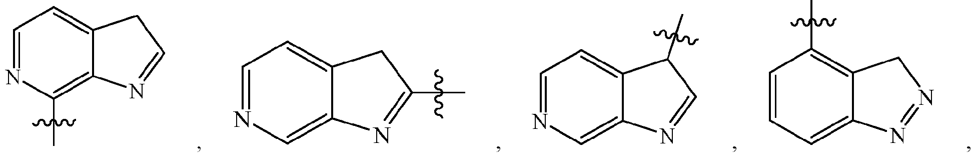

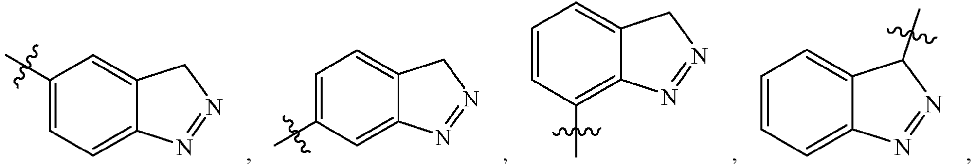

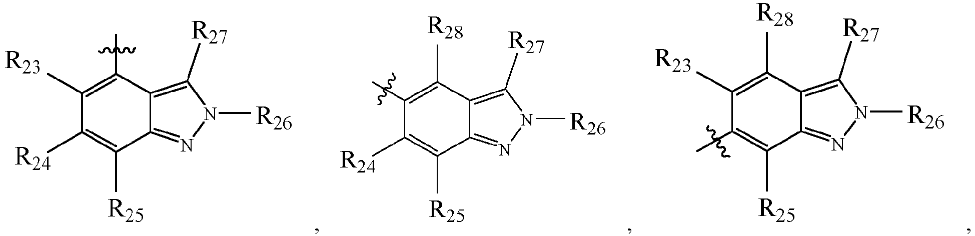

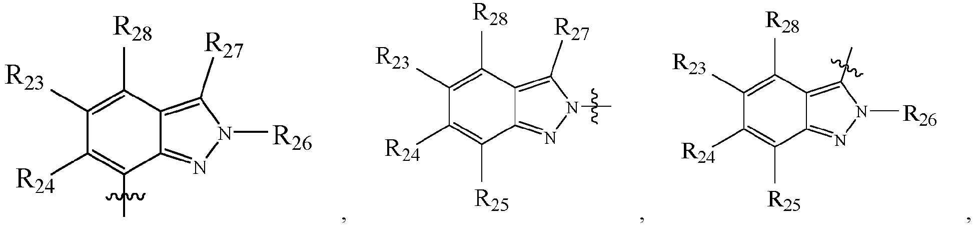

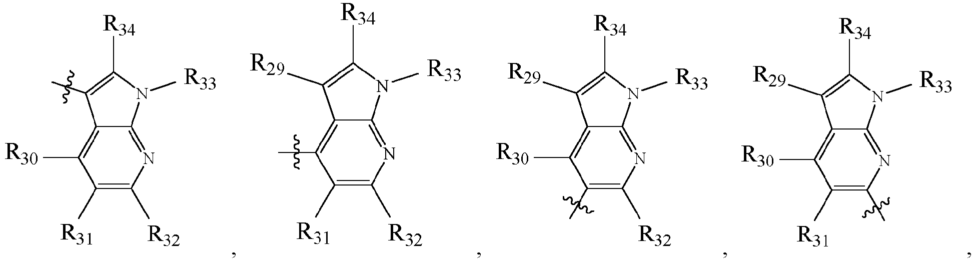

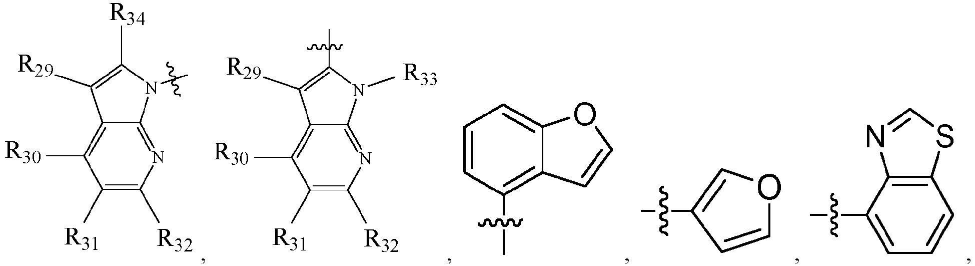

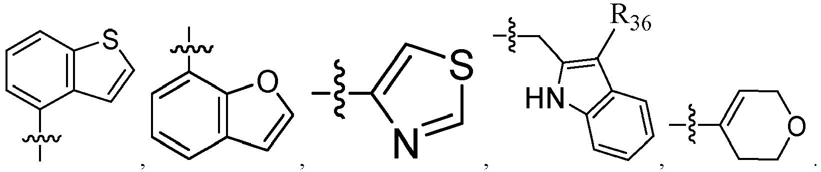

In another preferred embodiment, R₁, R₂, R₃ and R₄ are each independently

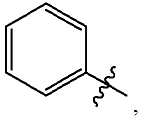

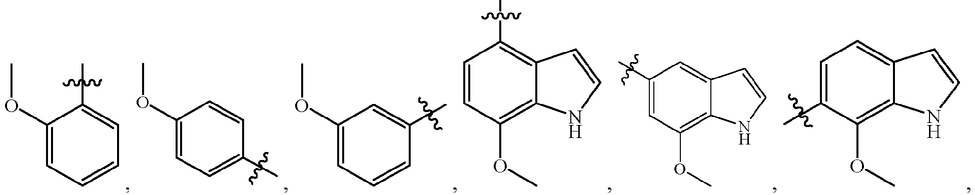

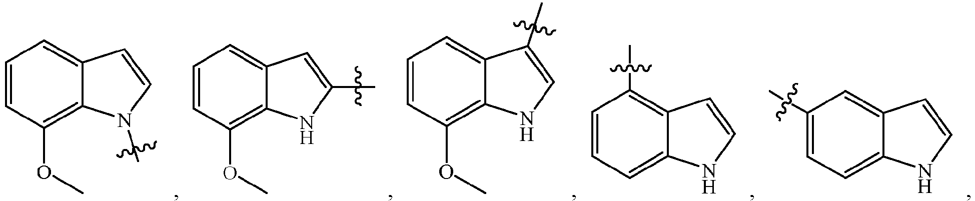

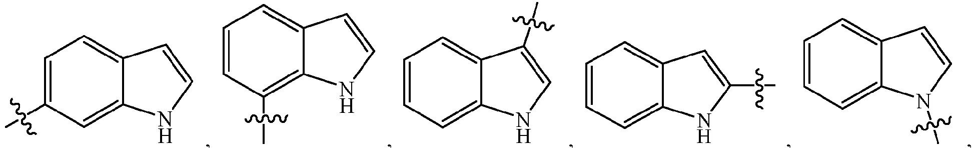

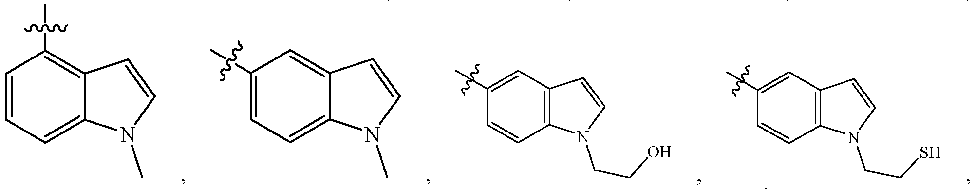

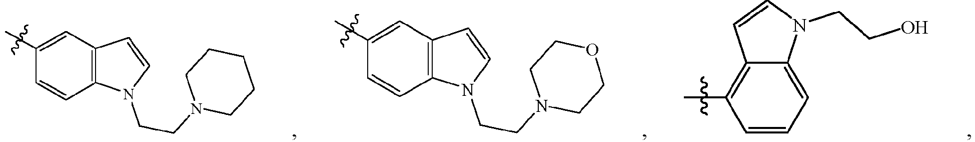

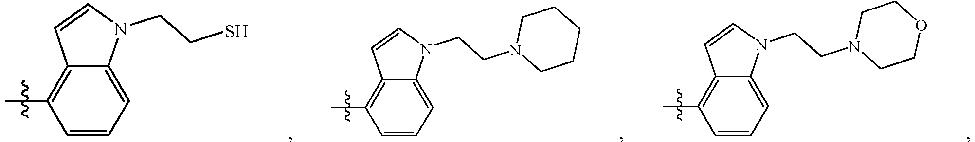

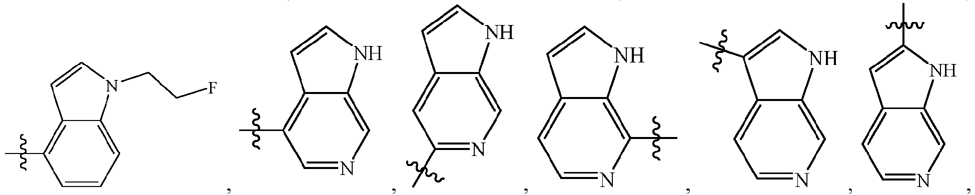

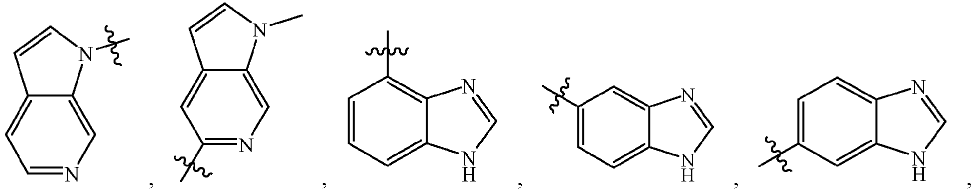

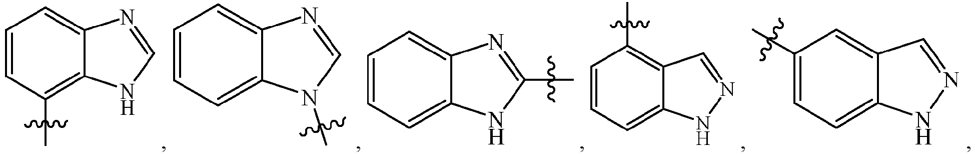

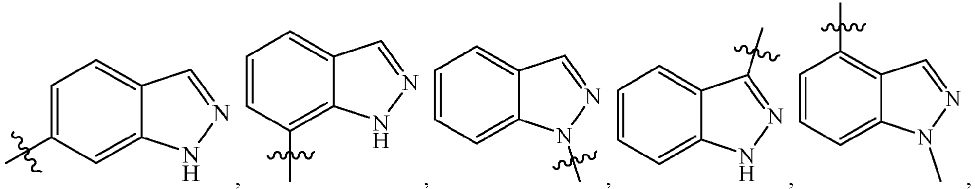

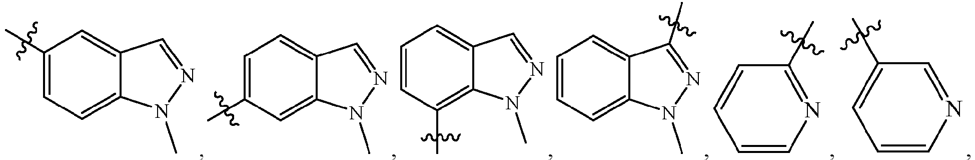

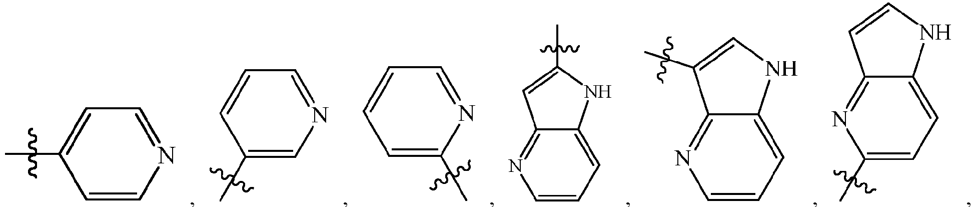

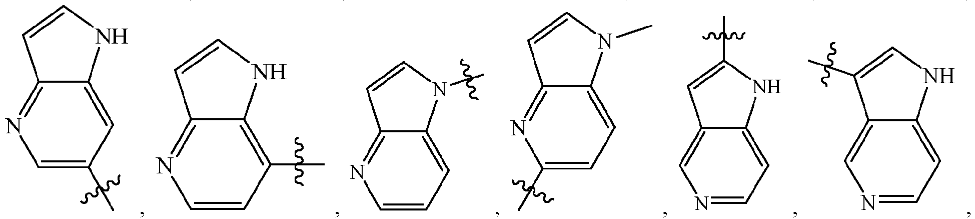

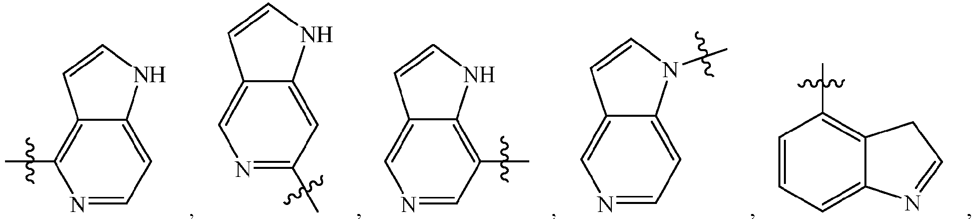

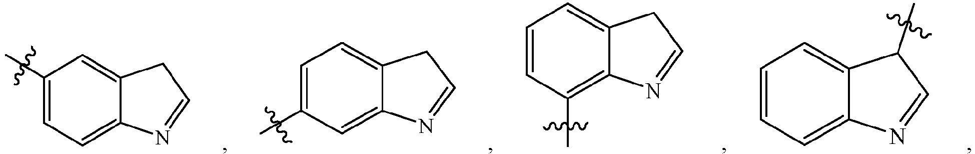

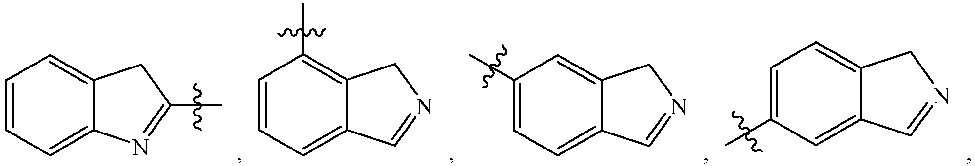

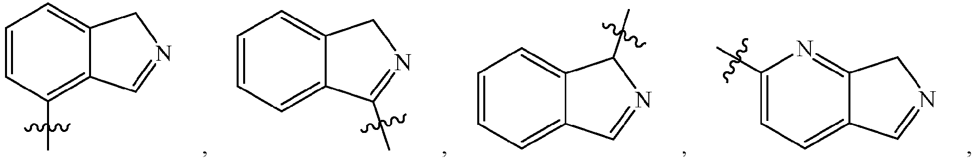

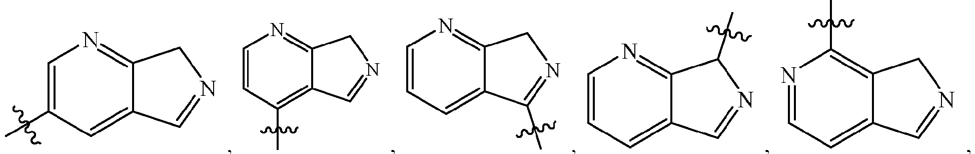

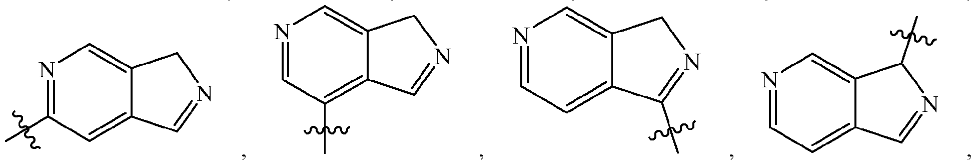

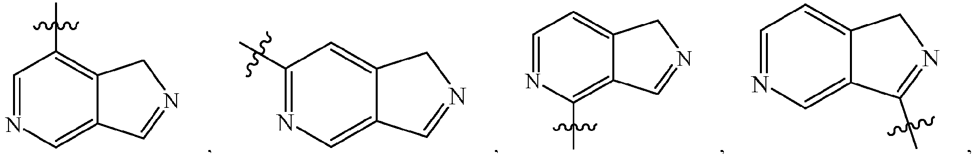

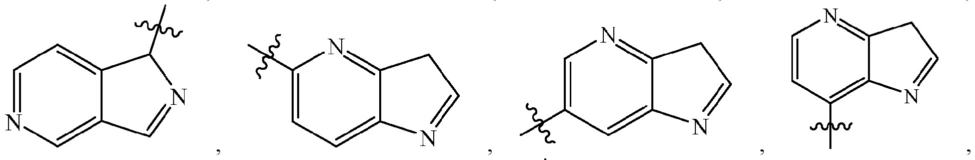

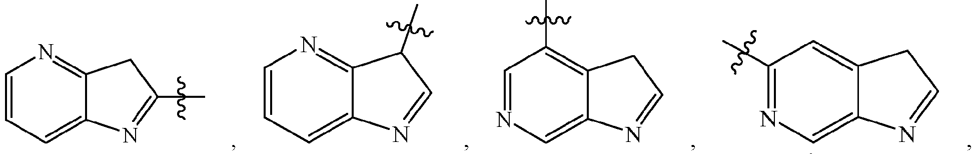

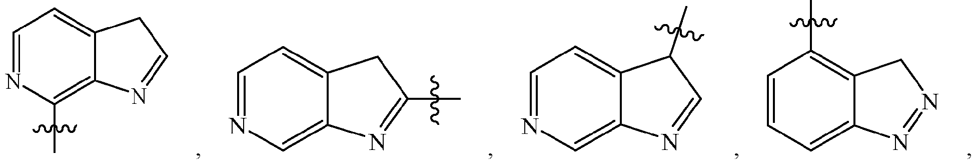

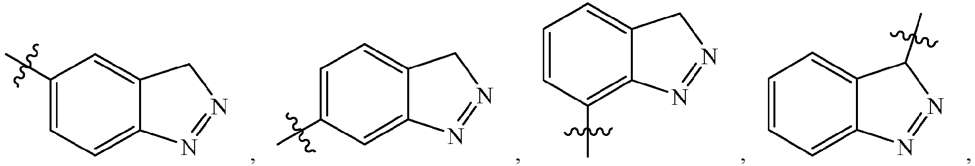

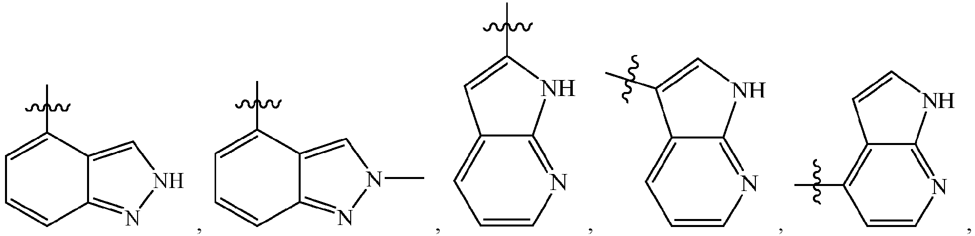

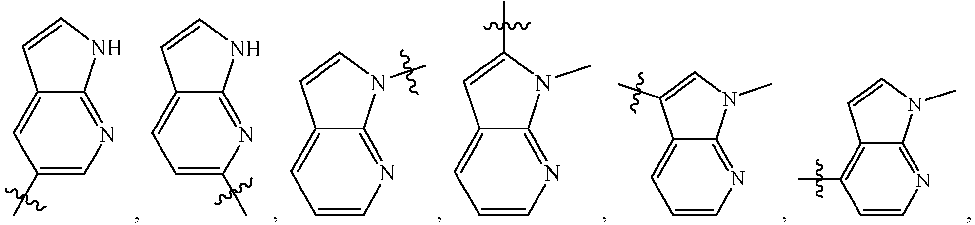

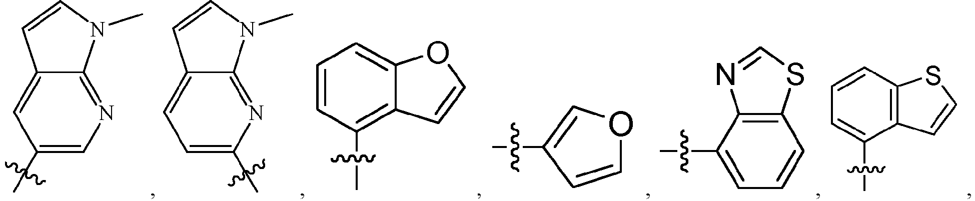

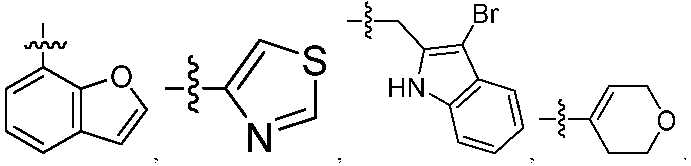

In another preferred embodiment, R₅, R₆, R₇, R₈ and R₉ are each independently hydrogen, C1-C10 alkyl, C1-C10 alkyl-O-, C1-C10 alkyl-S-.

In another preferred embodiment, R₅, R₆, R₇, R₈ and R₉ are each independently hydrogen, C1-C8 alkyl, C1-C8 alkyl-O-, C1-C8 alkyl-S-.

In another preferred embodiment, R₅, R₆, R₇, R₈ and R₉ are each independently hydrogen, C1-C6 alkyl, C1-C6 alkyl-O-, C1-C6 alkyl-S-.

In another preferred embodiment, R₅, R₆, R₇, R₈ and R₉ are each independently hydrogen, C1-C4 alkyl, C1-C4 alkyl-O-, C1-C4 alkyl-S-.

In another preferred embodiment, R₅, R₆, R₇, R₈ and R₉ are each independently hydrogen, methyl-O-, methyl-S-, methyl.

In another preferred embodiment, R₁₀, R₁₁, R₁₂, R₁₃, R₁₄, R₁₅ and R₁₆ are each independently hydrogen, C1-C10 alkyl, C1-C10 alkyl-O-, C1-C10 alkyl-S-, 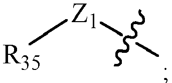
R₃₅ is hydrogen, hydroxyl, sulfhydryl, 3-12 membered heterocycloalkyl or halogen;
Z₁ is C1-C8 alkylidene.

In another preferred embodiment, R₁₀, R₁₁, R₁₂, R₁₃, R₁₄, R₁₅ and R₁₆ are each independently hydrogen, C1-C8 alkyl, C1-C8 alkyl-O-, C1-C8 alkyl-S-, 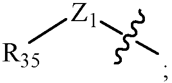
R₃₅ is hydrogen, hydroxyl, sulfhydryl, 3-12 membered heterocycloalkyl or halogen;
Z₁ is C1-C8 alkylidene.

In another preferred embodiment, R₁₀, R₁₁, R₁₂, R₁₃, R₁₄, R₁₅ and R₁₆ are each independently hydrogen, C1-C6 alkyl, C1-C6 alkyl-O-, C1-C6 alkyl-S-, 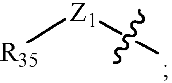
R₃₅ is hydrogen, hydroxyl, sulfhydryl, 3-12 membered heterocycloalkyl or halogen;
Z₁ is C1-C8 alkylidene.

In another preferred embodiment, R₁₀, R₁₁, R₁₂, R₁₃, R₁₄, R₁₅ and R₁₆ are each independently hydrogen, C1-C4 alkyl, C1-C4 alkyl-O-, C1-C4 alkyl-S-, 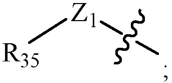
R₃₅ is hydrogen, hydroxyl, sulfhydryl, 3-12 membered heterocycloalkyl or halogen;
Z₁ is C1-C8 alkylidene.

In another preferred embodiment, R₁₀, R₁₁, R₁₂, R₁₃, R₁₄, R₁₅ and R₁₆ are each independently hydrogen, methyl-O-, methyl-S-, methyl, hydroxyl-ethyl-, sulfhydryl-ethyl-, piperidinyl-ethyl-, morpholinyl-ethyl-, haloethyl.

In another preferred embodiment the hydroxyl-ethyl- is

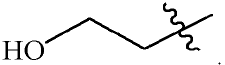

In another preferred embodiment, the sulfhydryl-ethyl- is

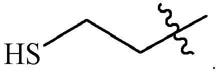

In another preferred embodiment, the piperidinyl-ethyl- is

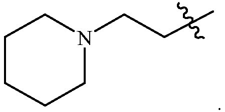

In another preferred embodiment, the morpholinyl-ethyl- is

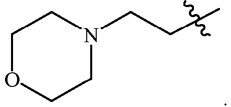

In another preferred embodiment, the haloethyl is fluoroethyl.

In another preferred embodiment, the haloethyl is

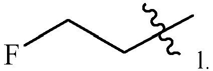

In another preferred embodiment, R₁₇, R₁₈, R₁₉, R₂₀, R₂₁ and R₂₂ are each independently hydrogen, C1-C10 alkyl, C1-C10 alkyl-O-, C1-C10 alkyl-S-.

In another preferred embodiment, R₁₇, R₁₈, R₁₉, R₂₀, R₂₁ and R₂₂ are each independently hydrogen, C1-C8 alkyl, C1-C8 alkyl-O-, C1-C8 alkyl-S-.

In another preferred embodiment, R₁₇, R₁₈, R₁₉, R₂₀, R₂₁ and R₂₂ are each independently hydrogen, C1-C6 alkyl, C1-C6 alkyl-O-, C1-C6 alkyl-S-.

In another preferred embodiment, R₁₇, R₁₈, R₁₉, R₂₀, R₂₁ and R₂₂ are each independently hydrogen, C1-C4 alkyl, C1-C4 alkyl-O-, C1-C4 alkyl-S-.

In another preferred embodiment, R₁₇, R₁₈, R₁₉, R₂₀, R₂₁ and R₂₂ are each independently hydrogen, methyl.

In another preferred embodiment, R₂₃, R₂₄, R₂₅, R₂₆, R₂₇ and R₂₈ are each independently hydrogen, C1-C10 alkyl, C1-C10 alkyl-O-, C1-C10 alkyl-S-.

In another preferred embodiment, R₂₃, R₂₄, R₂₅, R₂₆, R₂₇ and R₂₈ are each independently hydrogen, C1-C8 alkyl, C1-C8 alkyl-O-, C1-C8 alkyl-S-.

In another preferred embodiment, R₂₃, R₂₄, R₂₅, R₂₆, R₂₇ and R₂₈ are each independently hydrogen, C1-C6 alkyl, C1-C6 alkyl-O-, C1-C6 alkyl-S-.

In another preferred embodiment, R₂₃, R₂₄, R₂₅, R₂₆, R₂₇ and R₂₈ are each independently hydrogen, C1-C4 alkyl, C1-C4 alkyl-O-, C1-C4 alkyl-S-.

In another preferred embodiment, R₂₃, R₂₄, R₂₅, R₂₆, R₂₇ and R₂₈ are each independently hydrogen, methyl.

In another preferred embodiment, R₂₉, R₃₀, R₃₁, R₃₂, R₃₃ and R₃₄ are each independently hydrogen, C1-C10 alkyl, C1-C10 alkyl-O-, C1-C10 alkyl-S-.

In another preferred embodiment, R₂₉, R₃₀, R₃₁, R₃₂, R₃₃ and R₃₄ are each independently hydrogen, C1-C8 alkyl, C1-C8 alkyl-O-, C1-C8 alkyl-S-.

In another preferred embodiment, R₂₉, R₃₀, R₃₁, R₃₂, R₃₃ and R₃₄ are each independently hydrogen, C1-C6 alkyl, C1-C6 alkyl-O-, C1-C6 alkyl-S-.

In another preferred embodiment, R₂₉, R₃₀, R₃₁, R₃₂, R₃₃ and R₃₄ are each independently hydrogen, C1-C4 alkyl, C1-C4 alkyl-O-, C1-C4 alkyl-S-.

In another preferred embodiment, R₂₉, R₃₀, R₃₁, R₃₂, R₃₃ and R₃₄ are each independently hydrogen, methyl.

In another preferred embodiment, each "substituted" means that one or more (preferably 1, 2, 3, 4, 5, 6, 7, or 8) hydrogen atoms on the group are independently substituted by substituent selected from the group consisting of C1-C12 alkyl, C3-C8 cycloalkyl, C1-C12 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C8 ester group, C2-C4 amide group, C1-C12 alkyl-O-, C1-C12 alkyl-S-, C1-C12 haloalkoxyl, C1-C12 haloalkylthio, C6-C12 aryl, 5-12 membered heteroaryl, 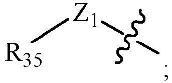
R₃₅ is hydrogen, hydroxyl, sulfhydryl, 3-12 membered heterocycloalkyl or halogen;
Z₁ is C1-C8 alkylidene.

In another preferred embodiment, each "substituted" means that one or more (preferably 1, 2, 3, 4, 5, 6, 7, or 8) hydrogen atoms on the group are independently substituted by substituent selected from the group consisting of C1-C10 alkyl, C3-C8 cycloalkyl, C1-C10 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C8 ester group, C2-C4 amide group, C1-C10 alkyl-O-, C1-C10 alkyl-S-, C1-C10 haloalkoxyl, C1-C10 haloalkylthio, C6-C12 aryl, 5-12 membered heteroaryl, 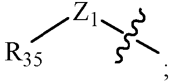
R₃₅ is hydrogen, hydroxyl, sulfhydryl, 3-12 membered heterocycloalkyl or halogen;
Z₁ is C1-C8 alkylidene.

In another preferred embodiment, each "substituted" means that one or more (preferably 1, 2, 3, 4, 5, 6, 7, or 8) hydrogen atoms on the group are independently substituted by substituent selected from the group consisting of C1-C8 alkyl, C3-C8 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C8 ester group, C2-C4 amide group, C1-C8 alkyl-O-, C1-C8 alkyl-S-, C1-C8 haloalkoxyl, C1-C8 haloalkylthio, C6-C10 aryl, 5-10 membered heteroaryl, 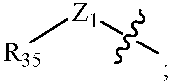
R₃₅ is hydrogen, hydroxyl, sulfhydryl, 3-12 membered heterocycloalkyl or halogen;
Z₁ is C1-C8 alkylidene.

In another preferred embodiment, each "substituted" means that one or more (preferably 1, 2, 3, 4, 5, 6, 7, or 8) hydrogen atoms on the group are independently substituted by substituent selected from the group consisting of C1-C6 alkyl, C3-C8 cycloalkyl, C1-C6 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C6 ester group, C2-C4 amide group, C1-C6 alkyl-O-, C1-C6 alkyl-S-, C1-C6 haloalkoxyl, C1-C6 haloalkylthio, C6-C10 aryl, 5-10 membered heteroaryl, 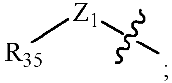
R₃₅ is hydrogen, hydroxyl, sulfhydryl, 3-12 membered heterocycloalkyl or halogen;
Z₁ is C1-C8 alkylidene.

In another preferred embodiment, each "substituted" means that one or more (preferably 1, 2, 3, 4, 5, 6, 7, or 8) hydrogen atoms on the group are independently substituted by substituent selected from the group consisting of C1-C4 alkyl, C3-C8 cycloalkyl, C1-C4 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C6 ester group, C2-C4 amide group, C1-C4 alkyl-O-, C1-C4 alkyl-S-, C1-C4 haloalkoxyl, C1-C4 haloalkylthio, C6-C10 aryl, 5-10 membered heteroaryl, 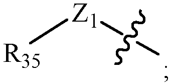
R₃₅ is hydrogen, hydroxyl, sulfhydryl, 3-12 membered heterocycloalkyl or halogen;
Z₁ is C1-C8 alkylidene.

In another preferred embodiment, R₃₅ is hydrogen, hydroxyl, sulfhydryl, 3-10 membered heterocycloalkyl or halogen.

In another preferred embodiment, R₃₅ is hydrogen, hydroxyl, sulfhydryl, 3-8 membered heterocycloalkyl or halogen.

In another preferred embodiment, R₃₅ is hydrogen, hydroxyl, sulfhydryl, 5-8 membered heterocycloalkyl or halogen.

In another preferred embodiment, R₃₅ is hydrogen, hydroxyl, sulfhydryl, 5 membered heterocycloalkyl, 6 membered heterocycloalkyl, 7 membered heterocycloalkyl, 8 membered heterocycloalkyl, 9 membered heterocycloalkyl, 10 membered heterocycloalkyl, 11 membered heterocycloalkyl, 12 membered heterocycloalkyl or halogen.

In another preferred embodiment, R₃₅ is hydrogen, hydroxyl, sulfhydryl, piperidinyl, morpholinyl or halogen.

In another preferred embodiment, R₃₆ is hydrogen or halogen.

In another preferred embodiment, R₃₆ is hydrogen or bromine.

In another preferred embodiment, halogen is fluorine, chlorine, bromine, or iodine.

In another preferred embodiment, halo is fluoro, chloro, bromo or iodo.

In another preferred embodiment, the halo is mono-halo, di-halo or full-halo.

In another preferred embodiment, Z₁ is C1-C6 alkylidene.

In another preferred embodiment, Z₁ is C1-C4 alkylidene.

In another preferred embodiment, Z₁ is ethylidene.

In another preferred embodiment, Z₁ is

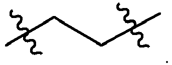

In another preferred embodiment, 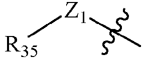 is

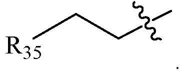

In another preferred embodiment, the heterocyclic ring of the heterocycloalkyl and heteroaryl has 1-4 (preferably 1, 2, 3 or 4) heteroatoms independently selected from the group consisting of N, O and S.

In another preferred embodiment, the heterocyclic ring of the heterocycloalkyl has 1-4 (preferably 1, 2, 3 or 4) heteroatoms independently selected from the group consisting of N, O and S.

In another preferred embodiment, the heterocyclic ring of the heteroaryl has 1-4 (preferably 1, 2, 3 or 4) heteroatoms independently selected from the group consisting of N, O and S.

In another preferred embodiment, the heterocycloalkyl has 0, 1 or 2 C=C ring double bonds.

In another preferred embodiment, alkyl is methyl.

In another preferred embodiment, the compound of formula I, or an optical isomer thereof, or a racemate thereof, or a solvate thereof, or a pharmaceutically acceptable salt thereof, or a deuterated compound thereof has the following structure of formula I-1: 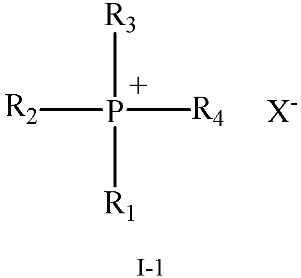
wherein, R₁, R₂ , R₃ and R₄ are each independently defined as described above;
X⁻ is anionic salt radical.

In another preferred embodiment, X⁻ is anionic acid radical.

In another preferred embodiment, the pharmaceutically acceptable salt of the compound of formula I comprises the salt formed by the compound of formula I and an acid.

In another preferred embodiment, the acid comprises one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, trifluoromethanesulfonic acid, benzenesulfonic acid, aspartic acid and glutamic acid.

In another preferred embodiment, the pharmaceutically acceptable salt of the compound of formula I comprises the salt formed by the compound of formula I and hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, trifluoromethanesulfonic acid, aspartic acid or glutamic acid.

In another preferred embodiment, the salt radical of the pharmaceutically acceptable salt of the compound of formula I comprises the salt radical formed by the loss of a H⁺ in the acid.

In another preferred embodiment, the salt radical of the pharmaceutically acceptable salt of the compound of formula I comprises the salt radical formed by the loss of a H⁺ in hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, trifluoromethanesulfonic acid, aspartic acid or glutamic acid.

In another preferred embodiment, the salt radical of the pharmaceutically acceptable salt of the compound of formula I comprises F-, Cl-, Br-, I-, HCOO-, CH₃COO-, 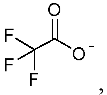 SO₄²⁻, NO³⁻ or

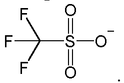

In another preferred embodiment, X⁻ is salt radical formed by the loss of a H⁺ in the acid.

In another preferred embodiment, X⁻ is salt radical formed by the loss of a H⁺ in the hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, trifluoromethanesulfonic acid, aspartic acid or glutamic acid.

In another preferred embodiment, X⁻ is F⁻, Cl⁻, Br⁻, I⁻, HCOO⁻, CH₃COO⁻, 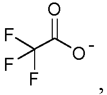 SO₄²-, NO₃⁻

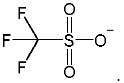 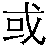

In another preferred embodiment, the compound of formula I, or an optical isomer thereof, or a racemate thereof, or a solvate thereof, or a pharmaceutically acceptable salt thereof, or a deuterated compound thereof is selected from the following group:

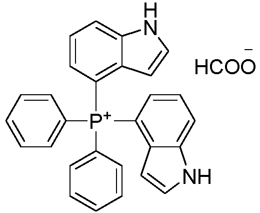

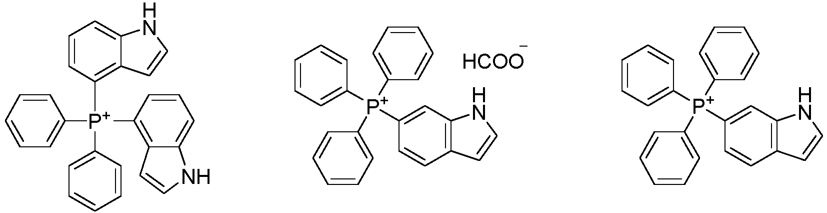

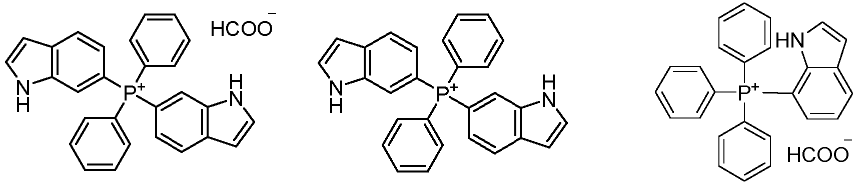

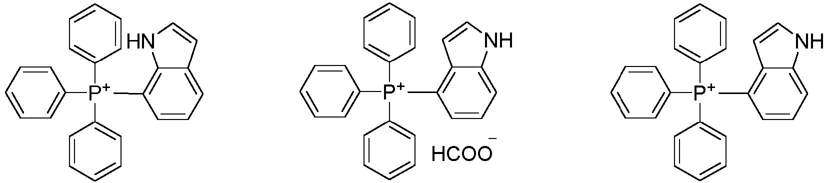

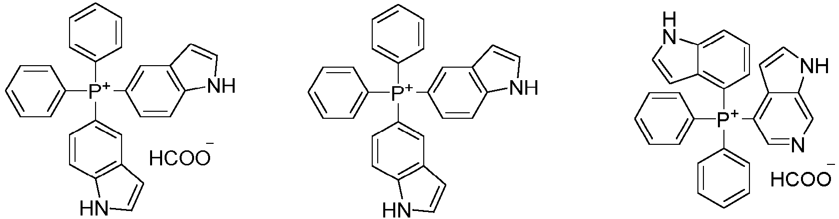

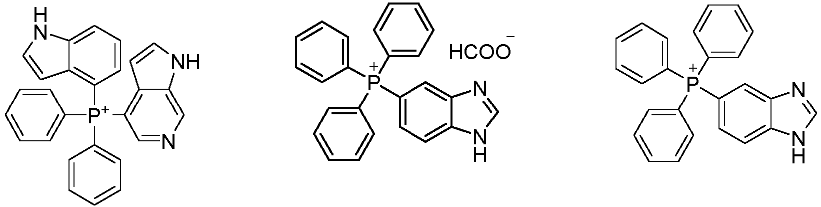

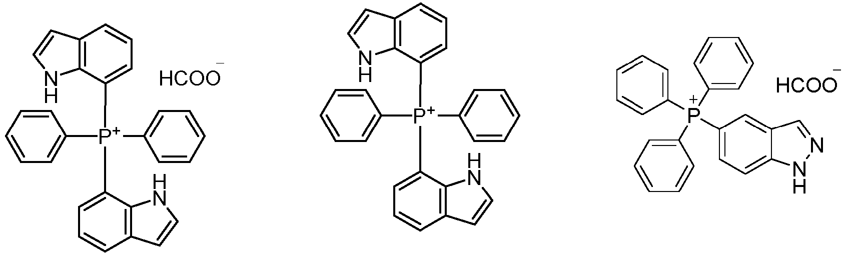

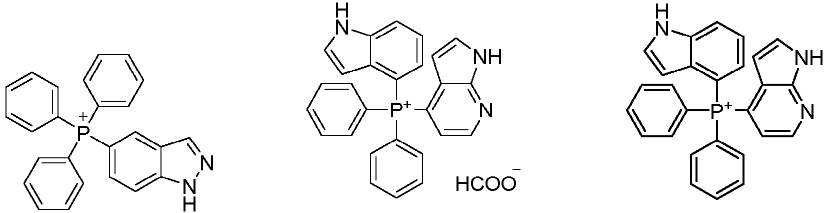

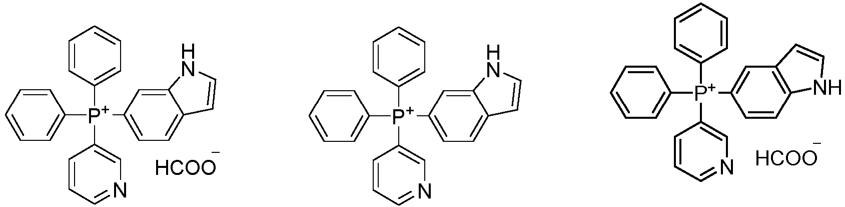

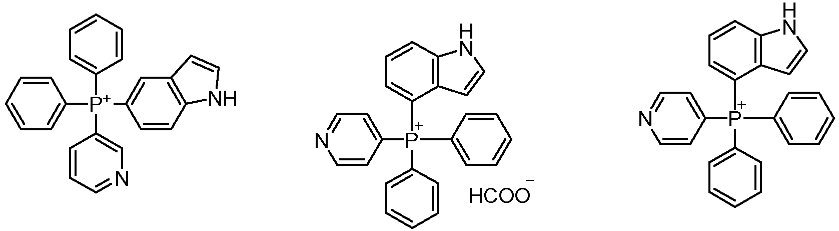

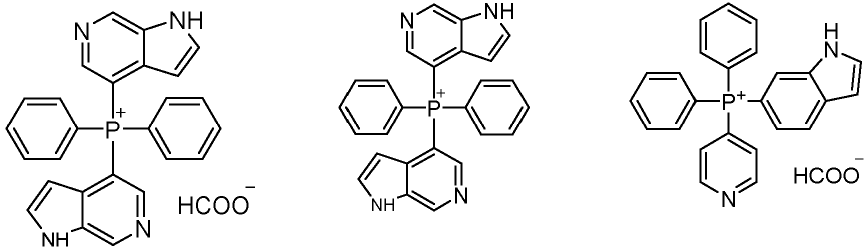

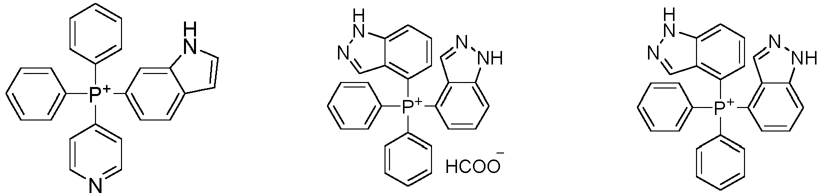

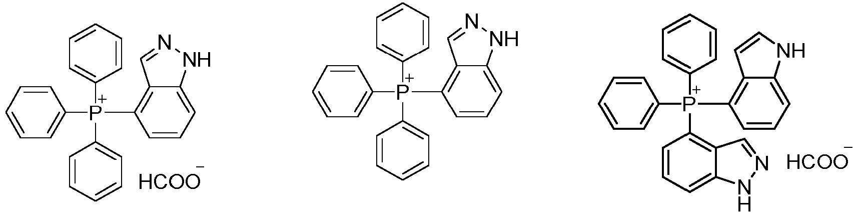

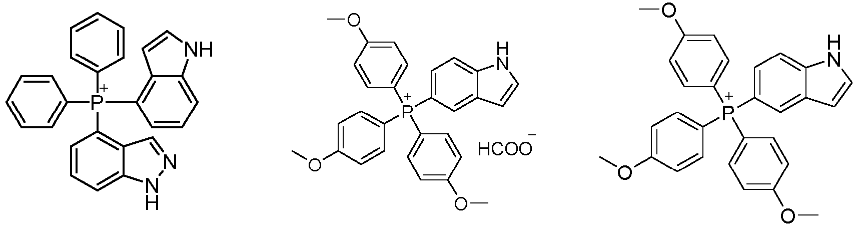

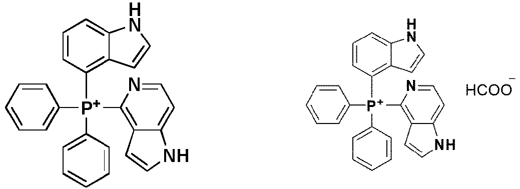

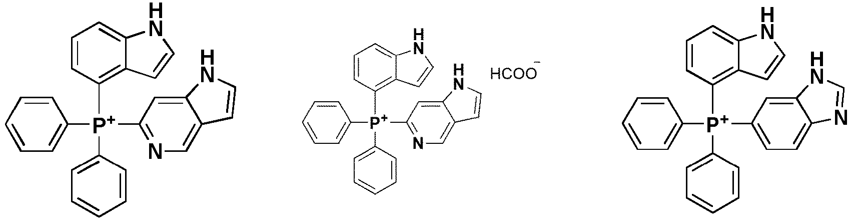

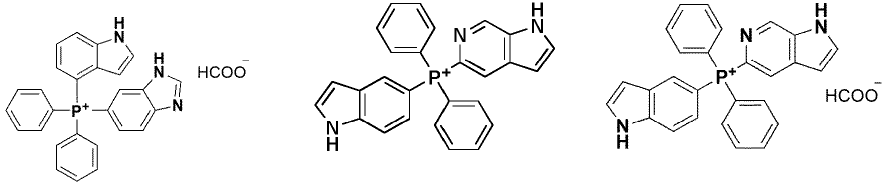

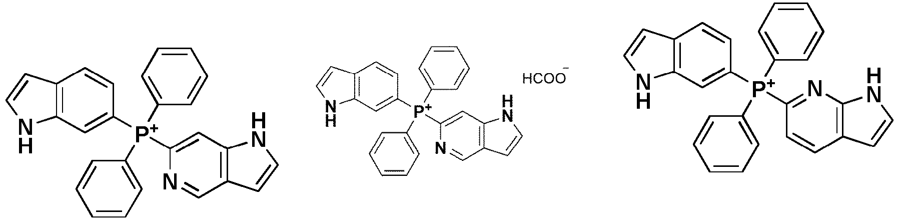

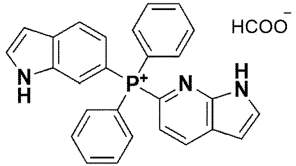

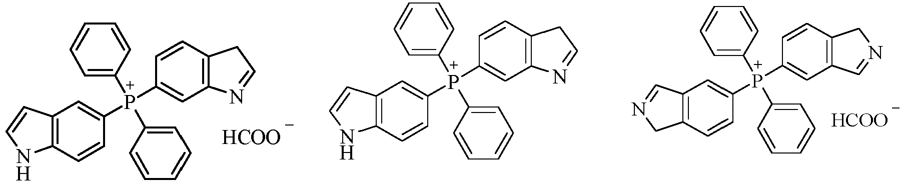

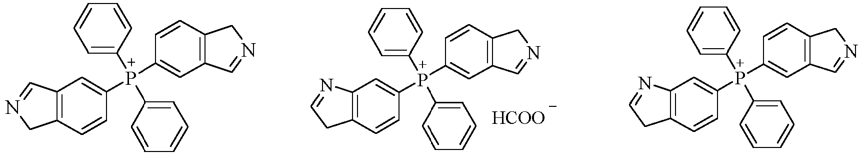

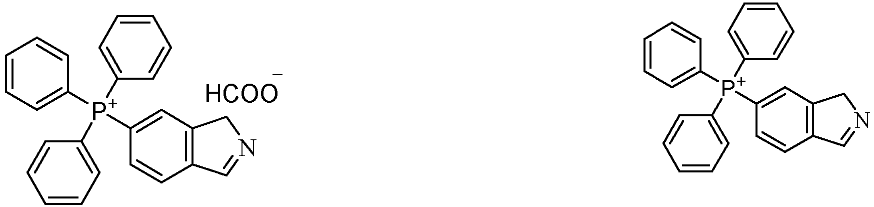

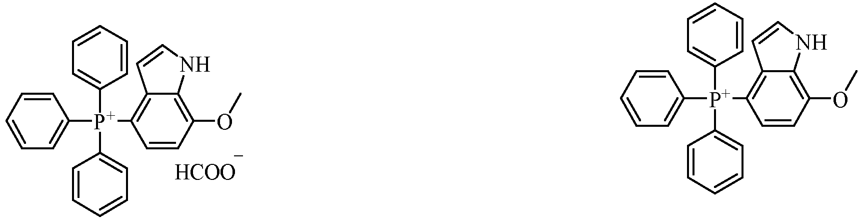

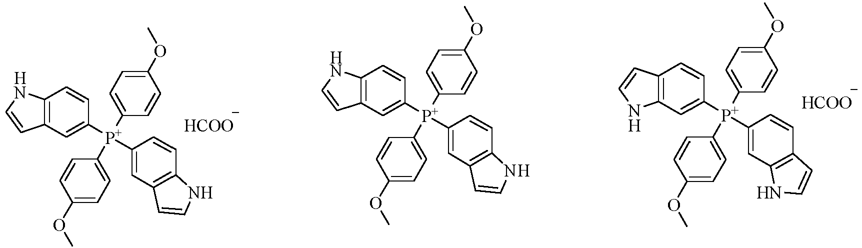

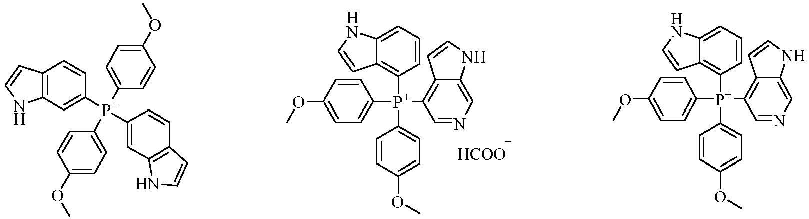

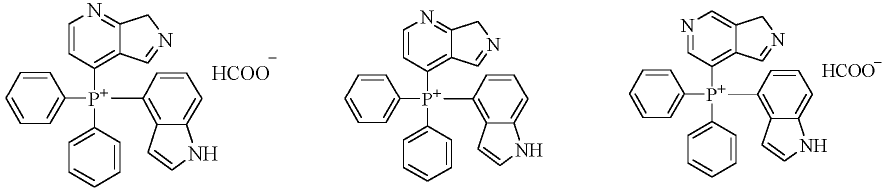

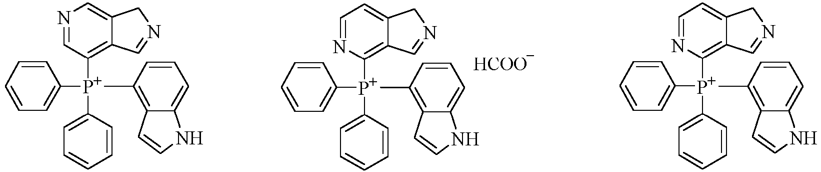

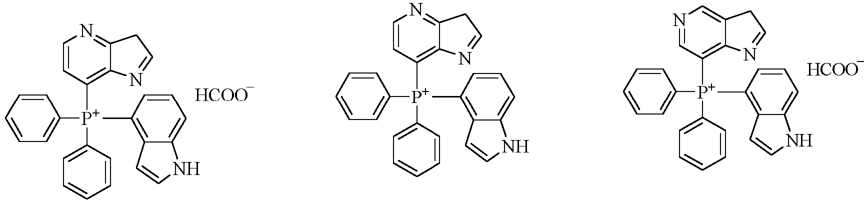

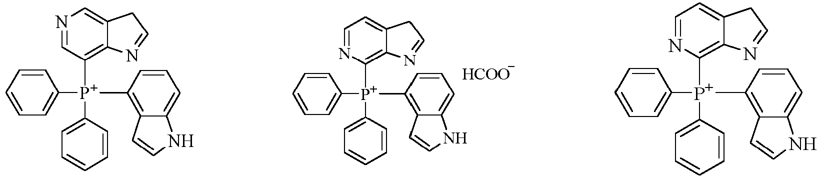

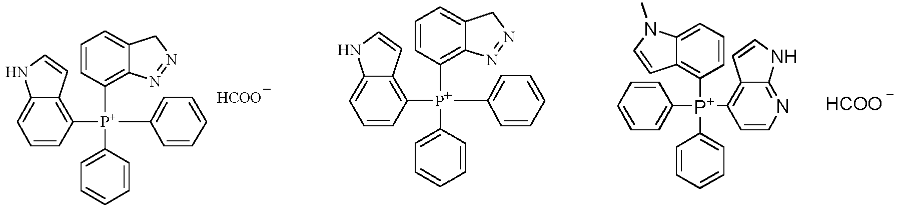

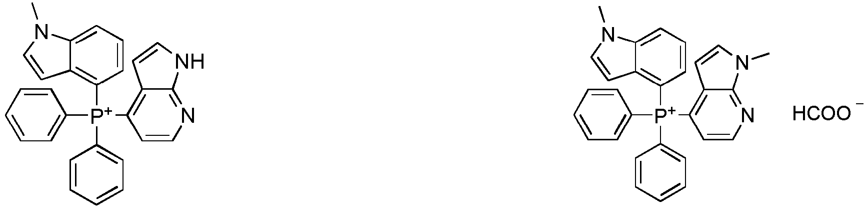

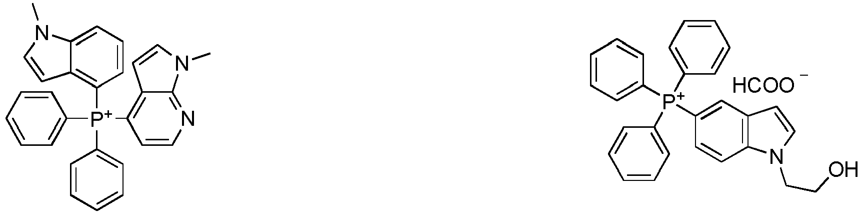

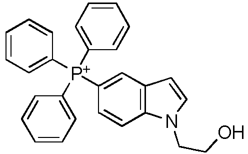

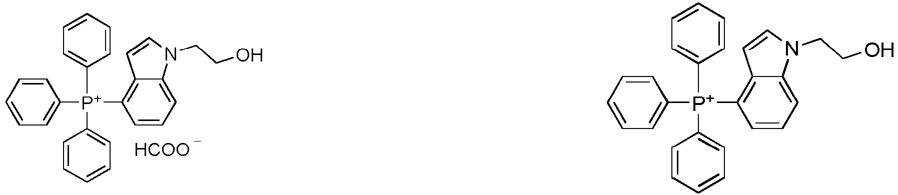

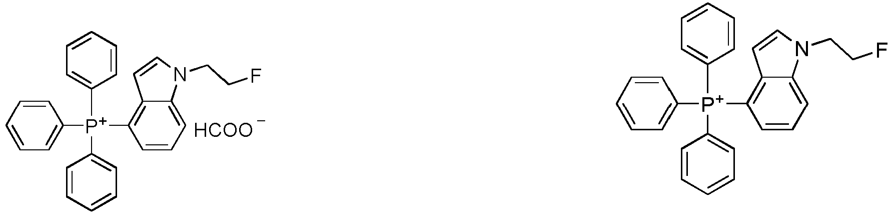

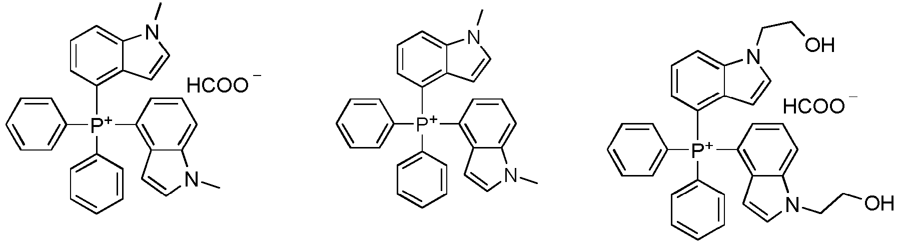

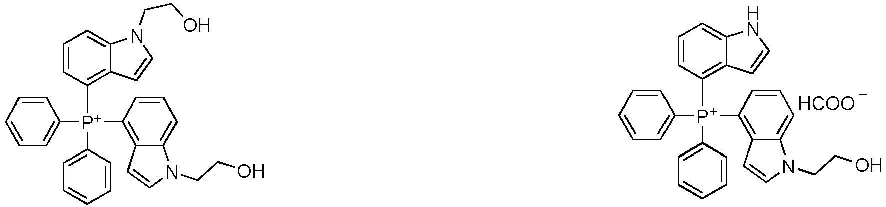

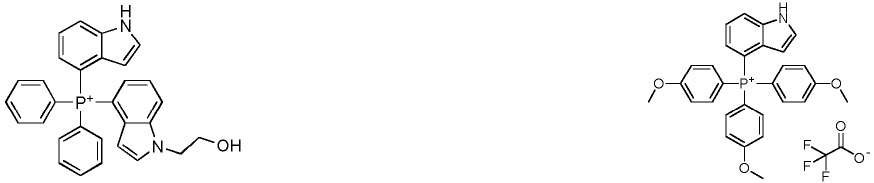

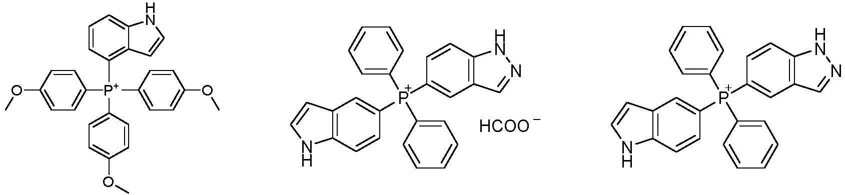

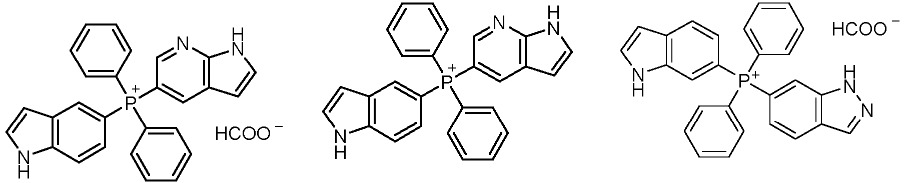

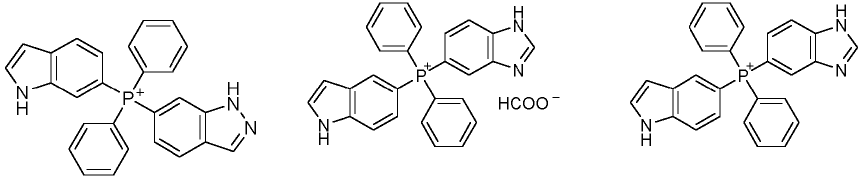

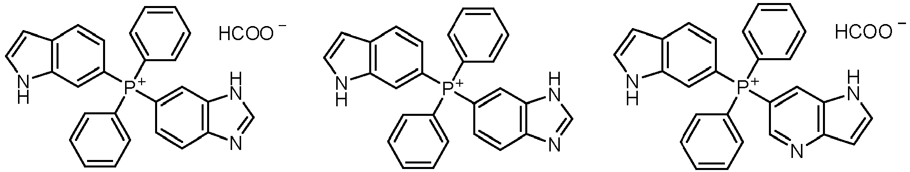

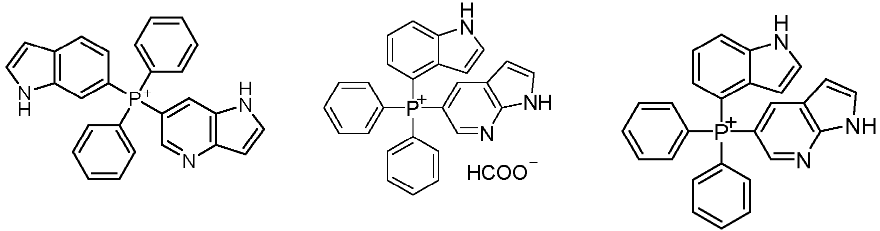

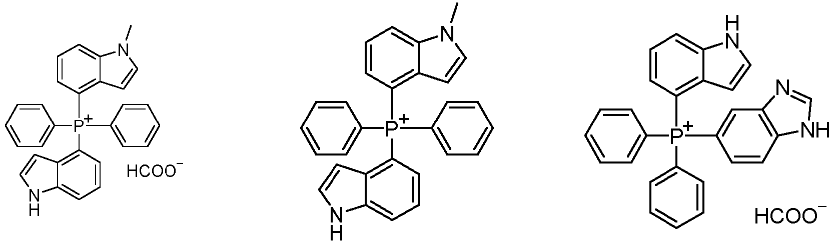

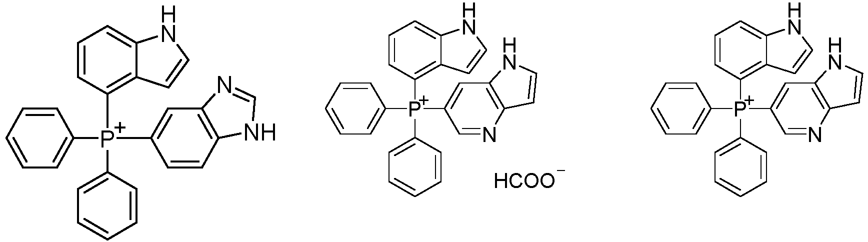

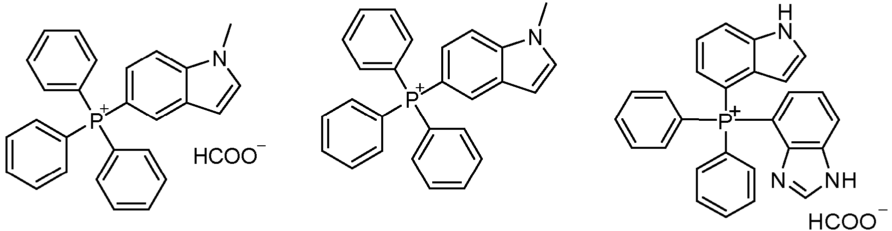

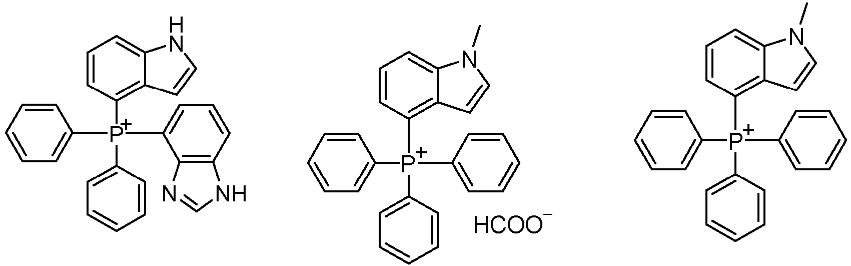

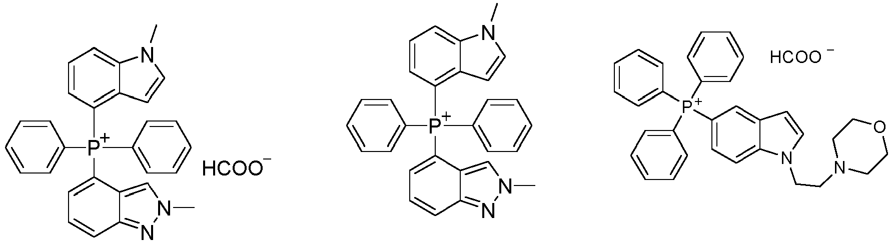

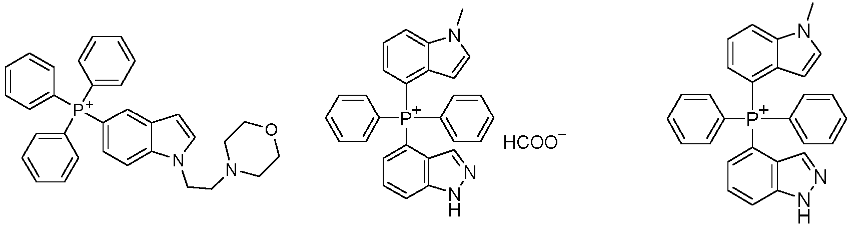

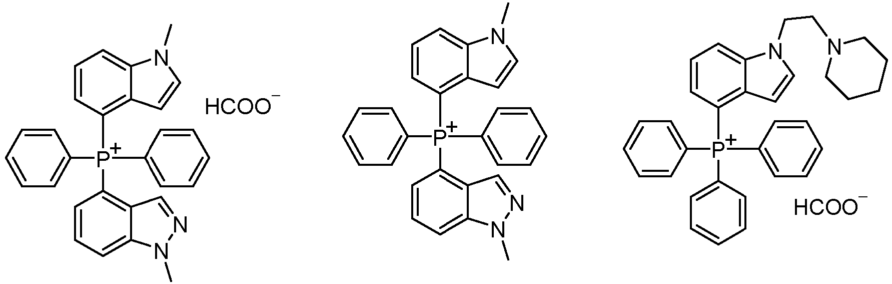

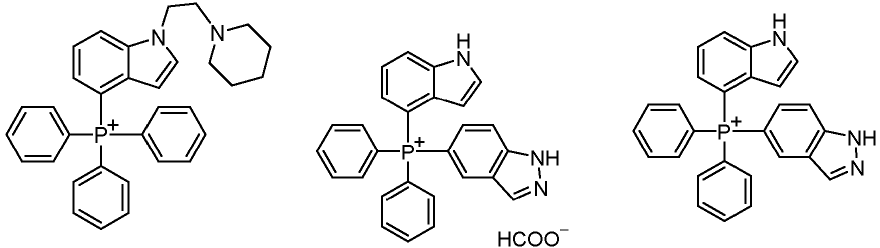

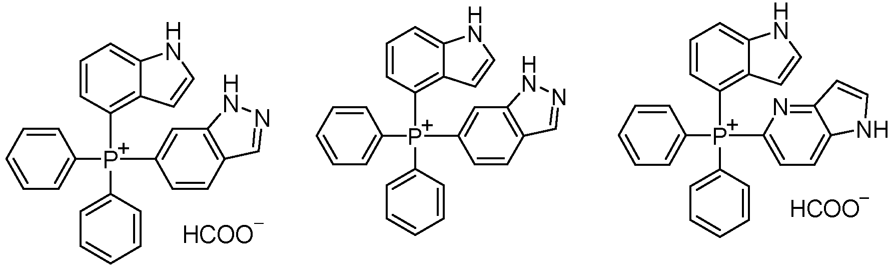

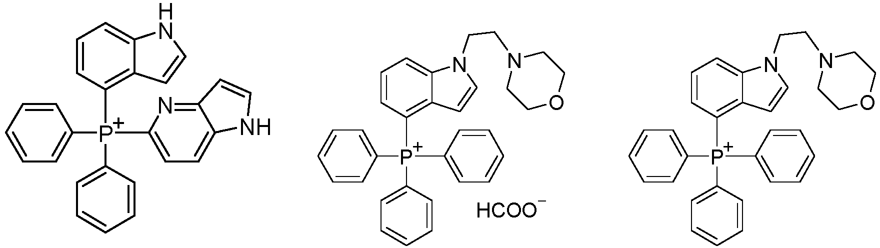

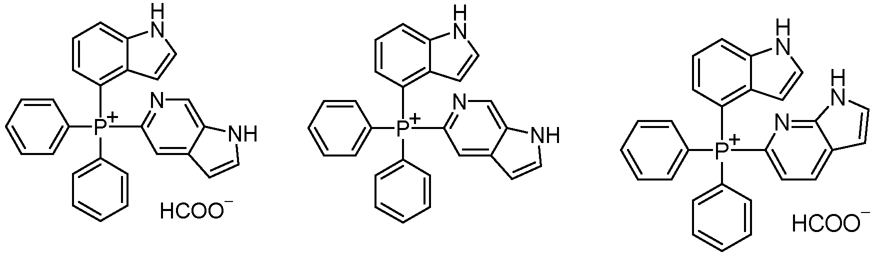

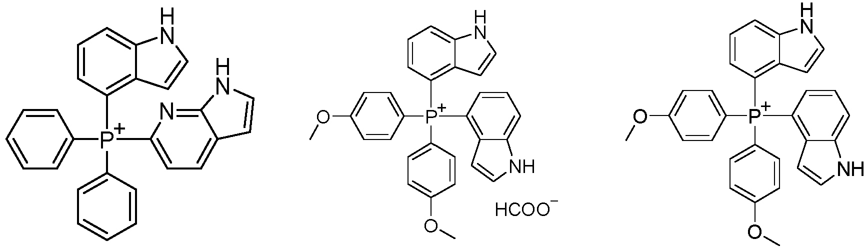

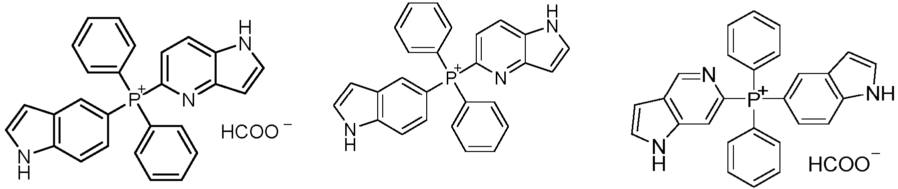

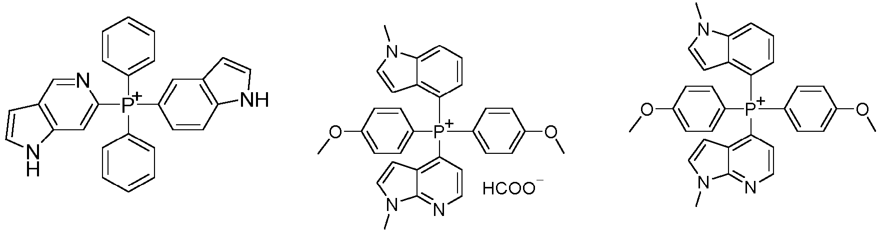

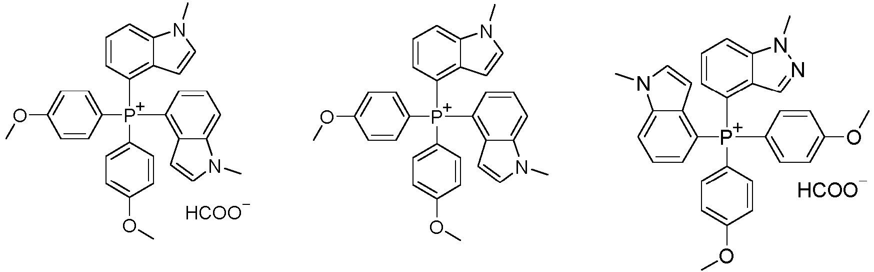

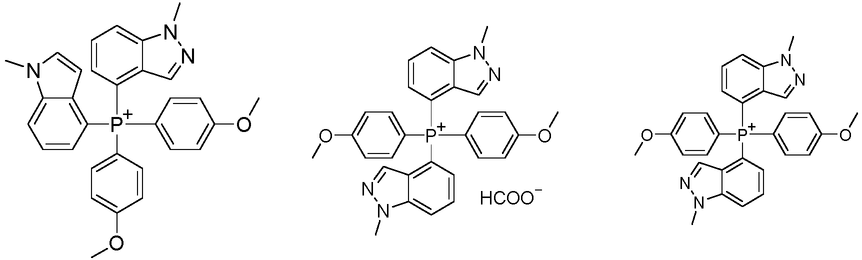

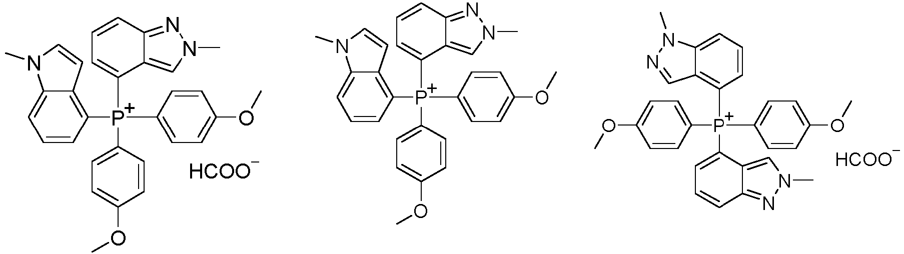

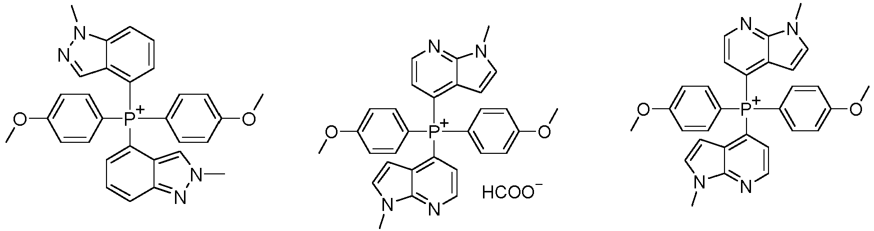

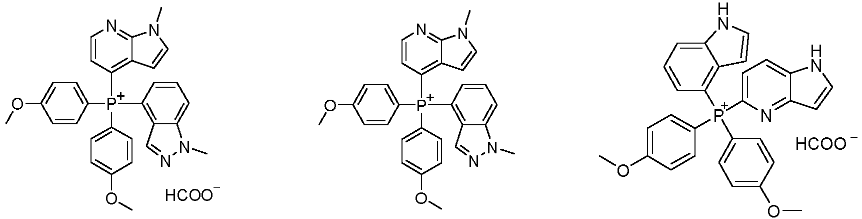

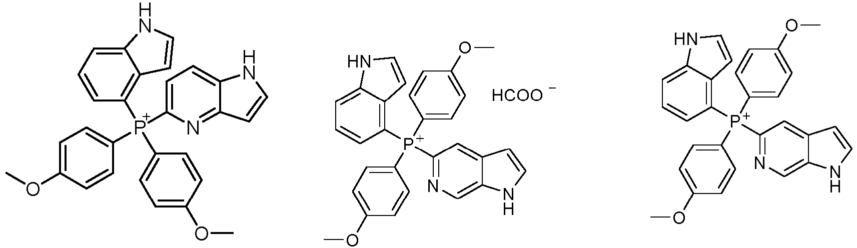

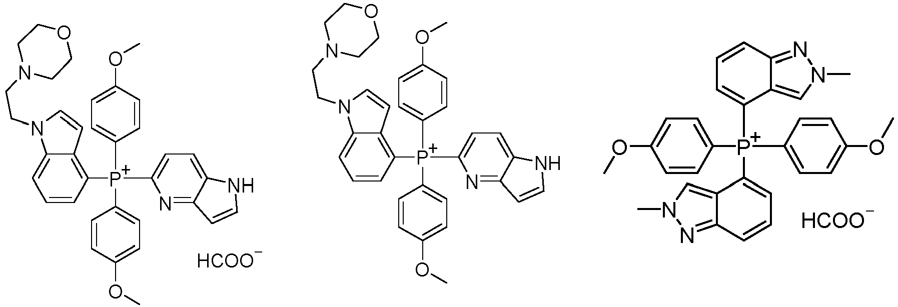

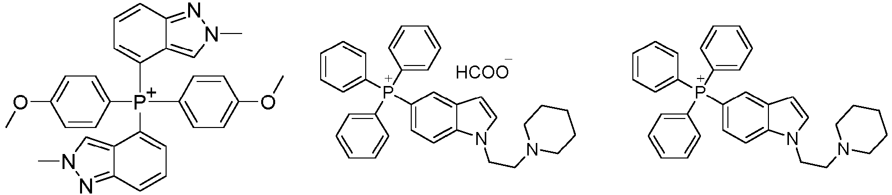

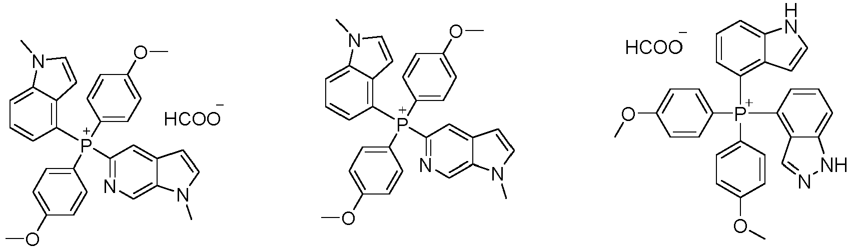

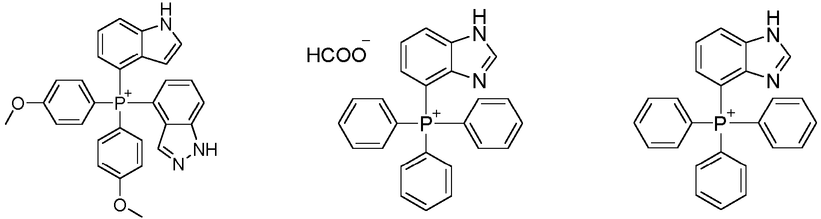

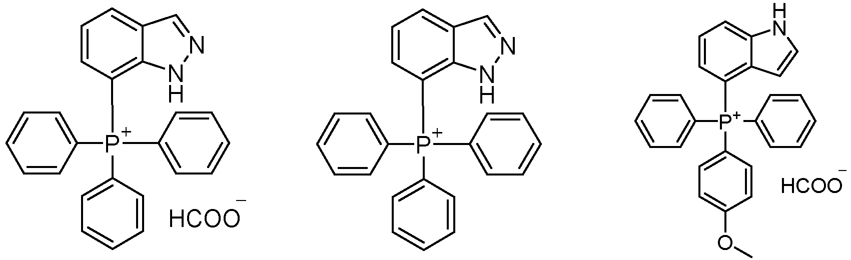

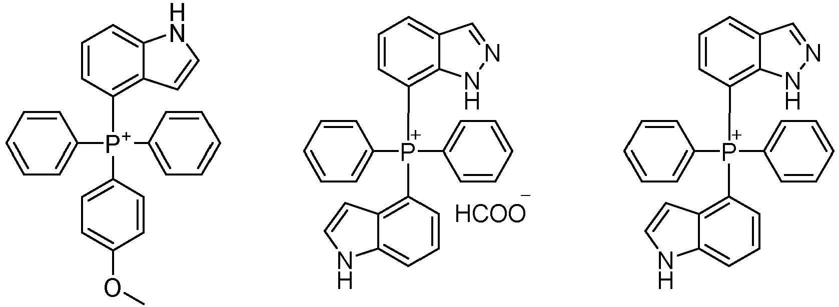

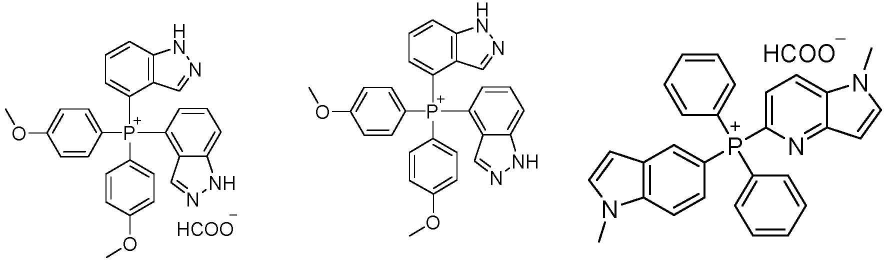

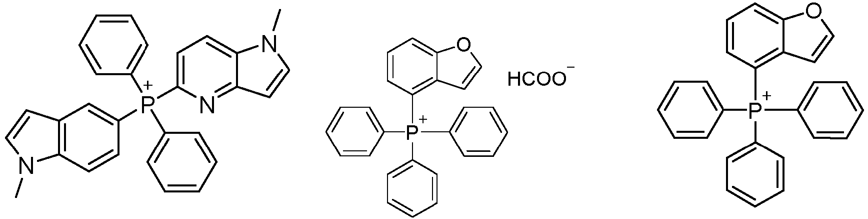

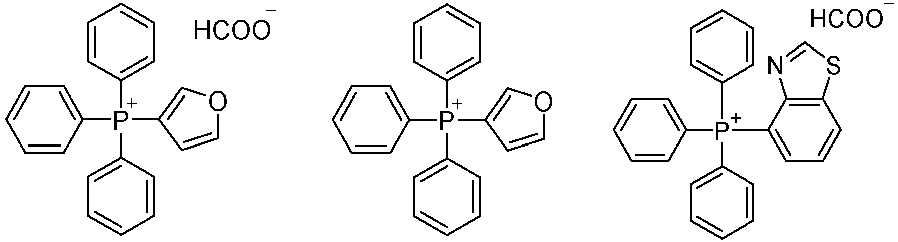

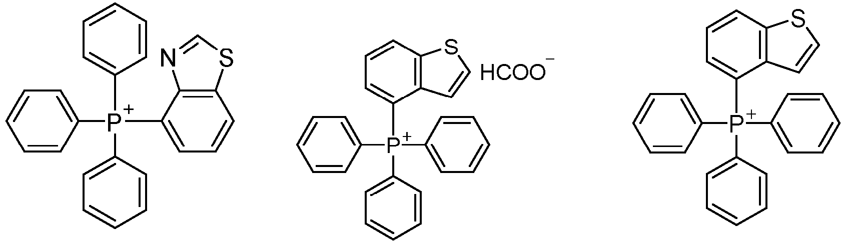

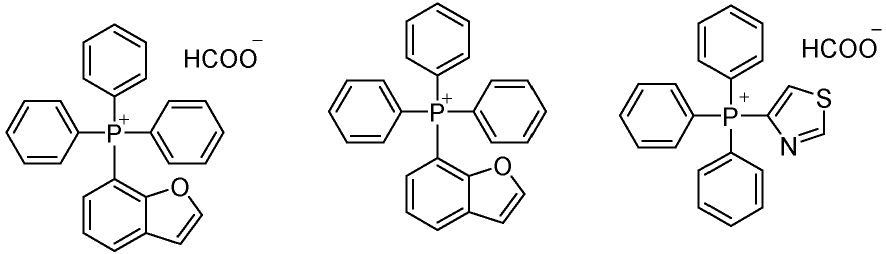

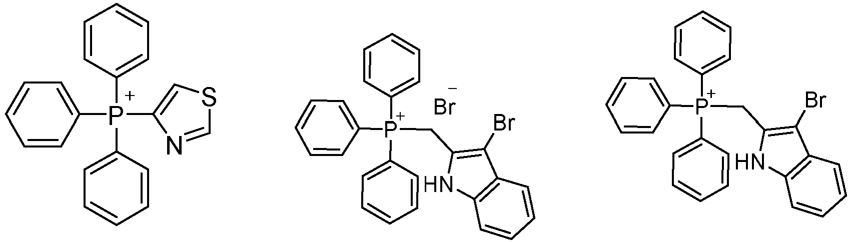

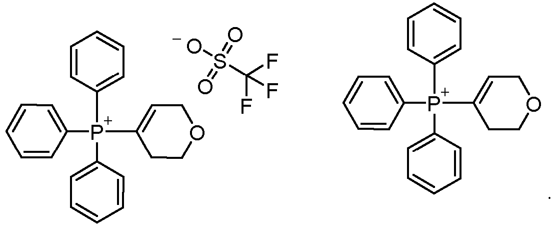

In the second aspect of the present invention, it provides a composition, the composition comprises (a) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the content of the (a) the compound of formula I, or an optical isomer thereof, or a racemate thereof, or a solvate thereof, or a pharmaceutically acceptable salt thereof, or a deuterated compound thereof according to the first aspect of the present invention is 0.001-99.9 wt%, based on the weight of the composition.

In another preferred embodiment, the composition is pharmaceutical composition.

In another preferred embodiment, the composition further comprises a pharmaceutically acceptable carrier.

In another preferred embodiment, the dosage form of the composition is a solid preparation, liquid preparation or semi-solid preparation.

In another preferred embodiment, the dosage form of the composition is oral preparation, external preparation or injection preparation.

In another preferred embodiment, the dosage form of the composition is tablet, injection, infusion, paste, gel, solution, microsphere or film.

In the third aspect of the present invention, it provides a use of the compound of formula I, or an optical isomer thereof, or a racemate thereof, or a solvate thereof, or a pharmaceutically acceptable salt thereof, or a deuterated compound thereof according to the first aspect of the present invention in the preparation of a composition or a preparation for preventing and/or treating tumor.

In another preferred embodiment, the tumor is human-derived tumor.

In another preferred embodiment, the tumor is human tumor.

In another preferred embodiment, the tumor comprises tumor with low expression, no expression, low activity or no activity of mitochondria permeability transition pore.

In another preferred embodiment, the tumor comprises tumor with low expression, no expression, low activity or no activity of peptidyl-prolyl cis-trans isomerase F.

In another preferred embodiment, the protein identification number of peptidyl-prolyl cis-trans isomerase F is UniProtKB/Swiss Prot: P30405, and the gene identification number of peptidyl-prolyl cis-trans isomerase F is NCBI Entrez Gene: 10105.

In another preferred embodiment, the tumor with low expression or low activity of mitochondria permeability transition pore means the expression level or activity level of mitochondria permeability transition pore in tumor cell is lower than the expression level or activity level of mitochondria permeability transition pore in the same type of cell or a normal cell.

In another preferred embodiment, the low expression or low activity of mitochondria permeability transition pore means the ratio (H1/H0) of the expression level or activity level H1of mitochondria permeability transition pore in a cell (e.g. tumor cell) to the expression level or activity level H0 of mitochondria permeability transition pore in the same type of cell or a normal cell is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell is tumor cell.

In another preferred embodiment, the same type of cell comprises the same type of tumor cell.

In another preferred embodiment, the same type of cell comprises the cell (e.g. the same type of tumor cell) with normal expression, high expression, normal activity or high activity of mitochondria permeability transition pore.

In another preferred embodiment, the same type of cell comprises the same type of cell with normal expression, high expression, normal activity or high activity of mitochondria permeability transition pore

In another preferred embodiment, the normal cell comprises normal tissue cell (e.g. tumor origin cell, tumor-adjacent cell or para-tumor tissue cell) with normal expression or normal activity of mitochondria permeability transition pore.

In another preferred embodiment, H0 refers to the expression level or activity level H0 of mitochondria permeability transition pore in the cell with normal expression, high expression, normal activity or high activity of mitochondria permeability transition pore.

In another preferred embodiment, the cell with normal expression, high expression, normal activity or high activity of mitochondria permeability transition pore comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with low expression or low activity of peptidyl-prolyl cis-trans isomerase F means the expression level or activity level of peptidyl-prolyl cis-trans isomerase F in tumor cell is lower than the expression level or activity level of peptidyl-prolyl cis-trans isomerase F in the same type of cell or a normal cell.

In another preferred embodiment, the low expression or low activity of peptidyl-prolyl cis-trans isomerase F means the ratio (C1/C0) of the expression level or activity level C1of peptidyl-prolyl cis-trans isomerase F in a cell (e.g. tumor cell) to the expression level or activity level C0 of peptidyl-prolyl cis-trans isomerase F in the same type of cell or a normal cell is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell is the same type of cell.

In another preferred embodiment, the same type of cell comprises the same type of tumor cell.

In another preferred embodiment, the same type of cell comprises the cell (e.g. the same type of tumor cell) with normal expression, high expression, normal activity or high activity of peptidyl-prolyl cis-trans isomerase F.

In another preferred embodiment, the same type of cell comprises the same type of cell with normal expression, high expression, normal activity or high activity of peptidyl-prolyl cis-trans isomerase F.

In another preferred embodiment, the normal cell comprises normal tissue cell (e.g. tumor origin cell, tumor-adjacent cell or para-tumor tissue cell) with normal expression or normal activity of peptidyl-prolyl cis-trans isomerase F.

In another preferred embodiment, C0 refers to the expression level or activity level of peptidyl-prolyl cis-trans isomerase F in the cell with normal expression, high expression, normal activity or high activity of peptidyl-prolyl cis-trans isomerase F.

In another preferred embodiment, the cell with normal expression, high expression, normal activity or high activity of peptidyl-prolyl cis-trans isomerase F comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the mitochondria permeability transition pore inhibitor is administered to achieve low expression, no expression, low activity or no activity of mitochondria permeability transition pore in tumor.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor is administered to achieve low expression, no expression, low activity or no activity of peptidyl-prolyl cis-trans isomerase F.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the inhibitor comprises specific inhibitor.

In another preferred embodiment, the inhibitor is selected from the group consisting of small molecule compound inhibitor, protein inhibitor, gene inhibitor, and combinations thereof.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the tumor comprises tumor with low or no expression of NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1.

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRFl.

In another preferred embodiment, the tumor comprises tumor with high methylation level of nucleotide site of NNMT gene.

In another preferred embodiment, the methylation of nucleotide site of NNMT gene comprises the methylation of cytosine nucleotide site of NNMT gene.

In another preferred embodiment, the methylation of nucleotide site of NNMT gene comprises the methylation of cytosine of NNMT gene.

In another preferred embodiment, the methylation of nucleotide site of NNMT gene comprises the methylation of the 5'carbon atom on the cytosine of the NNMT gene.

In another preferred embodiment, the tumor comprises tumor with high methylation level of DNA CpG site of NNMT gene.

In another preferred embodiment, the methylation of DNA CpG site of NNMT gene comprises the methylation of cytosine nucleotide site of DNA CpG site of NNMT gene.

In another preferred embodiment, the methylation of DNA CpG site of NNMT gene comprises the methylation of cytosine of DNA CpG site of NNMT gene.

In another preferred embodiment, the methylation of DNA CpG site of NNMT gene comprises the methylation of the 5'carbon atom on the cytosine of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell.

In another preferred embodiment, the low or no expression of NNMT gene means the ratio (E1/E0) of the expression E1 of NNMT gene in a cell (e.g. tumor cell) to the expression E0 of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the same type of cell comprises the same type of tumor cell.

In another preferred embodiment, the same type of cell comprises the cell (e.g. the same type of tumor cell) with normal or high expression of NNMT gene.

In another preferred embodiment, the same type of cell comprises the same type of cell with normal or high expression of NNMT gene.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of NNMT gene.

In another preferred embodiment, E0 refers to the expression level of NNMT gene in the cell with normal or high expression of NNMT gene.

In another preferred embodiment, the cell with normal or high expression of NNMT gene comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same type of cell or a normal cell.

In another preferred embodiment, the tumor with high expression of DNA methylase means the ratio (A1/A0) of the expression level A1 of DNA methylase in the tumor cell to the expression level A0 of DNA methylase in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g. the same type of tumor cell) with normal or low expression of DNA methylase.

In another preferred embodiment, the same type of cell comprises the same type of cell with normal or low expression of DNA methylase.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of DNA methylase.

In another preferred embodiment, A0 refers to the expression level of DNA methylase in the cell with normal or low expression of DNA methylase.

In another preferred embodiment, the cell with normal or low expression of DNA methylase comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor type of cell is higher than that in the same type of cell or a normal cell.

In another preferred embodiment, the tumor with high expression of DNMT1 means the ratio (B1/B0) of the expression level B1 of DNMT1 in the tumor cell to the expression level B0 of DNMT1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g. the same type of tumor cell ) with normal or low expression of DNMT1.

In another preferred embodiment, the same type of cell comprises the same type of cell with normal or low expression of DNMT1.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of DNMT1.

In another preferred embodiment, B0 refers to the expression level of DNMT1 in the cell with normal or low expression of DNMT1.

In another preferred embodiment, the cell with normal or low expression of DNMT1 comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNMT3a means that DNMT3a protein can be detected in 20 µg of protein extracted from tumor using DNMT3a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same type of cell or a normal cell.

In another preferred embodiment, the tumor with high expression of DNMT3a means the ratio (P1/P0) of the expression level P1 of DNMT3a in the tumor cell to the expression level P0 of DNMT3a in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g. the same type of tumor cell ) with normal or low expression of DNMT3a.

In another preferred embodiment, the same type of cell comprises the same type of cell with normal or low expression of DNMT3a.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of DNMT3a.

In another preferred embodiment, P0 refers to the expression level of DNMT3a in the cell with normal or low expression of DNMT3a.

In another preferred embodiment, the cell with normal or low expression of DNMT3a comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor type of cell is higher than that in the same type of cell or a normal cell.

In another preferred embodiment, the tumor with high expression of DNMT3b means the ratio (D1/D0) of the expression level D1 of DNMT3b in the tumor cell to the expression level D0 of DNMT3b in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g. the same type of tumor cell) with normal or low expression of DNMT3b.

In another preferred embodiment, the same type of cell comprises the same type of cell with normal or low expression of DNMT3b.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of DNMT3b

In another preferred embodiment, D0 refers to the expression level of DNMT3b in the cell with normal or low expression of DNMT3b.

In another preferred embodiment, the cell with normal or low expression of DNMT3b comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF 1 means the expression level of UHRF 1 in tumor type of cell is higher than that in the same type of cell or a normal cell.

In another preferred embodiment, the tumor with high expression of UHRF 1 means the ratio (F1/F0) of the expression level F1 of UHRF1 in the tumor cell to the expression level F0 of UHRF1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g. the same type of tumor cell) with normal or low expression of UHRF1.

In another preferred embodiment, the same type of cell comprises the same type of cell with normal or low expression of UHRF1.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal expression of UHRF1

In another preferred embodiment, F0 refers to the expression level of UHRF1 in the cell with normal or low expression of UHRF1.

In another preferred embodiment, the cell with normal or low expression of UHRF1 comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the high methylation level of nucleotide site of NNMT gene means the methylation level of nucleotide site of NNMT gene in a cell (e.g. tumor cell) is higher than that in the same type of cell or a normal cell.

In another preferred embodiment, the high methylation level of nucleotide site of NNMT gene means the ratio (L1/L0) of the methylation level L1 of nucleotide site of NNMT gene in a cell (e.g. tumor cell) to the methylation level L0 of nucleotide site of NNMT gene in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of nucleotide site of NNMT gene means the methylation level of nucleotide site of NNMT gene in a cell (e.g. tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type cell comprises the same type of tumor cell.

In another preferred embodiment, the same type of cell comprises the cell (e.g. the same type of tumor cell) with normal or low methylation level of nucleotide site of NNMT gene.

In another preferred embodiment, the same type of cell comprises the same type of cell with normal or low methylation level of nucleotide site of NNMT gene.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal methylation level of nucleotide site of NNMT gene.

In another preferred embodiment, L0 refers to the methylation level of nucleotide site of NNMT gene in the cell with normal or low methylation level of nucleotide site of NNMT gene.

In another preferred embodiment, the cell with normal or low methylation level of nucleotide site of NNMT gene comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the high methylation level of nucleotide site of NNMT gene means the methylation level (M%) of nucleotide site of NNMT gene in a cell (e.g. tumor cell) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell (e.g. tumor cell) is higher than that in the same type of cell or a normal cell.

In another preferred embodiment, the high methylation level of DNA CpG site of NNMT gene means the ratio (G1/G0) of the methylation level G1 of DNA CpG site of NNMT gene in a cell (e.g. tumor cell) to the methylation level G0 of DNA CpG site of NNMT gene in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of DNA CpG site of NNMT gene means the methylation level of DNA CpG site of NNMT gene in a cell (e.g. tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type of cell comprises the same type of tumor cell.

In another preferred embodiment, the same type of cell comprises the cell (e.g. the same type of tumor cell) with normal or low methylation level of DNA CpG site of NNMT gene.

In another preferred embodiment, the same type of cell comprises the same type of cell with normal or low methylation level of DNA CpG site of NNMT gene.

In another preferred embodiment, the normal cell comprises normal tissue cell (e.g. tumor origin cell, tumor-adjacent cell or para-tumor tissue cell).

In another preferred embodiment, the normal cell comprises normal tissue cell (e.g. tumor origin cell, tumor-adjacent cell or para-tumor tissue cell) with normal methylation level of DNA CpG site of NNMT gene.

In another preferred embodiment, G0 refers to the methylation level of DNA CpG site of NNMT gene in the cell with normal or low methylation level of DNA CpG site of NNMT gene

In another preferred embodiment, the cell with normal or low methylation level of DNA CpG site of NNMT gene comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the high methylation level of DNA CpG site of NNMT gene means the methylation level (M%) of DNA CpG site of NNMT gene in a cell (e.g. tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of DNA CpG site of NNMT gene refers to the ratio of the number of methylated DNA CpG nucleotides to the number of all CpG nucleotides in the NNMT gene.

In another preferred embodiment, the methylation level of DNA CpG site of NNMT gene refers to the ratio of the number of methylated DNA CpG sites to the number of all DNA CpG sites in the NNMT gene.

In another preferred embodiment, the methylation level of DNA CpG site of NNMT gene refers to the ratio of the number of methylated DNA CpG nucleotides to the number of all DNA CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of DNA CpG site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the NNMT gene inhibitor is administered to achieve low or no expression of the NNMT gene in tumor.

In another preferred embodiment, the DNA methylase promoter is administered to achieve high expression of DNA methylase in tumor.

In another preferred embodiment, the DNMT1 promoter is administered to achieve high expression of DNMT1 in tumor.

In another preferred embodiment, the DNMT3a promoter is administered to achieve high expression of DNMT3a in tumor.

In another preferred embodiment, the DNMT3b promoter is administered to achieve high expression of DNMT3b in tumor.

In another preferred embodiment, the UHRFl promoter is administered to achieve high expression of UHRFl in tumor.

In another preferred embodiment, the methylation promoter of nucleotide site of NNMT gene is administered to achieve high methylation level of nucleotide site of NNMT gene in tumor.

In another preferred embodiment, the methylation promoter of DNA CpG site of NNMT gene is administered to achieve high methylation level of DNA CpG site of NNMT gene in tumor.

In another preferred embodiment, the inhibitor comprises specific inhibitor.

In another preferred embodiment, the promoter comprises specific promoter.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNMT1 promoter comprises promoter that can achieve high expression of DNMT1 in tumor.

In another preferred embodiment, the DNMT3a promoter comprises promoter that can achieve high expression of DNMT3a in tumor.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation promoter of DNA CpG site of NNMT gene in tumor.

In another preferred embodiment, the tumor is selected from the group consisting of brain tumor, lung cancer, and combinations thereof.

In another preferred embodiment, the tumor comprises brain tumor and/or lung cancer.

In another preferred embodiment, the tumor comprises a tumor in cranial fossa.

In another preferred embodiment, the tumor comprises malignant tumor.

In another preferred embodiment, the brain tumor comprises glioma.

In another preferred embodiment, the brain tumor comprises a glioma in cranial fossa.

In another preferred embodiment, the brain tumor comprises medulloblastoma.

In another preferred embodiment, the brain tumor cell comprises Daoy cell.

In another preferred embodiment, the lung cancer is selected from the group consisting of non-small cell lung cancer, small cell lung cancer, and combinations thereof.

In another preferred embodiment, the lung cancer cell comprises NCI-H82 cell.

In another preferred embodiment, the level comprises protein level and/or mRNA level.

In another preferred embodiment, the expression comprises protein expression and/or mRNA expression.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the fourth aspect of the present invention, it provides a marker for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

In another preferred embodiment, the marker comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene in tumor cell.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" comprises "the patient tumor is sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" comprises "the patient tumor is not sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, the high expression or high activity of mitochondria permeability transition pore means the ratio (H1/H0) of the expression level or activity level H1of mitochondria permeability transition pore in a cell (e.g. tumor cell) to the expression level or activity level H0 of mitochondria permeability transition pore in the same type of cell or a normal cell is > 1. 0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the high expression or high activity of peptidyl-prolyl cis-trans isomerase F means the ratio (C1/C0) of the expression level or activity level C1of peptidyl-prolyl cis-trans isomerase F in a cell (e.g. tumor cell) to the expression level or activity level C0 of mitochondria permeability transition pore in the same type of cell or a normal cell is >1.0, preferably ≥1.2, more preferably ≥1.5, more preferably ≥2, more preferably ≥3, more preferably ≥5,more preferably ≥8, more preferably ≥10, more preferably ≥15, more preferably ≥20, more preferably ≥30, more preferably ≥50, such as 2-50.

In another preferred embodiment, the high expression of NNMT gene means the ratio (E1/E0) of the expression E1 of NNMT gene in a cell (e.g. tumor cell) to the expression E0 of NNMT gene in the same type of cell or a normal cell is >1.0, preferably ≥1.2, more preferably ≥1.5, more preferably ≥2, more preferably ≥3, more preferably ≥5,more preferably ≥8, more preferably ≥10, more preferably ≥15, more preferably ≥20, more preferably ≥30, more preferably ≥50, such as 2-50.

In another preferred embodiment, the tumor with low expression of DNA methylase means the ratio (A1/A0) of the expression level A1 of DNA methylase in the tumor cell to the expression level A0 of DNA methylase in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell (e.g. tumor cell) to the methylation level L0 of nucleotide site of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of DNA CpG site of NNMT gene means the ratio (G1/G0) of the methylation level G1 of DNA CpG site of NNMT gene in a cell (e.g. tumor cell) to the methylation level G0 of DNA CpG site of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In the fifth aspect of the present invention, it provides a detection kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the level comprises protein level and/or mRNA level.

In another preferred embodiment, the expression comprises mRNA expression and/or protein expression.

In the sixth aspect of the present invention, it provides a use of the detection kit according to the fifth aspect of the present invention in the preparation of concomitant diagnose kit for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" is as described in the fourth aspect of the invention.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" is as described in the fourth aspect of the invention.

In the seventh aspect of the present invention, it provides a medicine kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the detection sample comprises tumor.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In the eighth aspect of the present invention, it provides a method for preventing and/or treating tumor, which comprises administering the compound of formula I, or an optical isomer thereof, or a racemate thereof, or a solvate thereof, or a pharmaceutically acceptable salt thereof, or a deuterated compound thereof according to the first aspect of the present invention to a subject in need, thereby preventing and/or treating tumor.

In another preferred embodiment, the tumor is as described in the third aspect of the invention.

In another preferred embodiment, the subject is human and non-human mammals (rodent, rabbit, monkey, livestock, dog, cat, etc.).

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF 1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the mitochondria permeability transition pore inhibitor, peptidyl-prolyl cis-trans isomerase F inhibitor, NNMT gene inhibitor, DNA methylase promoter, UHRF 1 promoter, methylation promoter of nucleotide site of NNMT gene, and/or methylation promoter of DNA CpG site of NNMT gene is as described in the third aspect of the invention.

In the ninth aspect of the present invention, it provides a device or system, the device or system comprises:
(i) a detection module, the detection module is used to detect the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene;
(ii) an output module, the output module comprises the information as follows:
   the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, the detection sample comprises tumor.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In the tenth aspect of the present invention, it provides a use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the NNMT gene inhibitor comprises inhibitor that can achieve low or no expression of the NNMT gene in tumor.

In another preferred embodiment, the DNA methylase is selected from the group consisting of DNMT1, DNMT3a, DNMT3b, and combinations thereof.

In another preferred embodiment, the DNA methylase promoter comprises promoter that can achieve high expression of DNA methylase in tumor.

In another preferred embodiment, the DNA methylase promoter comprises DNMT1 promoter.

In another preferred embodiment, the DNMT1 promoter comprises promoter that can achieve high expression of DNMT1 in tumor.

In another preferred embodiment, the DNA methylase promoter comprises DNMT3a promoter.

In another preferred embodiment, the DNMT3a promoter comprises promoter that can achieve high expression of DNMT3a in tumor.

In another preferred embodiment, the DNA methylase promoter comprises DNMT3b promoter.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation level of nucleotide site of NNMT gene in tumor.

In another preferred embodiment, the inhibitor comprises specific inhibitor.

In another preferred embodiment, the promoter comprises specific promoter.

In another preferred embodiment, the anti-tumor drug comprises the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the tumor is as described in the third aspect of the invention.

In another preferred embodiment, the inhibitor is selected from the group consisting of small molecule compound inhibitor, protein inhibitor, gene inhibitor, and combinations thereof.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the eleventh aspect of the present invention, it provides an active ingredient combination, the active ingredient combination comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the molar ratio of the first active ingredient to the second active ingredient is 0.01-600:1, preferably 0.05-500: 1, more preferably 0.1-400:1, more preferably 0.2-200: 1, more preferably 0.5-100: 1, more preferably 0.5-80:1, most preferably 1-50: 1.

In another preferred embodiment, at least one active ingredient is independent in the active ingredient combination.

In another preferred embodiment, the first active ingredient and the second active ingredient are independent of each other in the active ingredient combination.

In the twelfth aspect of the present invention, it provides a composition, the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the composition is a pharmaceutical composition.

In another preferred embodiment, the composition further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In the thirteenth aspect of the present invention, it provides a medicine kit, the medicine kit comprises:
(A) a first preparation comprising a first active ingredient, the first active ingredient comprises anti-tumor drug; and
(B) a second preparation comprising a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anti-tumor drug.

In another preferred embodiment, the method for using together means the second preparation comprising a second active ingredient is administered firstly, then the first preparation comprising a first active ingredient is administered.

In the fourteenth aspect of the present invention, it provides a method for inhibiting tumor cell, which comprises contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method is vitro method.

In another preferred embodiment, the method for inhibiting tumor cell comprises non-therapeutic and non-diagnostic method for inhibiting tumor cell.

In another preferred embodiment, the contact is performed in vitro culture.

In another preferred embodiment, the tumor is as described in the third aspect of the invention.

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF 1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the tenth aspect of the invention.

In the fifteenth aspect of the present invention, it provides a use of medicine kit according to the seventh aspect of the present invention in the preparation of medicine box for preventing and/or treating tumor.

In another preferred embodiment, the medicine box further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In the present invention, each of the technical features specifically described above and below can be combined with each other, thereby constituting new or preferred technical solutions which need not be redundantly described one-by-one.

### DESCRIPTION OF THE DRAWINGS

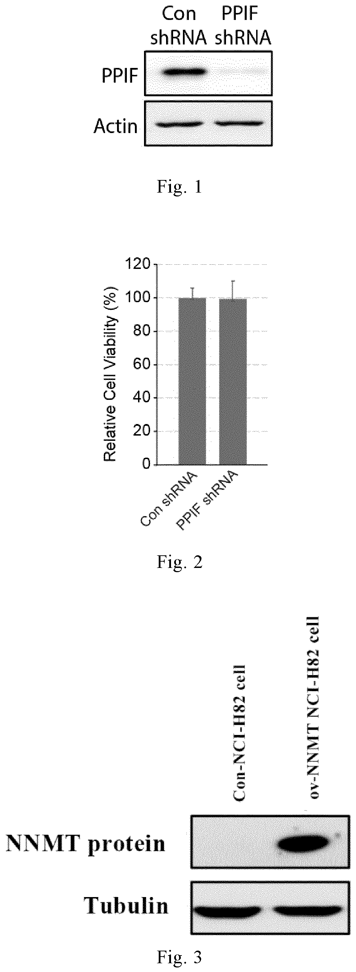

Fig.1 shows the expression content of PPIF protein using Western Blot test, wherein, Con shRNA refers to the expression content of PPIF protein in Daoy cell transfected with empty virus vector without carrying shRNA that can specificly induce the degradation of PPIF mRNA; PPIF shRNA refers to the expression content of PPIF protein in Daoy cell transfected with virus vector carrying shRNA that can specificly induce the degradation of PPIF mRNA.
Fig.2 shows the relative cell viability of Daoy cell having inactive mPTP and Daoy cell having active mPTP, wherein, Con shRNA refers to the relative viability of Daoy cell transfected with empty virus vector without carrying shRNA that can specificly induce the degradation of PPIF mRNA; PPIF shRNA refers to the relative viability of Daoy cell transfected with virus vector carrying shRNA that can specificly induce the degradation of PPIF mRNA.
Fig.3 shows the expression content of NNMT protein in Con-NCI-H82 cell and ov-NNMT NCI-H82 cell using Western Blot test, wherein, Con-NCI-H82 refers to the expression content of NNMT protein in NCI-H82 cell transfected with empty virus vector without carrying NNMT gene, which is used as control; ov-NNMT NCI-H82 refers to the expression content of NNMT protein in NCI-H82 cell transfected with virus vector carrying NNMT gene.

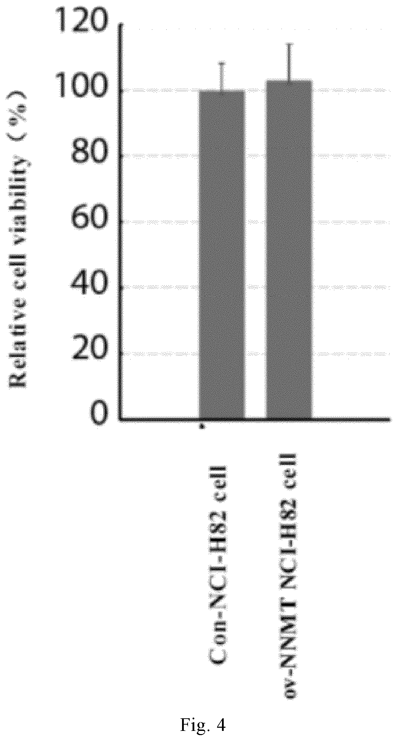

Fig.4 shows the relative cell viability of Con-NCI-H82 cell and ov-NNMT NCI-H82 cell, wherein, Con-NCI-H82 refers to the cell viability of NCI-H82 cell transfected with empty virus vector without carrying NNMT gene, which is used as control; ov-NNMT NCI-H82 refers to the cell viability of NCI-H82 cell transfected with virus vector carrying NNMT gene.

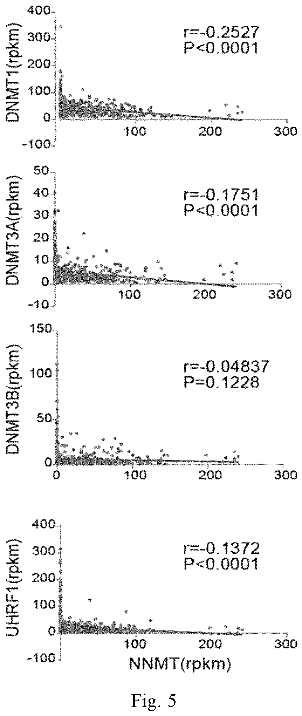

Fig.5 shows the correlation between the expression of NNMT and the expression of DNMT1, UHRF1, DNMT3a and DNMT3b in tumor cells.

### EMBODIMENTS FOR CARRYING OUT THE INVENTION

Based on an extensive and intensive research, the inventors have unexpectedly found that the compound of the present invention has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. On this basis, the inventors has completed the present invention.

### Terms

Unless otherwise defined, the meanings of all technical and scientific terms used in the invention are the same as those generally understood by the skilled in the art.

As used herein, the term "comprise", " comprising", and "containing" are used interchangeably, which not only comprise open definitions, but also semi-closed and closed definitions. In other words, the term comprises "consisting of" and "essentially consisting of".

As used herein, the term "cancer" and "tumor" are used interchangeably.

As used herein, the term "a cell" refers to a cell (e.g. single tumor cell) or a group of cells containing multiple similar cells ((e.g. a tumor tissue).

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, the term "high methylation level of DNA CpG site", "high level of DNA CpG site methylation" and "high methylation of DNA CpG site" are used interchangeably.

As used herein, the term "low methylation level of DNA CpG site", "low level of DNA CpG site methylation" and "low methylation of DNA CpG site" are used interchangeably.

As used herein, the term "methylation of CpG site", "methylation of CpG nucleotide" and "CpG methylation" are used interchangeably.

As used herein, the term "IC50" and "IC₅₀" are used interchangeably, which refers to 50% inhibiting concentration, ie, the concentration of the inhibitor when 50% inhibitory effect is achieved.

As used herein, the term "methylation of CpG site", "methylation of CpG nucleotide" and "CpG methylation" are used interchangeably.

As used herein, the term "P/S" refers to adding penicillin and streptomycin into the culture medium.

As used herein, "the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene" refers to one or more of the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene and high methylation level of DNA CpG site of NNMT gene.

As used herein, mitochondria permeability transition pore is abbreviated as mPTP.

As used herein, peptidyl-prolyl cis-trans isomerase F is abbreviated as PPIF.

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_000001405.25 (GRCh37. p13).

As used herein, the term "human chromosome 11" refers to human chromosome 11 according to human genome version GCF_000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide of site 114165695 on human chromosome 11, and the like.

As used herein, S-adenosyl methionine is abbreviated as SAM.

As used herein, the gene expression comprises the protein expression of the gene and/or the mRNA expression of the gene.

As used herein, the term "DNMT3a" and "DNMT3A" are used interchangeably, which refers to DNA methyltransferase 3a.

As used herein, the term "DNMT3b" and "DNMT3B" are used interchangeably, which refers to DNA methyltransferase 3b.

As used herein, the term "DNMT1" refers to DNA methyltransferase 1.

As used herein, the term "UHRF1" refers to ubiquitin-like with PHD and ring finger domain 1.

As used herein, the term "deuterated" refers to one or more hydrogen atoms in a compound or group are substituted by deuterium. The deuterated can be mono-substituted, di-substituted, multi-substituted or fully-substituted.

As used herein, the term "solvate" refers to a complex formed by the coordination of a compound with a solvent molecule in a specific ratio.

As used herein, the term "MS-ESI" refers to Electro Spray Ionization-Mass Spectroscopy.

As used herein, the term "¹H NMR" refers to H Nuclear Magnetic Resonance Spectra.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc. As used herein, the term "deuterated" refers to one or more hydrogen atoms in a compound or group are substituted by deuterium. The deuterated can be mono-substituted, di-substituted, multi-substituted or fully-substituted.

As used herein, "R₁", "R1" and "R¹" have the same meaning and can be used interchangeably, the other similar definitions have the same meaning. As used herein, " 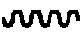 " denotes the linking site of the group.

As used herein, the term "alkyl" refers to a saturated hydrocarbon group with linear chain (ie, unbranched chain) or branched chain, or a combination of linear and branched chains, the alkyl only have carbon and hydrogen atoms. When the number of carbon atoms is limited in front of the alkyl (e.g. C1-C6 alkyl), it refers to the number of carbon atoms (e.g. 1-6) in the alkyl, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "alkylidene" refers to a group formed by removing one hydrogen on the alkyl, the alkyl is as defined above. When the number of carbon atoms is limited in front of the alkylidene (e.g. C1-C6 alkylidene), it refers to the number of carbon atoms (e.g. 1-6) in the alkylidene, for example, C1-C4 alkylidene refers to an alkylidene having 1-4 carbon atoms. Representative examples comprise but are not limited to methylidene, ethylidene, propylidene, isopropylidene, butylidene, isobutylidene, sec-butylidene, tert-butylidene, or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the haloalkyl (e.g. C1-C8 haloalkyl), it refers to the number of carbon atoms (e.g. 1-8) in the haloalkyl, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkyl" refers to a cyclic hydrocarbon group having a saturated or partially saturated monocyclic ring, bicyclic ring or polycyclic ring (fused ring, bridged ring or spiro ring). When the number of carbon atoms is limited in front of the cycloalkyl (e.g. C3-C12 cycloalkyl), it refers to the number of ring carbon atoms (e.g. 3-12) in the cycloalkyl. For example, C3-C8 cycloalkyl refers to a saturated or partially saturated monocycloalkyl or dicycloalkyl having 3-8 ring carbon atoms, comprising cyclopropyl, cyclobutyl, cyclopentyl, cycloheptyl, or the like. The term "spirocycloalkyl" refers to a bicyclic or polycyclic group that shares a carbon atom (referred to spiro-atom) between single rings, the spirocycloalkyl can have one or more double bonds, but no ring has a fully conjugated π electron system. Fused cycloalkyl refers to all carbon bicyclic or polycyclic group in which each rings in the system shares an adjacent pair of carbon atoms with other rings, one or more rings can have one or more double bonds, but no ring has a fully conjugated π electron system. Bridged cycloalkyl refers to all carbon polycyclic group in which any two rings share two non-directly connected carbon atoms, the bridged cycloalkyl can have one or more double bonds, but no ring has a fully conjugated π electron system. The representative examples of cycloalkyl comprise but are not limited to

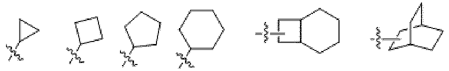

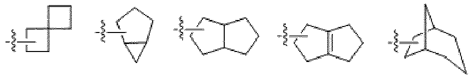

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the halocycloalkyl (e.g, C3-C8 halocycloalkyl), it refers to the number of ring carbon atoms (e.g. 3-8) in the halocycloalkyl, for example, C3-C8 halocycloalkyl refers to an halocycloalkyl having 3-8 ring carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-S- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "haloalkoxyl" refers to haloalkyl-O-, wherein the haloalkyl is as defined above. When the number of carbon atoms is limited in front of the haloalkoxyl, for example, C1-C6 haloalkoxyl refers to C1-C6 haloalkyl-O-, ie, the haloalkoxyl having 1-6 carbon atoms. Representative examples comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above. When the number of carbon atoms is limited in front of the haloalkylthio, for example, C1-C6 haloalkylthio refers to C1-C6 haloalkyl-S-, ie, the haloalkylthio having 1-6 carbon atoms. Representative examples of haloalkylthio comprise but are not limited to monofluoromethylthio, monofluoroethylthio, difluorobutylthio, or the like.

As used herein, the term "cycloalkoxyl" refers to R-O-, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 ring carbon atoms. Representative examples of cycloalkoxyl comprise but are not limited to cyclopropyloxyl, cyclobutoxyl, or the like.

As used herein, the term "cycloalkylthio" refers to R-S- group, wherein R is cycloalkyl, the cycloalkyl is as defined above. When the number of carbon atoms is limited in front of the cycloalkylthio, for example, C3-C8 cycloalkylthio means that the cycloalkyl in the cycloalkylthio has 3-8 ring carbon atoms. Representative examples of cycloalkylthio comprise but are not limited to cyclopropylthio, cyclobutythio, or the like.

As used herein, the term "halocycloalkoxyl" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkoxyl (e.g. C3-C8 halocycloalkoxyl), it refers to the number of ring carbon atoms (e.g. 3-8) in the halocycloalkoxyl, for example, C3-C8 halocycloalkoxyl refers to an halocycloalkoxyl having 3-8 ring carbon atoms. Representative examples comprises but are not limited to monofluorocyclopropyl-O-, monochlorocyclobutyl-O-, monofluorocyclopentyl-O-, difluorocycloheptyl-O -, or similar functional groups, or the like.

As used herein, the term "halocycloalkylthio" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkylthio (e.g. C3-C8 halocycloalkylthio), it refers to the number of ring carbon atoms (e.g. 3-8) in the halocycloalkylthio, for example, C3-C8 halocycloalkylthio refers to an halocycloalkylthio having 3-8 ring carbon atoms. Representative examples comprises but are not limited to monofluorocyclopropyl-S-, monochlorocyclobutyl-S-, monofluorocyclopentyl-S-, difluorocycloheptyl-S-, or similar functional groups, or the like.

As used herein, the term "heterocycloalkyl" refers to fully saturated or partially unsaturated cyclic (comprising but not limited to such as 3-7 membered monocyclic ring, 7-11 membered bicyclic ring, or 8-16 membered tricyclic ring) group, at least one heteroatom is present in a ring having at least one carbon atom, the linking site of the group is located on the ring having heteroatoms. When the number of members is limited in front of the heterocycloalkyl, it refers to the number of ring atoms in the heterocycloalkyl, for example, 3-16 membered heterocycloalkyl refers to a heterocycloalkyl having 3-16 ring atoms. Each heterocyclic ring having heteroatoms can have one or more (e.g. 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkyl comprise but are not limited to azetidinyl, oxetanyl, tetrahydrofuranyl, piperidinyl, piperazinyl. Polycyclic heterocycloalkyl comprises spiro, fused and bridged heterocyclyl, the spiro, fused and bridged heterocycloalkyl is optionally linked with other groups by single bond, or further linked with other cycloalkane rings and heterocycloalkane ring by any two or more atoms on the ring.

As used herein, the term "aryl" refers to an full carbon monocyclic ring or fused polycyclic ring (i.e., a ring that shares adjacent carbon atom pairs) group with a conjugated π electron system, which is aromatic cyclic hydrocarbon compound group. When the number of carbon atoms is limited in front of the aryl, it refers to the number of ring carbon atoms in the aryl, for example, C6-C12 aryl means that the aryl has 6-12 ring carbon atoms, such as phenyl and naphthyl.

As used herein, the term "heteroaryl" refers to aromatic heterocyclic ring group having one to more (preferably 1, 2, 3 or 4) ring heteroatoms, at least one heteroatom is present in a ring with at least one carbon atom. The heteroaryl can be monocyclic ring, or polycyclic ring (bicyclic, tricyclic or polycyclic ring) fused together or covalently connected. Each of heterocyclic ring having heteroatom can have one or more (e.g. 1, 2, 3, 4) heteroatoms independently selected from the group consisting of oxygen, sulfur and nitrogen. When the number of members is limited in front of the heteroaryl, it refers to the number of ring atoms in the heteroaryl, for example, 5-12 membered heteroaryl refers to a heteroaryl having 5-12 ring atoms. Representative examples comprise but are not limited to pyrrolyl, pyrazolyl, imidazolyl, thiazolyl, furanyl, pyridyl, pyrimidinyl, etc.

As used herein, the term "carboxyl" refers to -COOH or -alkyl-COOH, the alkyl is as defined above. For example, "C2-C4 carboxyl" refers to -C1-C3 alkyl-COOH. Representative examples of carboxyl comprise but are not limited to -COOH, -CH₂COOH, or the like.

As used herein, the term "ester group" refers to R-C(O)-O- or -C(O)-O-R, wherein, R is alkyl, the alkyl is defined as above. For example, "C₂-C₄ ester group" refers to C₁-C₃ alkyl-C(O)-O- or - C(O)-O-C₁-C₃ alkyl. Representative examples of ester group comprise but are not limited to CH₃C(O)O-, C₂H₅C(O)O-, (CH₃)₂CHC(O)O-, -C(O)OCH₃, -C(O)OC₂H₅, or the like.

As used herein, the term "amide group" refers to R-C(O)-N- or -C(O)-N-R , wherein, R is alkyl, the alkyl is defined as above. For example, "C₂-C₄ amide group" refers to C₁-C₃ alkyl-C(O)-N- or - C(O)-N-C₁-C₃ alkyl. Representative examples of amide group comprise but are not limited to CH₃C(O)-N-, C₂H₅C(O)-N-, (CH₃)₂CHC(O)-N-, -C(O)-N-CH₃, -C(O)-N-C₂H₅, or the like.

As used herein, the term "-C(O)-" refers to

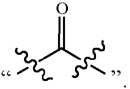

As used alone or as part of other substituent, the term "amino" refers to -NH₂.

As used alone or as part of other substituent, the term 'nitro' refers to -NO₂.

As used alone or as part of other substituent, the term "cyano" refers to -CN.

As used alone or as part of other substituent, the term "hydroxyl" refers to -OH.

As used alone or as part of other substituent, the term "sulfhydryl" refers to -SH.

In present invention, it should be understood that all substituents are unsubstituted, unless explicitly described herein as "substituted". The "substituted" means that one or more hydrogen atoms on specific group are substituted by substituent. The substituent can be the substituent as described above. Preferably, each "substituted" means that one or more (preferably 1, 2, 3, 4, 5, 6, 7, or 8) hydrogen atoms on the group are independently substituted by substituent selected from the group consisting of C1-C12 alkyl, C3-C8 cycloalkyl, C1-C12 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C8 ester group, C2-C4 amide group, C1-C12 alkyl-O-, C1-C12 alkyl-S-, C1-C12 haloalkoxyl, C1-C12 haloalkylthio, C6-C12 aryl, 5-12 membered heteroaryl, 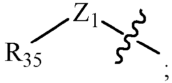 R₃₅ is hydrogen, hydroxyl, sulfhydryl, 3-12 membered heterocycloalkyl or halogen; Z₁ is C1-C8 alkylidene. Unless otherwise specified, each substituted group can have a substituent at any substituted position of the group, the substitution can be the same or different at each substituted position.

In the present invention, the term "prevention" refers to a method of preventing the occurrence of disease and/or its accompanying symptoms, or protecting a subject from getting disease. The term 'prevention' also comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100% inhibition, elimination and reversal. In some embodiments, compared to the level observed in the absence of the compound of present invention, the compound of present invention alleviates, inhibits and/or reverses related disease (e.g. tumor) and its accompanying symptoms, for example, by at least about 30%, at least about 50%, at least about 80%, at least about 90%, or 100%.

### Compound

As used herein, the terms "compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof, or a deuterated compound thereof.

In the present invention, the compound of formula I has the following structure:

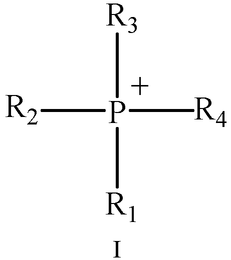

Specifically, the compound of formula I, or an optical isomer thereof, or a racemate thereof, or a solvate thereof, or a pharmaceutically acceptable salt thereof, or a deuterated compound thereof is as described above in the first aspect of the present invention.

Typically, the compound of formula I of the present invention is the compound prepared in the Examples of the present invention (comprising salt thereof or free form without salt radical) prepared in the Examples of the present invention..

The compound of formula I of the present invention can be prepared using the known organic synthesis method in the art.

The compound of formula I in present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

The compounds of the present invention is preferably prepared as described in the Examples of the present invention.

### Mitochondria permeability transition pore

In present invention, the mitochondria permeability transition pore is abbreviated as mPTP.

The compound of present invention has excellent precision treatment effect on tumor with low expression, no expression, low activity or no activity of mitochondria permeability transition pore, the tumor with low expression, no expression, low activity or no activity of mitochondria permeability transition pore is sensitive to the compound of present invention.

### Peptidyl-prolyl cis-trans isomerase F

In present invention, the peptidyl-prolyl cis-trans isomerase F is abbreviated as PPIF.

The compound of present invention has excellent precision treatment effect on tumor with low expression, no expression, low activity or no activity of peptidyl-prolyl cis-trans isomerase F, the tumor with low expression, no expression, low activity or no activity of peptidyl-prolyl cis-trans isomerase F is sensitive to the compound of present invention.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) and 499 bp after the transcription start site (non-bold section) in NNMT gene, the total length of promoter region of NNMT gene is 2500 bp, The nucleotide sequence of the promoter region of NNMT gene is as shown in SEQ ID NO: 1 as follows:

SEQ ID NO: 1:
TATCCAAGAGCTATCAGCACTCCCATGTTTATTGTAGCACTGTTCACAATAGC
CAAGATTTGGAAGTACTCTAAGTGTCCATTAGCAGATGAATGGATAAAGACAATG
TGGTAATACACATAATGGAGTACTATTCAGTCATAAAGAAGAATTAGATCCTGTC
ATTTGCAATAACATGGATGGAACTGGAGGTCATAATGTTGAGTGAAATAAACCAG
GCACAGAAAGACAAACTTTGCATGTTCTCACTTATTTATGGGAGCTAAAAACTAA
AATAACTGAACTCACAGAGATAGAGAGTAGAAGGATGGTTACGAGAGGATGGGA
AGGGTAGCGAGGTGGGTAGGGGGGATGTGGGGATCATTAATGGGTATAAAAAAT
AGTTAGAGGCCAGGCGCAGTGGCTCACGCCTGTAATCCCAGCACTTTGGGAGGC
CGAGGTAGGCGGAACACCTGAGGAGTTCAAGACCAGCCTGGCCAATATGATGAA
ACCCCGTCTCTACTAAAAATACAAAAATTAGCTGGGCGTGATGGTGTGCACCTGT
AGTCCCAGCTGCTTGGGAGGCTGAGGCAGGAGAATCGCTGGAACCCAAGAGGTG
AAGGTTGCAGTGAGCTGAGATCGCGTCACTGCACTCCAGCCTGGGTGACAGAGT
GAGACTCCACATCAAAAAAAAAAAAAAAAAGTTAGAAAGATTGAATAAGACCTAA
TATTTGCTAGCACAACAGGGTGAATATAGTAAAAAATAATTTATTTGTACCTTCA
AAAATAACTAGACAAGTATAATTGGGTTGTTTGTAACACACAAAAAATAAGTACT
TGAAGTGGTGGATACCCCATTTACCCTGATGTGATTATTTTGTATTGCAGGCCTC
TATCAGAATATCTCATGTAACCCATAAATATATACACCTACTCTGTACCCACAAAA
AGTTTTTAAAAAGAAAAATAAATAGCAACCGAAAAAAAAAGAGAGGGAGAAAAG
AAAAAAGAAAAAAAAATCAAGTGCCTGGCTGGGTAGAATAAATTCTAAGGCCAC
AATGTTACTGACCATGGGTTTTTTGGCTCTCAGTGTATAGAAATTGACACAAGGC
CAATAGTCTTCCCAAACATGCTTTACTGGAACTTACGCCCTGGCATAAGGGCCAC
AACAAAAGAGAGAGCGAATTCTCTGGCTTGCTGACTCCTTGGAAAAAACCGGTA
GGGATTTTTTTATTAGGCAAAGCACAGGAATTGACGTCAGAGGCAGGATGTGCT
GCTGGGCAAAGCATACGAGAAGTGGGGTATGCAGGTCAGCATTACTTGGTTGCA
ATGGTTATCTTGAGGAATGGGCCAACTGGTGGTCTGGCCAGTGGCAACAAGGCT
GTAAATCAATTATTCAGCATTCCTTCCCAAGGTGGGACACCCGGCAACATTGTTT
ATCTCCTAAGGCCAGTTCCTGGAATTAAGTGAAAGGATGACTAATGGACATGTTG
TCAGTGAGGTAGTGGTGTGGGTTTTGTGACCAGTGGGAATGCACGAAAGAATGC

TTTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGACA
GAGTCTTGCTCTGTCGCCCAGGCTGGAGTGCAGTGGCGCCATCTTGGCTCACTG
CAAGCTCCGCCCCCCGGGTTCATGCCATTCTCCTGCCTCAGCCTCCCAAGTAGCT
GGGACTGCAGGTGCCTACCACCAAGCCCAGCTAATTTTTTGTATTTTTTTTTTAG
TAGAGACGGGGTTTCACCATGTTAGCCAGGATGGTCTCGATCTCCTGACCTCGTG
ATCTGCCCGCCTTAGCCTCCCAAAGTGCTGGGATTACAGGCATGAGCCTCCGCC
CCCGGCCTTAAATAATTCTTAAAGGAAGTAAAGTTAACTTTGAAAGAACTATCAG
GATTTGGATTGACTGAAAGGAGTGGGGAAGCTTAGGGAGGAGGTGCTTGCCAGAC
ACTGGGTCATGGCAGTGGTCGGTGAAGCTGCAGTTGCCTAGGGCAGGGATGGAGAGA
GAGTCTGGGCATGAGGAGAGGGTCTCGGGATGTTTGGCTGGACTAGATTTTACAGAA
AGCCTTATCCAGGCTTTTAAAATTACTCTTTCCAGACTTCATCTGAGACTCCTTCTTCA
GCCAACATTCCTTAGCCCTGAATACATTTCCTATCCTCATCTTTCCCTTCTTTTTTTTCC
TTTCTTTTACATGTTTAAATTTAAACCATTCTTCGTGACCCCTTTTCTTGGGAGATTCAT
GGCAAGAACGAGAAGAATGATGGTGCTTGTTAGGGGATGTCCTGTCTCTCTGAACTTT
GGGGTCCTATGCATTAAATAATTTTCCTGACGAGCTCAAGTGCTCCCTCTGGTCTACAA
TCCCTGGCGGCTGGCCTTCATCCCTTGGGCAAGCATTGCATACAGCTCATGGCCCTCCC
TCTACCATACCC.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Corresponding to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence.

Typically, DNA methylation is the methylation of DNA CpG site. The distribution of CpG binucleotide is very uneven in the human genome, while CpG remains or is higher than normal level in some regions of the genome. The CpG site rich region (also known as CpG island) is mainly located in the promoter region and exon regions of the gene, which is a region rich in CpG dinucleotide. About 60% of the promoters of the gene contains CpG island. The CpG is the abbreviation of cytosine (C)-phosphate (p)-guanine (G).

### Tumor

As used herein, the term "tumor" and "cancer" are used interchangeably.

In a preferred embodiment of the present invention, the tumor comprises tumor with low expression, no expression, low activity or no activity of mitochondria permeability transition pore. Typically, the tumor with low expression, no expression, low activity or no activity of mitochondria permeability transition pore is as described above in the third aspect of the present invention.

In a preferred embodiment of the present invention, the tumor comprises tumor with low expression, no expression, low activity or no activity of peptidyl-prolyl cis-trans isomerase F. Typically, the tumor with low expression, no expression, low activity or no activity of peptidyl-prolyl cis-trans isomerase F is as described above in the third aspect of the present invention.

In a preferred embodiment of the present invention, the tumor comprises tumor with low or no expression of NNMT gene. Typically, the tumor with low or no expression of NNMT gene is as described above in the third aspect of the present invention.

In a preferred embodiment of the present invention, the tumor comprises tumor with high expression of DNA methylase. Typically, the tumor with high expression of DNA methylase is as described above in the third aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the third aspect of the present invention.

In a preferred embodiment of the present invention, the tumor comprises tumor with high expression of DNMT3a. Typically, the tumor with high expression of DNMT3a is as described above in the third aspect of the present invention.

In a preferred embodiment of the present invention, the tumor comprises tumor with high expression of DNMT3b. Typically, the tumor with high expression of DNMT3b is as described above in the third aspect of the present invention.

In a preferred embodiment of the present invention, the tumor comprises tumor with high expression of UHRF 1 (ubiquitin-like with PHD and ring finger domain 1). Typically, the tumor with high expression of UHRF 1 is as described above in the third aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the third aspect of the present invention.

In a preferred embodiment of the present invention, the tumor comprises tumor with high methylation level of DNA CpG site of NNMT gene. Typically, the tumor with high methylation level of DNA CpG site of NNMT gene is as described above in the third aspect of the present invention.

Typically, the tumor of present invention is as described above in the third aspect of the present invention.

In present invention, the type of tumors corresponding to tumor cell lines are shown in Table 1

**Table 1**

| Tumor cell line | The corresponding type of tumor |
|---|---|
| Daoy cell | Human medulloblastoma cell |
| NCI-H82 | Human small cell lung cancer cell |

### Anti-tumor drug

The anti-tumor drug can be the compound of formula I, or an optical isomer thereof, or a racemate thereof, or a solvate thereof, or a pharmaceutically acceptable salt thereof, or a deuterated compound thereof of the present invention.

### Use

The compound of present invention has more remarkable and excellent precison treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. That is, the tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is highly sensitive to the compound of the present invention.

The present invention further provides a method for preventing and/or treating tumor, which comprises administering the compound of the present invention to a subject in need.

The compound of present invention has more remarkable and excellent precison treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, therefore, during the prevention and/or treatment of tumor, firstly administering mitochondria permeability transition pore inhibitor, peptidyl-prolyl cis-trans isomerase F inhibitor, NNMT gene inhibitor, DNA methylase promoter, UHRF 1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of the present invention to prevent and/or treat tumor, thereby significantly enhancing the treatment effect of the compound of the present invention on tumors. Therefore, the present invention develops a compound that can significantly enhance anti-tumor effects when combined with mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene. The compound of the present invention can be combined with mitochondria permeability transition pore inhibitor, peptidyl-prolyl cis-trans isomerase F inhibitor, NNMT gene inhibitor, DNA methylase promoter, UHRF 1 promoter, methylation promoter of nucleotide site of NNMT gene, and/or methylation promoter of DNA CpG site of NNMT gene to significantly enhance the treatment effect of the compound on tumor.

In another preferred embodiment, the subject is human and non-human mammals (rodent, rabbit, monkey, livestock, dog, cat, etc.).

In the present invention, there are no special limitations to the method for achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor. For example, the expression and activity of mitochondria permeability transition pore and/or peptidyl-prolyl cis-trans isomerase F can be specifically achieved using methods such as gene knockout, or gene silencing (e.g, shRNA transfection), etc.

In another preferred embodiment, the peptidyl-prolyl cis-trans isomerase F inhibitor comprises shRNA.

Preferably, the nucleotide sequence of shRNA is GTTCTTCATCTGCACCATAAA.

### Marker

The present invention provides a marker for determining whether the compound of present invention is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

In a preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether the compound of present invention is suitable for use in the prevention and/or treatment of patient tumor, the method comprises as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

Specifically, the marker is as described above in the fourth aspect of the present invention.

### Composition

Preferably, the composition or preparation of the present invention is pharmaceutical composition or pharmaceutical preparation. The compositions or preparation of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and active ingredient in the composition or preparation can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g. methylcellulose, ethylcellulose, hydroxypropylmethylcellulose, sodium carboxymethylcellulose, etc.), gelatin, talc, solid lubricants (e.g. stearic acid, magnesium stearate), calcium sulfate, plant oil (e.g. soybean oil, sesame oil, peanut oil, olive oil, etc.), polyols (e.g. propylene glycol, glycerin, mannitol, sorbitol, etc.), emulsifier (e.g. Tween), wetting agent (e.g. sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

Typically, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 10 µg/kg.bw to 1 mg/kg.bw. Of course, the specific dose should also take into account the route of administration, the patient's health and other factors, which are within the skill range of skilled doctors.

### The main excellent technical effects of the present invention comprise:

1. The present invention has unexpectedly developed a compound having excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is highly sensitive to the compound of present invention, ie, the compound of present invention has more remarkable and excellent treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. Therefore, the compound of present invention can realize precise treatment on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, thus improving the treatment effect of the compound of present invention on tumor and avoiding administrating the compound of present invention to patient tumor insensitive to such anti-tumor drug. Therefore, the precision treatment of the compound of the present invention on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene has advantages such a more excellent prevention and treatment effect on tumors, low drug dose, and minimal side effects, which improve the precision prevention and treatment effect on tumor, reduce the side effects and improve patient compliance.

The present invention will be further illustrated below with reference to the specific examples. It should be understood that the following specific examples are based on the technical solution of the present invention and provide detailed embodiments and specific operation processes, but the scope of protection of the present invention is not limited to the examples.

### Example

Mitochondria permeability transition pore was abbreviated as mPTP.

Peptidyl-prolyl cis-trans isomerase F was abbreviated as PPIF.

DNMT3a refers to DNA methyltransferase 3a, NCBI entrez gene: 1788; Uniprotkb/Swiss-port: Q9Y6K1.

DNMT3b refers to DNA methyltransferase 3b, NCBI entrez gene: 1789; Uniprotkb/Swiss-port: Q9UBC3.

DNMT1 refers to DNA methyltransferase 1, NCBI entrez gene: 1786; Uniprotkb/Swiss-port: P26358.

UHRF1 refers to ubiquitin-like with PHD and ring finger domain 1, NCBI entrez gene: 29128; Uniprotkb/Swiss-port: Q96T88.

NNMT refers to Nicotinamide N-Methyltransferase.

### Example 1 Compound AB35419

The structure of compound AB35419 was as follows:

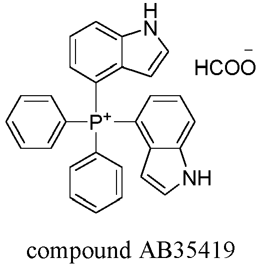

The synthetic route of compound AB35419 was as follows:

### Step (1):

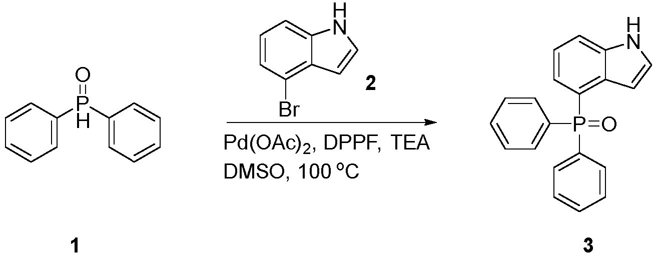

Compound 1 (700 mg, 3.46 mmol, 1 eq) was dissolved in dimethyl sulfoxide (10 mL) in a sealed tube, compound 2 (678 mg, 3.46 mmol, 1 eq), triethylamine (419 mg, 4.15 mmol, 1.2 eq), 1,1'-bis (diphenylphosphine) ferrocene (194 mg, 0.35 mmol, 0.1 eq) and palladium acetate (39 mg, 0.17 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

M-ESI: calculated [M+H]⁺: 318.10; found [M+H]⁺: 318.25.

### Step (2):

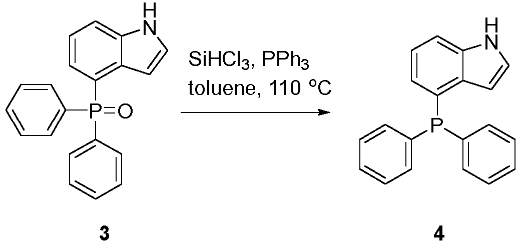

Compound 3 (700 mg, 2.2 mmol, 1 eq) was dissolved in toluene (10 mL) in a sealed tube, triphenylphosphine (1.1 g, 4.4 mmol, 2 eq) and trichlorosilane (5.9 g, 44 mmol, 20 eq) were added. The mixture was reacted at 110 °C for 16 h under N₂, the raction mixture was cooled and quenched with methanol (20 mL), and the pH was adjusted to neutral with 2N NaOH. The mixture was concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

MS-ESI: calculated [M+H]⁺: 302.11; found [M+H]⁺: 302.05.

### Step (3):

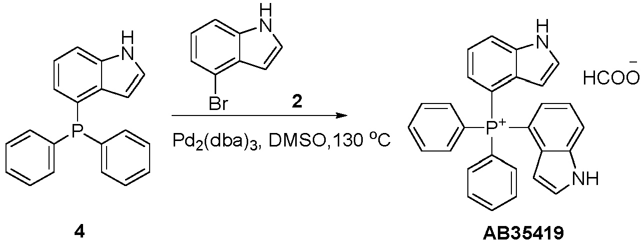

Compound 4 (243 mg, 0.81 mmol, 1 eq) was dissolved in DMSO (2 mL) in a sealed tube, compound 2 (158 mg, 0.81 mmol, 1 eq) and tris(dibenzylideneacetone)dipalladium (7 mg, 0.081 mmol, 0.01 eq) were added. The mixture was reacted at 130 °C for 5 h under N₂, the raction mixture was cooled, diluted with water and then extracted with dichloromethane. The organic phase was washed with brine, dried over anhydrous sodium sulfate, filtered and rotary dried. The crude product was purified by reversed phase preparative chromatography (acetonitrile/water+0.01% formic acid) to afford compound AB35419.

Compound AB35419:

MS-ESI: calculated [M]⁺: 417.15; found [M]⁺: 417.15.

¹H NMR (400 MHz, CDCl3) δ 12.68 (s, 2H), 8.86 (s, 1H), 8.01 (d, J= 4.0 Hz, 2H), 7.76-7.72 (m, 2H), 7.61-7.53 (m, 8H), 7.19 (s, 2H), 7.06-7.01 (m, 4H), 5.53 (d, J= 4.0 Hz, 2H).

### Example 2 Compound AB35431

The structure of compound AB35431 was as follows:

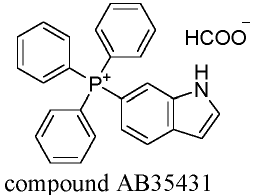

The synthetic route of compound AB35431 was as follows:

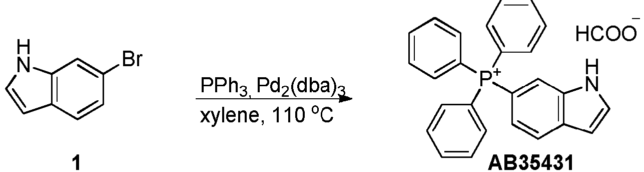

Compound 1 (200 mg, 1.02 mmol, 1 eq) was dissolved in xylene (2 mL) in a sealed tube, triphenylphosphine (268 mg, 1.02 mmol, 1 eq) and tris(dibenzylideneacetone)dipalladium (10 mg, 0.0102 mmol, 0.01 eq) were added. The mixture was reacted at 110 °C for 3 h under N₂, the raction mixture was cooled and concentrated, the crude product was purified by reversed phase preparative chromatography (acetonitrile/water+0.01% formic acid) to afford compound AB35431.

Compound AB35431:

MS-ESI: calculated [M]⁺: 378.14; found [M]⁺: 378.30.

¹H NMR (400 MHz, DMSO-d6) δ 8.84 (s, 1H), 8.23 (d, J= 12.0 Hz, 1H), 7.83-7.60 (m, 17H), 6.98-6.93 (m, 1H), 6.58 (s, 1H).

### Example 3 Compound AB35437

The structure of compound AB35437 was as follows:

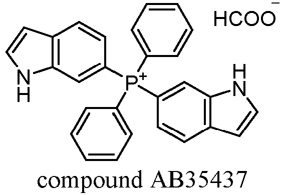

The synthetic route of compound AB35437 was as follows:

### Step (1):

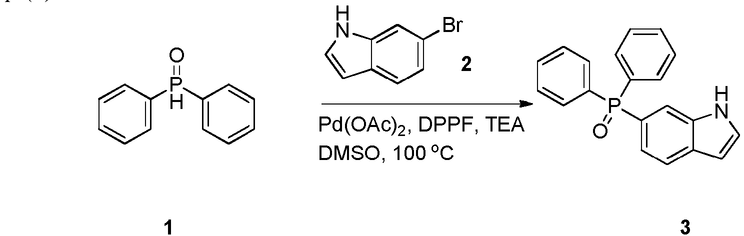

Compound 1 (500 mg, 2.47 mmol, 1 eq) was dissolved in dimethyl sulfoxide (5 mL) in a sealed tube, compound 2 (581 mg, 2.96 mmol, 1.2 eq), triethylamine (300 mg, 2.96 mmol, 1.2 eq), 1, 1'-bis(diphenylphosphine)ferrocene (137 mg, 0.24 mmol, 0.1 eq) and palladium acetate (28 mg, 0.12 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

M-ESI: calculated [M+H]⁺: 318.10; found [M+H]⁺: 318.25.

### Step (2):

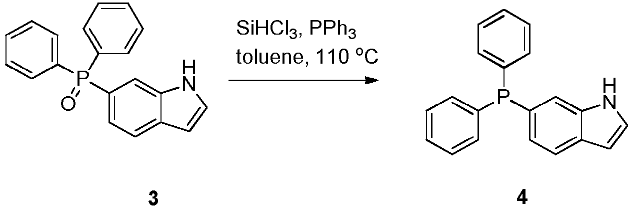

Compound 3 (700 mg, 2.2 mmol, 1 eq) was dissolved in toluene (10 mL) in a sealed tube, triphenylphosphine (1.1 g, 4.4 mmol, 2 eq) and trichlorosilane (5.9 g, 44 mmol, 20 eq) were added. The mixture was reacted at 110 °C for 16 h under N₂, the raction mixture was cooled and quenched with methanol (20 mL), and the pH was adjusted to neutral with 2N NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

MS-ESI: calculated [M+H]⁺: 302.11; found [M+H]⁺: 302.05.

### Step (3):

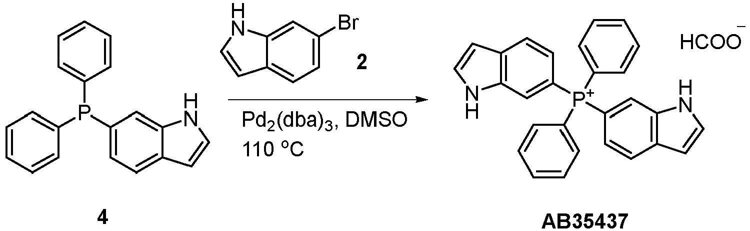

Compound 4 (100 mg, 0.33 mmol, 1 eq) was dissolved in DMSO (2 mL) in a sealed tube, compound 2 (65 mg, 0.33 mmol, 1 eq) and tris(dibenzylideneacetone)dipalladium (6 mg, 0.0066 mmol, 0.02 eq) were added. The mixture was reacted at 110 °C for 5 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35437.

Compound AB35437:

MS-ESI: calculated [M]⁺: 417.15; found [M]⁺: 417.30.

¹H NMR (400 MHz, CDCl3) δ 12.25 (s, 1H), 11.99 (s, 1H), 8.49 (s, 1H), 7.95-7.88 (m, 4H), 7.81-7.71 (m, 10H), 7.65-7.61 (m, 2H), 7.35-7.23 (m, 2H), 6.67-6.65 (m, 2H).

### Example 4 Compound AB35449

The structure of compound AB35449 was as follows:

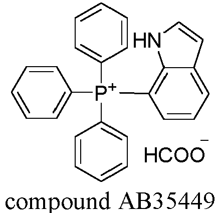

The synthetic route of compound AB35449 was as follows:

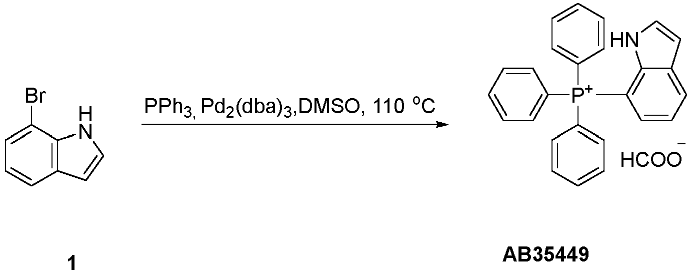

Compound 1 (200 mg, 1.02 mmol, 1 eq) was dissolved in anhydrous dimethyl sulfoxide (2 mL) in a sealed tube, triphenylphosphine (267 mg, 1.02 mmol, 1 eq) and tris(dibenzylideneacetone)dipalladium (9 mg, 0.0102 mmol, 0.01 eq) were added. The mixture was reacted at 110 °C for 5 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35449.

Compound AB35449:

MS-ESI: calculated [M]⁺: 378.14; found [M]⁺: 378.30.

¹H NMR (400 MHz, DMSO-d6) δ 11.20 (s, 1H), 8.46 (s, 1H), 8.18 (d, J= 4.0 Hz, 1H), 7.92-7.88 (m, 3H), 7.74-7.64 (m, 12H), 7.38 (d, J= 4.0 Hz, 1H), 7.30 (s, 1H), 6.85-6.84 (m, 1H), 6.72 (s, 1H).

### Example 5 Compound AB35450

The structure of compound AB35450 was as follows:

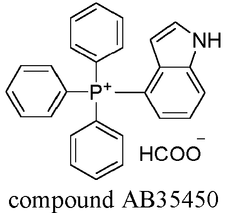

The synthetic route of compound AB35450 was as follows:

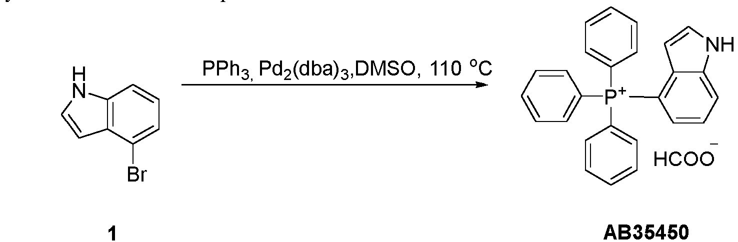

Compound 1 (300 mg, 1.5 mmol, 1 eq) was dissolved in anhydrous dimethyl sulfoxide (2 mL) in a sealed tube, triphenylphosphine (400 mg, 1.5 mmol, 1 eq) and tris(dibenzylideneacetone)dipalladium (15 mg, 0.015 mmol, 0.01 eq) were added. The mixture was reacted at 110 °C for 5 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35450.

Compound AB35450:

MS-ESI: calculated [M]⁺: 378.14; found [M]⁺: 378.15.

¹H NMR (400 MHz, DMSO-d6) δ 13.07 (s, 1H), 8.55 (s, 1H), 8.08 (d, J= 8.0 Hz, 1H), 7.95-7.91 (m, 3H), 7.77-7.70 (m, 12H), 7.57 (d, J= 4.0 Hz, 1H), 7.44-7.40 (m, 1H), 7.07-7.01 (m, 1H), 5.51 (s, 1H).

### Example 6 Compound AB35451

The structure of compound AB35451 was as follows:

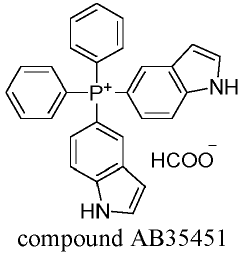

The synthetic route of compound AB35451 was as follows:

### Step (1):

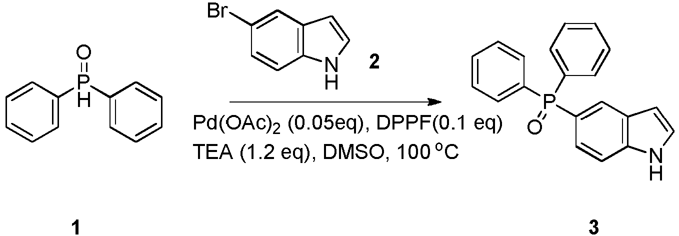

Compound 1 (1.5 g, 7.42 mmol, 1 eq) was dissolved in dimethyl sulfoxide (30 mL) in a sealed tube, compound 2 (1.6 g, 8.16 mmol, 1.1 eq), triethylamine (899 mg, 8.90 mmol, 1.2 eq), 1,1'-bis (diphenylphosphine)ferrocene (410 mg, 0.74 mmol, 0.1 eq) and palladium acetate (83 mg, 0.37 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

MS-ESI: calculated [M+H]⁺: 318.10; found [M+H]⁺: 318.25.

### Step (2):

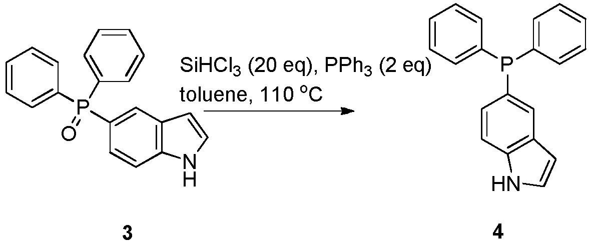

Compound 3 (1.3 g, 4.09 mmol, 1 eq) was dissolved in toluene (40 mL) in a sealed tube, triphenylphosphine (2.1 g, 8.18 mmol, 2 eq) and trichlorosilane (11.1 g, 81.8 mmol, 20 eq) were added. The mixture was reacted at 110 °C for 16 h under N₂, the raction mixture was cooled and quenched with methanol (50 mL), and the pH was adjusted to neutral with 2N NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

MS-ESI: calculated [M+H]⁺: 302.11; found [M+H]⁺: 302.05.

### Step (3):

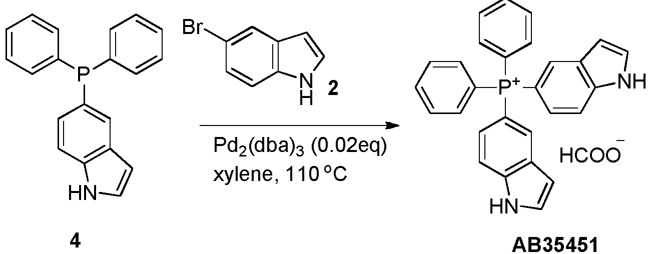

Compound 4 (200 mg, 0.66 mmol, 1 eq) was dissolved in xylene (3 mL) in a sealed tube, compound 2 (143 mg, 0.73 mmol, 1.1 eq) and tris(dibenzylideneacetone)dipalladium (12 mg, 0.013 mmol, 0.02 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35451.

Compound AB35451:

MS-ESI: calculated [M]⁺: 417.15; found [M]⁺: 417.10.

¹H NMR (400 MHz, DMSO-d6) δ 12.49 (s, 2H), 8.52 (s, 1H), 7.93-7.72 (m, 14H), 7.64 (s, 2H), 7.36-7.31 (m, 2H), 6.66 (s, 2H).

### Example 7 Compound AB35452

The structure of compound AB35452 was as follows:

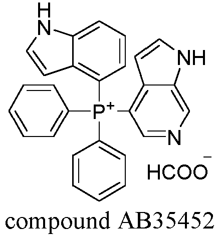

The synthetic route of compound AB35452 was as follows:

### Step (1):

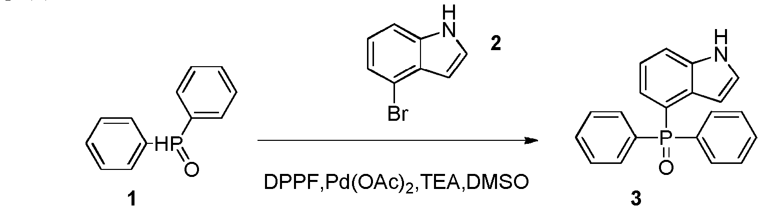

Compound 1 (2 g, 9.9 mmol, 1 eq) was dissolved in dimethyl sulfoxide (30 mL) in a sealed tube, compound 2 (2.33 g, 11.87 mmol, 1.2 eq), triethylamine (1.2 g, 11.87 mmol, 1.2 eq), 1,1'- bis (diphenylphosphine)ferrocene (550 mg, 0.99 mmol, 0.1 eq) and palladium acetate (111 mg, 0.495 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

MS-ESI: calculated [M+H]⁺: 318.10; found [M+H]⁺: 318.05.

### Step (2):

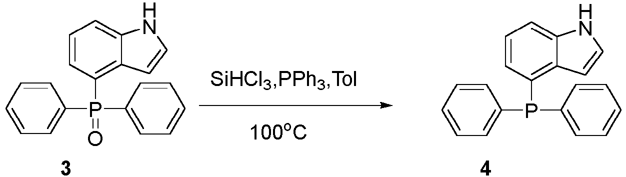

Compound 3 (2 g, 6.3 mmol, 1 eq) was dissolved in toluene (200 mL) in a sealed tube, triphenylphosphine (3.3 g, 12.6 mmol, 2 eq) and trichlorosilane (12.8 mL, 126 mmol, 20 eq) were added. The mixture was reacted at 100 °C for 16 h under N₂, the raction mixture was cooled and quenched with methanol (20 mL), and the pH was adjusted to neutral with saturated NaOH solution. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

MS-ESI: calculated [M+H]⁺: 301.11; found [M+H]⁺: 302.10.

### Step (3):

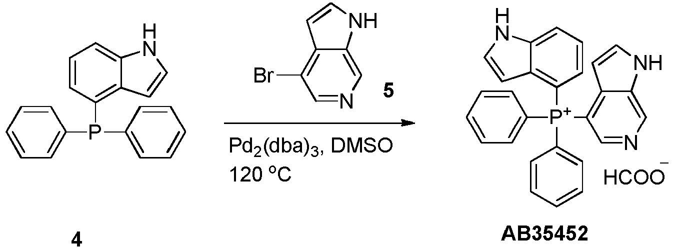

Compound 4 (200 mg, 0.66 mmol, 1 eq) was dissolved in dimethyl sulfoxide (2 mL) in a sealed tube, compound 5 (156 mg, 0.79 mmol, 1.2 eq) and tris(dibenzylideneacetone)dipalladium (60 mg, 0.066 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35452.

Compound AB35452:

MS-ESI: calculated [M]⁺: 418.15; found [M]⁺: 418.10.

¹H NMR (400 MHz, DMSO-d6) δ 12.49 (s, 1H), 9.22 (d, J= 4.0 Hz, 1H), 8.57 (s, 1H), 8.08-8.02 (m, 2H), 7.93-7.89 (m, 2H), 7.78-7.73 (m, 9H), 7.54 (d, J= 4.0 Hz, 1H), 7.42-7.38 (m, 1H), 7.22-7.16 (m, 1H), 5.60-5.56 (m, 2H).

### Example 8 Compound AB35461

The structure of compound AB35461 was as follows:

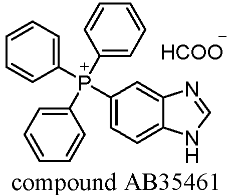

The synthetic route of compound AB35461 was as follows:

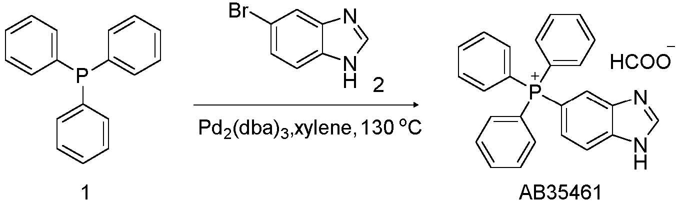

Compound 1 (200 mg, 0.76 mmol, 1 eq) was dissolved in xylene (2 mL) in a sealed tube, compound 2 (165 mg, 0.84 mmol, 1.1 eq) and tris(dibenzylideneacetone)dipalladium (35 mg, 0.038 mmol, 0.05 eq) were added. The mixture was reacted at 130 °C for 4 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35461.

Compound AB35461:

MS-ESI: calculated [M]⁺: 379.14; found [M]⁺: 379.20.

¹H NMR (400 MHz, DMSO-d6) δ 8.48 (s, 1H), 8.45 (s, 1H), 7.97-7.90 (m, 4H), 7.81-7.71 (m, 14H), 7.41-7.35 (m, 1H).

### Example 9 Compound AB35462

The structure of compound AB35462 was as follows:

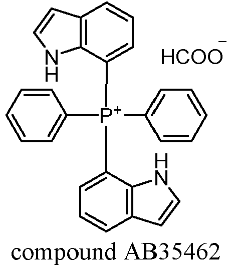

The synthetic route of compound AB35462 was as follows:

### Step (1):

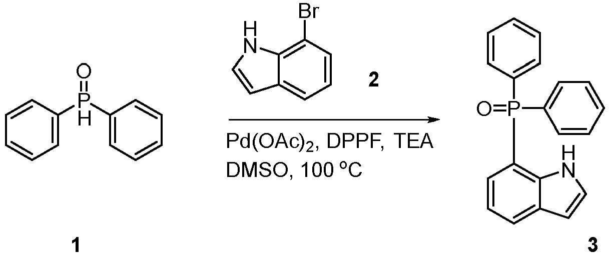

Compound 1 (560 mg, 2.77 mmol, 1 eq) was dissolved in dimethyl sulfoxide (10 mL) in a sealed tube, compound 2 (652 mg, 3.32 mmol, 1.2 eq), triethylamine (335 mg, 3.32 mmol, 1.2 eq), 1,1'- bis (diphenylphosphine)ferrocene (155 mg, 0.28 mmol, 0.1 eq) and palladium acetate (31 mg, 0.14 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3 (340mg, yield: 38.7%) as a white solid.

MS-ESI: calculated [M+H]⁺: 318.10; found [M+H]⁺: 318.05.

### Step (2):

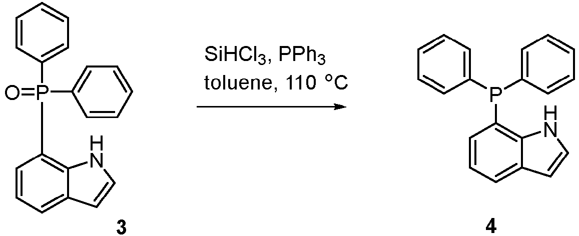

Compound 3 (340 mg, 1.07 mmol, 1 eq) was dissolved in toluene (10 mL) in a sealed tube, triphenylphosphine (561 mg, 2.14 mmol, 2 eq) and trichlorosilane (2.9 g, 21.4 mmol, 20 eq) were added. The mixture was reacted at 110 °C for 16 h under N₂, the raction mixture was cooled and quenched with methanol (50 mL), and the pH was adjusted to neutral with 2M NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4 (300 mg).

MS-ESI: calculated [M+H]⁺: 302.11; found [M+H]⁺: 302.05.

### Step (3):

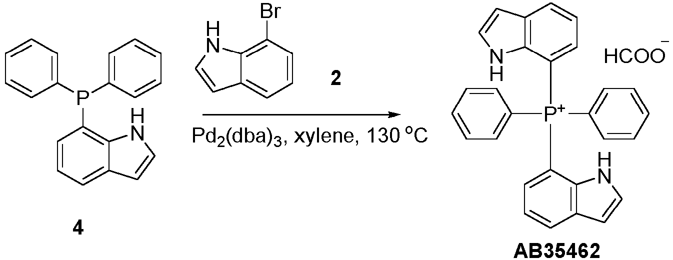

Compound 4 (200 mg, 0.66 mmol, 1 eq) was dissolved in xylene (1 mL) in a sealed tube, compound 2 (156 mg, 0.79 mmol, 1.2 eq) and tris(dibenzylideneacetone)dipalladium (64 mg, 0.07 mmol, 0.1 eq) were added. The mixture was reacted at 130 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35462.

Compound AB35462:

MS-ESI: calculated [M]⁺: 417.15; found [M]⁺: 417.10.

¹H NMR (400 MHz, DMSO-d6) δ 11.01 (s, 1H), 8.41 (s, 1H), 8.17 (d, J=8.0 Hz, 2H), 7.87-7.85 (m, 2H), 7.75-7.62 (m, 9H), 7.3-7.25 (m, 4H), 7.06-7.03 (m, 2H), 6.71 (s, 2H).

### Example 10 Compound AB35470

The structure of compound AB35470 was as follows:

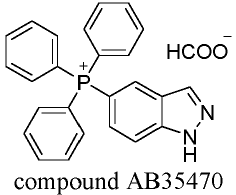

The synthetic route of compound AB35470 was as follows:

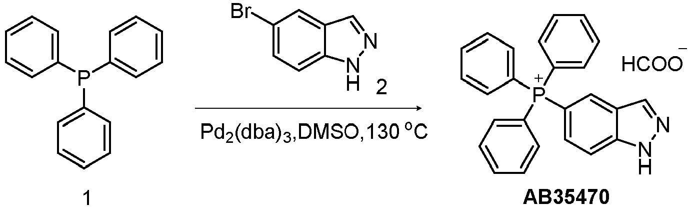

Compound 1 (200 mg, 0.76 mmol, 1 eq) was dissolved in dimethyl sulfoxide (2 mL) in a sealed tube, compound 2 (166 mg, 0.84 mmol, 1.1 eq) and tris(dibenzylideneacetone)dipalladium (35 mg, 0.038 mmol, 0.05 eq) were added. The mixture was reacted at 130 °C for 4 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35470.

Compound AB35470:

MS-ESI: calculated [M]⁺: 379.14; found [M]⁺: 379.10.

¹H NMR (400 MHz, DMSO-d6) δ 8.51 (s, 1H), 8.32 (s, 1H), 7.94 (d, J= 8.0 Hz, 1H), 7.81 (d, J= 4.0 Hz, 1H), 7.79-7.77 (m, 3H), 7.76-7.77 (m, 12H), 7.57-7.52 (m, 1H).

### Example 11 Compound AB35471

The structure of compound AB35471 was as follows:

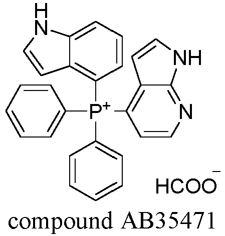

The synthetic route of compound AB35471 was as follows:

### Step (1):

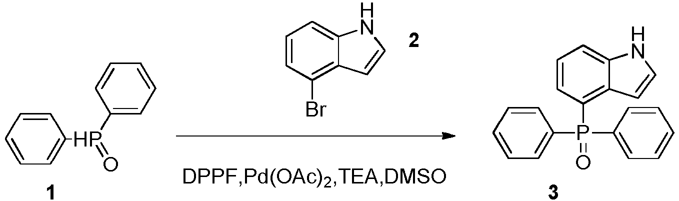

Compound 1 (2 g, 9.9 mmol, 1 eq) was dissolved in dimethyl sulfoxide (30 mL) in a sealed tube, compound 2 (2.33 g, 11.87 mmol, 1.2 eq), triethylamine (1.2 g, 11.87 mmol, 1.2 eq), 1,1 '-bis (diphenylphosphine)ferrocene (550 mg, 0.99 mmol, 0.1 eq) and palladium acetate (111 mg, 0.495 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

MS-ESI: calculated [M+H]⁺: 318.10; found [M+H]⁺: 318.05

### Step (2):

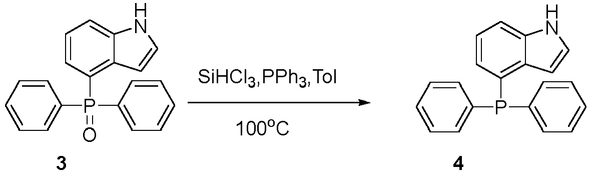

Compound 3 (2 g, 6.3 mmol, 1 eq) was dissolved in toluene (200 mL) in a sealed tube, triphenylphosphine (3.3 g, 12.6 mmol, 2 eq) and trichlorosilane (12.8 mL, 126 mmol, 20 eq) were added. The mixture was reacted at 100 °C for 16 h under N₂, the raction mixture was cooled and quenched with methanol (50 mL), and the pH was adjusted to neutral with saturated NaOH solution. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

MS-ESI: calculated [M+H]⁺: 302.11; found [M+H]⁺: 302.10.

### Step (3):

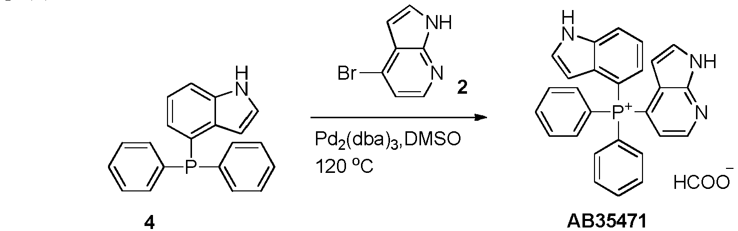

Compound 4 (100 mg, 0.33 mmol, 1 eq) was dissolved in dimethyl sulfoxide (1 mL) in a sealed tube, compound 2 (78 mg, 0.39 mmol, 1.2 eq) and tris(dibenzylideneacetone)dipalladium (30 mg, 0.033 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB35471.

Compound AB35471:

MS-ESI: calculated [M]⁺: 418.15; found [M]⁺: 418.10.

¹H NMR (400 MHz, DMSO-d6) δ 8.54-8.51 (m, 2H), 8.01 (d, J= 4.0 Hz, 1H), 7.90-7.80 (m, 2H), 7.75-7.68 (m, 10H), 7.52 (s, 1H), 7.40-7.38 (m, 1H), 7.19-7.14 (m, 2H), 5.56 (s, 1H), 5.47 (s, 1H).

### Example 12 Compound AB35472

The structure of compound AB35472 was as follows:

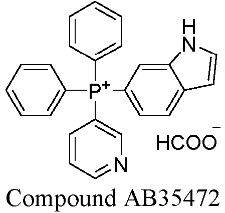

The synthetic route of compound AB35472 was as follows:

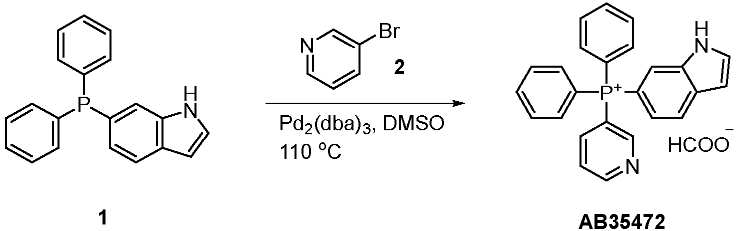

Compound 1 (100 mg, 0.33 mmol, 1 eq) was dissolved in dimethyl sulfoxide (1 mL) in a sealed tube, compound 2 (63 mg, 0.40 mmol, 1.2 eq) and tris(dibenzylideneacetone)dipalladium (27 mg, 0.03 mmol, 0.1 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB35472.

Compound AB35472:

MS-ESI: calculated [M]⁺:379.14; found [M]⁺:379.10.

¹H NMR (400 MHz, DMSO-d6) δ 12.93 (s, 1H), 9.05 (s, 1H), 8.86 (d, J= 4.0 Hz, 1H), 8.47 (s, 1H), 8.20-8.18 (m, 1H), 7.91-7.85 (m, 3H), 7.80-7.77 (m, 11H), 7.28-7.23 (m, 1H), 6.67 (s, 1H).

### Example 13 Compound AB35473

The structure of compound AB35473 was as follows:

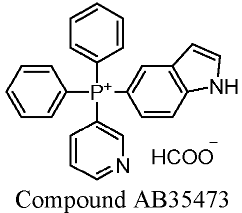

The synthetic route of compound AB35473 was as follows:

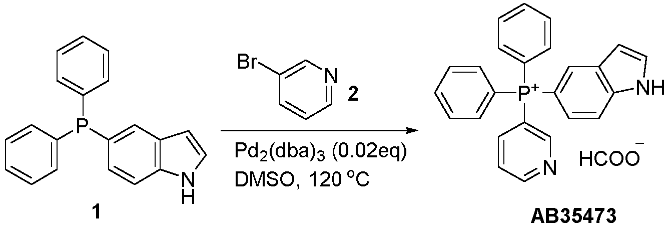

Compound 1 (100 mg, 0.33 mmol, 1 eq) was dissolved in dimethyl sulfoxide (1 mL) in a sealed tube, compound 2 (63 mg, 0.40 mmol, 1.2 eq) and tris(dibenzylideneacetone)dipalladium (30 mg, 0.033 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB35473.

Compound AB35473:

MS-ESI: calculated [M]⁺:379.14; found [M]⁺:379.30.

¹H NMR (400 MHz, DMSO-d6) δ 12.86 (s, 1H), 9.03 (s, 1H), 8.86 (d, J= 4.0 Hz, 1H), 8.53 (s, 1H), 8.19-8.16 (m, 1H), 7.85-7.78 (m, 13H), 7.63 (s, 1H), 7.36-7.31 (m, 1H), 6.64 (s, 1H).

### Example 14 Compound AB35474

The structure of compound AB35474 was as follows:

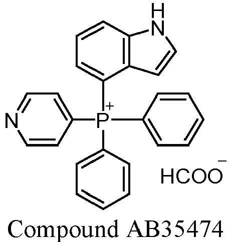

The synthetic route of compound AB35474 was as follows:

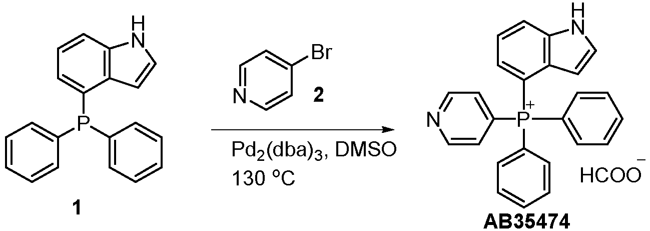

Compound 1 (100 mg, 0.33 mmol, 1 eq) was dissolved in dimethyl sulfoxide (2 mL) in a sealed tube, compound 2 (63 mg, 0.40 mmol, 1.2 eq) and tris(dibenzylideneacetone)dipalladium (30 mg, 0.033 mmol, 0.1 eq) were added. The mixture was reacted at 130 °C for 4 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB35474.

Compound AB35474:

MS-ESI: calculated [M]⁺:379.14; found [M]⁺:379.10.

¹H NMR (400 MHz, DMSO-d6) δ 12.39 (s, 1H), 8.98-8.96 (m, 2H), 8.50 (s, 1H), 7.94-7.72 (m, 14H), 7.64 (s, 1H), 7.35-7.30 (m, 1H), 6.65 (s, 1H).

### Example 15 Compound AB35477

The structure of compound AB35477 was as follows:

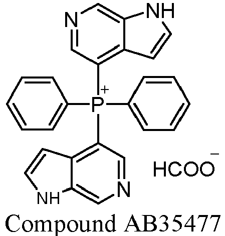

The synthetic route of compound AB35477 was as follows:

### Step (1):

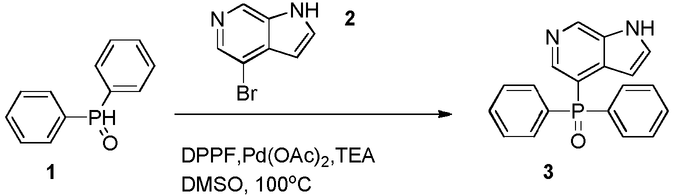

Compound 1 (2 g, 9.89 mmol, 1 eq) was dissolved in dimethyl sulfoxide (50 mL) in a sealed tube, compound 2 (2.3 g, 11.87 mmol, 1.2 eq), triethylamine (1.2 g, 11.87 mmol, 1.2 eq), 1,1'- bis (diphenylphosphine)ferrocene (549 mg, 0.99 mmol, 0.1 eq) and palladium acetate (110 mg, 0.49 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

### Step (2):

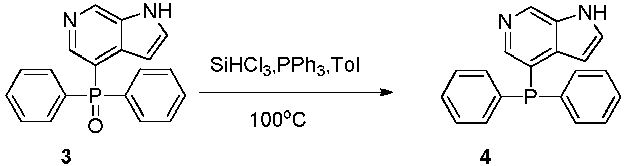

Compound 3 (2 g, 6.28 mmol, 1 eq) was dissolved in toluene (50 mL) in a sealed tube, triphenylphosphine (3.3 g, 12.56 mmol, 2 eq) and trichlorosilane (17.0 g, 125.6 mmol, 20 eq) were added. The mixture was reacted at 100 °C for 16 h under N₂, the raction mixture was cooled and quenched with methanol (50 mL), and the pH was adjusted to neutral with 2N NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

### Step (3):

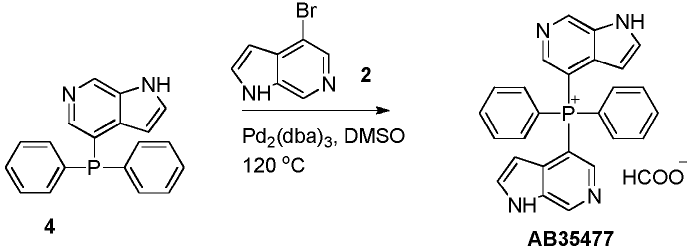

Compound 4 (100 mg, 0.33 mmol, 1 eq) was dissolved in DMSO (2 mL) in a sealed tube, compound 2 (65 mg, 0.33 mmol, 1 eq) and tris(dibenzylideneacetone)dipalladium (30 mg, 0.066 mmol, 0.1 eq) were added. The mixture was reacted at 120 °C for 5 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35477.

Compound AB35477:

MS-ESI: calculated [M]⁺: 419.14; found [M]⁺: 419.15.

¹H NMR (400 MHz, DMSO-d6) δ 9.20 (s, 2H), 8.49 (s, 3H), 8.06 (d, J= 8.0 Hz, 2H), 7.92-7.88 (m, 2H), 7.83-7.73 (m, 10H), 5.62 (s, 2H).

### Example 16 Compound AB35479

The structure of compound AB35479 was as follows:

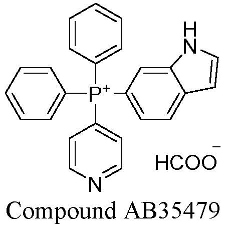

The synthetic route of compound AB35479 was as follows:

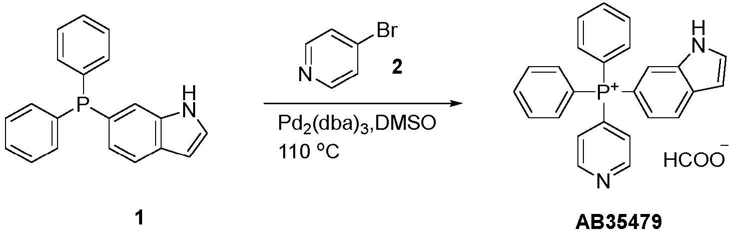

Compound 1 (100 mg, 0.33 mmol, 1 eq) was dissolved in dimethyl sulfoxide (1 mL) in a sealed tube, compound 2 (63 mg, 0.40 mmol, 1.2 eq) and tris(dibenzylideneacetone)dipalladium (20 mg, 0.033 mmol, 0.1 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35479.

Compound AB35479:

MS-ESI: calculated [M]⁺:379.14; found [M]⁺:379.10.

¹H NMR (400 MHz, DMSO-d6) δ 12.79 (s, 1H), 8.99-8.97 (m, 2H), 8.47 (s, 1H), 7.95-7.93 (m, 3H), 7.79-7.73(m, 12H), 7.27-7.22 (m, 1H), 6.68 (s, 1H).

### Example 17 Compound AB35480

The structure of compound AB35480 was as follows:

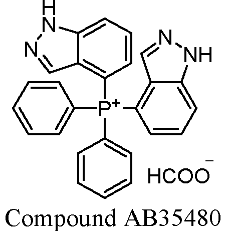

The synthetic route of compound AB35480 was as follows:

### Step (1):

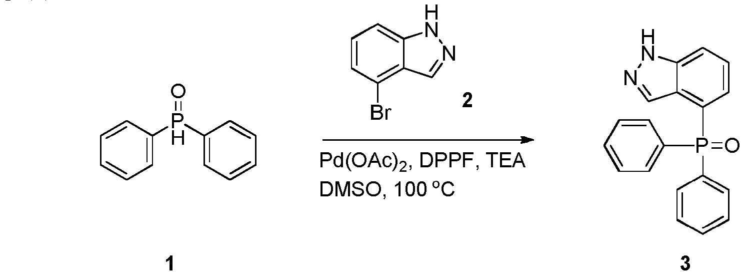

Compound 1 (5 g, 24.73 mmol, 1 eq) was dissolved in dimethyl sulfoxide (50 mL) in a sealed tube, compound 2 (5.8 g, 29.67 mmol, 1.2 eq), triethylamine (3.0 g, 29.67 mmol, 1.2 eq), 1,1'- bis (diphenylphosphine)ferrocene (1.3 g, 2.47 mmol, 0.1 eq) and palladium acetate (276 mg, 1.23 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

### Step (2):

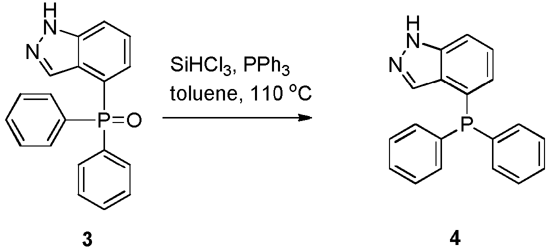

Compound 3 (8.0 g, 25.13 mmol, 1 eq) was dissolved in toluene (100 mL) in a sealed tube, triphenylphosphine (13.2 g, 50.26 mmol, 2 eq) and trichlorosilane (68 g, 502.6 mmol, 20 eq) were added. The mixture was reacted at 100 °C for 16 h under N₂, the raction mixture was cooled and quenched with methanol (200 mL), and the pH was adjusted to neutral with 2N NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

### Step (3):

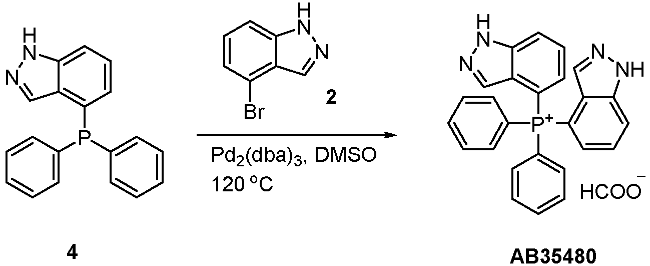

Compound 4 (100 mg, 0.33 mmol, 1 eq) was dissolved in DMSO (2 mL) in a sealed tube, compound 2 (65 mg, 0.33 mmol, 1 eq) and tris(dibenzylideneacetone)dipalladium (30 mg, 0.033 mmol, 0.1 eq) were added. The mixture was reacted at 120 °C for 5 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35480.

Compound AB35480:

MS-ESI: calculated [M]⁺: 419.14; found [M]⁺: 419.10.

¹H NMR (400 MHz, DMSO-d6) δ 8.53 (s, 1H), 8.23 (d, J= 4.0 Hz, 2H), 7.93-7.91 (m, 2H), 7.8-7.74 (m, 9H), 7.63-7.61 (m, 2H), 7.41-7.35 (m, 2H), 7.07 (s, 2H).

### Example 18 Compound AB35481

The structure of compound AB35481 was as follows:

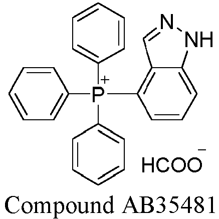

The synthetic route of compound AB35481 was as follows:

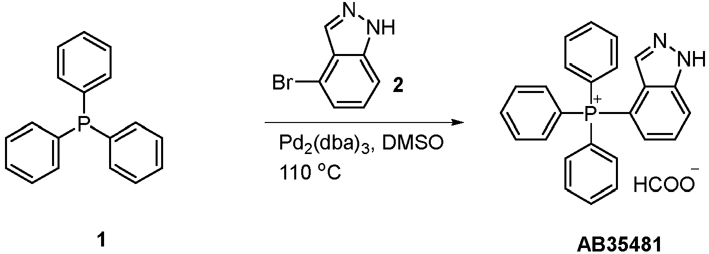

Compound 1 (100 mg, 0.38 mmol, 1 eq) was dissolved in dimethyl sulfoxide (1 mL) in a sealed tube, compound 2 (89 mg, 0.45 mmol, 1.2 eq) and tris(dibenzylideneacetone)dipalladium (35 mg, 0.038 mmol, 0.1 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35481.

Compound AB35481:

MS-ESI: calculated [M]⁺:379.14; found [M]⁺:379.15.

¹H NMR (400 MHz, DMSO-d6) δ 8.51 (s, 1H), 8.22 (d, J= 8.0 Hz, 1H), 8.94-8.92 (m, 3H), 7.76-7.66 (m, 12H), 7.63-7.61 (m, 1H), 7.24-7.20 (m, 1H), 7.02 (s, 1H).

### Example 19 Compound AB35482

The structure of compound AB35482 was as follows:

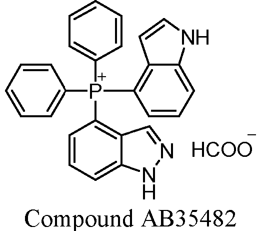

The synthetic route of compound AB35482 was as follows:

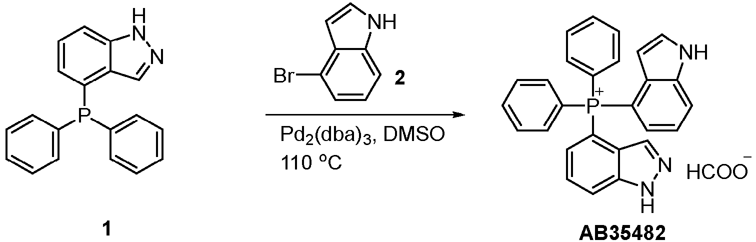

Compound 1 (100 mg, 0.33 mmol, 1 eq) was dissolved in dimethyl sulfoxide (1 mL) in a sealed tube, compound 2 (78 mg, 0.40 mmol, 1.2 eq) and tris(dibenzylideneacetone)dipalladium (30 mg, 0.033 mmol, 0.1 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35482.

Compound AB35482:

MS-ESI: calculated [M]⁺:418.15; found [M]⁺:418.25.

¹H NMR (400 MHz, DMSO-d6) δ 12.13 (s, 1H), 8.51 (s, 1H), 8.20-8.00 (m, 2H), 7.95-7.91 (m, 2H), 7.74-7.64 (m, 10H), 7.51 (s, 1H), 7.41-7.37 (m, 2H), 7.19-7.14 (m, 1H), 7.00 (s, 1H), 5.56 (s, 1H).

### Example 20 Compound AB35495

The structure of compound AB35495 was as follows:

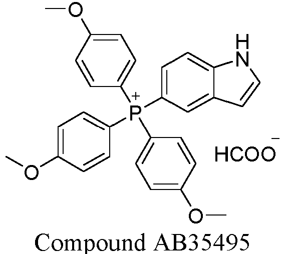

The synthetic route of compound AB35495 was as follows:

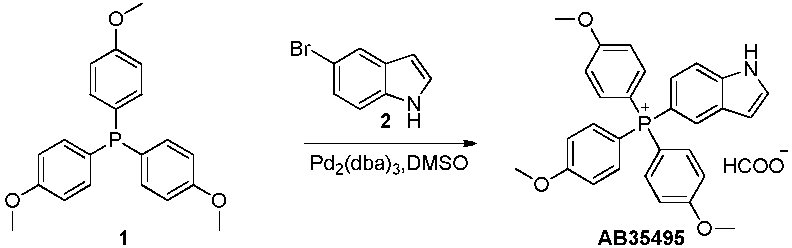

Compound 1 (200 mg, 0.57 mmol, 1 eq) was dissolved in dimethyl sulfoxide (2 mL) in a sealed tube, compound 2 (134 mg, 0.68 mmol, 1.2 eq) and tris(dibenzylideneacetone)dipalladium (52 mg, 0.057 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35495.

Compound AB35495:

MS-ESI: calculated [M]⁺: 468.17; found [M]⁺: 468.40.

¹H NMR (400 MHz, DMSO-d6) δ 8.49 (s, 1H), 7.81-7.77 (m, 2H), 7.55-7.53 (m, 7H), 7.30-7.22 (m, 7H), 6.62 (s, 1H), 3.87 (s, 9H).

### Example 21 Compound AB35503

The structure of compound AB35503 was as follows:

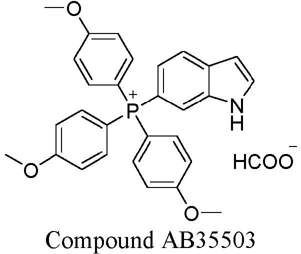

The synthetic route of compound AB35503 was as follows:

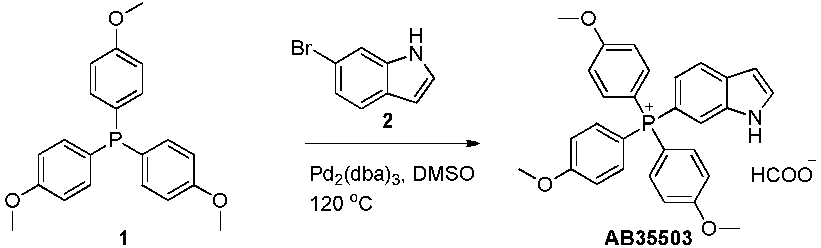

Compound 1 (200 mg, 0.57 mmol, 1 eq) was dissolved in dimethyl sulfoxide (2 mL) in a sealed tube, compound 2 (134 mg, 0.68 mmol, 1.2 eq) and tris(dibenzylideneacetone)dipalladium (52 mg, 0.057 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35503.

Compound AB35503:

MS-ESI: calculated [M]⁺: 468.17; found [M]⁺: 468.20.

¹H NMR (400 MHz, DMSO-d6) δ 12.55 (s, 1H), 8.49 (s, 1H), 7.91-7.89 (m, 1H), 7.62-7.50 (m, 8H), 7.30-7.25 (m, 6H), 7.16-7.13 (m, 1H), 6.64 (s, 1H), 3.87 (s, 9H).

### Example 22 Compound AB35507

The structure of compound AB35507 was as follows:

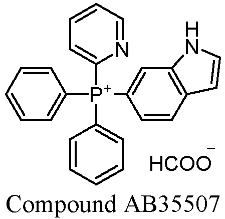

The synthetic route of compound AB35507 was as follows:

### Step (1):

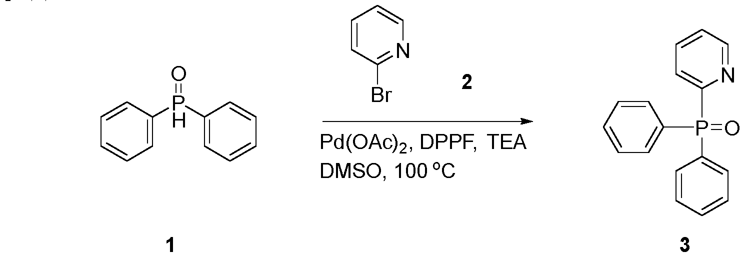

Compound 1 (3.5 g, 17.31 mmol, 1 eq) was dissolved in dimethyl sulfoxide (50 mL), compound 2 (3.3 g, 20.77 mmol, 1.2 eq), triethylamine (2.1 g, 20.77 mmol, 1.2 eq), 1,1'-bis (diphenylphosphine)ferrocene (959 mg, 1.73 mmol, 0.1 eq) and palladium acetate (194 mg, 0.86 mmol, 0.05 eq) were added. The mixture was reacted at 100 °C for overnight under N₂, the raction mixture was diluted with dichloromethane and washed with water twice, the organic phase was washed with brine twice, dried over anhydrous sodium sulfate, filtered and rotary dried, the crude product was purified by fast chromatography (DCM/MeOH=100/1 to 50/1) to afford compound 3.

### Step (2):

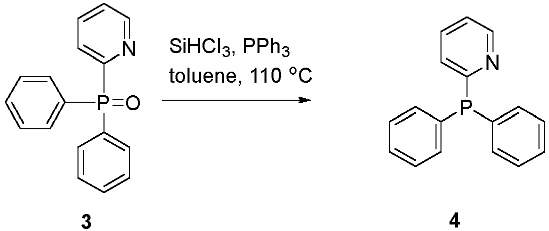

Compound 3 (2 g, 7.16 mmol, 1 eq) was dissolved in toluene (200 mL) in a sealed tube. triphenylphosphine (3.7 g, 14.32 mmol, 2 eq) and trichlorosilane (19.4 ml, 143.2 mmol, 20 eq) were added. The mixture was reacted at 110 °C for 16 h under N₂, the raction mixture was quenched with methanol (50 mL), and the pH was adjusted to neutral with 2N NaOH. The mixture was filtered to remove solid, concentrated, diluted with water and then extracted with dichloromethane. The organic phase was dried over anhydrous sodium sulfate, filtered and rotary dried, the crude product was purified by fast chromatography (PE/EA=25/1) to afford compound 4.

### Step (3):

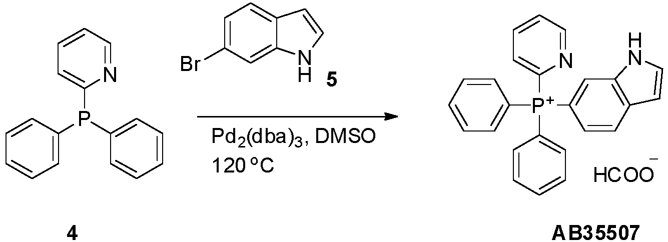

Compound 4 (100 mg, 0.38 mmol, 1 eq) was dissolved in dimethyl sulfoxide (2 mL) in a sealed tube, compound 5 (89 mg, 0.45 mmol, 1.2 eq) and tris(dibenzylideneacetone)dipalladium (35 mg, 0.038 mmol, 0.1 eq) were added. The mixture was reacted at 120 °C for 5 h under N₂, the raction mixture was diluted with water and then extracted with dichloromethane. The organic phase was dried over anhydrous sodium sulfate, filtered and rotary dried, the crude product was purified by reversed phase preparative chromatography (acetonitrile/water+0.1% formic acid) to afford compound AB35507.

Compound AB35507:

MS-ESI: calculated [M]⁺: 379.14; found [M]⁺: 379.10.

¹H NMR (400 MHz, DMSO-d6) δ 8.96 (d, J= 4.0 Hz, 1H), 8.47 (s, 1H), 8.15-8.13 (m, 1H), 7.83-7.71 (m, 15H), 7.29-7.24 (m, 1H), 6.66 (s, 1H).

### Example 23 Compound 24

Compound 24 was synthesized using conventional synthetic methods.

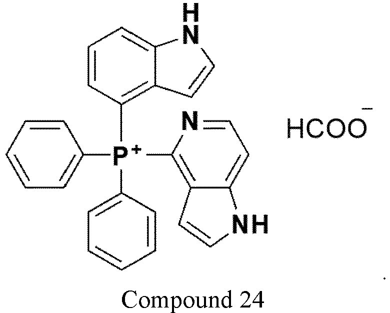

### Example 24 Compound 33

Compound 33 was synthesized using conventional synthetic methods.

The structure of compound 33 was as follows:

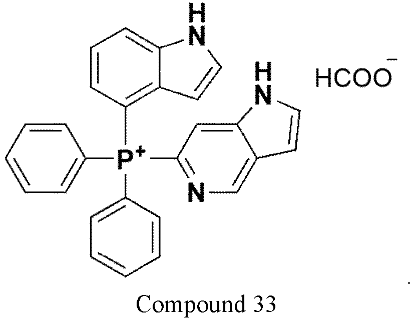

### Example 25 Compound 35

Compound 35 was synthesized using conventional synthetic methods.

The structure of compound 35 was as follows:

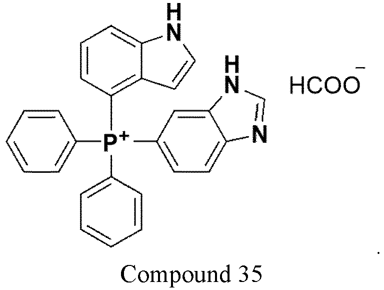

### Example 26 Compound 36

Compound 36 was synthesized using conventional synthetic methods.

The structure of compound 36 was as follows:

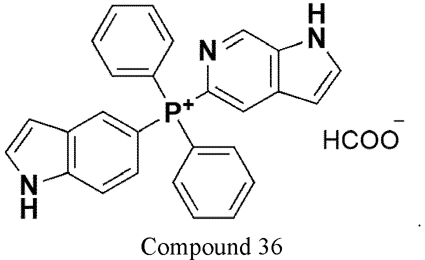

### Example 27 Compound 42

Compound 42 was synthesized using conventional synthetic methods.

The structure of compound 42 was as follows:

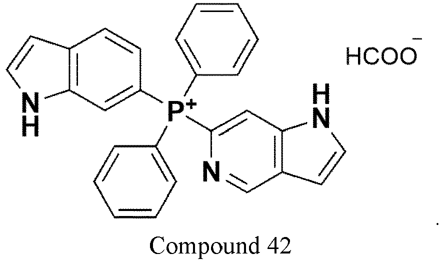

### Example 28 Compound 43

Compound 43 was synthesized using conventional synthetic methods.

The structure of compound 43 was as follows:

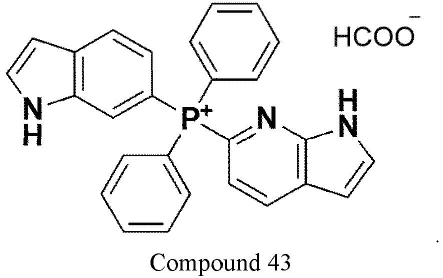

### Example 29 Compound 46

Compound 46 was synthesized using conventional synthetic methods.

The structure of compound 46 was as follows:

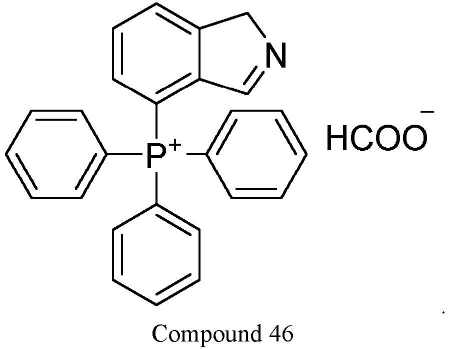

### Example 30 Compound 47

Compound 47 was synthesized using conventional synthetic methods.

The structure of compound 47 was as follows:

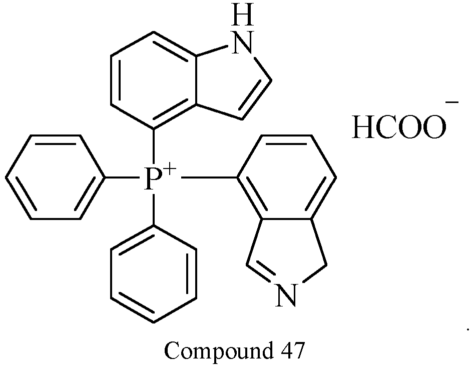

### Example 31 Compound 48

Compound 48 was synthesized using conventional synthetic methods.

The structure of compound 48 was as follows:

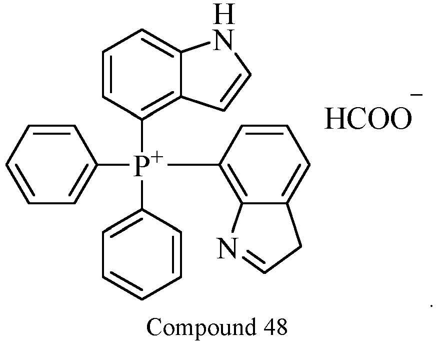

### Example 32 Compound 49

Compound 49 was synthesized using conventional synthetic methods.

The structure of compound 49 was as follows:

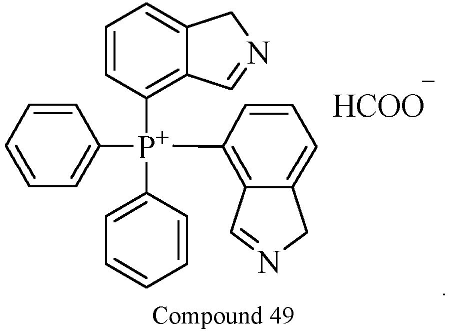

### Example 33 Compound 50

Compound 50 was synthesized using conventional synthetic methods.

The structure of compound 50 was as follows:

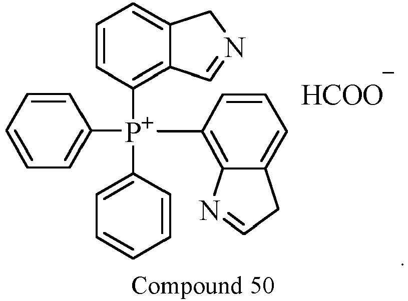

### Example 34 Compound 51

Compound 51 was synthesized using conventional synthetic methods.

The structure of compound 51 was as follows:

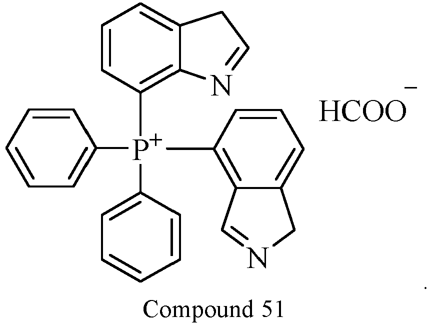

### Example 35 Compound 52

Compound 52 was synthesized using conventional synthetic methods.

The structure of compound 52 was as follows:

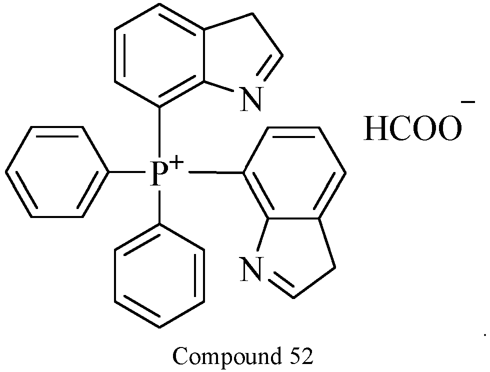

### Example 36 Compound 53

Compound 33 was synthesized using conventional synthetic methods.

The structure of compound 53 was as follows:

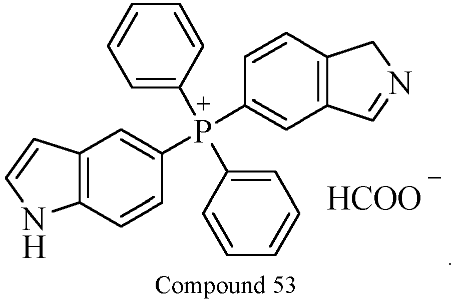

### Example 37 Compound 54

Compound 54 was synthesized using conventional synthetic methods.

The structure of compound 54 was as follows:

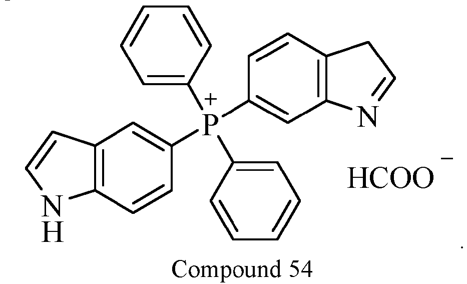

### Example 38 Compound 55

Compound 55 was synthesized using conventional synthetic methods.

The structure of compound 55 was as follows:

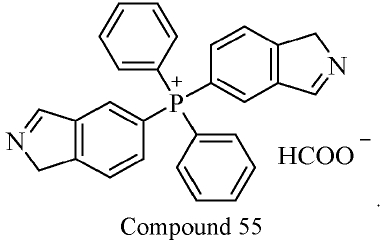

### Example 39 Compound 56

Compound 56 was synthesized using conventional synthetic methods.

The structure of compound 56 was as follows:

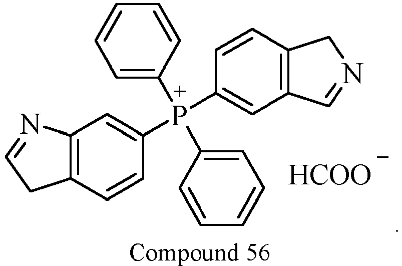

### Example 40 Compound 57

Compound 57 was synthesized using conventional synthetic methods.

The structure of compound 57 was as follows:

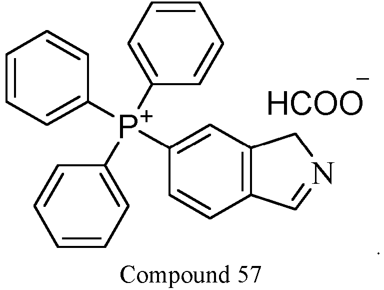

### Example 41 Compound 59

Compound 59 was synthesized using conventional synthetic methods.

The structure of compound 59 was as follows:

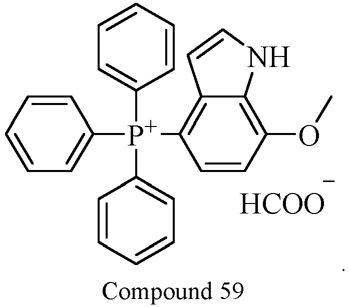

### Example 42 Compound 60

Compound 60 was synthesized using conventional synthetic methods.

The structure of compound 60 was as follows:

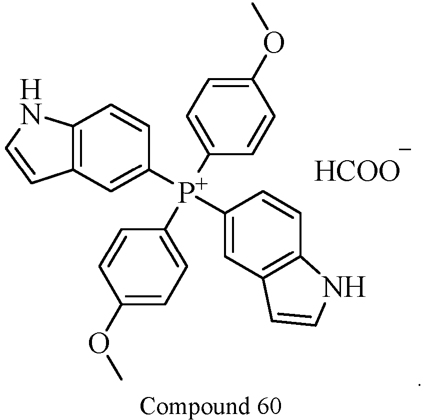

### Example 43 Compound 61

Compound 61 was synthesized using conventional synthetic methods.

The structure of compound 61 was as follows:

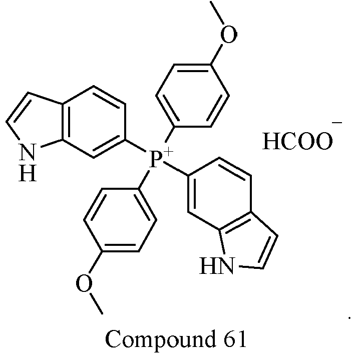

### Example 44 Compound 62

Compound 62 was synthesized using conventional synthetic methods.

The structure of compound 62 was as follows:

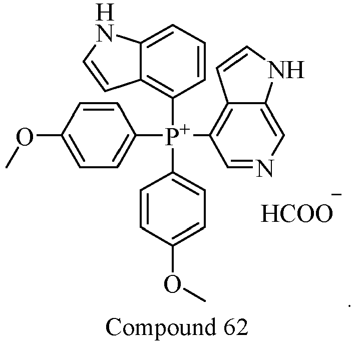

### Example 45 Compound 63

Compound 63 was synthesized using conventional synthetic methods.

The structure of compound 63 was as follows:

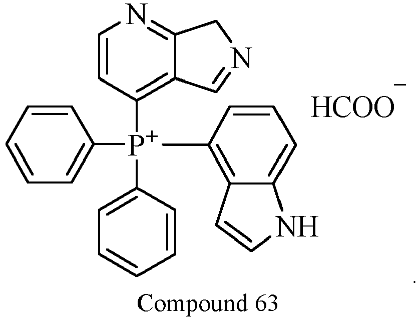

### Example 46 Compound 64

Compound 64 was synthesized using conventional synthetic methods.

The structure of compound 64 was as follows:

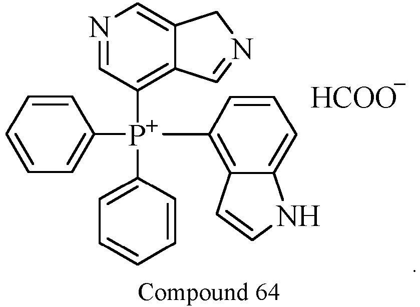

### Example 47 Compound 65

Compound 65 was synthesized using conventional synthetic methods.

The structure of compound 65 was as follows:

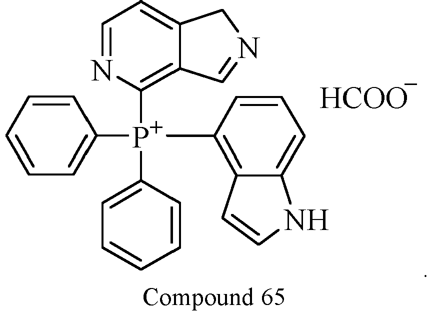

### Example 48 Compound 66

Compound 66 was synthesized using conventional synthetic methods.

The structure of compound 66 was as follows:

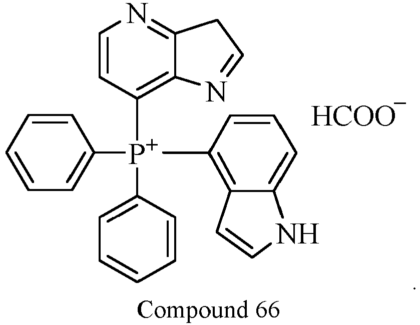

### Example 49 Compound 67

Compound 67 was synthesized using conventional synthetic methods.

The structure of compound 67 was as follows:

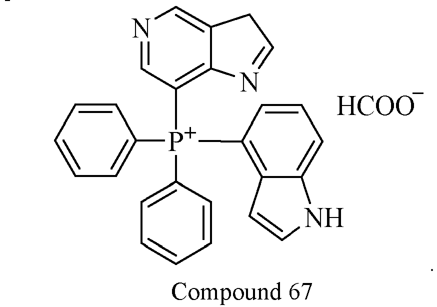

### Example 50 Compound 68

Compound 68 was synthesized using conventional synthetic methods.

The structure of compound 68 was as follows:

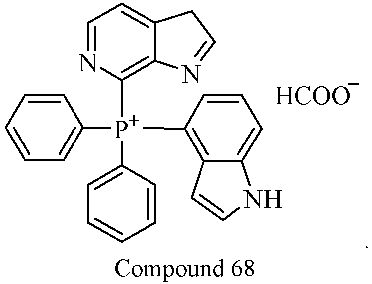

### Example 51 Compound 69

Compound 69 was synthesized using conventional synthetic methods.

The structure of compound 69 was as follows:

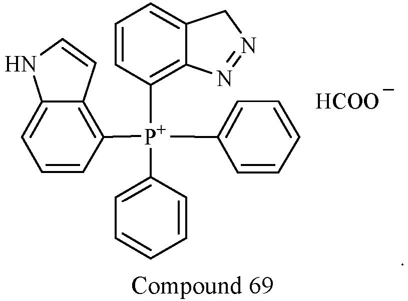

### Example 52 Compound 73

Compound 73 was synthesized using conventional synthetic methods.

The structure of compound 73 was as follows:

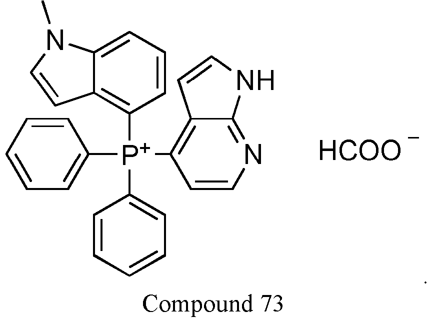

### Example 53 Compound 74

Compound 74 was synthesized using conventional synthetic methods.

The structure of compound 74 was as follows:

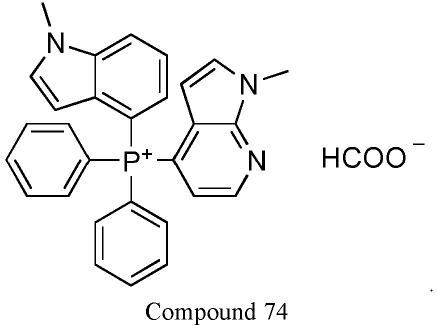

### Example 54 Compound 76

Compound 76 was synthesized using conventional synthetic methods.

The structure of compound 76 was as follows:

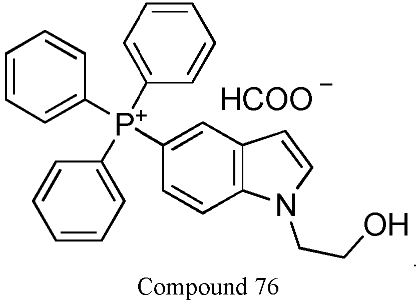

### Example 55 Compound 80

Compound 80 was synthesized using conventional synthetic methods.

The structure of compound 80 was as follows:

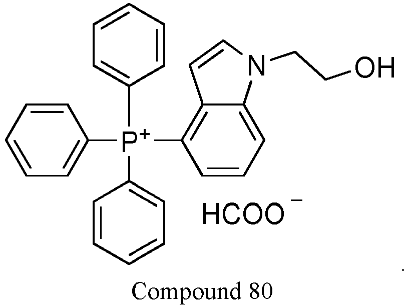

### Example 56 Compound 83

Compound 83 was synthesized using conventional synthetic methods.

The structure of compound 83 was as follows:

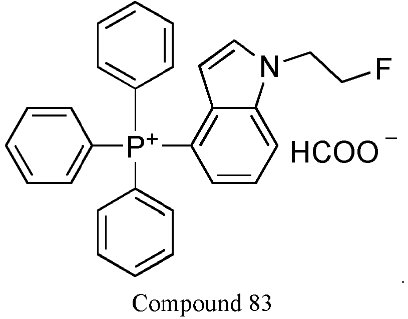

### Example 57 Compound 84

Compound 84 was synthesized using conventional synthetic methods.

The structure of compound 84 was as follows:

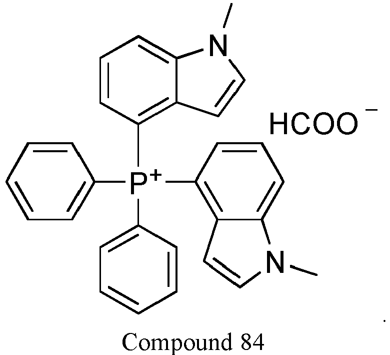

### Example 58 Compound 85

Compound 85 was synthesized using conventional synthetic methods.

The structure of compound 85 was as follows:

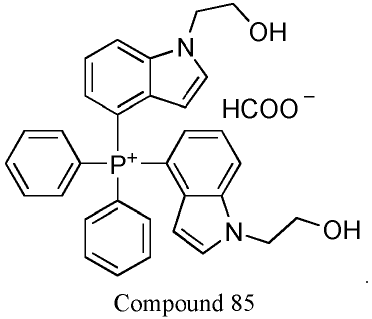

### Example 59 Compound 87

Compound 87 was synthesized using conventional synthetic methods.

The structure of compound 87 was as follows:

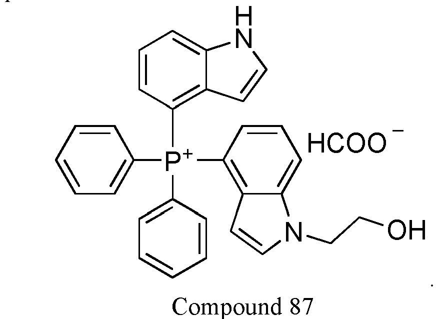

### Example 60 Compound AB35527

The structure of compound AB35527 was as follows:

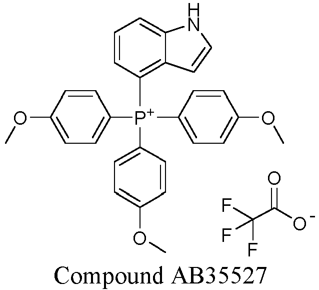

The synthetic route of compound AB35527 was as follows:

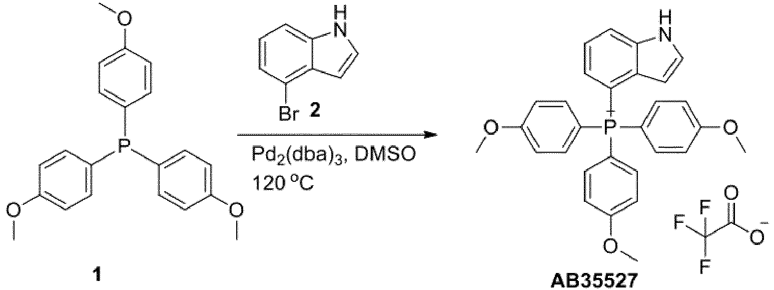

Compound 1 (200 mg, 0.57 mmol, 1 eq) was dissolved in dimethyl sulfoxide (3 mL) in a sealed tube, compound 2 (111 mg, 0.57 mmol, 1 eq) and tris(dibenzylideneacetone)dipalladium (52 mg, 0.057 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.1% trifluoroacetic acid) to afford compound AB35527.

Compound AB35527:

MS-ESI: calculated [M]⁺: 468.17; found [M]⁺: 468.20.

¹H NMR (400 MHz, DMSO-d6) δ 12.09 (s, 1H), 7.95 (d, J= 8.0 Hz, 1H), 7.52-7.50 (m, 7H), 7.26-7.24 (m, 1H), 7.02-6.95 (m, 6H), 7.05-7.00 (m, 1H), 5.53 (s, 1H), 3.85 (s, 9H).

### Example 61 Compound AB35529

The structure of compound AB35529 was as follows:

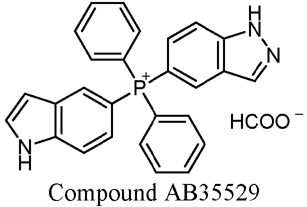

The synthetic route of compound AB35529 was as follows:

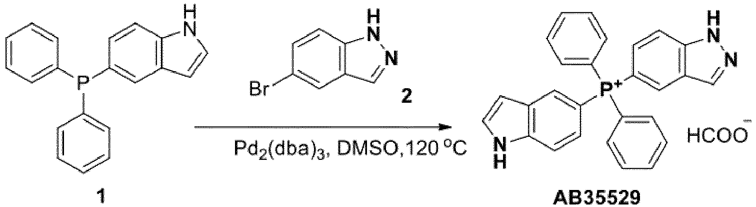

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35529.

Compound AB35529:

MS-ESI: calculated [M]⁺: 418.15; found [M]⁺: 418.15.

¹H NMR (400 MHz, DMSO-d6) δ 12.40 (s, 1H), 8.59 (s, 1H), 8.34 (s, 1H), 8.15 (d, *J*= 12 Hz, 1H), 7.98-7.73 (m, 14H), 7.65 (d, *J=* 4 Hz, 1H), 7.58-7.55 (m, 1H), 7.38-7.32 (m, 1H), 6.67 (d, *J*= 4 Hz, 1H).

### Example 62 Compound AB35537

The structure of compound AB35537 was as follows:

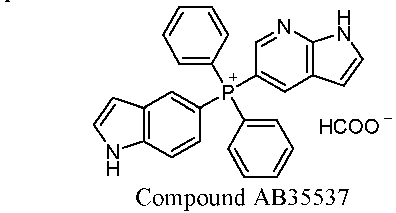

The synthetic route of compound AB35537 was as follows:

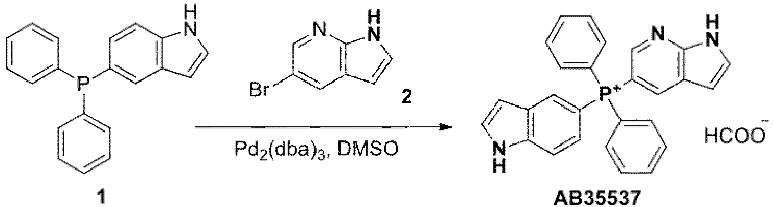

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35537.

Compound AB35537:

MS-ESI: calculated [M]⁺: 418.15; found [M]⁺: 418.15.

¹H NMR (400 MHz, DMSO-d6) δ 12.29 (s, 1H), 8.53 (s, 1H), 8.37-8.27 (m, 2H), 8.79-7.80 (m, 3H), 7.77-7.73 (m, 11H), 7.62 (s, 1H), 7.34-7.32 (m, 1H), 6.67-6.64 (m, 2H).

### Example 63 Compound AB35538

The structure of compound AB35538 was as follows:

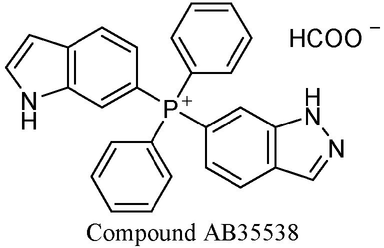

The synthetic route of compound AB35538 was as follows:

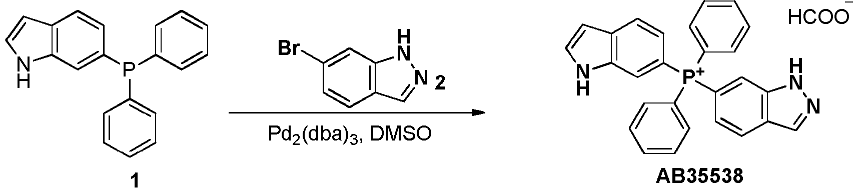

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35538.

Compound AB35538:

MS-ESI: calculated [M]⁺: 418.15; found [M]⁺: 418.15.

¹H NMR (400 MHz, DMSO-d6) δ 12.37 (s, 1H), 8.52 (s, 1H), 8.32 (s, 1H), 8.16-8.13 (m, 1H), 7.92-7.87 (m, 4H), 7.83-7.75 (m, 10H), 7.63 (d, *J=* 4.0 Hz, 1H), 7.37-7.32 (m, 2H).

### Example 64 Compound AB35539

The structure of compound AB35539 was as follows:

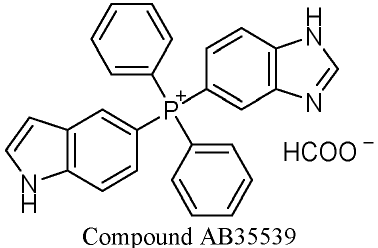

The synthetic route of compound AB35539 was as follows:

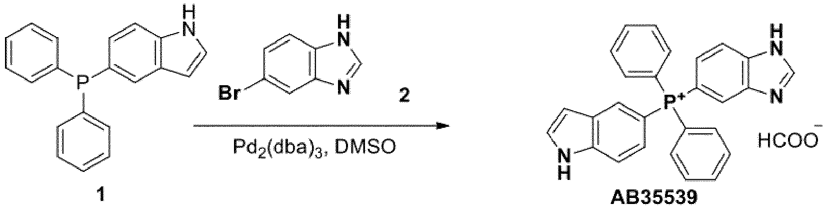

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35539.

Compound AB35539:

MS-ESI: calculated [M]⁺: 418.15; found [M]⁺: 418.10.

¹H NMR (400 MHz, DMSO-d6) δ 12.39 (s, 1H), 8.55 (s, 1H), 8.47 (m, 1H), 7.99-7.96 (m, 1H), 7.91-7.74 (m, 14H), 7.65-7.64 (d, *J=* 4.0 Hz, 1H), 7.46-7.33 (m, 2H), 6.67 (d, *J=* 4.0 Hz, 1H),.

### Example 65 Compound AB35548

The structure of compound AB35548 was as follows:

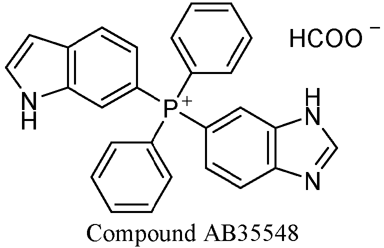

The synthetic route of compound AB35548 was as follows:

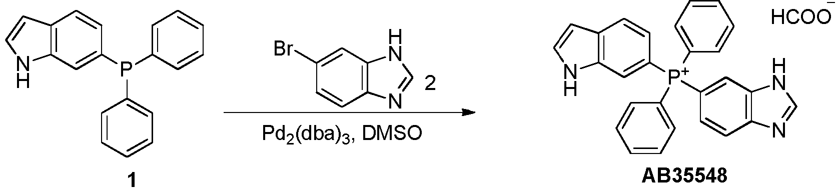

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35548.

Compound AB35548:

MS-ESI: calculated [M]⁺: 418.15; found [M]⁺: 418.15.

¹H NMR (400 MHz, DMSO-d6) δ 12.41 (s, 1H), 8.57 (s, 1H), 8.50 (s, 1H), 8.02-7.67 (m, 15H), 7.67 (d, *J=* 4.0 Hz, 1H), 7.49-7.36 (m, 2H), 7.70 (d, *J=* 4.0 Hz, 1H).

### Example 66 Compound AB35549

The structure of compound AB35549 was as follows:

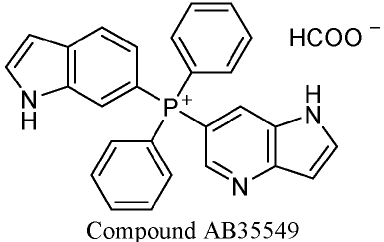

The synthetic route of compound AB35549 was as follows:

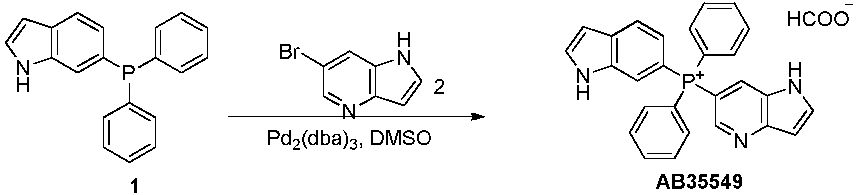

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35549.

Compound AB35549:

MS-ESI: calculated [M]⁺: 418.15; found [M]⁺: 418.10.

¹H NMR (400 MHz, DMSO-d6) δ 12.49 (s, 1H), 8.54-8.50 (m, 3H), 8.12-8.08 (m, 2H), 7.94-7.91 (m, 3H), 7.82-7.79 (m, 10H), 7.66 (d, *J=* 4.0 Hz, 1H), 7.41-7.36 (m, 1H), 6.82 (d, *J*= 4.0 Hz, 1H), 6.68 (d, *J=* 4.0 Hz, 1H).

### Example 67 Compound AB35563

The structure of compound AB35563 was as follows:

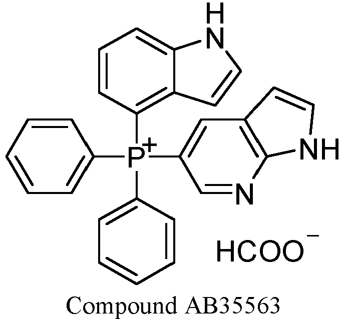

The synthetic route of compound AB35563 was as follows:

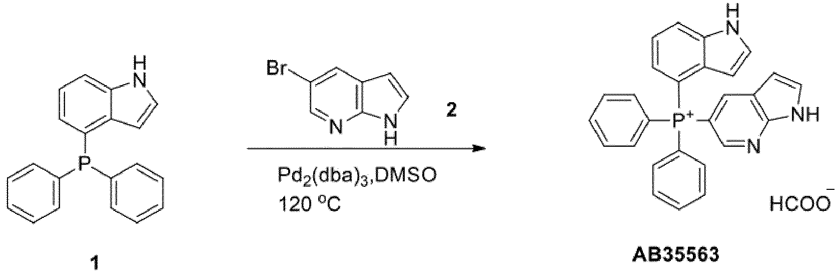

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N2, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35563.

Compound AB35563:

MS-ESI: calculated [M]⁺: 418.15; found [M]⁺: 418.10.

¹H NMR (400 MHz, DMSO-d6) δ 8.57 (s, 1H), 8.37-8.31 (m, 2H), 8.05 (d, *J=* 8.0 Hz, 1H), 7.94-7.91 (m, 2H), 7.79-7.75 (m, 10H), 7.56 (d, *J=* 4.0 Hz, 1H), 7.44-7.41 (m, 1H), 7.11-7.05 (m, 1H), 6.68 (d, *J=* 4.0 Hz, 1H), 5.56 (d, *J=* 4.0 Hz, 1H).

### Example 68 Compound AB35564

The structure of compound AB35564 was as follows:

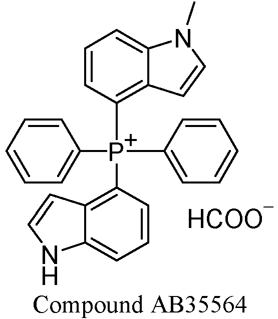

The synthetic route of compound AB35564 was as follows:

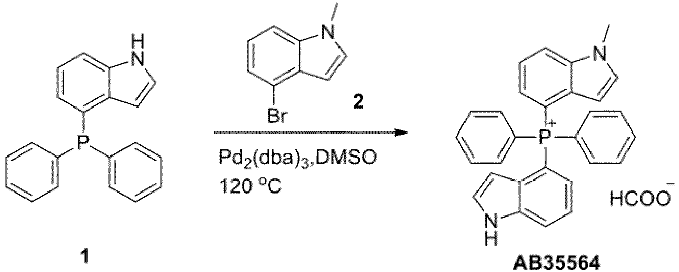

Compound 1 (100 mg, 0.33 mmol, 1 eq) was dissolved in dimethyl sulfoxide (3 mL) in a sealed tube, compound 2 (69 mg, 0.33 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35564.

Compound AB35564:

MS-ESI: calculated [M]⁺: 431.17; found [M]⁺: 431.15.

¹H NMR (400 MHz, DMSO-d6) δ 12.66 (s, 1H), 8.57 (s, 1H), 8.09-8.01 (m, 2H), 7.91-7.87 (m, 2H), 7.74-7.68 (m, 8H), 7.51-7.44 (m, 3H), 7.41-7.36 (m, 1H), 7.25-7.14 (m, 2H), 5.54 (m, 2H), 3.89 (m, 3H).

### Example 69 Compound AB35569

The structure of compound AB35569 was as follows:

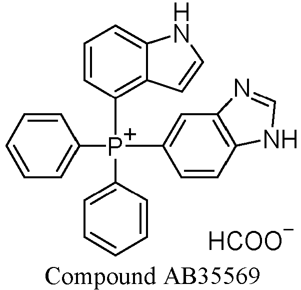

The synthetic route of compound AB35569 was as follows:

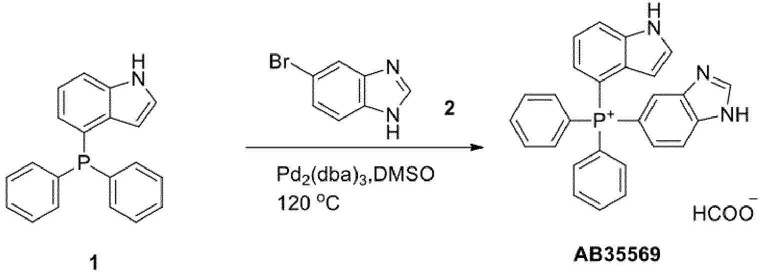

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35569.

Compound AB35569:

MS-ESI: calculated [M]⁺: 418.15; found [M]⁺: 418.05.

¹H NMR (400 MHz, DMSO-d6) δ 8.55 (s, 1H), 8.47 (s, 1H), 8.03 (d, *J=* 8.0 Hz, 1H), 7.92-7.84 (m, 4H), 7.77-7.72 (m, 8H), 7.55 (d, *J=* 4.0 Hz, 1H), 7.45-7.39 (m, 2H), 7.10-7.04 (m, 1H), 5.54 (d, *J=* 4.0 Hz, 1H).

### Example 70 Compound AB35570

The structure of compound AB35570 was as follows:

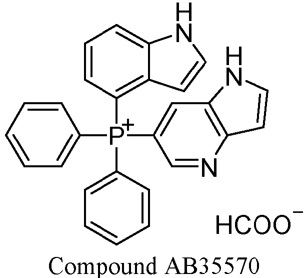

The synthetic route of compound AB35570 was as follows:

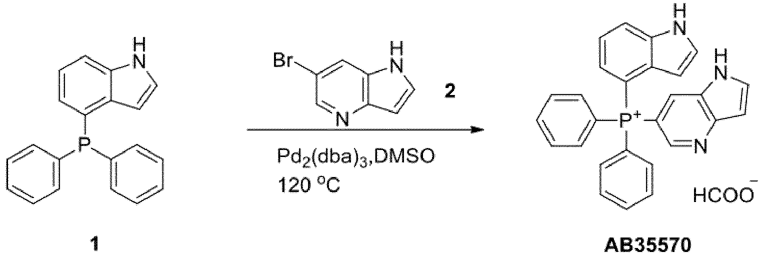

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35570.

Compound AB35570:

MS-ESI: calculated [M]⁺: 418.15; found [M]⁺: 418.10.

¹H NMR (400 MHz, DMSO-d6) δ 8.56 (s, 1H), 8.46 (d, *J=* 4.0 Hz, 1H), 8.14-8.06 (m, 3H), 7.96-7.91 (m, 2H), 7.79-7.77 (m, 9H), 7.55 (s, 1H), 7.43-7.40 (m, 1H), 7.11-7.05 (m, 1H), 6.79 (s, 1H), 5.57 (s, 1H).

### Example 71 Compound AB35571

The structure of compound AB35571 was as follows:

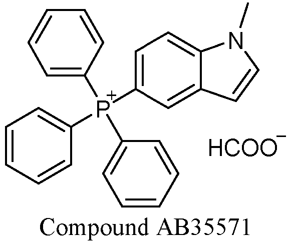

The synthetic route of compound AB35571 was as follows:

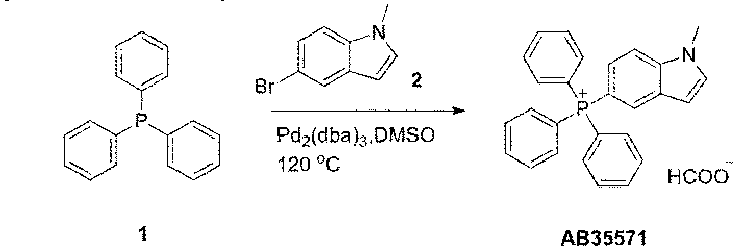

Compound 1 (100 mg, 0.38 mmol, 1 eq) was dissolved in dimethyl sulfoxide (3 mL) in a sealed tube, compound 2 (88 mg, 0.42 mmol, 1.1 eq) and tris(dibenzylideneacetone)dipalladium (7 mg, 0.008 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35571.

Compound AB35571:

MS-ESI: calculated [M]⁺: 392.16; found [M]⁺: 392.10.

¹H NMR (400 MHz, DMSO-d6) δ 8.53 (s, 1H), 7.96-7.93 (m, 3H), 7.89-7.86 (m, 2H), 7.82-7.71 (m, 12H), 7.66 (d, *J=* 4.0 Hz, 1H), 7.43-7.37 (m, 1H), 6.70 (d, *J=* 4.0 Hz, 1H), 3.91 (s, 3H).

### Example 72 Compound AB35572

The structure of compound AB35572 was as follows:

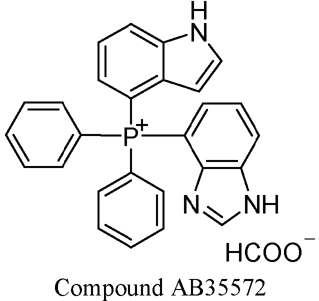

The synthetic route of compound AB35572 was as follows:

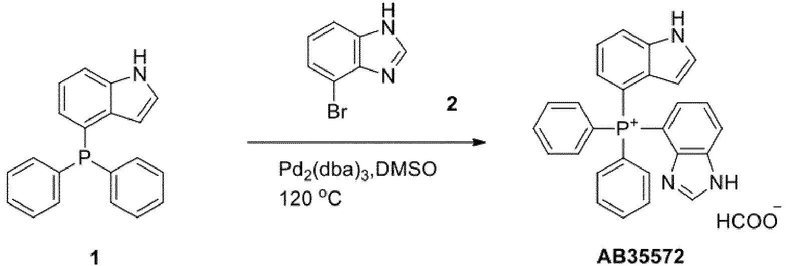

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35572.

Compound AB35572:

MS-ESI: calculated [M]⁺: 418.15; found [M]⁺: 418.10.

¹H NMR (400 MHz, DMSO-d6) δ 12.50 (s, 1H), 8.54 (s, 1H), 8.25 (s, 1H), 8.11 (d, *J*= 8.0 Hz, 1H), 7.98 (d, *J=* 8.0 Hz, 1H), 7.85-7.81 (m, 2H), 7.74-7.66 (m, 9H), 7.48 (d, *J*= 4.0 Hz, 1H), 7.39-7.33 (m, 2H), 7.12-7.03 (m, 2H), 5.49 (d, *J=* 4.0 Hz, 1H).

### Example 73 Compound AB35573

The structure of compound AB35573 was as follows:

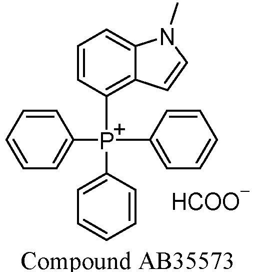

The synthetic route of compound AB35573 was as follows:

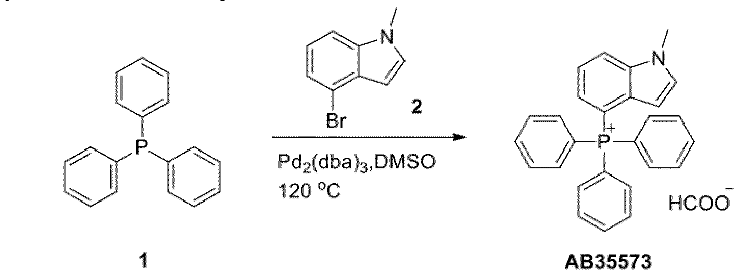

Compound 1 (100 mg, 0.38 mmol, 1 eq) was dissolved in dimethyl sulfoxide (3 mL) in a sealed tube, compound 2 (88 mg, 0.42 mmol, 1.1 eq) and tris(dibenzylideneacetone)dipalladium (7 mg, 0.008 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35573.

Compound AB35573:

MS-ESI: calculated [M]⁺: 392.16; found [M]⁺: 392.10.

¹H NMR (400 MHz, DMSO-d6) δ 8.54 (s, 1H), 8.13 (d, *J=* 8.0 Hz, 1H), 7.96-7.92 (m, 3H), 7.78-7.70 (m, 12H), 7.57-7.49 (m, 2H), 7.12-7.06 (m, 1H), 5.52 (d, *J=* 4.0 Hz, 1H), 3.92 (s, 3H).

### Example 74 Compound AB35574

The structure of compound AB35574 was as follows:

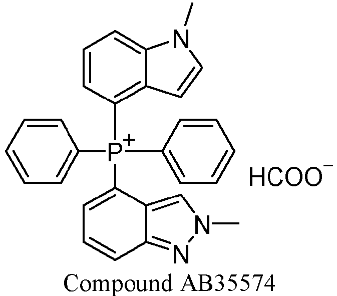

The synthetic route of compound AB35574 was as follows:

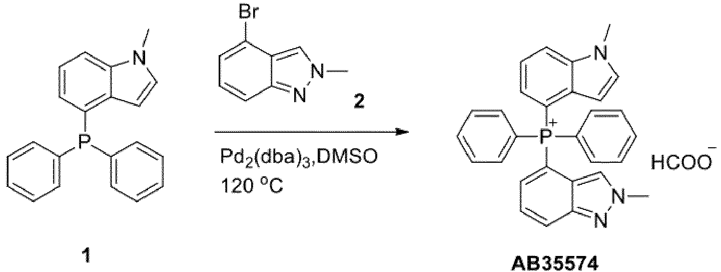

Compound 1 (100 mg, 0.32 mmol, 1 eq) was dissolved in dimethyl sulfoxide (3 mL) in a sealed tube, compound 2 (70 mg, 0.35 mmol, 1.1 eq) and tris(dibenzylideneacetone)dipalladium (6 mg, 0.006 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried, the crude product was purified by reversed phase preparative chromatography (acetonitrile/water+0.01% formic acid) to afford compound AB35574.

Compound AB35574:

MS-ESI: calculated [M]⁺: 446.18; found [M]⁺: 446.05.

¹H NMR (400 MHz, DMSO-d6) δ 8.50 (s, 1H), 8.23-8.19 (m, 1H), 8.11 (d, *J=* 8.0 Hz, 1H), 7.93-7.90 (m, 2H), 7.76-7.71(m, 8H), 7.64 (s, 1H), 7.56-7.46 (m, 3H), 7.42-7.36 (m, 1H), 7.27-7.21 (m, 1H), 5.60 (d, *J=* 4.0 Hz, 1H), 4.05 (s, 3H), 3.90 (s, 3H).

### Example 75 Compound AB35576

The structure of compound AB35576 was as follows:

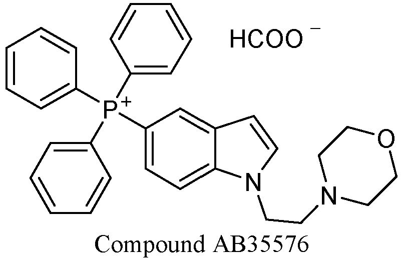

The synthetic route of compound AB35576 was as follows:

### Step (1):

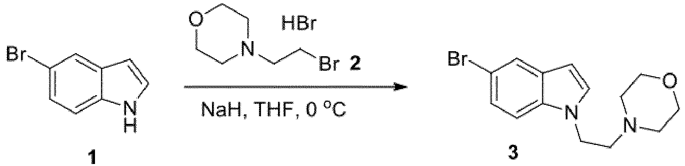

Compound 1 (500 mg, 2.55 mmol, 1 eq) was dissolved in tetrahydrofuran (10 mL) in a sealed tube, and sodium hydride (153 mg, 3.82 mmol, 1.5 eq, 60% in mineral oil) was added at 0 ° C. The mixture was reacted for 0.5 h, a solution of compound 2 (771 mg, 2.81 mmol, 1.1 eq) in tetrahydrofuran (5 mL) was added to the above raction mixture at 0 °C, and the mixture was reacted for 1 h. The raction mixture was quenched with saturated ammonium chloride aqueous solution and then extracted with ethyl acetate. The organic phase was dried over anhydrous sodium sulfate, filtered and rotary dried, the crude product was purified by fast chromatography (dichloromethane: methanol=20:1) to afford compound 3.

### Step (2):

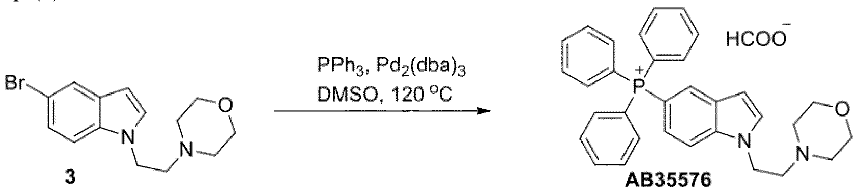

Compound 3 (100 mg, 0.32 mmol, 1 eq) was dissolved in dimethyl sulfoxide (3 mL) in a sealed tube, triphenylphosphine (84 mg, 0.32 mmol, 1 eq) and tris(dibenzylideneacetone)dipalladium (7 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35576.

Compound AB35576:

MS-ESI: calculated [M]⁺: 491.22; found [M]⁺: 491.20.

¹H NMR (400 MHz, DMSO-d6) δ 8.34 (s, 1H), 7.96-7.93 (m, 4H), 7.77-7.71 (m, 14H), 7.40-7.36 (m, 1H), 6.70 (d, *J=* 4.0 Hz, 1H), 4.43-4.40 (m, 2H), 3.54-3.51 (m, 4H), 2.70-2.67 (m, 2H), 2.45-2.43 (m, 4H).

### Example 76 Compound AB35577

The structure of compound AB35577 was as follows:

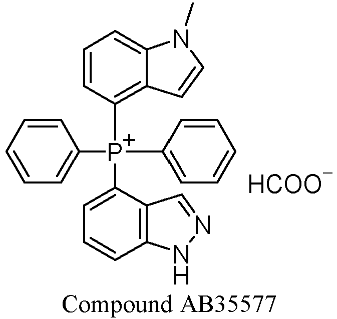

The synthetic route of compound AB35577 was as follows:

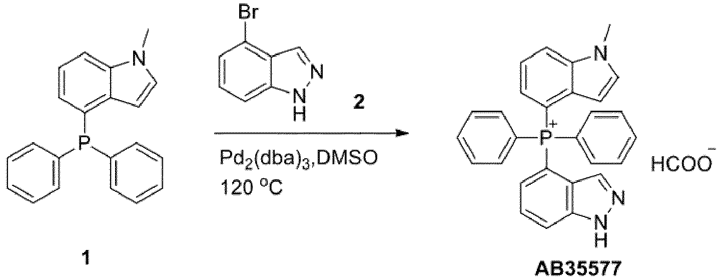

Compound 1 (100 mg, 0.32 mmol, 1 eq) was dissolved in dimethyl sulfoxide (3 mL) in a sealed tube, compound 2 (62 mg, 0.32 mmol, 1 eq) and tris(dibenzylideneacetone)dipalladium (6 mg, 0.006 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35577.

Compound AB35577:

MS-ESI: calculated [M]⁺: 432.16; found [M]⁺: 432.15.

¹H NMR (400 MHz, DMSO-d6) δ 8.44 (s, 1H), 8.21-8.11 (m, 2H), 7.95-7.91 (m, 2H), 7.77-7.65 (m, 8H), 7.69-7.65 (m, 1H), 7.53-7.48 (m, 2H), 7.44-7.38 (m, 1H), 7.25-7.20 (m, 1H), 7.03 (m, 1H), 5.58 (d, J= 4.0 Hz, 1H), 3.90 (s, 3H).

### Example 77 Compound AB35578

The structure of compound AB35578 was as follows:

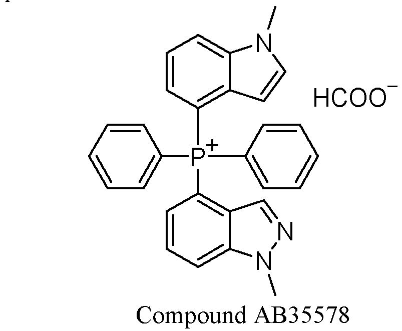

The synthetic route of compound AB35578 was as follows:

### Step (1):

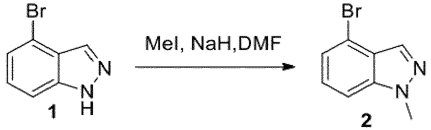

Compound 1 (500 mg, 2.55 mmol, 1 eq) was dissolved in N, N-dimethylformamide (10 mL) in a sealed tube, and sodium hydride (153 mg, 3.82 mmol, 1.5 eq, 60% in mineral oil) was added at 0 °C. The mixture was reacted for 0.5 h, a solution of MeI (771 mg, 2.81 mmol, 1.1 eq) in N, N-dimethylformamide (5 mL) was added to the above raction mixture at 0 °C, and the mixture was reacted for 1 h. The raction mixture was quenched with saturated ammonium chloride aqueous solution and then extracted with ethyl acetate. The organic phase was dried over anhydrous sodium sulfate, filtered and rotary dried, the crude product was purified by fast chromatography (dichloromethane: methanol=20:1) to afford compound 2.

### Step (2):

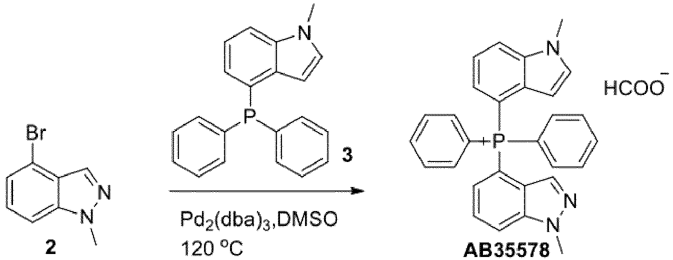

Compound 2 (100 mg, 0.47 mmol, 1 eq) was dissolved in dimethyl sulfoxide (3 mL) in a sealed tube, compound 3 (149 mg, 0.47 mmol, 1 eq) and tris(dibenzylideneacetone)dipalladium (9 mg, 0.009 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35578.

Compound AB35578:

MS-ESI: calculated [M]⁺: 446.18; found [M]⁺: 446.95.

¹H NMR (400 MHz, DMSO-d6) δ 8.54 (s, 1H), 8.23-8.11 (m, 2H), 7.94-7.90 (m, 2H), 7.76-7.71 (m, 8H), 7.66 (s, 1H), 7.55-7.47 (m, 3H), 7.42-7.36 (m, 1H), 7.27-7.21 (m, 1H), 5.60 (d, J= 4.0 Hz, 1H), 4.05 (s, 3H), 3.90 (s, 3H).

### Example 78 Compound AB35579

The structure of compound AB35579 was as follows:

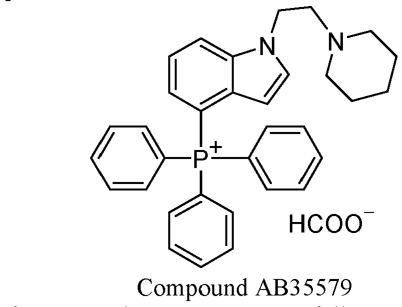

The synthetic route of compound AB35579 was as follows:

### Step (1):

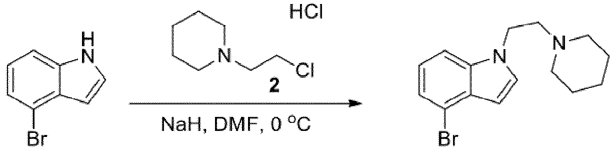

Compound 1 (500 mg, 2.55 mmol, 1 eq) was dissolved in N, N-dimethylformamide (10 mL) in a sealed tube, and sodium hydride (153 mg, 3.83 mmol, 1.5 eq, 60% in mineral oil) was added at 0 °C. The mixture was reacted for 0.5 h, a solution of compound 2 (516 mg, 2.81 mmol, 1.1 eq) in N, N-dimethylformamide (5 mL) was added to the above raction mixture at 0 °C, and the mixture was reacted for 1 h. The raction mixture was quenched with saturated ammonium chloride aqueous solution and then extracted with ethyl acetate. The organic phase was dried over anhydrous sodium sulfate, filtered and rotary dried, the crude product was purified by fast chromatography (dichloromethane: methanol=20:1) to afford compound 3.

### Step (2):

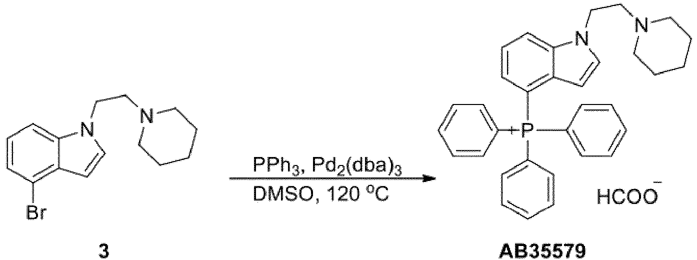

Compound 3 (100 mg, 0.32 mmol, 1 eq) was dissolved in dimethyl sulfoxide (3 mL) in a sealed tube, triphenylphosphine (84 mg, 0.32 mmol, 1 eq) and tris(dibenzylideneacetone)dipalladium (7 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35579.

Compound AB35579:

MS-ESI: calculated [M]⁺: 489.25; found [M]⁺: 489.20.

¹H NMR (400 MHz, DMSO-d6) δ 8.36 (s, 1H), 8.18 (d, *J=* 8.0 Hz, 1H), 7.95-7.91 (m, 3H), 7.78-7.69 (m, 12H), 6.62 (d, *J=* 4.0 Hz, 1H), 7.49-7.46 (m, 1H), 7.10-7.04 (m, 1H), 5.53 (d, *J*= 4.0 Hz, 1H), 4.40-4.37 (m, 2H), 2.66-2.62 (m, 2H), 2.37-2.34 (m, 4H), 1.45-1.32 (m, 6 H).

### Example 79 Compound AB35580

The structure of compound AB35580 was as follows:

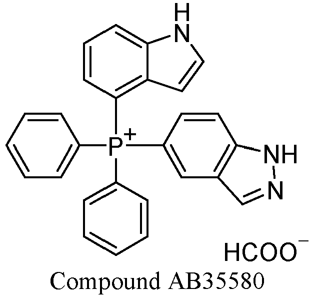

The synthetic route of compound AB35580 was as follows:

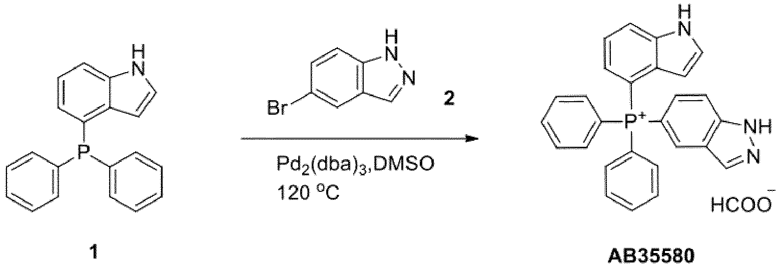

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35580.

Compound AB35580:

MS-ESI: calculated [M]⁺: 418.15; found [M]⁺: 418.10.

¹H NMR (400 MHz, DMSO-d6) δ 8.59 (s, 1H), 8.32 (s, 1H), 8.20 (d, *J=* 12.0 Hz, 1H), 8.05 (d, *J=* 4.0 Hz, 1H), 7.97-7.90 (m, 3H), 7.77-7.72 (m, 9H), 7.56-7.54 (m, 2H), 7.44-7.40 (m, 1H), 7.10-7.04 (m, 1H), 5.54 (d, *J=* 4.0 Hz, 1H).

### Example 80 Compound AB35581

The structure of compound AB35581 was as follows:

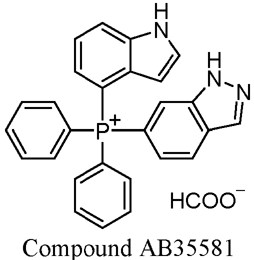

The synthetic route of compound AB35581 was as follows:

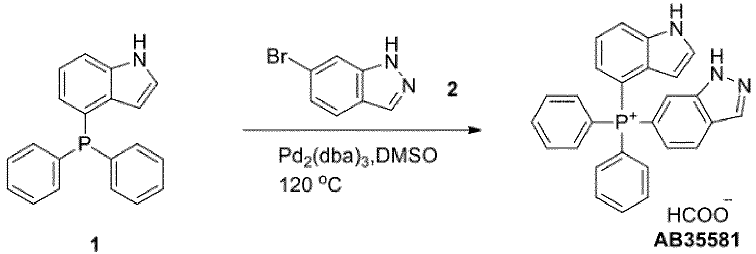

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35581.

Compound AB35581:

MS-ESI: calculated [M]⁺: 418.15; found [M]⁺: 418.10.

¹H NMR (400 MHz, DMSO-d6) δ 8.54 (s, 1H), 8.35 (s, 1H), 8.17-8.15 (m, 1H), 8.06 (d, *J=* 4.0 Hz, 1H), 7.93-7.89 (m, 3H), 7.79-7.76 (m, 8H), 7.56 (d, *J=* 4.0 Hz, 1H), 7.43-7.41 (m, 1H), 7.38-7.33 (m, 1H), 7.13-7.07 (m, 1H), 5.56 (d, *J=* 4.0 Hz, 1H).

### Example 81 Compound AB35584

The structure of compound AB35584 was as follows:

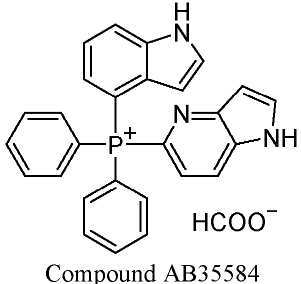

The synthetic route of compound AB35584 was as follows:

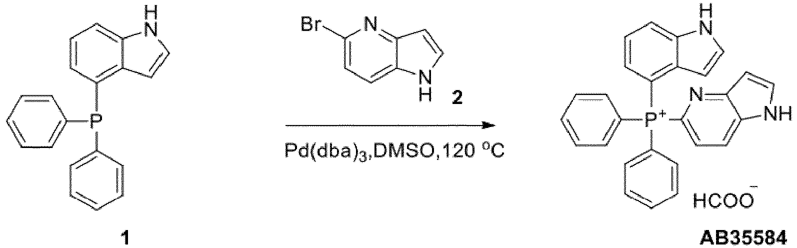

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35584.

Compound AB35584:

MS-ESI: calculated [M]⁺: 418.15; found [M]⁺: 418.10.

¹H NMR (400 MHz, DMSO-d6) δ 8.54 (s, 1H), 8.12-8.08 (m, 1H), 8.04-8.02 (m, 2H), 7.91-7.88 (m, 2H), 7.82-7.74 (m, 8H), 7.53 (d, *J=* 4.0 Hz, 1H), 7.45-7.38 (m,2H), 7.09-7.03 (m, 1H), 6.84 (d, *J=* 4.0 Hz, 1H), 5.56 (d, *J=* 4.0 Hz, 1H).

### Example 82 Compound AB35585

The structure of compound AB35585 was as follows:

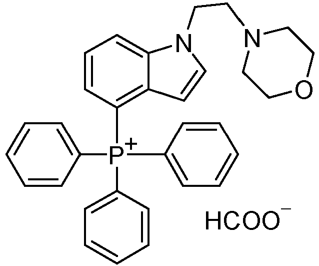

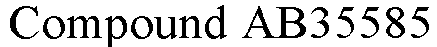

The synthetic route of compound AB35585 was as follows:

### Step (1):

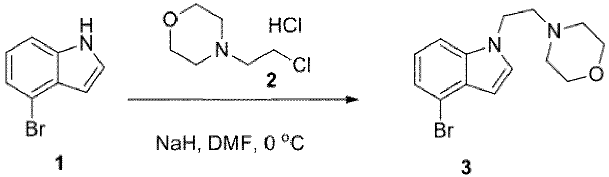

Compound 1 (500 mg, 2.55 mmol, 1 eq) was dissolved in N, N-dimethylformamide (10 mL) in a sealed tube, and sodium hydride (153 mg, 3.83 mmol, 1.5 eq, 60% in mineral oil) was added at 0 °C. The mixture was reacted for 0.5 h, a solution of compound 2 (523 mg, 2.81 mmol, 1.1 eq) in N, N-dimethylformamide (5 mL) was added to the above raction mixture at 0 °C, and the mixture was reacted for 1 h. The raction mixture was quenched with saturated ammonium chloride aqueous solution and then extracted with ethyl acetate. The organic phase was dried over anhydrous sodium sulfate, filtered and rotary dried, the crude product was purified by fast chromatography (dichloromethane: methanol=20:1) to afford compound 3.

### Step (2):

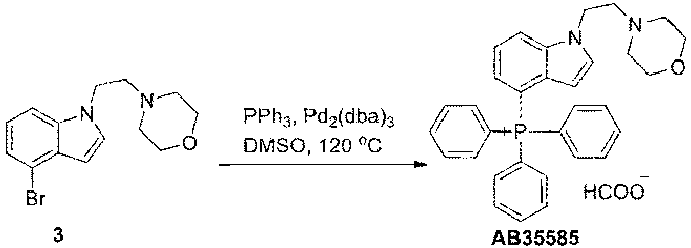

Compound 3 (140 mg, 0.45 mmol, 1 eq) was dissolved in dimethyl sulfoxide (3 mL) in a sealed tube, triphenylphosphine (119 mg, 0.45 mmol, 1 eq) and tris(dibenzylideneacetone)dipalladium (8 mg, 0.009 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35585.

Compound AB35585:

MS-ESI: calculated [M]⁺: 491.22; found [M]⁺: 489.20.

¹H NMR (400 MHz, DMSO-d6) δ 8.42 (s, 1H), 8.20 (d, *J=* 8.0 Hz, 1H), 7.95-7.92 (m, 3H), 7.79-7.70 (m, 12H), 7.64 (d, *J=* 4.0 Hz, 1H), 7.51-7.46 (m, 1H), 7.11-7.05 (m, 1H), 5.54 (d, *J*= 4.0 Hz, 1H), 4.43-4.40 (m, 2H), 3.47 (s, 4H), 2.71-2.68 (m, 2H), 2.41-2.38 (m, 4H).

### Example 83 Compound AB35588

The structure of compound AB35588 was as follows:

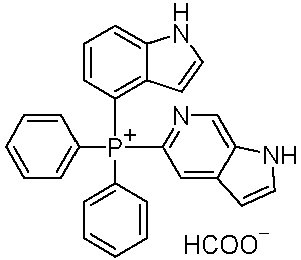

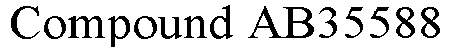

The synthetic route of compound AB35588 was as follows:

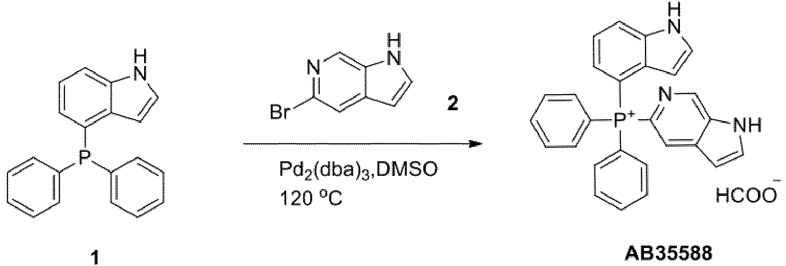

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35588

Compound AB35588:

MS-ESI: calculated [M]⁺: 418.15; found [M]⁺: 418.05.

¹H NMR (400 MHz, DMSO-d6) δ 9.24 (s, 1H), 8.55 (s, 1H), 8.03 (d, *J=* 8.0 Hz, 1H), 7.96 (d, *J=* 8.0 Hz, 1H), 7.90-7.87 (m, 3H), 7.81-7.70 (m, 8H), 7.52 (d, *J=* 4.0 Hz, 1H), 7.44-7.39 (m, 1H), 7.10-7.05 (m, 1H), 6.73 (d, *J=* 4.0 Hz, 1H), 5.57 (d, *J=* 4.0 Hz, 1H).

### Example 84 Compound AB35589

The structure of compound AB35589 was as follows:

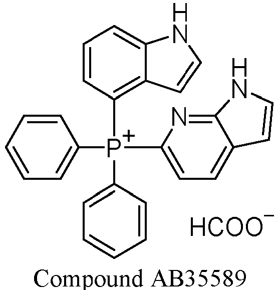

The synthetic route of compound AB35589 was as follows:

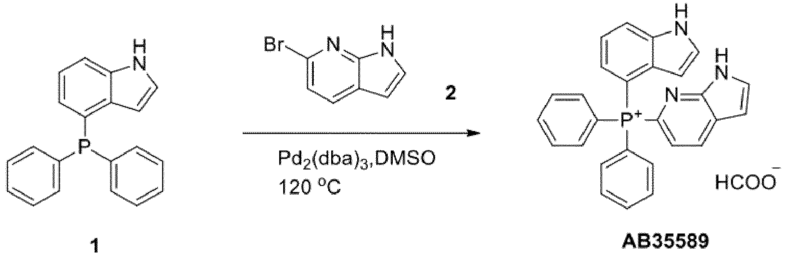

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35589.

Compound AB35589:

MS-ESI: calculated [M]⁺: 418.15; found [M]⁺: 418.00.

¹H NMR (400 MHz, DMSO-d6) δ 8.55 (s, 1H), 8.29-8.26 (m, 1H), 8.04 (d, *J=* 8.0 Hz, 1H), 7.93-7.89 (m, 3H), 7.83-7.73 (m, 8H), 7.53 (d, *J=* 4.0 Hz, 1H), 7.44-7.36 (m, 2H), 7.12-7.06 (m, 1H), 6.72 (d, *J=* 4.0 Hz, 1H), 5.57 (d, *J=* 4.0 Hz, 1H).

### Example 85 Compound AB35596

The structure of compound AB35596 was as follows:

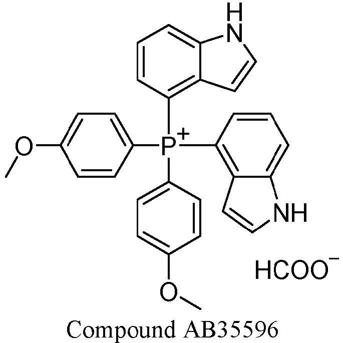

The synthetic route of compound AB35596 was as follows:

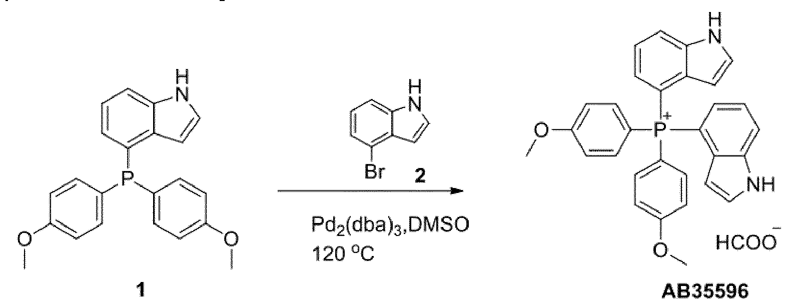

Compound 1 (100 mg, 0.28 mmol, 1 eq) was dissolved in dimethyl sulfoxide (3 mL) in a sealed tube, compound 2 (60 mg, 0.31 mmol, 1.1 eq) and tris(dibenzylideneacetone)dipalladium (5 mg, 0.006 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35596.

Compound AB35596:

MS-ESI: calculated [M]⁺: 477.17; found [M]⁺: 477.55.

¹H NMR (400 MHz, DMSO-d6) δ 12.36 (s, 2H), 8.54 (s, 1H), 7.97 (d, *J=* 8.0 Hz, 2H), 7.61-7.56 (m, 4H), 7.50 (d, *J=* 4.0 Hz, 2H), 7.39-7.35 (m, 2H), 7.28-7.25 (m, 4H), 7.18-7.12 (m, 2H), 5.57 (d, *J=* 4.0 Hz, 2H), 3.87 (s, 6H).

### Example 86 Compound AB35597

The structure of compound AB35597 was as follows:

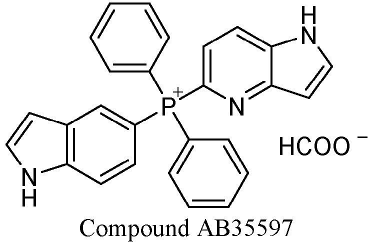

The synthetic route of compound AB35597 was as follows:

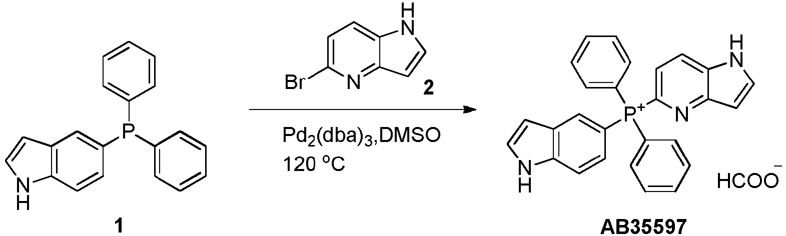

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35597.

Compound AB35597:

MS-ESI: calculated [M]⁺: 418.15; found [M]⁺: 418.10.

¹H NMR (400 MHz, DMSO-d6) δ 9.21 (s, 1H), 8.50 (s, 1H), 7.95 (d, *J=* 8.0 Hz, 1H), 7.89-7.85 (m, 4H), 7.78-7.71 (m, 10H), 7.60 (d, *J=* 4.0 Hz, 1H), 7.37-7.32 (m, 1H), 6.73 (d, *J=* 4.0 Hz, 1H), 6.63 (d, *J=* 4.0 Hz, 1H).

### Example 87 Compound AB36405

The structure of compound AB36405 was as follows:

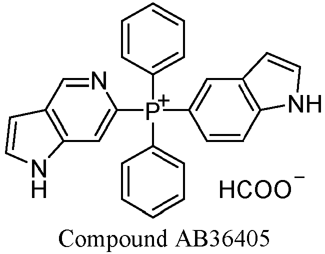

The synthetic route of compound AB36405 was as follows:

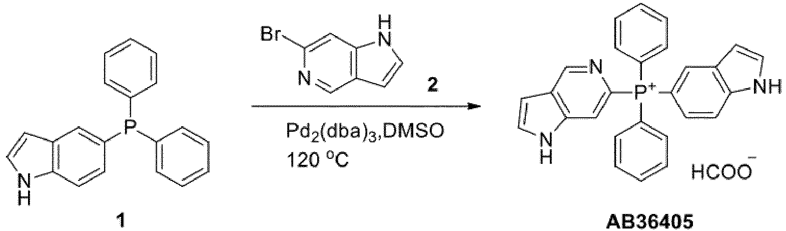

Compound 1 (100 mg, 0.33 mmol, 1 eq) was dissolved in dimethyl sulfoxide (3 mL) in a sealed tube, compound 2 (71 mg, 0.36 mmol, 1.1 eq) and tris(dibenzylideneacetone)dipalladium (6 mg, 0.007 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36405.

Compound AB36405:

MS-ESI: calculated [M]⁺: 418.15; found [M]⁺: 418.15.

¹H NMR (400 MHz, DMSO-d6) δ 12.17 (s, 1H), 9.27 (s, 1H), 8.49 (s, 1H), 7.90-7.87 (m, 3H), 7.79-7.72 (m, 12H), 7.61 (d, *J=* 4.0 Hz, 1H), 7.39-7.34 (m, 1H), 6.83 (d, *J=* 4.0 Hz, 1H), 6.64 (d, *J*= 4.0 Hz, 1H).

### Example 88 Compound AB36407

The structure of compound AB36407 was as follows:

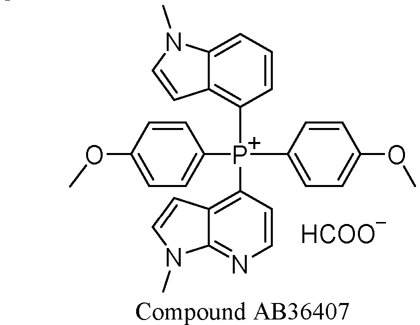

The synthetic route of compound AB36407 was as follows:

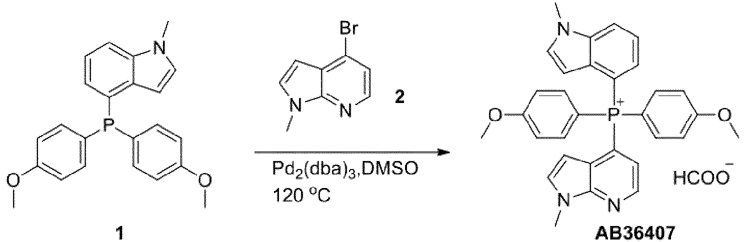

Compound 1 (500 mg, 1.33 mmol, 1 eq) was dissolved in dimethyl sulfoxide (3 mL) in a sealed tube, compound 2 (281 mg, 1.33 mmol, 1 eq) and tris(dibenzylideneacetone)dipalladium (24 mg, 0.027 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36407.

Compound AB36407:

MS-ESI: calculated [M]⁺: 506.20; found [M]⁺: 506.35.

¹H NMR (400 MHz, DMSO-d6) δ 8.60-8.57 (m, 1H), 8.42 (s, 1H), 8.09 (d, *J=* 8.0 Hz, 1H), 7.75 (d, *J=* 4.0 Hz, 1H), 7.65-7.59 (m, 4H), 7.53 (d, *J=* 4.0 Hz, 1H), 7.49-7.45 (m, 1H), 7.30-7.27 (m, 4H), 7.20-7.17 (m, 2H), 5.60-5.53 (m, 2H), 3.90 (s, 6H), 3.88 (s, 6H).

### Example 89 Compound AB36420

The structure of compound AB36420 was as follows:

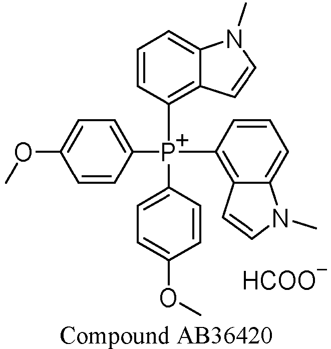

The synthetic route of compound AB36420 was as follows:

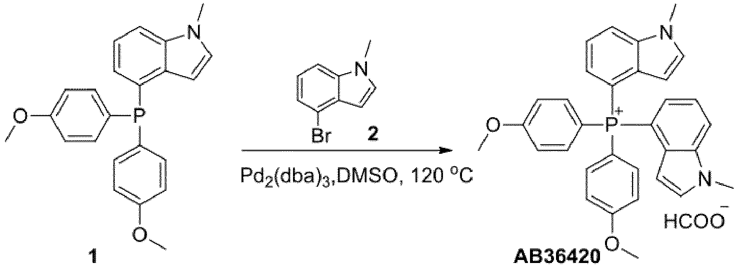

Compound 1 (200 mg, 0.53 mmol, 1 eq) was dissolved in dimethyl sulfoxide (3 mL) in a sealed tube, compound 2 (112 mg, 0.53 mmol, 1 eq) and tris(dibenzylideneacetone)dipalladium (6 mg, 0.01 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36420.

Compound AB36420:

MS-ESI: calculated [M]⁺: 505.20; found [M]⁺: 505.10.

¹H NMR (400 MHz, DMSO-d6) δ 8.54 (s, 1H), 8.05 (d, *J=* 8.0 Hz, 2H), 7.60-7.55 (m, 4H), 7.50 (d, *J=* 4.0 Hz, 2H), 7.47-7.42 (m, 2H), 7.28-7.25 (m, 4H), 7.22-7.16 (m, 2H), 5.56 (d, *J*= 4.0 Hz, 2H), 3.89 (s, 6H), 3.87 (s, 6H).

### Example 90 Compound AB36438

The structure of compound AB36438 was as follows:

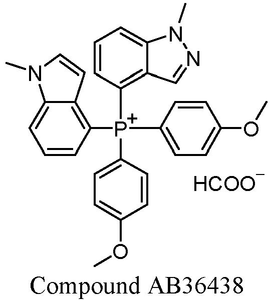

The synthetic route of compound AB36438 was as follows:

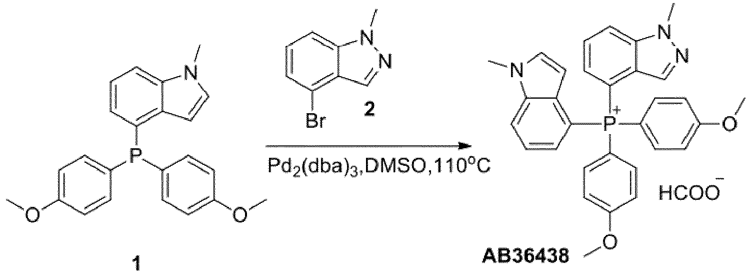

Compound 1 (200 mg, 0.53 mmol, 1 eq) was dissolved in dimethyl sulfoxide (3 mL) in a sealed tube, compound 2 (112 mg, 0.53 mmol, 1 eq) and tris(dibenzylideneacetone)dipalladium (6 mg, 0.01 mmol, 0.02 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36438.

Compound AB36438:

MS-ESI: calculated [M]⁺: 506.20; found [M]⁺: 506.65.

¹H NMR (400 MHz, DMSO-d6) δ 8.47 (s, 1H), 8.28 (d, *J=* 8.0 Hz, 1H), 8.09 (d, *J=* 8.0 Hz, 1H), 7.73-7.70 (m, 1H), 7.64-7.59 (m, 4H), 7.53 (d, *J=* 4.0 Hz, 1H), 7.47-7.37 (m, 2H), 7.30-7.27 (m, 4H), 7.22-7.16 (m, 1H), 7.00 (s, 1H), 5.60 (d, *J=* 4.0 Hz, 1H), 4.13 (s, 3H), 3.90 (s, 3H), 3.88 (s, 6H).

### Example 91 Compound AB36439

The structure of compound AB36439 was as follows:

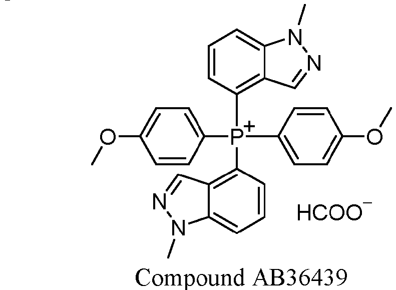

The synthetic route of compound AB36439 was as follows:

### Step (1):

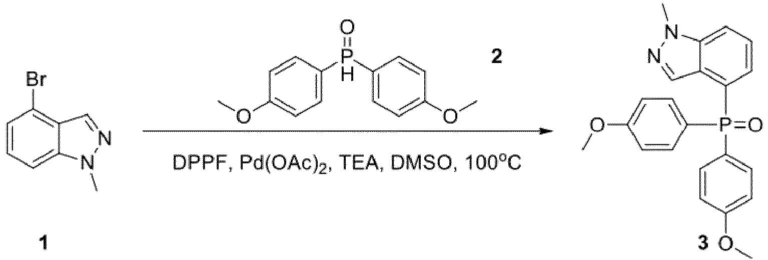

Compound 1 (2 g, 9.47 mmol, 1 eq) was dissolved in dimethyl sulfoxide (20 mL) in a sealed tube, compound 2 (2.5 g, 9.47 mmol, 1 eq), triethylamine (1.1 g, 11.36 mmol, 1.2 eq), 1,1'-bis (diphenylphosphine)ferrocene (527 mg, 0.95 mmol, 0.1 eq) and palladium acetate (106 mg, 0.47 mmol, 0.05 eq) were added. The mixture was reacted at 100 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

### Step (2):

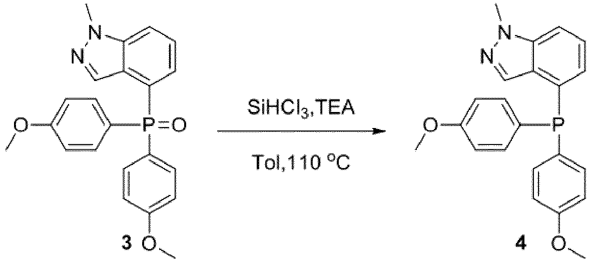

Compound 3 (3 g, 7.64 mmol, 1 eq) was dissolved in toluene (50 mL) in a sealed tube, triethylamine (1.5 g, 15.28 mmol, 2 eq) and trichlorosilane (10.3 g, 76.4 mmol, 10 eq) were added. The mixture was reacted at 110 °C for 16 h under N₂, the raction mixture was cooled and quenched with methanol (20 mL), and the pH was adjusted to neutral with 2N NaOH solution. The mixture was concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

### Step (3):

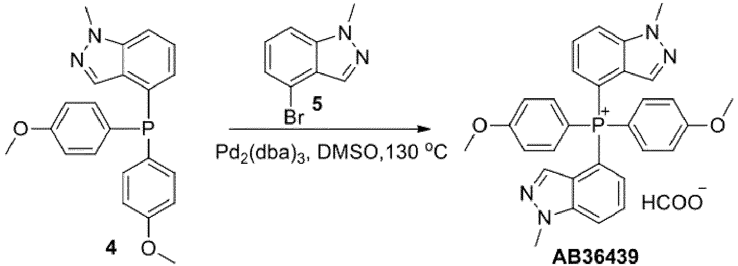

Compound 4 (200 mg, 0.53 mmol, 1 eq) was dissolved in dimethyl sulfoxide (2 mL) in a sealed tube, compound 5 (112 mg, 0.53 mmol, 1 eq) and tris(dibenzylideneacetone)dipalladium (48 mg, 0.053 mmol, 0.01 eq) were added. The mixture was reacted at 130 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36439.

Compound AB36439:

MS-ESI: calculated [M]⁺: 507.19; found [M]⁺: 507.20.

¹H NMR (400 MHz, DMSO-d6) δ 8.49 (s, 1H), 8.31 (d, *J=* 12.0 Hz, 2H), 7.73-7.63 (m, 6H), 7.44-7.38 (m, 2H), 7.31-7.28 (m, 4H), 7.10 (s, 2H),4.14 (s, 6H), 3.89 (s, 6H).

### Example 92 Compound AB36444

The structure of compound AB36444 was as follows:

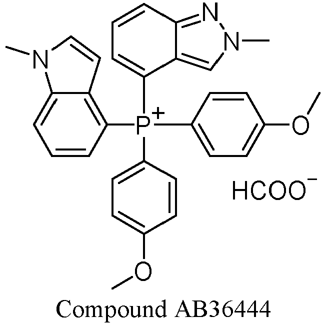

The synthetic route of compound AB36444 was as follows:

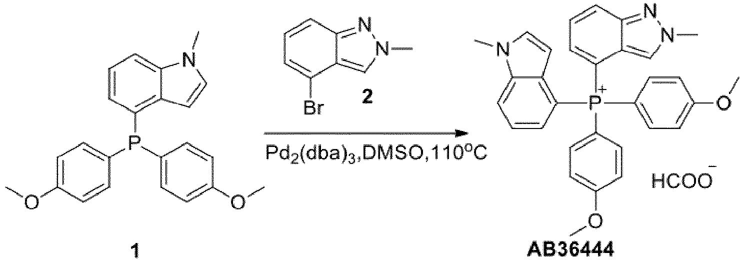

Compound 1 (200 mg, 0.53 mmol, 1 eq) was dissolved in dimethyl sulfoxide (3 mL) in a sealed tube, compound 2 (112 mg, 0.53 mmol, 1 eq) and tris(dibenzylideneacetone)dipalladium (6 mg, 0.01 mmol, 0.02 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36444.

Compound AB36444:

MS-ESI: calculated [M]⁺: 506.20; found [M]⁺: 506.30.

¹H NMR (400 MHz, DMSO-d6) δ 8.47 (s, 1H), 8.19-8.07 (m, 2H), 7.64-7.53 (m, 5H), 7.49-7.44 (m, 3H), 7.33-7.32 (m, 1H), 7.29-7.27 (m, 4H), 7.22-7.17 (m, 1H), 5.62 (d, *J*= 4.0 Hz, 1H), 4.07 (s, 3H), 3.89 (s, 3H), 3.88 (s, 6H).

### Example 93 Compound AB36445

The structure of compound AB36445 was as follows:

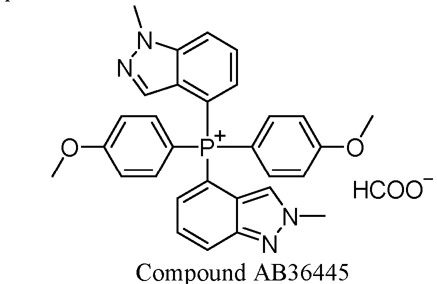

The synthetic route of compound AB36445 was as follows:

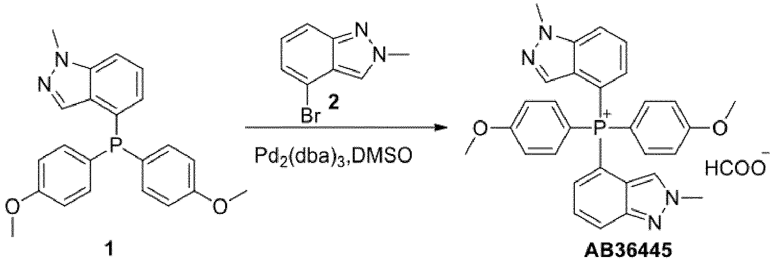

Compound 1 (200 mg, 0.53 mmol, 1 eq) was dissolved in dimethyl sulfoxide (2 mL) in a sealed tube, compound 2 (112 mg, 0.53 mmol, 1 eq) and tris(dibenzylideneacetone)dipalladium (48 mg, 0.053 mmol, 0.01 eq) were added. The mixture was reacted at 130 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36445.

Compound AB36445:

MS-ESI: calculated [M]⁺: 507.19; found [M]⁺: 507.25.

¹H NMR (400 MHz, DMSO-d6) δ 8.48 (s, 1H), 8.31-8.28 (m, 1H), 8.22-8.19 (m, 1H), 7.73 (s, 2H), 7.68-7.63 (m, 4H), 7.57-7.52 (m, 1H), 7.42-7.35 (m, 2H), 7.31-7.28 (m, 4H), 7.11 (s, 1H), 4.14 (s, 3H), 4.07 (s, 3H), 3.88 (s, 6H).

### Example 94 Compound AB36448

The structure of compound AB36448 was as follows:

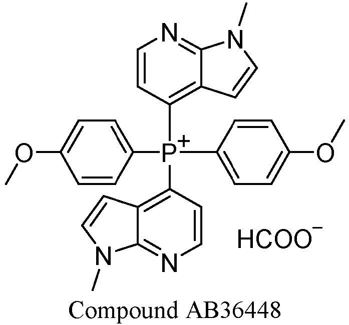

The synthetic route of compound AB36448 was as follows:

### Step (1):

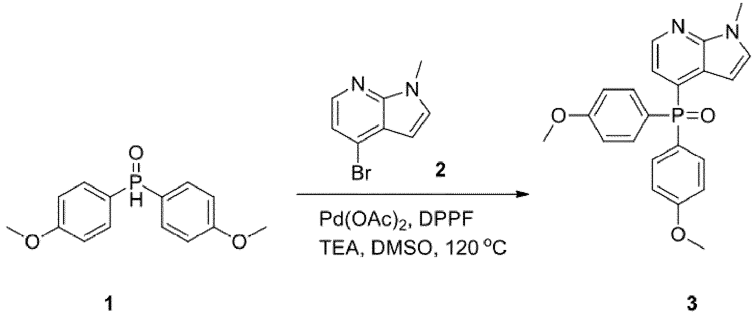

Compound 1 (3 g, 11.44 mmol, 1 eq) was dissolved in dimethyl sulfoxide (30 mL) in a sealed tube, compound 2 (2.4 g, 11.44 mmol, 1 eq), triethylamine (1.4 g, 13.73 mmol, 1.2 eq), 1,1'- bis (diphenylphosphine)ferrocene (632 mg, 1.14 mmol, 0.1 eq) and palladium acetate (128 mg, 0.57 mmol, 0.05 eq) were added. The mixture was reacted at 120 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

### Step (2):

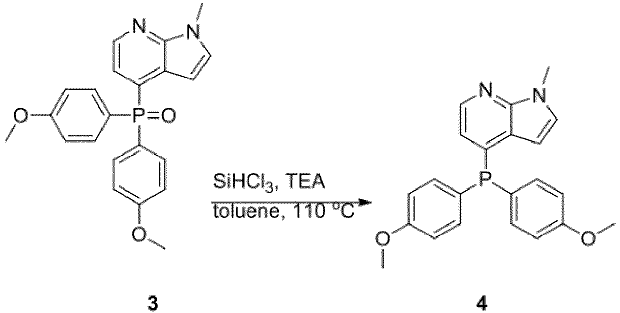

Compound 3 (3.7 g, 9.43 mmol, 1 eq) was dissolved in toluene (50 mL) in a sealed tube, triethylamine (1.9 g, 18.86 mmol, 2 eq) and trichlorosilane (12.8 g, 94.3 mmol, 10 eq) were added. The mixture was reacted at 110 °C for 16 h under N₂, the raction mixture was cooled and quenched with methanol (50 mL), and the pH was adjusted to neutral with 2N NaOH solution. The mixture was concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

### Step (3):

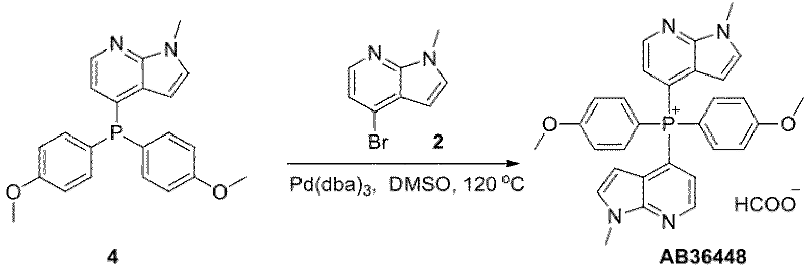

Compound 4 (200 mg, 0.53 mmol, 1 eq) was dissolved in dimethyl sulfoxide (2 mL) in a sealed tube, compound 2 (112 mg, 0.53 mmol, 1 eq) and tris(dibenzylideneacetone)dipalladium (48 mg, 0.053 mmol, 0.01 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36448.

Compound AB36448:

MS-ESI: calculated [M]⁺: 507.19; found [M]⁺: 507.20.

¹HNMR (400 MHz, DMSO-d6) δ 8.61-8.59 (m, 1H), 8.39 (s, 1H), 8.23-8.20 (m, 1H), 7.77-7.75 (m, 2H), 7.68-7.63 (m, 4H), 7.57-7.53 (m, 1H), 7.40-7.34 (m, 1H), 7.31-7.28 (m, 4H), 7.24-7.19 (m, 1H), 5.61 (d, *J=* 4.0 Hz, 1H), 4.07 (s, 3H), 3.90 (s, 3H), 3.89 (s, 6H).

### Example 95 Compound AB36449

The structure of compound AB36449 was as follows:

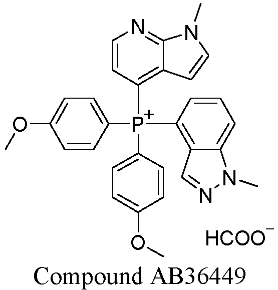

The synthetic route of compound AB36449 was as follows:

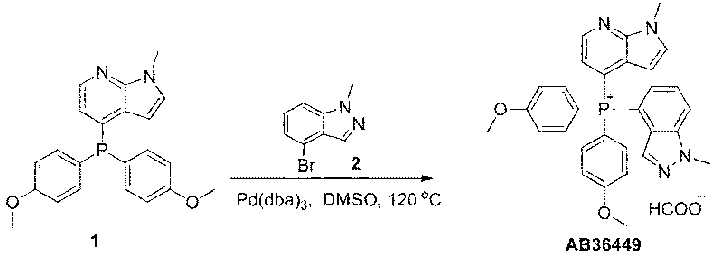

Compound 1 (200 mg, 0.53 mmol, 1 eq) was dissolved in dimethyl sulfoxide (2 mL) in a sealed tube, compound 2 (112 mg, 0.53 mmol, 1 eq) and tris(dibenzylideneacetone)dipalladium (48 mg, 0.053 mmol, 0.01 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36449.

Compound AB36449:

MS-ESI: calculated [M]⁺: 507.19; found [M]⁺: 507.20.

¹HNMR (400 MHz, DMSO-d6) δ 8.61-8.59 (m, 1H), 8.39 (s, 1H), 8.33-8.31 (m, 1H), 7.78-7.64 (m, 6H), 7.44-7.38 (m, 1H), 7.31-7.29 (m, 4H), 7.22-7.19 (m, 1H), 7.13 (s, 1H), 5.60 (d, *J*= 4.0 Hz, 1H), 4.15 (s, 3H), 3.91 (s, 3H), 3.89 (s, 6H).

### Example 96 Compound AB36452

The structure of compound AB36452 was as follows:

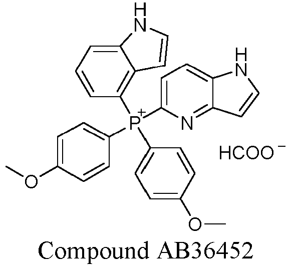

The synthetic route of compound AB36452 was as follows:

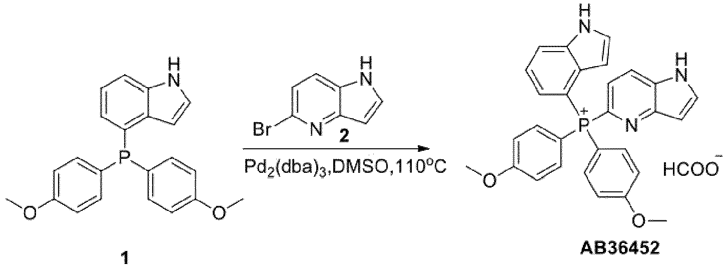

Compound 1 (200 mg, 0.55 mmol, 1 eq) was dissolved in dimethyl sulfoxide (2 mL) in a sealed tube, compound 2 (108 mg, 0.55 mmol, 1 eq) and tris(dibenzylideneacetone)dipalladium (5 mg, 0.0055 mmol, 0.01 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36452.

Compound AB36452:

MS-ESI: calculated [M]⁺: 478.17; found [M]⁺: 478.35.

¹H NMR (400 MHz, CDCl₃) δ 12.68 (s, 1H), 8.54 (s, 1H), 8.11-8.00 (m, 3H), 7.70-7.65 (m, 4H), 7.51 (d, *J=* 4.0 Hz, 1H), 7.40-7.34 (m, 2H), 7.29-7.27 (m, 4H), 7.06-7.01 (m, 1H), 6.79 (d, *J*= 4.0 Hz, 1H), 5.57 (d, *J=* 4.0 Hz, 1H), 3.87 (s, 6H).

### Example 97 Compound AB36453

The structure of compound AB36453 was as follows:

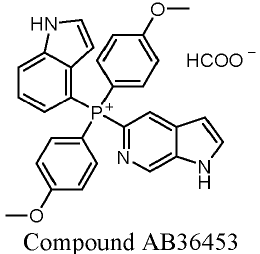

The synthetic route of compound AB36453 was as follows:

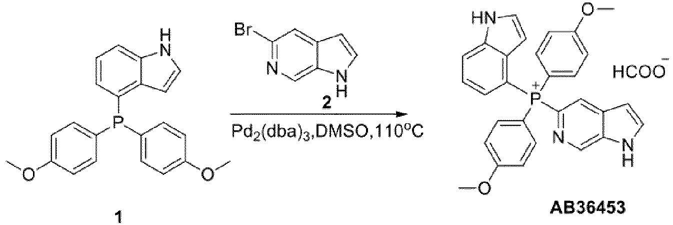

Compound 1 (200 mg, 0.55 mmol, 1 eq) was dissolved in dimethyl sulfoxide (2 mL) in a sealed tube, compound 2 (108 mg, 0.55 mmol, 1 eq) and tris(dibenzylideneacetone)dipalladium (5 mg, 0.0055 mmol, 0.01 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36453.

Compound AB36453:

MS-ESI: calculated [M]⁺: 478.17; found [M]⁺: 478.30.

¹H NMR (400 MHz, CDCl₃) δ 12.73 (s, 1H), 9.24 (s, 1H), 8.60 (s, 1H), 8.02 (d, *J=* 8.0 Hz, 1H), 7.92 (d, *J=* 8.0 Hz, 1H), 7.85 (d, *J=* 4.0 Hz, 1H), 7.70-7.65 (m, 4H), 7.51 (d, *J=* 4.0 Hz, 1H), 7.39-7.35 (m, 1H), 7.28-7.25 (m, 4H), 7.08-7.02 (m, 1H), 6.69 (d, *J=* 4.0 Hz, 1H), 5.59 (d, *J=* 4.0 Hz, 1H), 3.87 (s, 6H).

### Example 98 Compound AB36459

The structure of compound AB36459 was as follows:

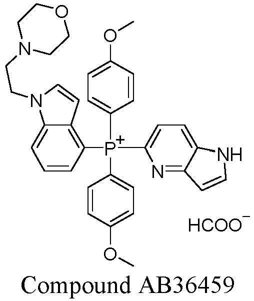

The synthetic route of compound AB36459 was as follows:

### Step (1):

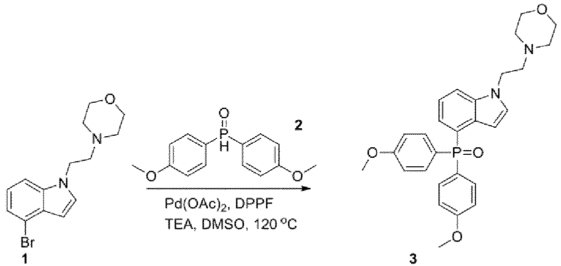

Compound 1 (2 g, 6.47 mmol, 1 eq) was dissolved in dimethyl sulfoxide (30 mL) in a sealed tube, compound 2 (1.7 g, 6.47 mmol, 1 eq), triethylamine (784 mg, 7.76 mmol, 1.2 eq), 1,1'- bis (diphenylphosphine)ferrocene (355 mg, 0.64 mmol, 0.1 eq) and palladium acetate (74 mg, 0.32 mmol, 0.05 eq) were added. The mixture was reacted at 120 °C for 16 h under N₂, the raction mixture was cooled, diluted with dichloromethane and washed with water three times, the organic phase was dried over anhydrous sodium sulfate, filtered and rotary dried, the crude product was purified by fast chromatography (DCM/MeOH=100/1 to 50/1) to afford compound 3.

### Step (2):

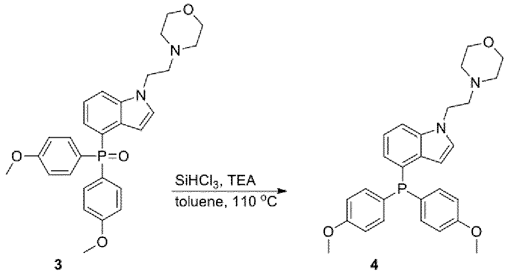

Compound 3 (1.8 g, 3.67 mmol, 1 eq) was dissolved in toluene (30 mL) in a sealed tube, triethylamine (741 mg, 7.34 mmol, 2 eq) and trichlorosilane (5.0 g, 36.7 mmol, 10 eq) were added. The mixture was reacted at 110 °C for 16 h under N₂, the raction mixture was cooled and quenched with methanol (50 mL), and the pH was adjusted to neutral with 2N NaOH solution. The mixture was concentrated, diluted with water and then extracted with dichloromethane. The organic phase was dried over anhydrous sodium sulfate, filtered and rotary dried, the crude product was purified by fast chromatography (PE/EA=100/1 to 25/1) to afford compound 4.

### Step (3):

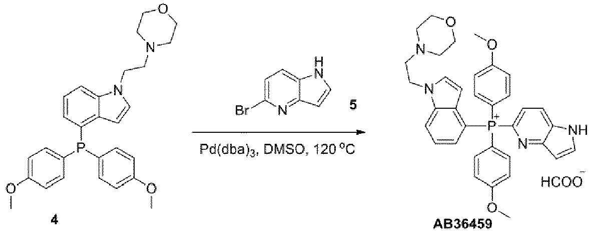

Compound 4 (200 mg, 0.42 mmol, 1 eq) was dissolved in dimethyl sulfoxide (2 mL) in a sealed tube, compound 5 (83 mg, 0.42 mmol, 1 eq) and tris(dibenzylideneacetone)dipalladium (4 mg, 0.0042 mmol, 0.01 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36459.

Compound AB36459:

MS-ESI: calculated [M]⁺: 591.25; found [M]⁺: 519.35.

¹H NMR (400 MHz, DMSO-d6) δ 12.59 (s, 1H), 8.42 (s, 1H), 8.15-8.07 (m, 2H), 8.01 (d, *J=* 4.0 Hz, 1H), 7.70-7.65 (m, 4H), 7.58 (d, *J=* 4.0 Hz, 1H), 7.46-7.42 (m, 1H), 7.38-7.35 (m, 1H), 7.30-7.27 (m, 4H), 7.09-7.04 (m, 1H), 6.80 (d, *J=* 4.0 Hz, 1H), 5.58 (d, *J=* 4.0 Hz, 1H), 4.41-4.38 (m, 2H), 3.88 (s, 6H), 3.55 (s, 4H), 2.70-2.67 (m, 2H), 2.41 (d, *J=* 4.0 Hz, 4H).

### Example 99 Compound AB36471

The structure of compound AB36471 was as follows:

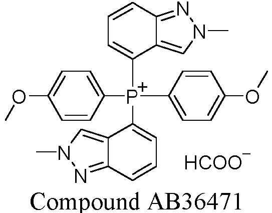

The synthetic route of compound AB36471 was as follows:

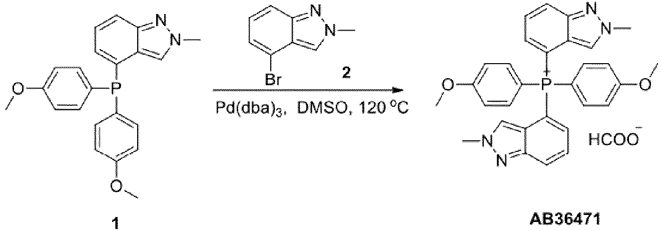

Compound 1 (100 mg, 0.26 mmol, 1 eq) was dissolved in dimethyl sulfoxide (2 mL) in a sealed tube, compound 2 (56 mg, 0.26 mmol, 1 eq) and tris(dibenzylideneacetone)dipalladium (16 mg, 0.026 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36471.

Compound AB36471:

MS-ESI: calculated [M]⁺: 507.19; found [M]⁺: 507.20.

¹H NMR (400 MHz, CDCl₃) δ 8.49 (s, 1H), 8.32 (d, *J=* 8.0 Hz, 2H), 7.75-7.64 (m, 6H), 7.45-7.39 (m, 2H), 7.30 (d, *J=* 4.0 Hz, 4H), 7.11 (s, 2H), 4.15 (s, 6H), 3.89 (s, 6H).

### Example 100 Compound AB35591

The structure of compound AB35591 was as follows:

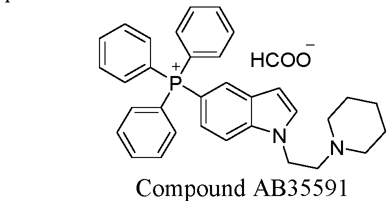

The synthetic route of compound AB35591 was as follows:

### Step (1):

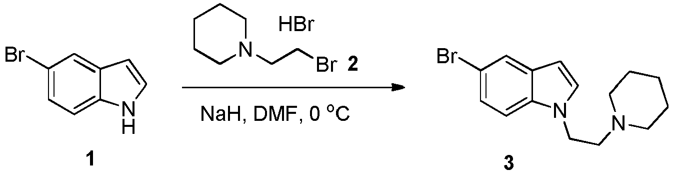

Compound 1 (700 mg, 3.57 mmol, 1 eq) was dissolved in N, N-dimethylformamide (10 mL) in a sealed tube, sodium hydride (214 mg, 5.36 mmol, 1.5 eq, 60% in mineral oil) was added at 0 °C. The mixture was reacted for 0.5 h, a solution of compound 2 (1.1 g, 3.93 mmol, 1.1 eq) in N, N-dimethylformamide (5 mL) was added to the above raction mixture at 0 °C, and the mixture was reacted for 1 h. The raction mixture was quenched with saturated ammonium chloride aqueous solution and then extracted with ethyl acetate. The organic phase was dried over anhydrous sodium sulfate, filtered and rotary dried, the crude product was purified by fast chromatography (dichloromethane: methanol=20:1) to afford compound 3.

### Step (2):

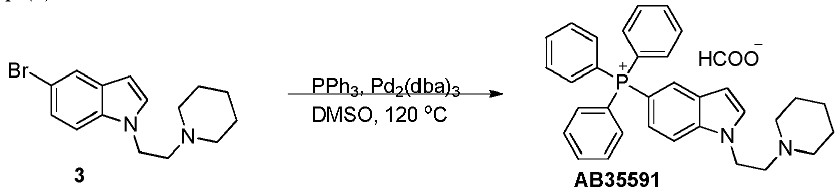

Compound 3 (200 mg, 0.65 mmol, 1 eq) was dissolved in dimethyl sulfoxide (5 mL) in a sealed tube, triphenylphosphine (170 mg, 0.65 mmol, 1 eq) and tris(dibenzylideneacetone)dipalladium (12 mg, 0.013 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB35591.

MS-ESI: calculated [M]⁺: 489.25; found [M]⁺: 489.30.

¹H NMR (400 MHz, DMSO-d6) δ 8.43 (s, 1H), 8.07-7.80 (m, 21H), 7.51-7.46 (m, 1H), 6.80 (d, *J=* 4.0 Hz, 1H), 4.51-4.48 (m, 2H), 2.76-2.73 (m, 2H), 2.61 (s, 1H), 1.55-1.46 (m, 6H).

### Example 101 Compound AB36458

The structure of compound AB36458 was as follows:

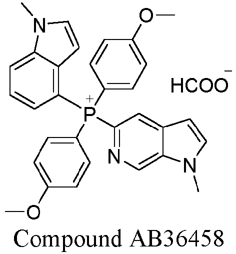

The synthetic route of compound AB36458 was as follows:

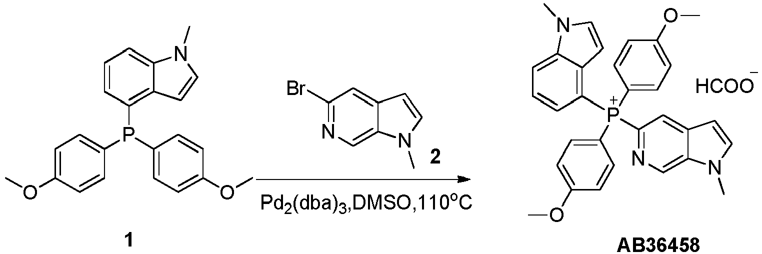

Compound 1 (200 mg, 0.53 mmol, 1 eq) was dissolved in dimethyl sulfoxide (3 mL) in a sealed tube, compound 2 (112 mg, 0.53 mmol, 1 eq) and tris(dibenzylideneacetone)dipalladium (6 mg, 0.01 mmol, 0.02 eq) were added. The mixture was reacted at 110 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36458.

MS-ESI: calculated [M]⁺: 506.20; found [M]⁺: 506.30.

¹H NMR (400 MHz, DMSO-d6) δ 9.29 (s, 1H), 8.43 (s, 1H), 8.07 (d, *J=* 8.0 Hz, 1H), 7.94 (d, *J=* 8.0 Hz, 1H), 7.84 (d, *J=* 4.0 Hz, 1H), 7.69-7.64 (m, 4H), 7.50-7.44 (m, 2H), 7.29-7.26 (m, 4H), 7.12-7.06 (m, 1H), 6.73 (d, *J=* 4.0 Hz, 1H), 5.58 (d, *J=* 4.0 Hz, 1H), 4.03 (s, 3H), 3.91 (s, 3H), 3.88 (s,6H).

### Example 102 Compound AB36498

The structure of compound AB36498 was as follows:

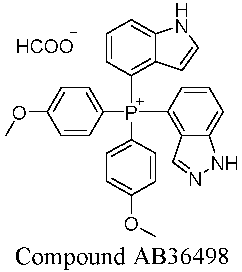

The synthetic route of compound AB36498 was as follows:

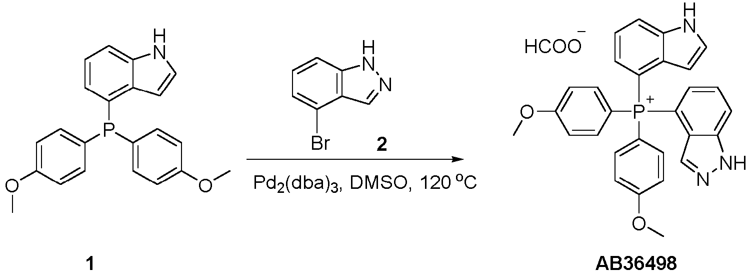

Compound 1 (150 mg, 0.41 mmol, 1 eq) was dissolved in dimethyl sulfoxide (2 mL) in a sealed tube, compound 2 (82 mg, 0.41 mmol, 1 eq) and tris(dibenzylideneacetone)dipalladium (37 mg, 0.041 mmol, 0.1 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36498.

MS-ESI: calculated [M]⁺: 478.17; found [M]⁺: 478.25.

¹H NMR (400 MHz, DMSO-d6) δ 8.51 (s, 1H), 8.16 (s, 1H), 8.10 (d, *J=* 8.0 Hz, 1H), 7.63-7.52 (m, 5H), 7.53 (d, *J=* 4.0 Hz, 1H), 7.40-7.34 (m, 2H), 7.30-7.27 (m, 4H), 7.18-7.12 (m, 1H), 7.03 (s, 1H), 5.60 (s, 1H), 3.88 (s, 6H).

### Example 103 Compound AB36504

The structure of compound AB36504 was as follows:

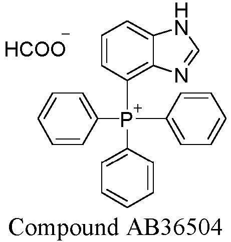

The synthetic route of compound AB36504 was as follows:

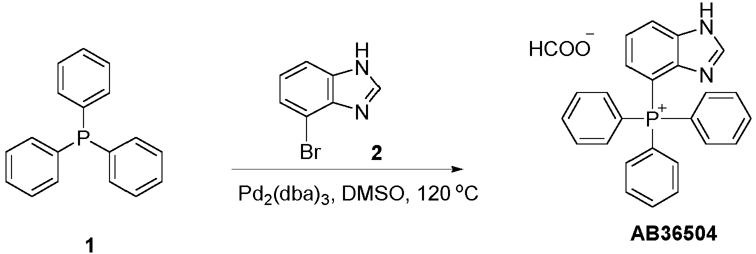

Compound 1 (200 mg, 0.76 mmol, 1.0 eq) and compound 2 (150 mg, 0.76 mmol, 1.0 eq) were dissolved in dimethyl sulfoxide (3 mL) in a sealed tube, and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36504.

MS-ESI: calculated [M]⁺: 379.14;found: 379.25.

¹H NMR (400 MHz, DMSO-d6) δ 8.44 (s, 1H), 8.18 (d, *J=* 8.0 Hz, 1H), 7.92-7.88 (m, 3H), 7.74-7.69 (m, 13H), 7.59-7.55 (m, 1H), 7.17-7.11(m, 1H).

### Example 104 Compound AB36511

The structure of compound AB36511 was as follows:

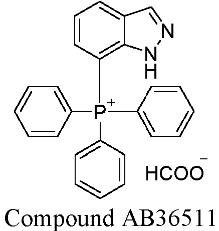

The synthetic route of compound AB36511 was as follows:

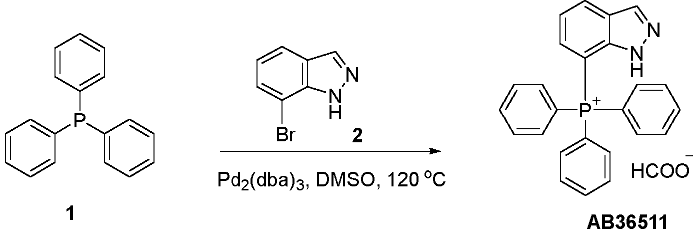

Compound 1 (200 mg, 0.76 mmol, 1.0 eq) and compound 2 (150 mg, 0.76 mmol, 1.0 eq) were dissolved in dimethyl sulfoxide (3 mL) in a sealed tube, and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36511.

MS-ESI: calculated [M]⁺: 379.14; found: 379.15.

¹H NMR (400 MHz, DMSO-d6) δ 8.59 (s, 1H), 8.44 (s, 1H), 8.41-8.39 (m, 1H), 7.92-7.89 (m, 3H), 7.76-7.70 (m, 12H), 7.37-7.32 (m, 1H), 7.27-7.23 (m, 1H).

### Example 105 Compound AB36513

The structure of compound AB36513 was as follows:

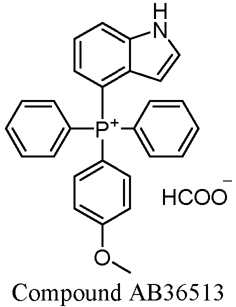

The synthetic route of compound AB36513 was as follows:

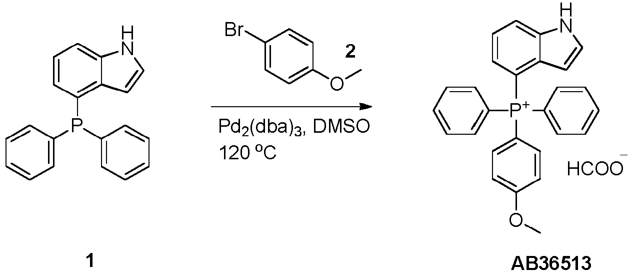

Compound 1 (200 mg, 0.66 mmol, 1.0 eq) and compound 2 (123 mg, 0.66 mmol, 1.0 eq) were dissolved in dimethyl sulfoxide (3 mL) in a sealed tube, and tris(dibenzylideneacetone)dipalladium (30 mg, 0.033 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36513.

MS-ESI: calculated [M]⁺: 408.15; found: 408.20.

¹H NMR (400 MHz, DMSO-d6) δ 12.86 (s, 1H), 8.51 (s, 1H), 8.06-8.01 (m, 1H), 7.93-7.89 (m, 2H), 7.79-7.50 (m, 11H), 7.44-7.31 (m, 3H), 7.20-7.00 (m, 1H), 5.53 (s, 1H), 3.89 (s, 3H).

### Example 106 Compound AB36514

The structure of compound AB36514 was as follows:

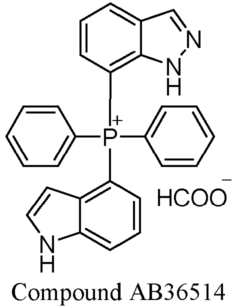

The synthetic route of compound AB36514 was as follows:

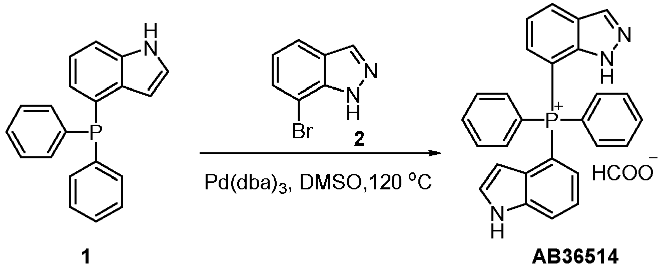

Compound 1 (200 mg, 0.66 mmol, 1.0 eq) and compound 2 (130 mg, 0.66 mmol, 1.0 eq) were dissolved in dimethyl sulfoxide (3 mL), and tris(dibenzylideneacetone)dipalladium (30 mg, 0.033 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36514.

MS-ESI: calculated [M]⁺: 418.15; found: 418.10.

¹H NMR (400 MHz, DMSO-d6) δ 12.18 (s, 1H), 8.56 (s, 1H), 8.47 (s, 1H), 8.38 (d, J= 8.0 Hz, 1H), 7.99 (d, J= 8.0 Hz, 1H), 7.88-7.85 (m, 2H), 7.76-7.67 (m, 8H), 7.50 (d, J= 4.0 Hz, 1H), 7.41-7.30 (m, 3H), 7.15-7.10 (m, 1H), 5.53 (d, J= 4.0 Hz, 1H).

### Example 107 Compound AB36516

The structure of compound AB36516 was as follows:

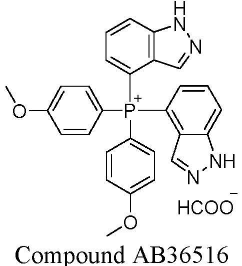

The synthetic route of compound AB36516 was as follows:

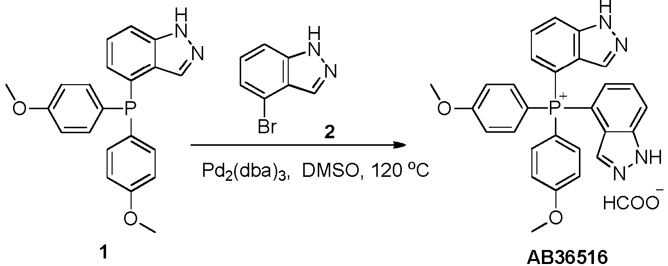

Compound 1 (300 mg, 0.83 mmol, 1.0 eq) and compound 2 (164 mg, 0.83 mmol, 1.0 eq) were dissolved in dimethyl sulfoxide (5 mL), and tris(dibenzylideneacetone)dipalladium (38 mg, 0.041 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36516.

MS-ESI: calculated [M]⁺: 479.16; found: 479.30.

¹H NMR (400 MHz, DMSO-d6) δ 8.54 (s, 1H), 8.19-8.17 (m, 2H), 7.66-7.61 (m, 6H), 7.37-7.31 (m, 2H), 7.29-7.26 (m, 4H), 7.08 (s, 2H), 3.86 (s, 6H).

### Example 108 Compound AB36518

The structure of compound AB36518 was as follows:

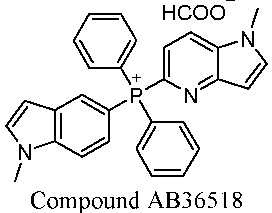

The synthetic route of compound AB36518 was as follows:

### Step (1):

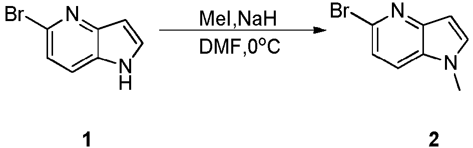

Compound 1 (1.0 g, 5.07 mmol, 1.0 eq) was dissolved in N, N-dimethylformamide (10 mL) and sodium hydride (406 mg, 10.14 mmol, 2.0 eq, 60% in mineral oil) was added at 0 °C. The mixture was reacted for 0.5 h, and iodomethane (863 mg, 6.08 mmol, 1.2 eq) was added, and the mixture was reacted at room temperature for 1 h. The raction mixture was quenched with saturated ammonium chloride aqueous solution and then extracted with ethyl acetate twice. The organic phase was dried over anhydrous sodium sulfate, filtered and rotary dried, the crude product was purified by normal phase preparative chromatography (petroleum ether/ethyl acetate=0% -10%) to obtain compound 2.

### Step (2):

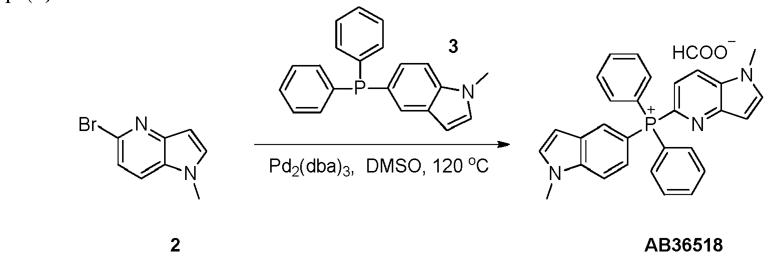

Compound 2 (133 mg, 0.63 mmol, 1.0 eq) and compound 3 (200 mg, 0.63 mmol, 1.0 eq) were dissolved in dimethyl sulfoxide (5 mL), and tris(dibenzylideneacetone)dipalladium (29 mg, 0.032 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36518.

MS-ESI: calculated [M]⁺: 446.18; found: 446.15.

¹H NMR (400 MHz, DMSO-d6) δ 8.26-8.23 (m, 1H), 8.05 (d, J= 4.0 Hz, 1H), 7.91-7.86 (m, 4H), 7.79-7.75 (m, 8H), 7.65 (d, J= 4.0 Hz, 1H), 7.52-7.42 (m, 2H), 6.86 (d, J= 4.0 Hz, 1H), 6.68 (d, J= 4.0 Hz, 1H), 3.93 (s, 3H), 3.91 (s, 3H).

### Example 109 Compound AB36521

The structure of compound AB36521 was as follows:

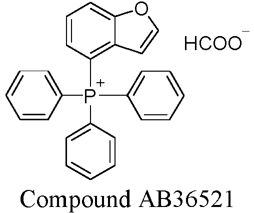

The synthetic route of compound AB36521 was as follows:

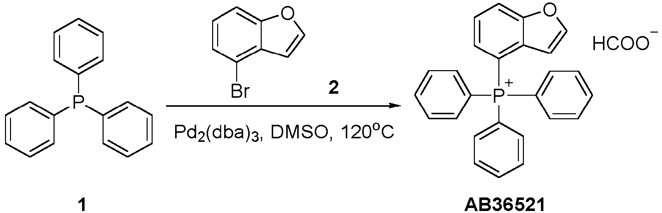

Compound 1 (200 mg, 0.76 mmol, 1.0 eq) and compound 2 (150 mg, 0.76 mmol, 1.0 eq) were dissolved in dimethyl sulfoxide (5 mL), and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36521.

MS-ESI: calculated [M]⁺: 379.12; found: 379.05.

¹H NMR (400 MHz, DMSO-d6) δ 8.29 (d, J= 8.0 Hz, 1H), 8.22 (s, 1H), 7.97-7.95 (m, 3H), 7.80-7.70 (m, 14H), 7.36-7.30 (m, 1H), 5.98 (s, 1H).

### Example 110 Compound AB36533

The structure of compound AB36533 was as follows:

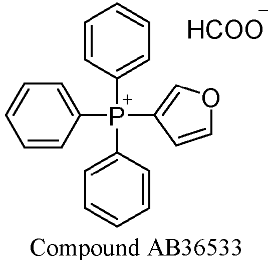

The synthetic route of compound AB36533 was as follows:

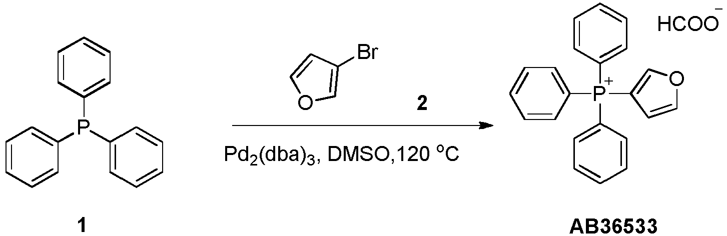

Compound 1 (200 mg, 0.76 mmol, 1.0 eq) and compound 2 (111 mg, 0.76 mmol, 1.0 eq) were dissolved in dimethyl sulfoxide (5 mL), tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36533.

MS-ESI: calculated [M]⁺: 329.11; found: 329.10.

¹H NMR (400 MHz, DMSO-d6) δ 8.65 (s, 1H), 8.07 (s, 1H), 8.00-7.98 (m, 1H), 7.91-7.88 (m, 3H), 7.80-7.75 (m, 6H), 7.69-7.64 (m, 6H), 6.98-6.96 (m, 1H).

### Example 111 Compound AB36534

The structure of compound AB36534 was as follows:

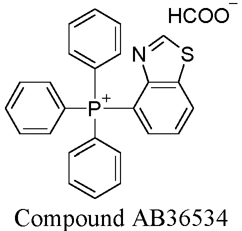

The synthetic route of compound AB36534 was as follows:

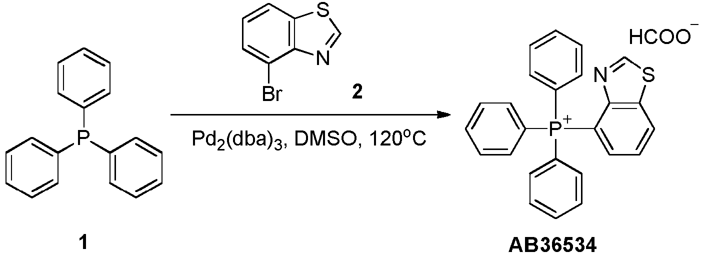

Compound 1 (500 mg, 1.9 mmol, 1.0 eq) and compound 2 (407 mg, 1.9 mmol, 1.0 eq) were dissolved in dimethyl sulfoxide (10 mL), tris(dibenzylideneacetone)dipalladium (87 mg, 0.095 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36534.

MS-ESI: calculated [M]⁺: 396.10; found: 396.20.

¹H NMR (400 MHz, DMSO-d6) δ 9.51 (s, 1H), 8.87 (d, J= 8.0 Hz, 1H), 8.46 (s, 1H), 7.93-7.89 (m, 3H), 7.87-7.73 (m, 1H), 7.75-7.68 (m, 12H), 7.48-7.43 (m, 1H).

### Example 112 Compound AB36535

The structure of compound AB36535 was as follows:

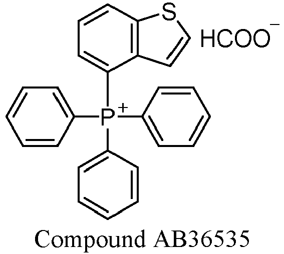

The synthetic route of compound AB36535 was as follows:

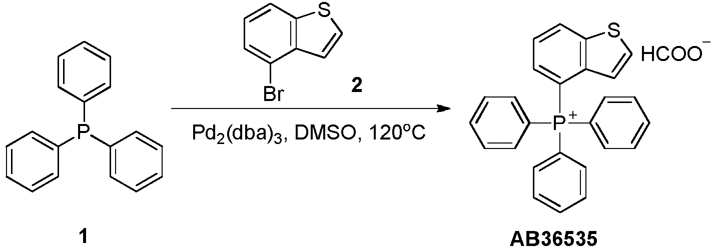

Compound 1 (200 mg, 0.76 mmol, 1.0 eq) and compound 2 (162 mg, 0.76 mmol, 1.0 eq) were dissolved in dimethyl sulfoxide (5 mL), and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36535.

MS-ESI: calculated [M]⁺: 395.10; found: 395.10_{∘}

¹H NMR (400 MHz, DMSO-d6) δ 8.72 (d, J= 8.0 Hz, 1H), 8.50 (s, 1H), 8.03 (d, J= 8.0 Hz, 1H), 7.98-7.94 (m, 3H), 7.80-7.72 (m, 13H), 7.41-7.35 (m, 1H), 6.62 (d, J= 4.0 Hz, 1H).

### Example 113 Compound AB36536

The structure of compound AB36536 was as follows:

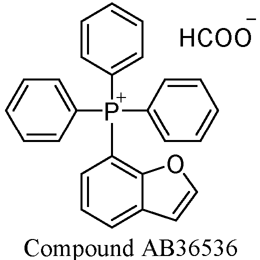

The synthetic route of compound AB36536 was as follows:

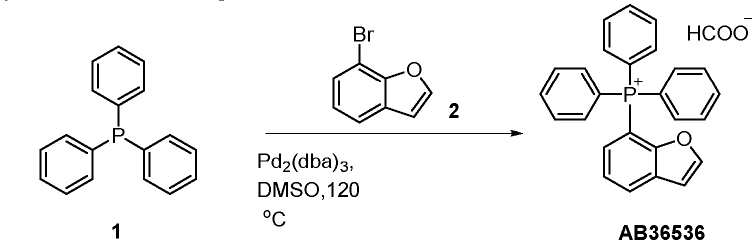

Compound 1 (200 mg, 0.76 mmol, 1.0 eq) and compound 2 (150 mg, 0.76 mmol, 1.0 eq) were dissolved in dimethyl sulfoxide (5 mL), and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36536.

MS-ESI: calculated [M]⁺: 379.12; found: 379.05.

¹H NMR (400 MHz, DMSO-d6) δ 8.33-8.28 (m, 2H), 8..00 (s, 1H), 7.93-7.90 (m, 3H), 7.78-7.75 (m, 5H), 7.74-7.72 (m, 3H), 7.70-7.67 (m, 4H), 7.63-7.59 (m, 1H),7.23-7.17 (m, 2H).

### Example 114 Compound AB36537

The structure of compound AB36537 was as follows:

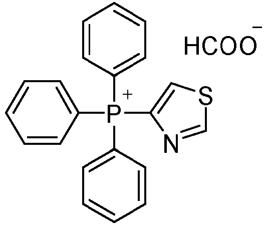

### Compound AB36537

The synthetic route of compound AB36537 was as follows:

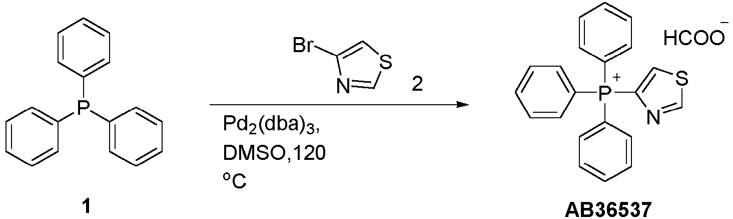

Compound 1 (200 mg, 0.76 mmol, 1.0 eq) and compound 2 (125 mg, 0.76 mmol, 1.0 eq) were dissolved in dimethyl sulfoxide (5 mL), and tris(dibenzylideneacetone)dipalladium (35 mg, 0.038 mmol, 0.05 eq) was added. The mixture was reacted at 120 °C for 16 h, the raction mixture was diluted with water and then extracted with dichloromethane. The organic phase was dried over anhydrous sodium sulfate, filtered and rotary dried. The crude product was purified by reversed phase preparative chromatography (acetonitrile/water+0.1% formic acid) to afford compound AB36537.

MS-ESI: calculated [M]⁺: 346.08; found: 346.10.

¹H NMR (400 MHz, CDCl₃) δ 9.29 (s, 1H), 8.73 (s, 1H), 7.89-7.87 (m, 3H), 7.75-7.66 (m, 12H), 7.23 (d, J=8.0 Hz, 1H).

### Example 115 Compound AB36542

The structure of compound AB36542 was as follows:

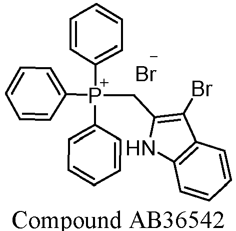

The synthetic route of compound AB36542was as follows:

### Step (1):

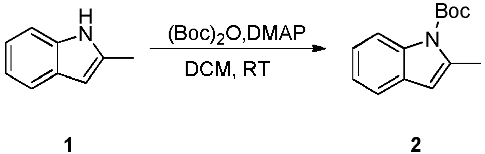

Compound 1 (500 mg, 3.8 mmol, 1.0 eq) was dissolved in dichloromethane (10 mL), di-tert butyl dicarbonate (996 mg, 4.56 mmol, 1.2 eq) and 4-dimethylaminopyridine (23 mg, 0.19 mmol, 0.05 eq) were added. The mixture was reacted at room temperature for 16 h, the raction mixture was diluted with water and then extracted with dichloromethane. The organic phase was dried over anhydrous sodium sulfate, filtered and rotary dried. The crude product was purified by normal phase preparative chromatography (dichloromethane/methanol=50/1) to afford compound 2.

### Step (2):

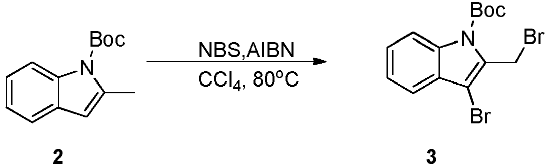

Compound 2 (100 mg, 0.43 mmol, 1.0 eq) was dissolved in tetrachloromethane (5 mL), and N-bromosuccinimide (76 mg, 0.43 mmol, 1.0 eq) and azobisisobutyronitrile (7 mg, 0.043 mmol, 0.1 eq) were added. The mixture was reacted at 80 °C for 1 h, the raction mixture was diluted with water and then extracted with dichloromethane. The organic phase was dried over anhydrous sodium sulfate, filtered and rotary dried. The crude product was purified by thin-layer chromatography (petroleum ether/ethyl acetate=10/1) to afford compound 3.

### Step (3):

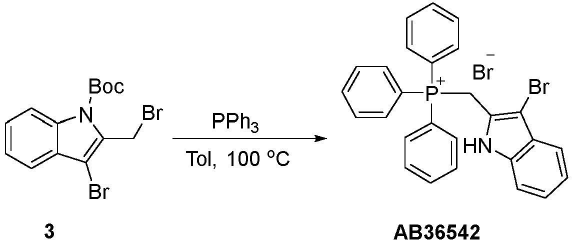

Compound 3 (90 mg, 0.23 mmol, 1.0 eq) and triphenylphosphine (60 mg, 0.23 mmol, 1.0 eq) were dissolved in toluene (5 mL). The mixture was reacted at 100 °C for 3 h, the raction mixture was diluted with water and then extracted with dichloromethane. The organic phase was dried over anhydrous sodium sulfate, filtered and rotary dried. The crude product was purified by thin-layer chromatography (dichloromethane/methanol=15/1) to obtain compound AB36542.

MS-ESI: calculated [M]⁺: 470.07; found: 469.95.

¹H NMR (400 MHz, DMSO-d6) δ 11.31 (s, 1H), 7.93-7.89 (m, 3H), 7.73-7.69 (m, 6H), 7.65-7.60 (m, 6H), 7.34-7.28 (m, 2H), 7.20-7.10 (m, 2H), 5.20 (d, J= 12.0 Hz, 2H).

### Example 116 Compound AB36546

The structure of compound AB36546 was as follows:

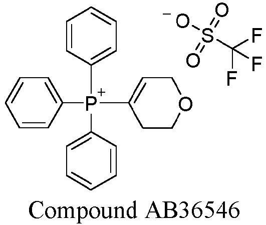

The synthetic route of compound AB36546 was as follows:

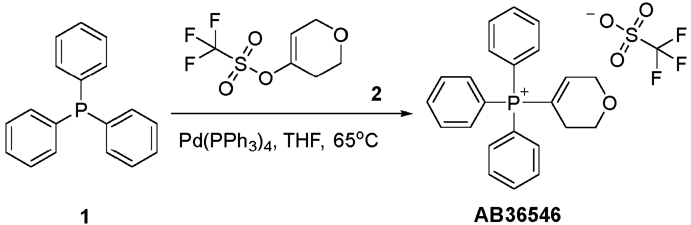

Compound 1 (200 mg, 0.76 mmol, 1.0 eq) and compound 2 (176 mg, 0.76 mmol, 1.0 eq) were dissolved in tetrahydrofuran (5 mL), tetrakis(triphenylphosphine)palladium (44 mg, 0.038 mmol, 0.05 eq) was added. The mixture was reacted at 65 °C for 16 h, the raction mixture was diluted with water and then extracted with dichloromethane. The organic phase was dried over anhydrous sodium sulfate, filtered and rotary dried. The crude product was purified by normal phase preparative chromatography (dichloromethane/methanol=15/1) to obtain compound AB36546.

MS-ESI: calculated [M]⁺: 345.14; found: 345.00.

¹H NMR (400 MHz, DMSO-d6) δ 7.97-7.93 (m, 3H), 7.83-7.73 (m, 12H), 6.85 (d, J= 24 Hz, 1H), 4.41 (s, 2H), 3.88-3.86 (m, 2H), 2.32-2.30 (m, 2H).

### Example 117 Compound AB36410

The structure of compound AB36410 was as follows:

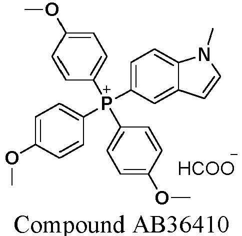

The synthetic route of compound AB36410 was as follows:

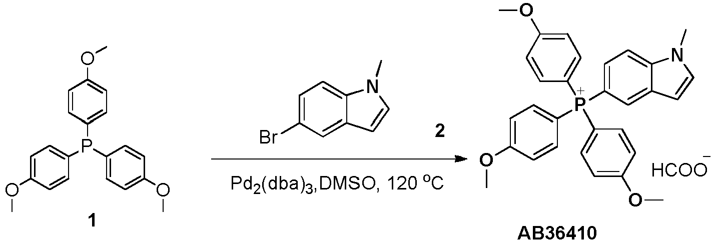

Compound 1 (200 mg, 0.57 mmol, 1 eq) was dissolved in dimethyl sulfoxide (3 mL) in a sealed tube, compound 2 (119 mg, 0.57 mmol, 1 eq) and tris(dibenzylideneacetone)dipalladium (10 mg, 0.01 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36410.

MS-ESI: calculated [M]⁺: 482.19; found [M]⁺: 482.30.

¹H NMR (400 MHz, DMSO-d6) δ 8.51 (s, 1H), 7.87-7.81 (m, 2H), 7.64-7.57 (m, 7H), 7.37-7.31 (m, 7H), 6.68 (d, *J=* 4.0 Hz, 1H), 3.91 (s, 3H), 3.90 (m, 9H).

### Example 118 Compound AB36411

The structure of compound AB36411 was as follows:

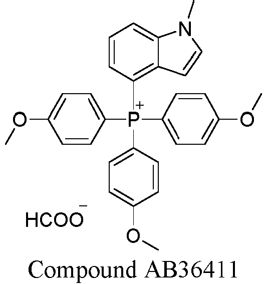

The synthetic route of compound AB36411 was as follows:

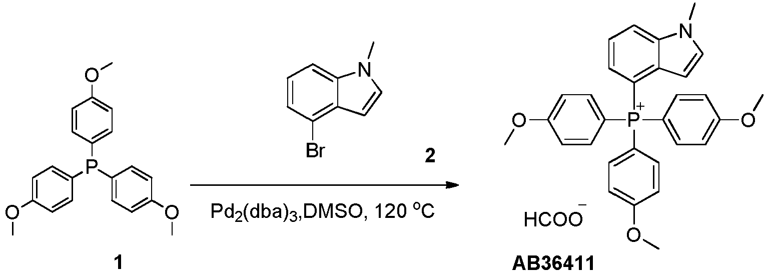

Compound 1 (100 mg, 0.28 mmol, 1 eq) was dissolved in dimethyl sulfoxide (3 mL) in a sealed tube, compound 2 (59 mg, 0.28 mmol, 1 eq) and tris(dibenzylideneacetone)dipalladium (5 mg, 0.005 mmol, 0.02 eq) were added. The mixture was reacted at 120 °C for 16 h under N₂, the raction mixture was cooled, diluted with water and then extracted with dichloromethane. The organic phase was dried over anhydrous sodium sulfate, filtered and rotary dried. The crude product was purified by reversed phase preparative chromatography (acetonitrile/water+0.01% formic acid) to afford compound AB36411.

MS-ESI: calculated [M]⁺: 482.19; found [M]⁺: 482.10.

¹H NMR (400 MHz, DMSO-d6) δ 8.51 (s, 1H), 8.08 (d, *J=* 8.0 Hz, 1H), 7.58-7.55 (m, 7H), 7.50-7.45 (m, 1H), 7.31-7.28 (m, 6H), 7.09-7.05 (m, 1H), 75.56 (d, *J=* 4.0 Hz, 1H), 3.91 (s, 3H), 3.88 (s, 9H).

### Example 119 Compound SJ00001

Compound SJ00001 was obtained by commercial purchase.

The structure of compound SJ00001 was as follows:

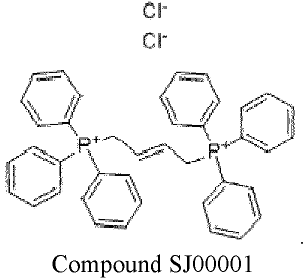

### Example 120 Compound SJ00002

Compound SJ00002 was obtained by commercial purchase.

The structure of compound SJ00002 was as follows:

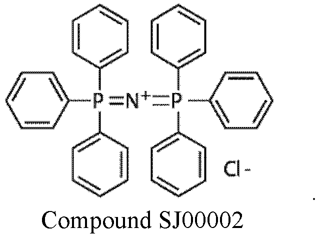

### (CAS number: 21050-13-5)

### Example 121 Compound SJ00003

Compound SJ00003 was obtained by commercial purchase.

The structure of compound SJ00003 was as follows:

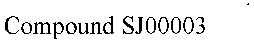

### Example 122 Compound SJ00004

Compound SJ00004 was obtained by commercial purchase.

The structure of compound SJ00004 was as follows:

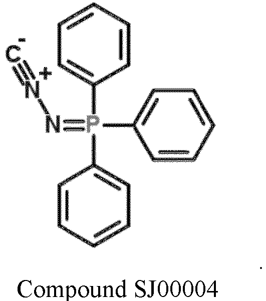

### Example 123 Compound SJ00005

Compound SJ00005 was obtained by commercial purchase.

The structure of compound SJ00005 was as follows:

### Compound SJ00005

### Example 124 Compound SJ00006

Compound SJ00006 was obtained by commercial purchase.

The structure of compound SJ00006 was as follows:

### Compound SJ00006

### (CAS number: 25791-20-2)

### Example 125

**The activity of mitochondria permeability transition pore in related cell was investigated.**

Experimental background:
The mitochondria permeability transition pore (mPTP) was a non-specific channel on the inner membrane of mitochondria, which could allow small molecules with molecular less than 1.5KD to pass freely, and its activity was affected by peroxides (such as H₂O₂), pH and calcium ions in mitochondria. Some cells had active mitochondria permeability transition pore, while some cells had inactive mitochondria permeability transition pore. For cells with active mitochondria permeability transition pore, adding peroxide (such as H₂O₂) could increase the activity of mitochondria permeability transition pore, resulting in a decrease in mitochondria membrane potential. For cells with inactive mitochondria permeability transition pore, adding peroxide (such as H₂O₂) had no significant effect on the activity of the mitochondria permeability transition pore, and the mitochondria membrane potential had no significant change. Based on this principle, whether the mPTP was active or inactive in specific cell could be determined by measuring the change of potential difference of the mitochondria membrane under peroxide stimulation

### Experimental method and result:

Daoy cell (human medulloblastoma cell, ATCC no. HTB-186) were cultured in 10% fetal bovine serum-containing DMEM (+p/s), and then 1.5 µM Cyclosporin A (CSA, CSA could effectively inhibit the activity of mitochondria permeability transition pore) was added to the medium, and the cell cultured in medium without the addition of Cyclosporin A (CSA) was used as blank control. The mitochondria membrane potential difference was detected by Tetramethylrhodamine (TMRM) in Daoy cell after different treatments, the high fluorescence intensity of TMRM represented the high membrane potential difference, the result was shown in Table 3.

**Table 3 Relative TMRM signal intensity (%) in Daoy cell after different treatments**

| **Cell** | **Daoy cell** | | | |
|---|---|---|---|---|
| CsA | - | - | + | + |
| H₂O₂ | - | + | - | + |
| mean value | 100 | 82 | 125 | 121 |
| Standard deviation | 7 | 11 | 14 | 11 |
| Repeat times | 3 | 3 | 3 | 3 |
| P value | | <0.05 | <0.01 | |

| | | | | |
|---|---|---|---|---|
| Remarks: "+" represented existence, "-" represented none. | | | | |

It could be seen from Table 3 that as for normal Daoy cell cultured in medium without the addition of CSA, the membrane potential was significantly down-regulated under the action of H₂O₂, indicating that the mPTP was active in Daoy cell. However, as for Daoy cell cultured in medium with the addition of CSA (CsA could inhibit the activity of mitochondria permeability transition pore (mPTP)), the membrane potential was not significant changed under the action of H₂O₂, indicating that active mPTP of Daoy cell was inhibited by CsA and the mPTP of Daoy cell become inactive, H₂O₂ did not cause a decrease in membrane potential in this case.

Therefore, Table 3 showed that the mitochondria permeability transition pore was active in Daoy cell.

### Example 126

The inhibitory effect of the compounds prepared in the Examples of the present invention on the mPTP-active Daoy cell, and the mPTP-inactive Daoy cell which was constructed by transfecting the Daoy cell with shRNA that specificly induce the degradation of PPIF mRNA.

The peptidyl-prolyl cis-trans isomerase F was abbreviated as PPIF, the protein identification number was UniProtKB/Swiss Prot: P30405, and the gene identification number was NCBI Entrez Gene: 10105

### 1. Construction of Daoy cell having inactive mitochondria permeability transition pore (mPTP)

### 1.1 Experimental background:

The activity of mitochondria permeability transition pore (mPTP) was regulated by the PPIF protein. When PPIF protein was inhibited, mPTP activity was significantly decreased. PPIF protein activity was restricted by the expression level of PPIF protein in cell. The PPIF protein expression level in Daoy cell can be specifically decreased by transfecting the Daoy cell with shRNA that could specificly induce the degradation of PPIF mRNA, thereby constructing Daoy cell having inactive mitochondria permeability transition pore (mPTP) (abbreviated as mPTP-inactive Daoy cell).

### 1.2 Experiment method and result:

A viral vector carrying shRNA that could specifically induces the degradation of PPIF mRNA was obtained using cloning technology, the shRNA (the nucleotide sequence of shRNA was GTTCTTCATCTGCACCATAAA (SEQ ID NO: 2)) carried by viral vector could specifically induce the degradation of PPIF mRNA, the Daoy cell was tranfected with the viral vector carring shRNA that could specifically induce the degradation of PPIF mRNA, and the Daoy cell transfected with empty virus vector without carrying shRNA that can specificly induce the degradation of PPIF mRNA was used as control. The expression level of PPIF protein in Daoy cell was detected using Western blot technique, and the result was shown in Fig. 1. The Fig.1 showed the PPIF in the Daoy cell tranfected with the shRNA that could specifically induce the degradation of PPIF (mPTP regulated protein) mRNA was not expressed (i.e. PPIF shRNA in Fig.1), while the PPIF in the Daoy cell not tranfected with the shRNA that could specifically induce the degradation of PPIF mRNA was normally expressed (i.e. con shRNA in Fig.1, as the control).

The Daoy cell transfected with empty virus vector without carrying shRNA that could specificly induce the degradation of PPIF mRNA and the Daoy cell transfected with virus vector carrying shRNA that could specificly induce the degradation of PPIF mRNA were cultured in 10% fetal bovine serum-containing DMEM (+p/s) respectively, and then 1.5 µM Cyclosporin A (CSA, CSA could effectively inhibit the activity of mitochondria permeability transition pore) was added to the medium, and the cell cultured in medium without the addition of Cyclosporin A (CSA) were used as blank control. The mitochondria membrane potential difference was detected in the Daoy cell transfected with empty virus vector without carrying shRNA that could specificly induce the degradation of PPIF mRNA and the Daoy cell transfected with virus vector carrying shRNA that could specificly induce the degradation of PPIF mRNA using Tetramethylrhodamine (TMRM) according to the method described in Example 125 above, the high fluorescence intensity of TMRM represented the high membrane potential difference, the results were shown in Table 4 and Table 5.

**Table 4 Relative TMRM signal intensity (%) in the Daoy cell transfected with empty virus vector without carrying shRNA that could specificly induce the degradation of PPIF mRNA after different treatments**

| **Cell** | The Daoy cell transfected with empty virus vector without carrying shRNA that could specificly induce the degradation of PPIF mRNA | | | |
|---|---|---|---|---|
| CsA | - | - | + | + |
| H₂O₂ | - | + | - | + |
| mean value | 100 | 79 | 131 | 126 |
| Standard deviation | 6 | 7 | 10 | 8 |
| Repeat times | 3 | 3 | 3 | 3 |

**Table 5 Relative TMRM signal intensity (%) in the Daoy cell transfected with virus vector carrying shRNA that could specificly induce the degradation of PPIF mRNA after different treatments**

| **Cell** | The Daoy cell transfected with virus vector carrying shRNA that could specificly induce the degradation of PPIF mRNA | | | |
|---|---|---|---|---|
| CsA | - | - | + | + |
| H₂O₂ | - | + | - | + |
| mean value | 100 | 95 | 105 | 101 |
| Standard deviation | 5 | 6 | 8 | 11 |
| Repeat times | 3 | 3 | 3 | 3 |

The Table 4 and Table 5 showed the mitochondria permeability transition pore (mPTP) is active in the Daoy cell transfected with empty virus vector without carrying shRNA that could specificly induce the degradation of PPIF mRNA, however, cell membrane potential is not affected by Cyclosporin A (CSA) and H₂O₂ in the Daoy cell transfected with virus vector carrying shRNA that could specificly induce the degradation of PPIF mRNA, indicating the mitochondria permeability transition pore (mPTP) is inactive in the Daoy cell transfected with virus vector carrying shRNA that could specificly induce the degradation of PPIF mRNA, therefore, the Daoy cell having inactive mitochondria permeability transition pore (mPTP) was successfully constructed by transfecting the Daoy cell with shRNA that could specificly induce the degradation of PPIF (mPTP regulated protein) mRNA.

The cell viability of the mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNAwas detected using the Promega CellTiter-Glo kit which reflected cell viability by measuring intracellular ATP content, the results was shown in Fig.2, it could be seen from Fig.2 that cell viability of mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNA was almost the same, and the difference in cell viability was not statistically significant.

### 2. The correlation between the inhibitory effect of the compound sprepared in the Examples of the present invention on tumor cell and the activity level of mPTP was investigated

### 2.1 Experimental background:

The inhibitory effect (IC₅₀ value) of the compounds prepared in the Examples of the present invention on the mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA was detected using Promega CellTiter-Glo kit which refleced cell viability by directly measured intracellular ATP content.

### 2.2 Experiment method and result:

The mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA were cultured in 10% fetal bovine serum-containing DMEM (+p/s), the 50% inhibiting concentration (IC₅₀) of the compounds prepared in the Examples of present invention on the two types of cells was detected, and the experimental results were shown in Table 6:

**Table 6 the inhibitory effect of the compounds prepared in the Examples of present invention on the mPTP-active Daoy cell and mPTP-inactive Daoy cell (IC₅₀, µM)**

| Compound | mPTP-active Daoy cell (IC₅₀, µM) | mPTP-inactive Daoy cell (IC₅₀, µM) |
|---|---|---|
| compound AB35419 | 0.74 | 0.15 |
| compound AB35431 | 2.00 | 0.65 |
| compound AB35437 | 0.92 | 0.30 |
| compound AB35449 | 7.12 | 2.44 |
| compound AB35450 | 0.43 | 0.10 |
| compound AB35451 | 1.22 | 0.28 |
| compound AB35452 | 5.39 | 0.82 |
| compound AB35461 | >50 | 7.06 |
| compound AB35462 | 28.86 | 12.69 |
| compound AB35470 | 12.16 | 2.36 |
| compound AB35471 | 4.35 | 0.81 |
| compound AB35472 | 38.45 | 10.66 |
| compound AB35473 | 18.05 | 2.95 |
| compound AB35474 | 18.40 | 2.45 |
| compound AB35477 | 24.23 | 10.31 |
| compound AB35479 | 25.10 | 6.51 |
| compound AB35480 | >50 | 6.26 |
| compound AB35481 | 16.70 | 2.50 |
| compound AB35482 | 3.97 | 0.61 |
| compound AB35495 | 1.35 | 0.33 |
| compound AB35503 | 1.30 | 0.39 |
| compound AB35507 | 4.08 | 1.73 |
| compound AB35527 | 1.21 | 0.25 |
| compound AB35529 | 4.11 | 0.88 |
| compound AB35537 | 2.24 | 0.79 |
| compound AB35538 | 5.94 | 1.71 |
| compound AB35539 | 12.22 | 3.88 |
| compound AB35548 | 8.09 | 2.64 |
| compound AB35549 | 10.53 | 3.85 |
| compound AB35563 | 1.51 | 0.56 |
| compound AB35564 | 0.39 | 0.05 |
| compound AB35569 | 12.23 | 2.34 |
| compound AB35570 | 15.65 | 3.99 |
| compound AB35571 | 1.38 | 0.27 |
| compound AB35572 | 10.26 | 3.34 |
| compound AB35573 | 1.26 | 0.24 |
| compound AB35574 | 8.35 | 2.45 |
| compound AB35576 | 1.60 | 0.46 |
| compound AB35577 | 3.89 | 1.21 |
| compound AB35578 | 3.80 | 1.45 |
| compound AB35579 | 1.21 | 0.27 |
| compound AB35580 | 4.57 | 1.16 |
| compound AB35581 | 5.34 | 1.74 |
| compound AB35584 | 1.34 | 0.46 |
| compound AB35585 | 3.54 | 1.22 |
| compound AB35588 | 1.14 | 0.38 |
| compound AB35589 | 1.53 | 0.36 |
| compound AB35596 | 1.32 | 0.25 |
| compound AB35597 | 1.18 | 0.24 |
| compound AB36405 | 5.45 | 1.89 |
| compound AB36407 | 1.13 | 0.24 |
| compound AB36420 | 1.09 | 0.19 |
| compound AB36438 | 1.03 | 0.24 |
| compound AB36439 | 4.36 | 1.29 |
| compound AB36444 | 1.99 | 0.55 |
| compound AB36445 | 13.93 | 3.02 |
| compound AB36448 | 7.24 | 2.00 |
| compound AB36449 | 2.16 | 0.57 |
| compound AB36452 | 1.82 | 0.30 |
| compound AB36453 | 1.85 | 0.29 |
| compound AB36459 | 1.56 | 0.32 |
| compound AB36471 | 6.36 | 1.48 |
| compound AB35591 | 3.02 | 0.87 |
| compound AB36458 | 1.19 | 0.23 |
| compound AB36498 | 6.65 | 1.32 |
| compound AB36504 | 25.31 | 5.12 |
| compound AB36511 | 13.36 | 4.03 |
| compound AB36513 | 1.96 | 0.38 |
| compound AB36514 | 10.23 | 1.19 |
| compound AB36516 | 33.12 | 9.58 |
| compound AB36518 | 1.52 | 0.45 |
| compound AB36521 | 2.45 | 0.75 |
| compound AB36533 | 11.91 | 3.52 |
| compound AB36534 | 3.66 | 1.21 |
| compound AB36535 | 1.56 | 0.37 |
| compound AB36536 | 1.39 | 0.31 |
| compound AB36537 | 14.38 | 3.55 |
| compound AB36542 | 8.37 | 3.68 |
| compound AB36546 | 35.05 | 7.28 |
| compound AB36410 | 0.57 | 0.62 |
| compound AB36411 | 0.52 | 0.68 |
| compound SJ00001 | 3.71 | 3.44 |
| compound SJ00002 | 0.44 | 0.46 |
| compound SJ00003 | 4.91 | 5.18 |
| compound SJ00004 | 9.01 | 7.99 |
| compound SJ00005 | 0.32 | 0.33 |
| compound SJ00006 | 0.29 | 0.34 |

| | | |
|---|---|---|
| Noted: IC₅₀ refered to 50% inhibiting concentration, ie, the concentration of the inhibitory compound when 50% inhibitory effect was achieved. The mPTP-active Daoy cell refered to the Daoy cell transfected with empty virus vector without carrying shRNA that can specificly induce the degradation of PPIF mRNA; the mPTP-inactive Daoy cell refered to the Daoy cell transfected with virus vector carrying shRNA that can specificly induce the degradation of PPIF mRNA. | | |

As was shown in Table 6, the compounds prepared in the Examples of the present invention had more significant inhibitory effect on mitochondria permeability transition pore (mPTP)-inactive Daoy cell, while the compounds prepared in the Examples of the present invention had weaker inhibitory effect on mPTP-active Daoy cell, indicating decreasing the mPTP activity in Daoy cell could significantly improve the inhibitory effect of the compounds prepared in the Examples of the present invention on tumor, therefore, the compounds prepared in the Examples of the present invention had more significant inhibitory effect on mPTP-inactive Daoy cell, the mPTP-inactive Daoy cell was more sensitive to the compounds prepared in the Examples of the present invention. Therefore, the compounds prepared in the Examples of the present invention had excellent precision treatment effect on mPTP-inactive Daoy cell. As mentioned above, when PPIF protein was inhibited, mPTP activity was significantly decreased, inhibiting the expression level of PPIF protein could decrease the mPTP activity in Daoy cell. Therefore, the compounds prepared in the Examples of the present invention had more significant inhibitory effect on Daoy cell with low expression of PPIF protein, while the compounds prepared in the Examples of the present invention had weaker inhibitory effect on Daoy cell with normal expression of PPIF protein, indicating that decreasing the expression level of PPIF protein could significantly improve the inhibitory effect of the compounds prepared in the Examples of the present invention on tumnor. Therefore, the compounds prepared in the Examples of the present invention had more significant inhibitory effect on Daoy cell with low expression of PPIF protein, i.e, the Daoy cell with low expression of PPIF protein was more sensitive to the compounds prepared in the Examples of the present invention compared with the Daoy cell with normal expression of PPIF protein. The compounds prepared in the Example of the present invention had excellent precision treatment on Daoy cell with low expression of PPIF protein.

### Example 127

The role of expression level of NNMT (Nicotinamide N-Methyltransferase) in the sensitivity of NCI-H82 cell (human small cell lung cancer cell) to the compounds prepared in the Examples of the present invention was determined

### Experiment method and result:

The expression of NNMT protein in NCI-H82 cell was overexpressed by inserting the NNMT gene into NCI-H82 cell using a viral vector, and the NNMT protein-overexpressing NCI-H82 cell (ov-NNMT NCI-H82 cell) was obtained. The control NCI-H82 cell (Con-NCI-H82 cell) was obtained by transfecting NCI-H82 cell with empty virus vector without carrying NNMT gene. The expressing content of NNMT protein in the Con-NCI-H82 cell and ov-NNMT NCI-H82 cell was detected using Western Blot assay (as shown in Fig.3), it could be seen from Fig.3 that the NNMT protein was overexpressed in the ov-NNMT NCI-H82 cell compared with the expression level of NNMT protein in the Con-NCI-H82 cell.

The cell viability of Con-NCI-H82 cell and ov-NNMT NCI-H82 cell was detected using the Promega CellTiter-Glo kit which reflected cell viability by measuring intracellular ATP content (as shown in Fig.4), it could be seen from Fig.4 that the cell viability of Con-NCI-H82 cell and ov-NNMT NCI-H82 cell was almost the same, and the difference in cell viability was not statistically significant.

The inhibitory effect (IC₅₀) of the compounds prepared in the Examples of the present invention on Con-NCI-H82 cell and ov-NNMT NCI-H82 cell was detected using Promega CellTiter-Glo kit which directly measured intracellular ATP content, the result was shown in Table 7 below:

**Table 7 the inhibitory effect of the compounds prepared in the Examples of the present invention on Con-NCI-H82 cell and ov-NNMT NCI-H82 cell (IC₅₀,**

| Compound | Con-NCI-H82 (µM) | ov-NNMT NCI-H82 (µM) |
|---|---|---|
| compound AB35419 | 0.05 | 0.56 |
| compound AB35431 | 0.32 | 1.03 |
| compound AB35437 | 0.27 | 1.21 |
| compound AB35449 | 2.17 | 6.47 |
| compound AB35450 | 0.23 | 1.56 |
| compound AB35451 | 0.13 | 0.96 |
| compound AB35452 | 0.63 | 2.34 |
| compound AB35461 | 8.49 | 20.73 |
| compound AB35462 | 6.48 | 20.56 |
| compound AB35470 | 2.92 | 7.02 |
| compound AB35471 | 0.34 | 2.16 |
| compound AB35472 | 8.11 | 19.43 |
| compound AB35473 | 3.27 | 12.33 |
| compound AB35474 | 3.66 | 13.55 |
| compound AB35477 | 25.34 | >50 |
| compound AB35479 | 6.36 | 25.51 |
| compound AB35480 | 13.77 | 40.05 |
| compound AB35481 | 2.45 | 9.82 |
| compound AB35482 | 0.41 | 2.13 |
| compound AB35495 | 0.25 | 1.88 |
| compound AB35503 | 0.45 | 3.22 |
| compound AB35507 | 0.78 | 3.96 |
| compound AB35527 | 0.36 | 1.45 |
| compound AB35529 | 0.38 | 1.87 |
| compound AB35537 | 0.51 | 2.33 |
| compound AB35538 | 0.97 | 3.86 |
| compound AB35539 | 1.55 | 6.03 |
| compound AB35548 | 2.22 | 7.14 |
| compound AB35549 | 3.18 | 9.57 |
| compound AB35563 | 0.46 | 2.09 |
| compound AB35564 | 0.13 | 1.32 |
| compound AB35569 | 1.66 | 5.34 |
| compound AB35570 | 3.34 | 11.85 |
| compound AB35571 | 0.19 | 1.43 |
| compound AB35572 | 0.28 | 1.95 |
| compound AB35573 | 0.14 | 1.37 |
| compound AB35574 | 0.41 | 2.56 |
| compound AB35576 | 0.51 | 2.93 |
| compound AB35577 | 1.08 | 4.54 |
| compound AB35578 | 1.27 | 4.93 |
| compound AB35579 | 0.51 | 2.77 |
| compound AB35580 | 0.55 | 3.66 |
| compound AB35581 | 1.26 | 4.81 |
| compound AB35584 | 0.24 | 1.73 |
| compound AB35585 | 0.52 | 3.34 |
| compound AB35588 | 0.21 | 1.78 |
| compound AB35589 | 0.32 | 2.34 |
| compound AB35596 | 0.25 | 1.56 |
| compound AB35597 | 0.18 | 1.85 |
| compound AB36405 | 0.98 | 3.72 |
| compound AB36407 | 0.24 | 1.55 |
| compound AB36420 | 0.14 | 0.98 |
| compound AB36438 | 0.32 | 1.53 |
| compound AB36439 | 3.07 | 7.83 |
| compound AB36444 | 0.86 | 3.91 |
| compound AB36445 | 6.92 | 18.34 |
| compound AB36448 | 4.23 | 11.26 |
| compound AB36449 | 1.49 | 5.91 |
| compound AB36452 | 0.29 | 1.36 |
| compound AB36453 | 0.35 | 1.62 |
| compound AB36459 | 0.48 | 2.09 |
| compound AB36471 | 2.86 | 10.98 |
| compound AB 35591 | 0.38 | 1.25 |
| compound AB 36458 | 0.19 | 0.98 |
| compound AB 36498 | 1.01 | 3.43 |
| compound AB 36504 | 3.38 | 10.68 |
| compound AB 36511 | 3.32 | 9.74 |
| compound AB 36513 | 0.11 | 0.86 |
| compound AB 36514 | 0.26 | 1.35 |
| compound AB 36516 | 6.47 | 17.89 |
| compound AB 36518 | 0.26 | 1.36 |
| compound AB 36521 | 0.47 | 1.92 |
| compound AB 36533 | 3.09 | 8.97 |
| compound AB 36534 | 1.35 | 5.11 |
| compound AB 36535 | 0.46 | 2.32 |
| compound AB 36536 | 0.56 | 1.89 |
| compound AB 36537 | 3.97 | 8.16 |
| compound AB 36542 | 3.25 | 9.22 |
| compound AB 36546 | 4.72 | 10.51 |
| compound AB 36410 | 0.19 | 0.21 |
| compound AB 36411 | 0.17 | 0.16 |
| compound SJ00001 | 0.68 | 0.79 |
| compound SJ00002 | 0.18 | 0.21 |
| compound SJ00003 | 3.24 | 2.98 |
| compound SJ00004 | 5.51 | 5.32 |
| compound SJ00005 | 0.19 | 0.17 |
| compound SJ00006 | 0.13 | 0.14 |

| | | |
|---|---|---|
| Note: IC₅₀ refered to 50% inhibiting concentration, ie, the concentration of the compound when 50% inhibitory effect was achieved. Con-NCI-H82 refered to the NCI-H82 cell transfected with empty virus vector without carrying NNMT gene, which was used as control; ov-NNMT NCI-H82 refered to the NCI-H82 cell transfected with virus vector carrying NNMT gene, the NNMT protein was overexpressed in the ov-NNMT NCI-H82 cell. | | |

As was shown in Table 7, the NNMT protein in NCI-H82 cell was overexpressed in the Example, the results further confirmed that the compounds prepared in the Examples of the present invention had significant inhibitory effect on tumor cell with low or no expression of NNMT gene, while the compounds prepared in the Examples of the present invention had weak inhibitory effect on tumor cells with high expression of NNMT gene. Decreasing the expression of NNMT gene in tumor could significantly improve the inhibitory effect of the compounds prepared in the Examples of the present invention on tumor, the expression level of NNMT gene in tumor cell was significantly negative correlation with the sensitivity of tumor cell to the compounds prepared in the Examples of the present invention. Therefore, the compounds prepared in the Examples of the present invention had more significant inhibitory effect on tumor cell with low or no expression of NNMT gene, the tumor cell with low or no expression of NNMT gene were highly sensitive to the compounds prepared in the Examples of the present invention. Therefore, the compounds prepared in the Examples of the present invention has excellent precision treatment on the tumor cell with low expression of NNMT gene.

### Example 128

The methylation level of DNA in cell was maintained by DNA methylation enzymes DNMT3a (DNA methyltransferase 3a), DNMT3b (DNA methyltransferase 3b) and DNMT1 (DNA methyltransferase 1). The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumor was determined in the Example.

### Experiment method and result:

The expression of NNMT gene, DNMT1, UHRF1, DNMT3a and DNMT3b in various cells were obtained from a public database (Cancer Cell Line Encyclopedia, CCLE, 1019 cells in total). Then, the correlation between expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.5.

The Fig.5 showed the expression of NNMT was negatively correlated with the expression of DNA methylase (DNMT3a, DNMT3b and DNMT1) and UHRF1 in each cell. Therefore, the tumor with high expression of DNA methylase (DNMT3a, DNMT3b and DNMT1) and UHRF1 was highly sensitive to the compounds prepared in the Examples of the present invention, the compounds prepared in the Examples of the present invention had excellent precision treatment on the tumor with high expression of DNA methylase (DNMT3a, DNMT3b and DNMT1) and UHRF1.

The above is an embodiment designed for a specific case of the present invention. It should be understood for the skilled in the art the improvements can be made without departing from the principles of the present invention, and these improvements should also be considered as the scope of protection of the present invention.

## Claims

1. A compound of formula I, or an optical isomer thereof, or a racemate thereof, or a solvate thereof, or a pharmaceutically acceptable salt thereof, or a deuterated compound thereof; 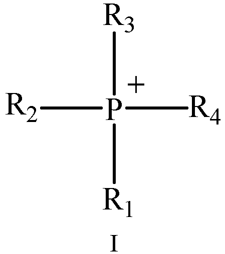 wherein,
R₁, R₂, R₃ and R₄ are each independently substituted or unsubstituted C3-C16 cycloalkyl, substituted or unsubstituted 3-16 membered heterocycloalkyl, substituted or unsubstituted C6-C16 aryl, substituted or unsubstituted 5-16 membered heteroaryl, or substituted or unsubstituted 5-16 membered heteroaryl-substituted or unsubstituted C1-C8 alkyl-;
the heterocyclic ring of the heterocycloalkyl and heteroaryl has 1-4 (preferably 1, 2, 3 or 4) heteroatoms independently selected from the group consisting of N, O and S;
each "substituted" means that one or more (preferably 1, 2, 3, 4, 5, 6, 7, or 8) hydrogen atoms on the group are independently substituted by substituent selected from the group consisting of C1-C12 alkyl, C3-C8 cycloalkyl, C1-C12 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C8 ester group, C2-C4 amide group, C1-C12 alkyl-O-, C1-C12 alkyl-S-, C1-C12 haloalkoxyl, C1-C12 haloalkylthio, C6-C12 aryl, 5-12 membered heteroaryl, 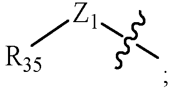
R₃₅ is hydrogen, hydroxyl, sulfhydryl, 3-12 membered heterocycloalkyl or halogen;
Z₁ is C1-C8 alkylidene.

2. The compound of formula I, or an optical isomer thereof, or a racemate thereof, or a solvate thereof, or a pharmaceutically acceptable salt thereof, or a deuterated compound thereof, wherein the compound of formula I, or an optical isomer thereof, or a racemate thereof, or a solvate thereof, or a pharmaceutically acceptable salt thereof, or a deuterated compound thereof is selected from the group consisting of

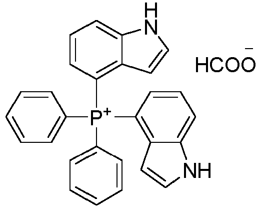

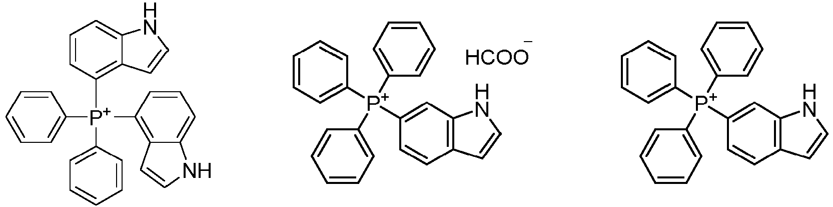

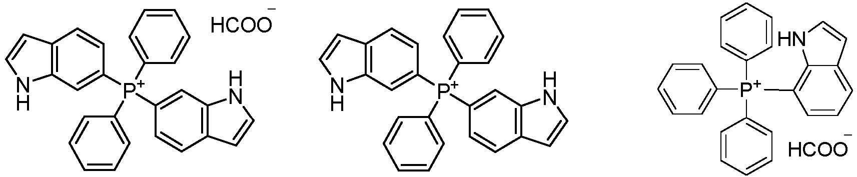

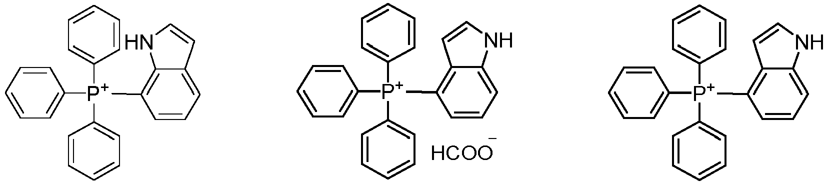

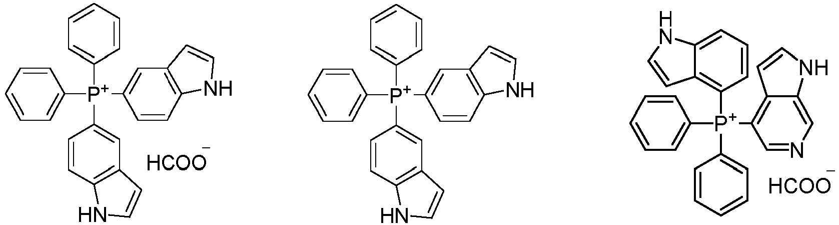

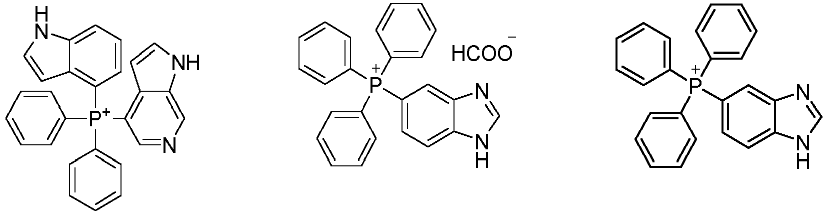

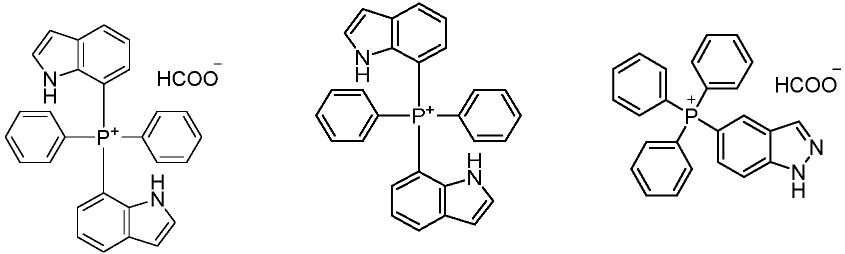

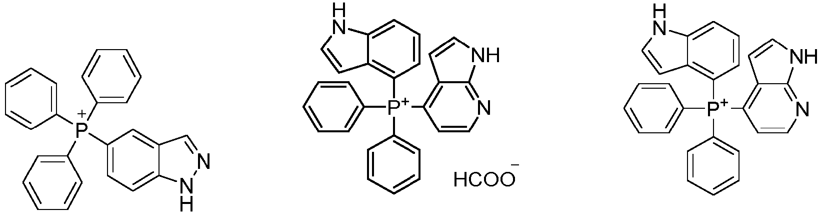

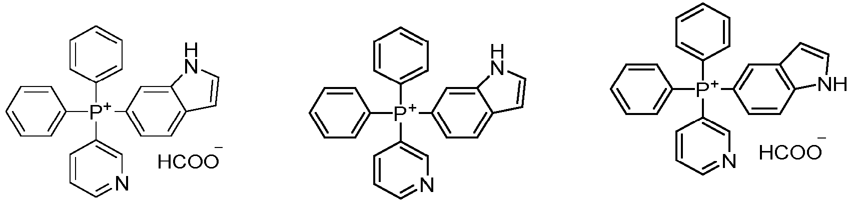

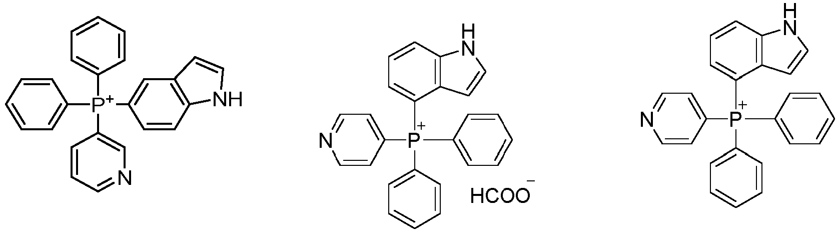

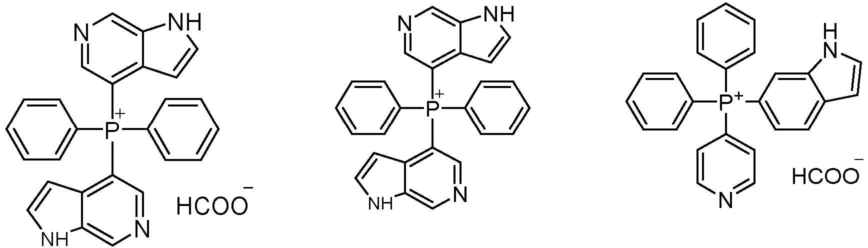

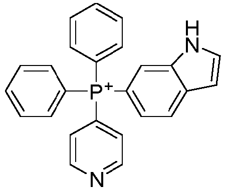

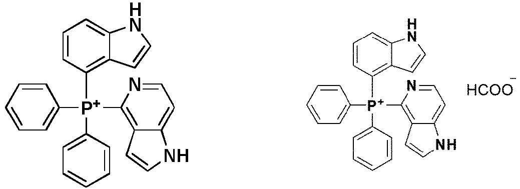

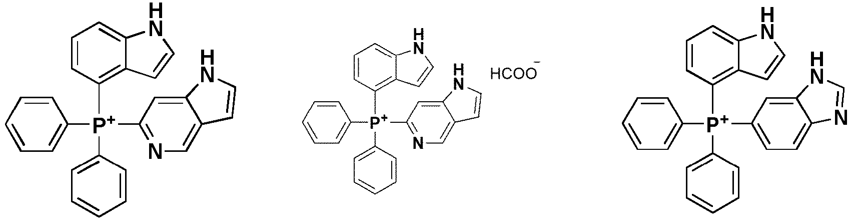

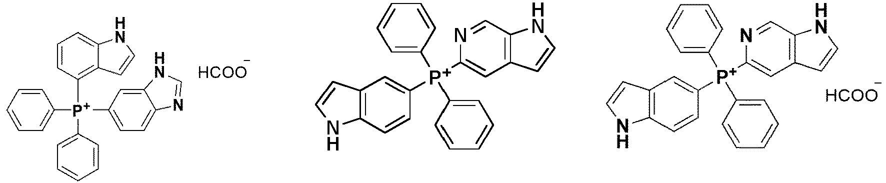

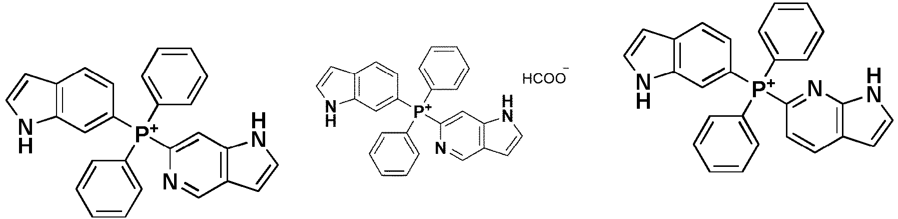

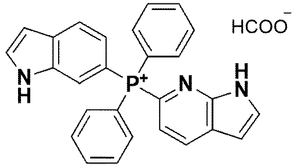

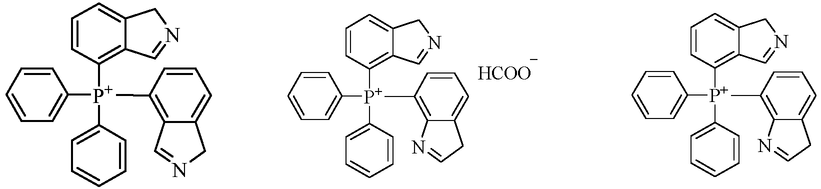

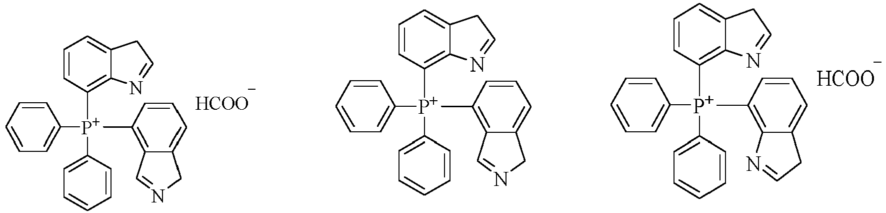

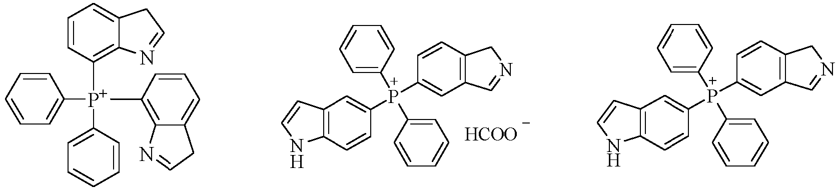

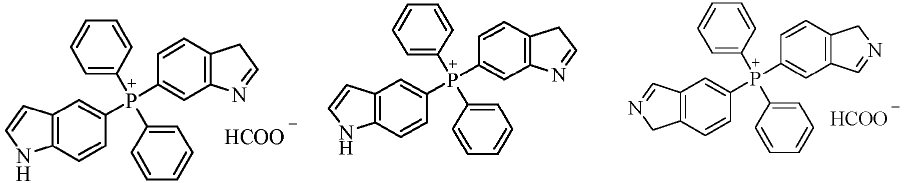

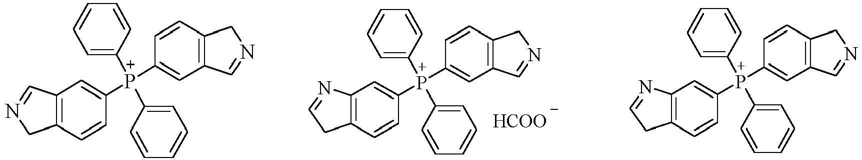

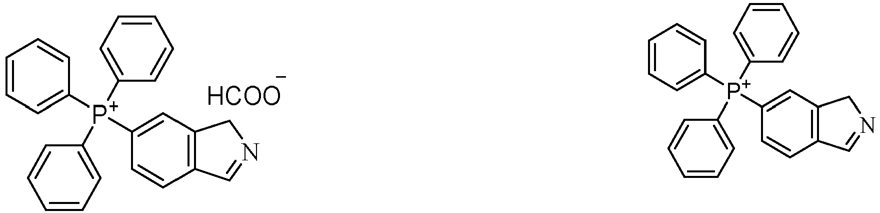

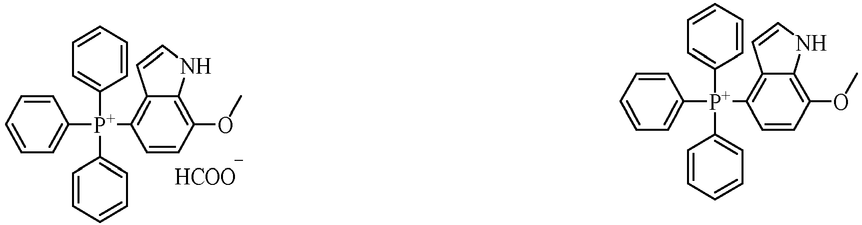

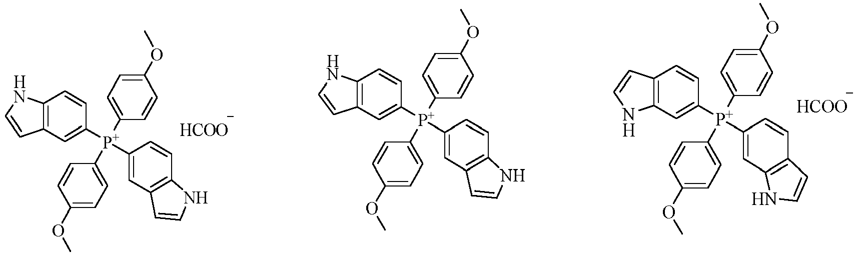

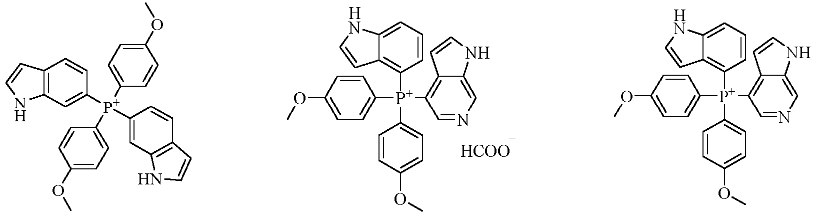

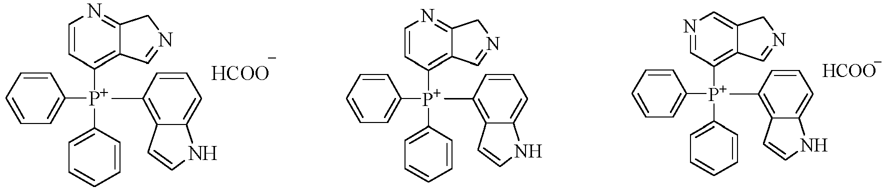

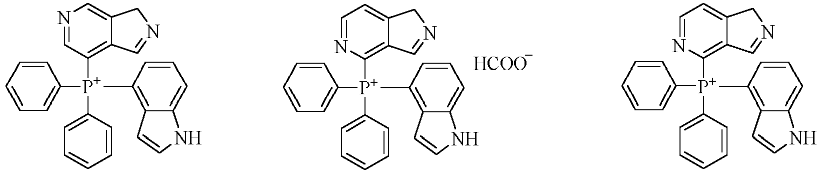

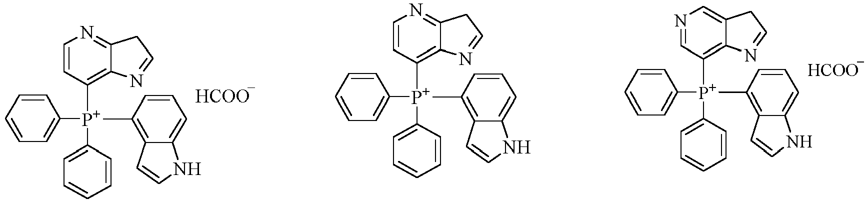

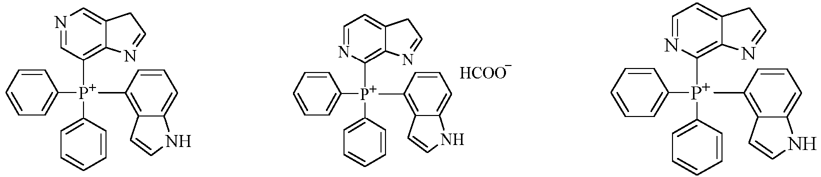

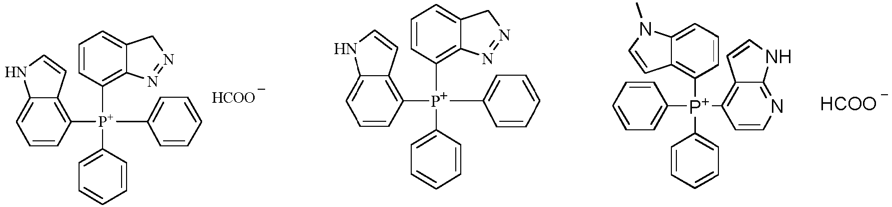

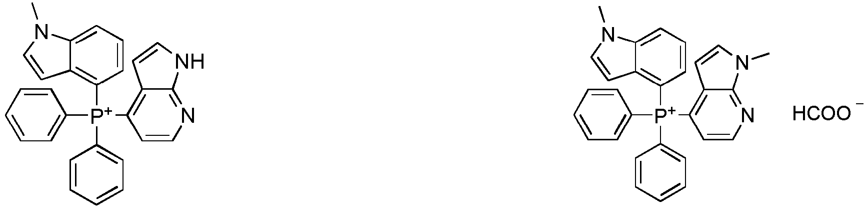

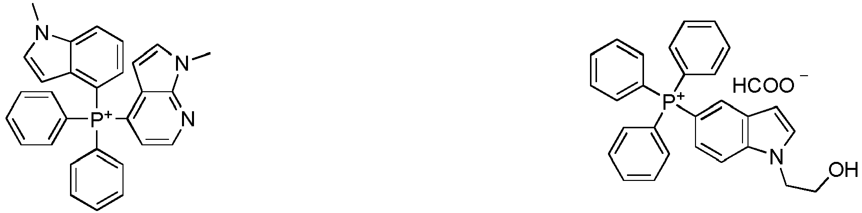

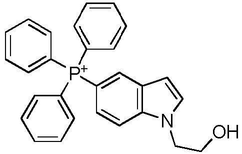

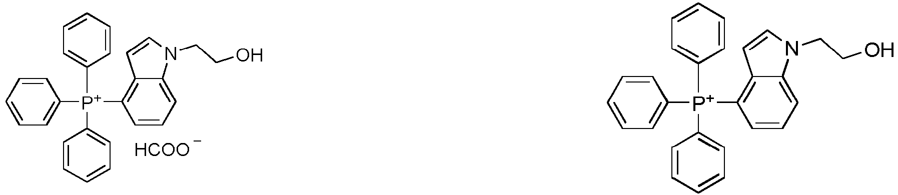

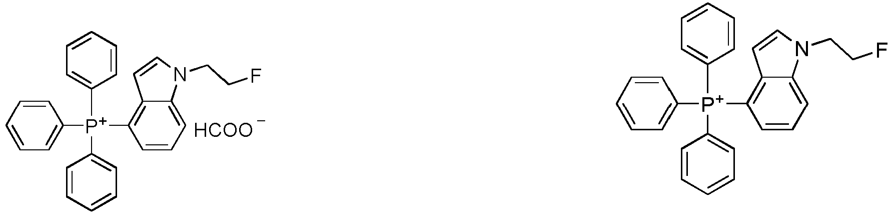

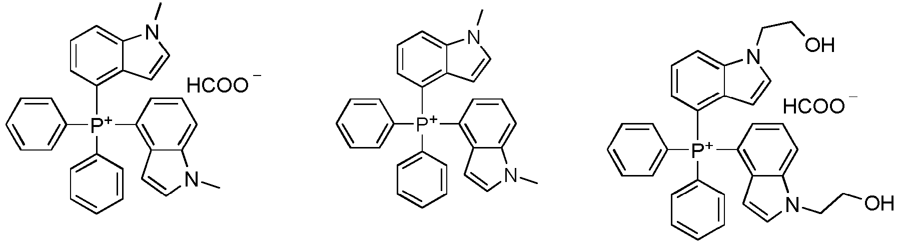

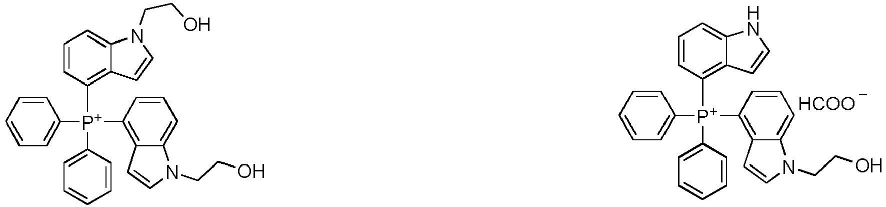

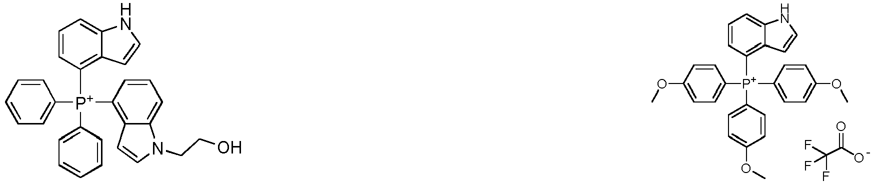

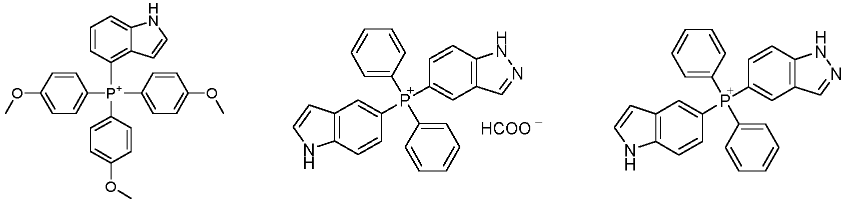

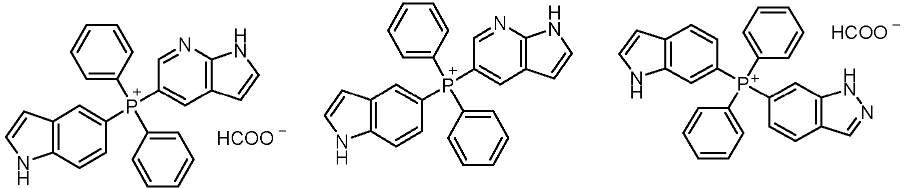

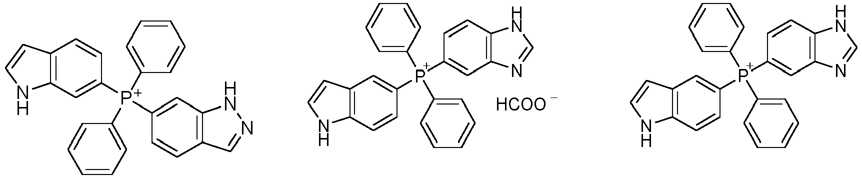

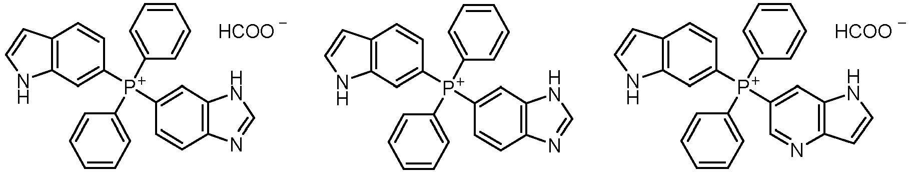

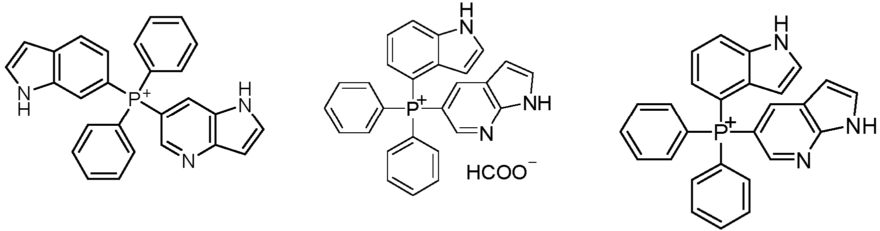

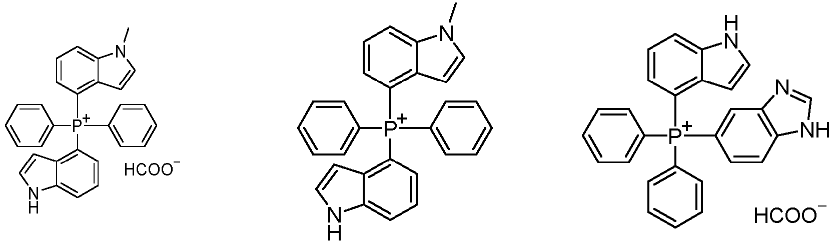

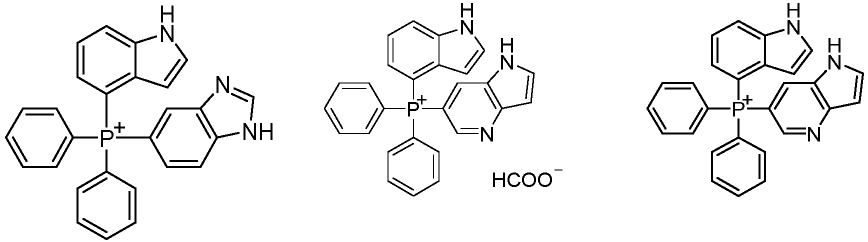

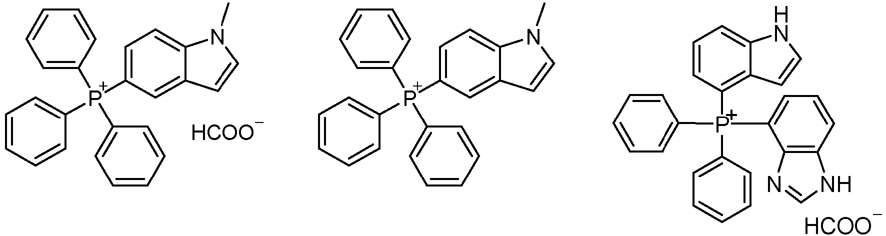

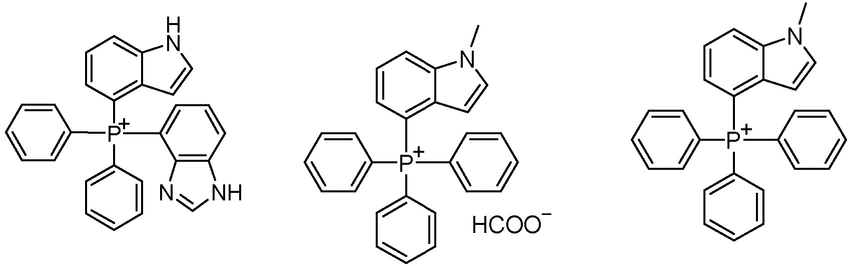

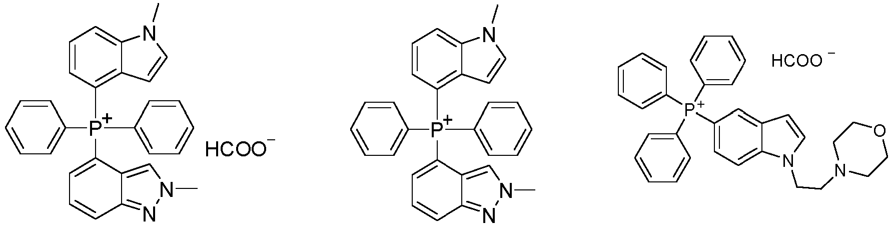

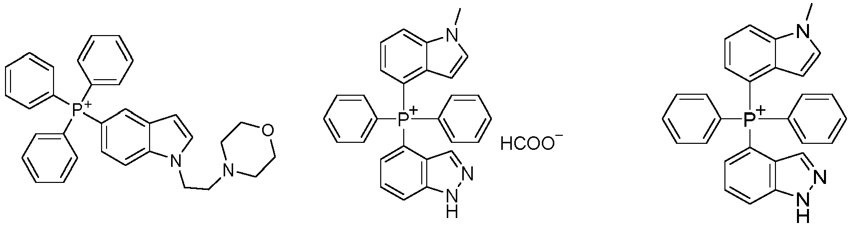

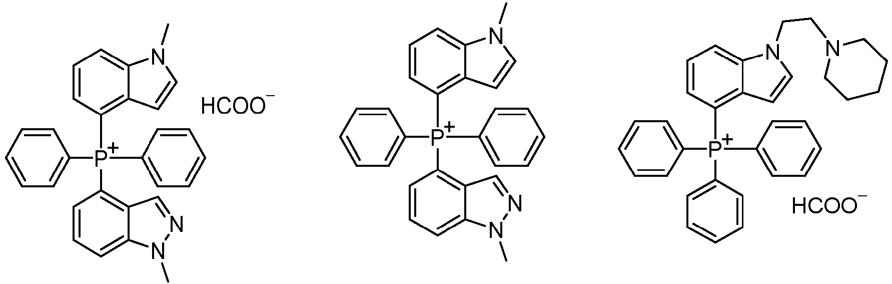

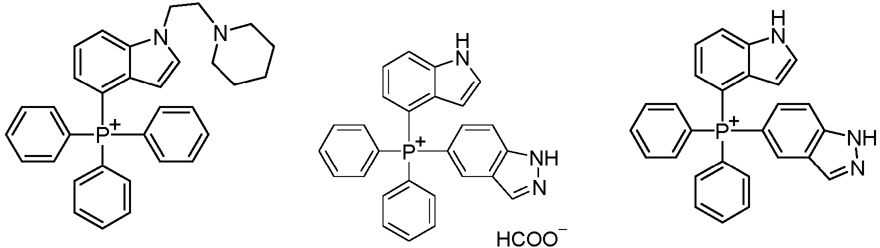

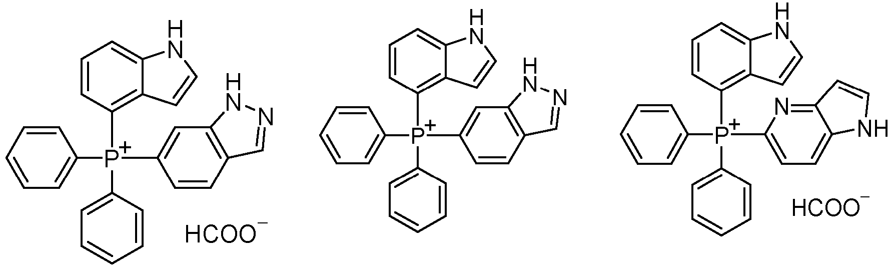

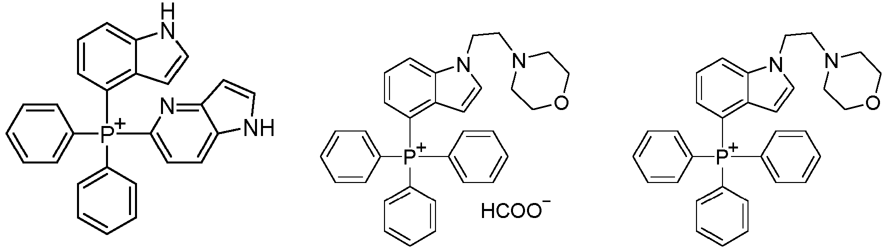

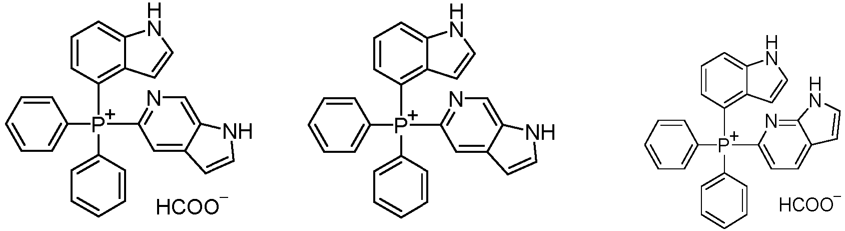

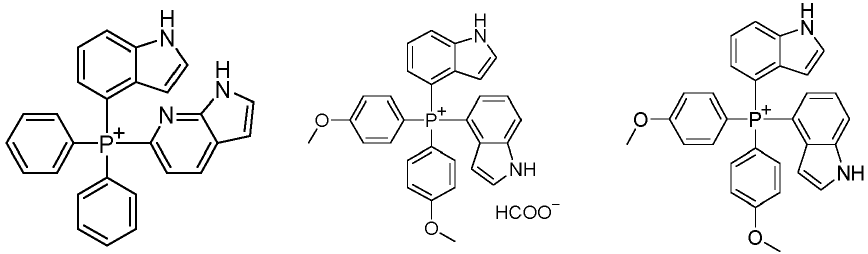

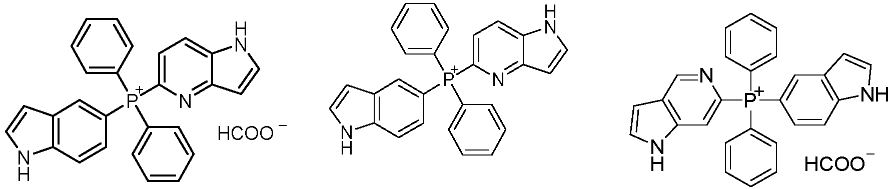

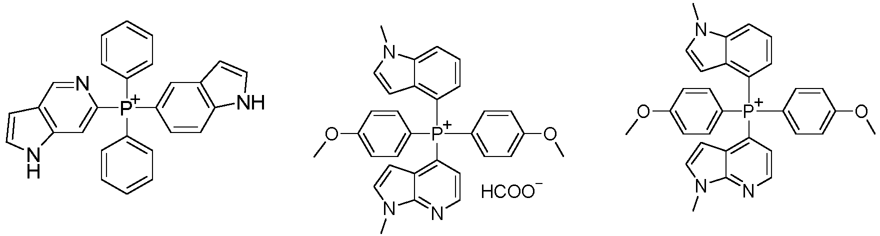

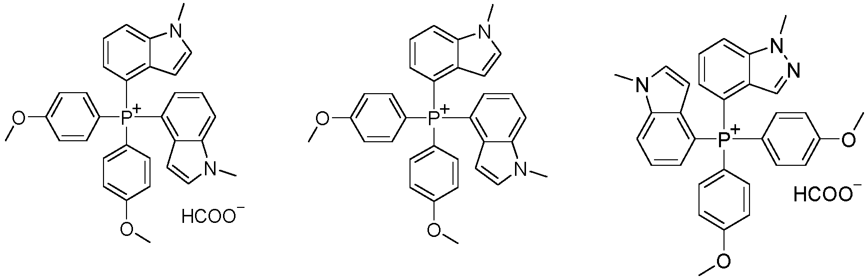

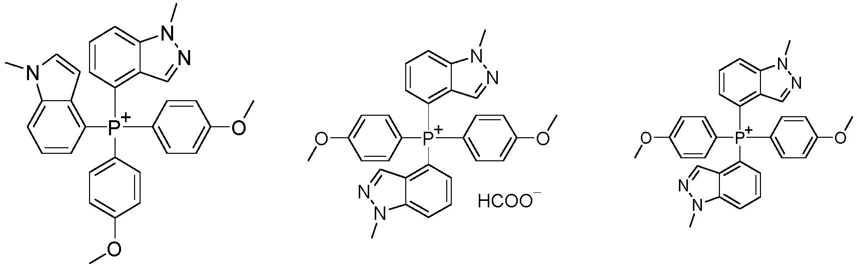

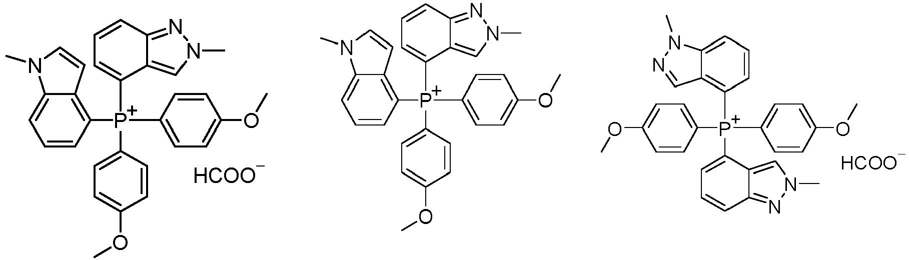

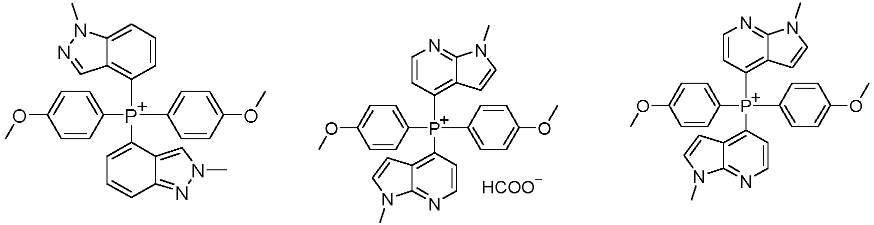

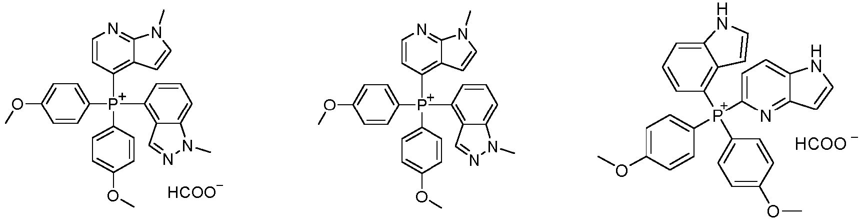

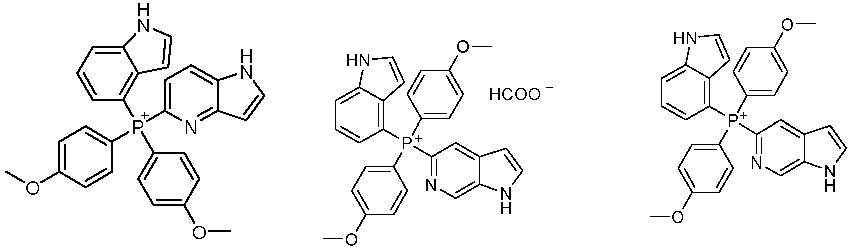

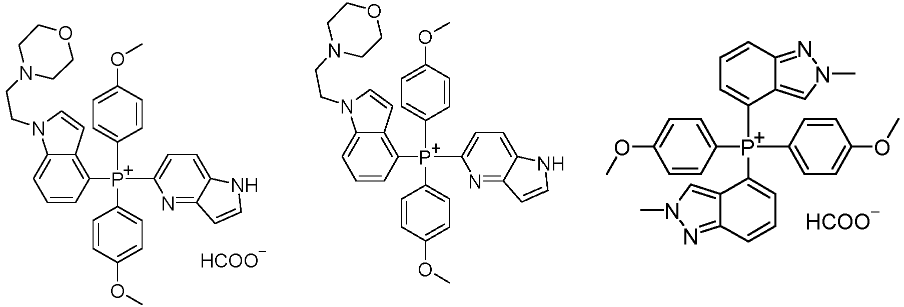

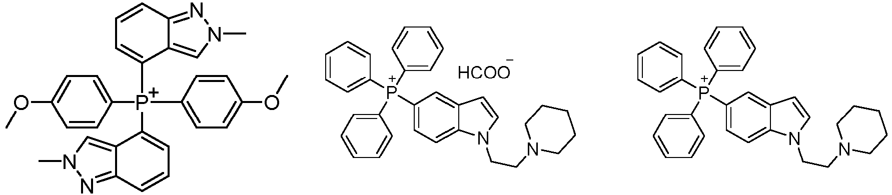

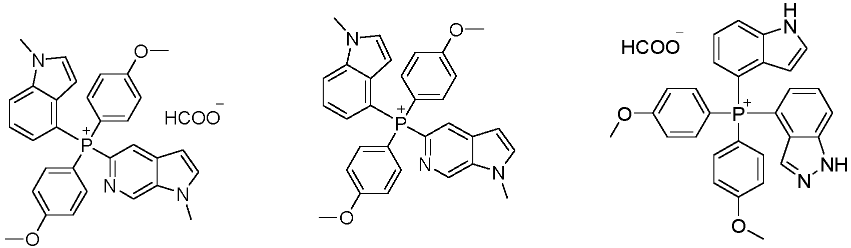

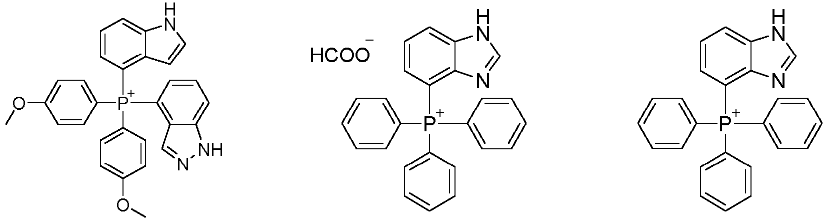

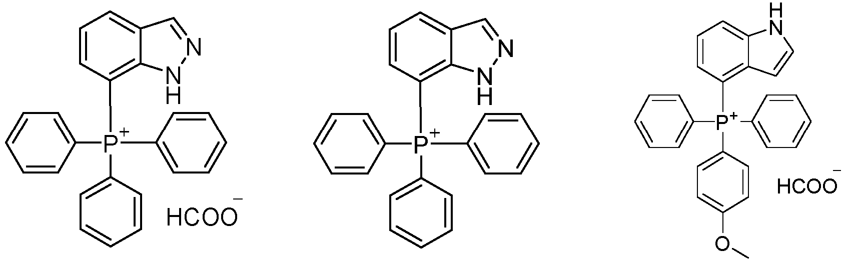

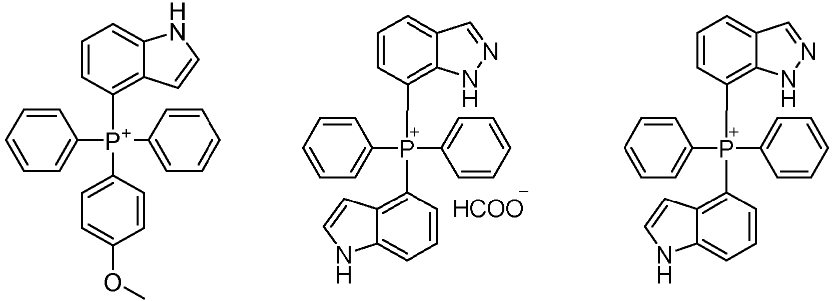

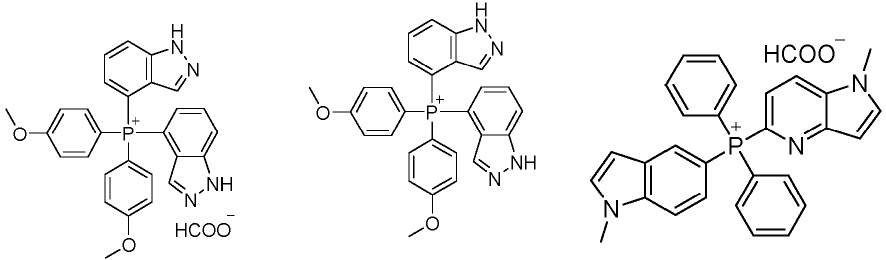

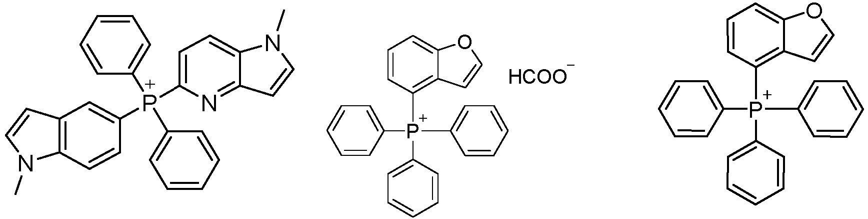

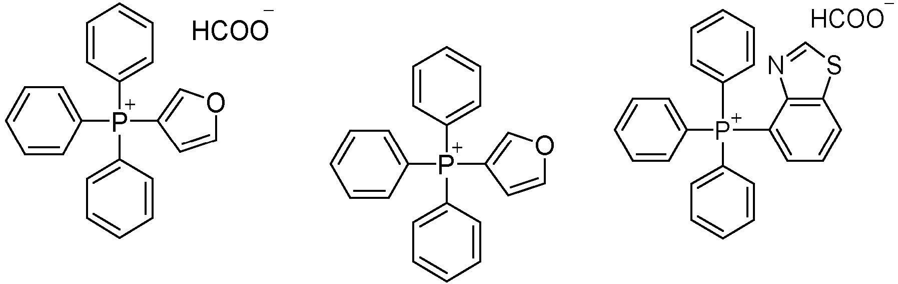

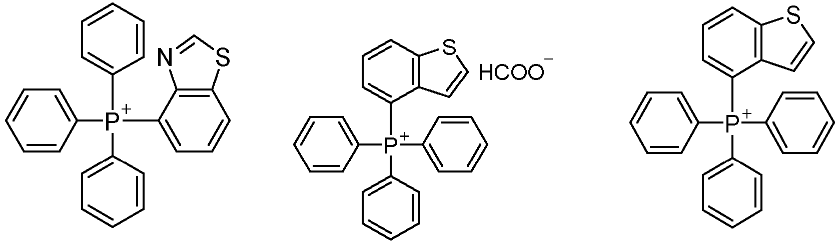

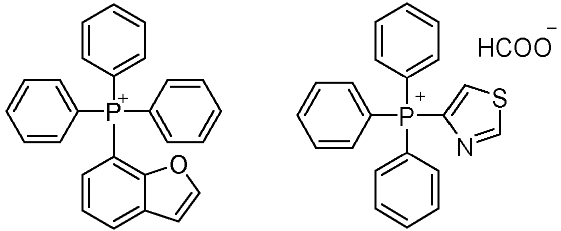

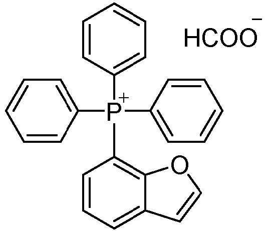

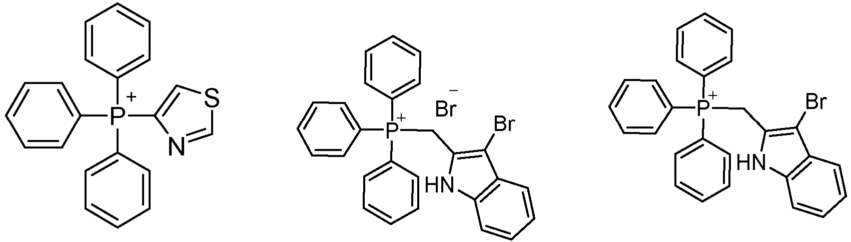

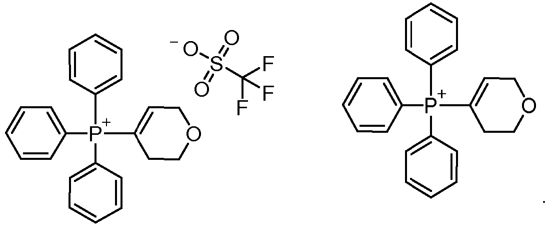

3. A use of the compound of formula I, or an optical isomer thereof, or a racemate thereof, or a solvate thereof, or a pharmaceutically acceptable salt thereof, or a deuterated compound thereof of claim 1 in the preparation of a composition or a preparation for preventing and/or treating tumor.

4. The use of claim 3, wherein the tumor comprises tumor with low expression, no expression, low activity or no activity of mitochondria permeability transition pore;
the tumor comprises tumor with low expression, no expression, low activity or no activity of peptidyl-prolyl cis-trans isomerase F;
the tumor comprises tumor with low or no expression of NNMT gene;
the tumor comprises tumor with high expression of DNA methylase;
the tumor comprises tumor with high expression of DNMT1;
the tumor comprises tumor with high expression of DNMT3a;
the tumor comprises tumor with high expression of DNMT3b;
the tumor comprises tumor with high expression of UHRFl;
the tumor comprises tumor with high methylation level of nucleotide site of NNMT gene; and/or
the tumor comprises tumor with high methylation level of DNA CpG site of NNMT gene.

5. The use of claim 4, wherein the low expression or low activity of mitochondria permeability transition pore means the ratio (H1/H0) of the expression level or activity level H1of mitochondria permeability transition pore in the tumor cell to the expression level or activity level H0 of mitochondria permeability transition pore in the same type of cell or a normal cell is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low expression or low activity of peptidyl-prolyl cis-trans isomerase F means the ratio (C1/C0) of the expression level or activity level C1of peptidyl-prolyl cis-trans isomerase F in the tumor cell to the expression level or activity level C0 of peptidyl-prolyl cis-trans isomerase F in the same type of cell or a normal cell is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low or no expression of NNMT gene means the ratio (E1/E0) of the expression E1 of NNMT gene in the tumor cell to the expression E0 of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the tumor with high expression of DNA methylase means the ratio (A1/A0) of the expression level A1 of DNA methylase in the tumor cell to the expression level A0 of DNA methylase in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of DNMT1 means the ratio (B1/B0) of the expression level B1 of DNMT1 in the tumor cell to the expression level B0 of DNMT1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of DNMT3a means the ratio (P1/P0) of the expression level P1 of DNMT3a in the tumor cell to the expression level P0 of DNMT3a in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of DNMT3b means the ratio (D1/D0) of the expression level D1 of DNMT3b in the tumor cell to the expression level D0 of DNMT3b in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of UHRF1 means the ratio (F1/F0) of the expression level F1 of UHRF1 in the tumor cell to the expression level F0 of UHRF1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the high methylation level of nucleotide site of NNMT gene means the ratio (L1/L0) of the methylation level L1 of nucleotide site of NNMT gene in the tumor cell to the methylation level L0 of nucleotide site of NNMT gene in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50; and/or
the high methylation level of DNA CpG site of NNMT gene means the ratio (G1/G0) of the methylation level G1 of DNA CpG site of NNMT gene in the tumor cell to the methylation level G0 of DNA CpG site of NNMT gene in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

6. The use of claim 5, wherein the same type of cell comprises the same type of tumor cell with normal expression, high expression, normal activity or high activity of mitochondria permeability transition pore;
the same type of cell comprises the same type of tumor cell with normal expression, high expression, normal activity or high activity of peptidyl-prolyl cis-trans isomerase F;
the same type of cell comprises the same type of tumor cell with normal or high expression of NNMT gene;
the same type of cell comprises the same type of tumor cell with normal or low expression of DNA methylase;
the same type of cell comprises the same type of tumor cell with normal or low expression of DNMT1;
the same type of cell comprises the the same type of tumor cell with normal or low expression of DNMT3a;
the same type of cell comprises the same type of tumor cell with normal or low expression of DNMT3b;
the same type of cell comprises the same type of tumor cell with normal or low expression of UHRF 1;
the same type of cell comprises the same type of tumor cell with normal or low methylation level of nucleotide site of NNMT gene; and/or
the same type of cell comprises the same type of tumor cell with normal or low methylation level of DNA CpG site of NNMT gene.

7. The use of claim 3, wherein the tumor is selected from the group consisting of brain tumor, lung cancer, and combinations thereof.

8. A marker for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

9. A use of a detection kit in the preparation of concomitant diagnose kit for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor;
the detection kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.
the concomitant diagnose kit further comprises instruction or label, the instruction or label records as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRF1, high methylation level of nucleotide site of NNMT gene, and/or high methylation level of DNA CpG site of NNMT genel; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRF1, low methylation level of nucleotide site of NNMT gene, and/or low methylation level of DNA CpG site of NNMT gene.

10. A medicine kit, wherein the medicine kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof according to the first aspect of the present invention.

11. A use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug;
the anti-tumor drug is the compound of formula I, or an optical isomer thereof, or a racemate thereof, or a solvate thereof, or a pharmaceutically acceptable salt thereof, or a deuterated compound thereof of claim 1.

12. A composition, wherein the composition comprises:
(1) a first active ingredient, the first active ingredient comprises anti-tumor drug, the anti-tumor drug is the compound of formula I, or an optical isomer thereof, or a racemate thereof, or a solvate thereof, or a pharmaceutically acceptable salt thereof, or a deuterated compound thereof of claim 1; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.
